# EUROPEAN PATENT SPECIFICATION

(11) **EP 2 004 667 B1**
(45) Date of publication and mention of the grant of the patent: **04.09.2013**
(21) Application number: 07736511.2
(22) Date of filing: 29.01.2007
(51) Int. Cl.: C07J 3/00, C07J 31/00, C07J 41/00, C07J 71/00

(54) **NOVEL 11 BETA - HYDROXYANDROSTA-4-ENE-3-ONES**
NEUARTIGE 11 BETA - HYDROXYANDROSTA-4-EN-3-ONE
NOUVELLES 11 BETA - HYDROXYANDROSTA-4-ENE-3-ONES

(30) Priority: 27.01.2006 IN MU01312006
(43) Date of publication of application: 24.12.2008
(73) Proprietor: Sun Pharma Advanced Research Company Limited, Mumbai 400 093 (IN)
(72) Inventor: PATEL, Jiten Ranchhodbhai, Baroda 390 020 (IN); PATEL, Gopalkumar Chimanlal, Baroda 390 020 (IN); SHETH, Gaurav Sanjivkumar, Baroda 390 020 (IN); SHAH, Samir Rameshchandra, Baroda 390 020 (IN); MANDHANE, Sanjay Nandlal, Baroda 390 020 (IN); CHITTURI, Trinadha Rao, Baroda 390 020 (IN); THENNATI, Rajamannar, Baroda 390 020 (IN)
(74) Representative: Atkinson, Jonathan David Mark
(86) International application number: PCT/IN2007/000039
(87) International publication number: WO 2007/099548

(56) References cited:
- BE-A1- 889 563
- GB-A- 2 079 755
- US-A- 4 996 335
- DATABASE CAPLUS [Online] LITTLE R.J. ET AL.: 'Soft drugs based on hydrocortisone: the inactive metabolite approach and its application to steroidal antiinflammatory agents', XP009000424 Retrieved from STN Database accession no. (1999:408919) & PHARMACEUTICAL RESEARCH vol. 16, no. 6, 1999, pages 961 - 967

## Description

The present invention relates to novel anti-inflammatory and antiallergic analogues of the 11β-hydroxyandrosta-4-ene-3-ones, represented as formula I and physiologically acceptable salt thereof, having insignificant or no noteworthy systemic effects at multiples of efficacious dose,

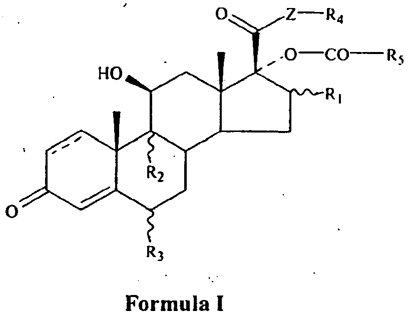

### BACKGROUND OF THE INVENTION

Corticosteroids (or glucocorticoids) having anti-inflammatory properties are widely used for the treatment of inflammatory conditions or disorders of skin, airways, eye, GI tract, joints, CNS etc, and several autoimmune disorders. Some of the inflammatory skin disorders where treatment with glucocorticoids is prescribed are eczema, psoriasis, allergic dermatitis, pruritis, hypersensitivity reactions etc. Inflammatory or allergic conditions of the airways for which glucocorticoids are used include disorders of nose, throat or lungs such as rhinitis (including hay fever), nasal polyps, asthma (including allergen-induced asthmatic reaction), chronic obstructive pulmonary disease, interstitial lung disease, fibrosis, etc. Glucocrticoid administration is also used for inflammatory bowel disorders such as ulcerative colitis and Crohn's diseases; and inflammatory joint disorders such as rheumatoid arthritis which are autoimmune diseases. However, administration of corticosteroids in general may cause, in addition to the desired pharmacological effect, undesirable or adverse side effects at sites distant from the target tissue, the so-called systemic effects. Some of the undesired systemic effects encountered include widespread immunosuppression, increased bone turnover, impaired growth, skin thinning, diabetes, obesity, water retention, progesterone and estrogen related disorders. It is therefore desirable to have glucocorticoids which possess potent anti-inflammatory activity at the target tissue, with minimal or preferably no systemic activity at therapeutic doses when used for chronic treatment.

### Classical glucocorticoids are described in

- **United States patent No.** 4335121 (the '121 patent; Indian reference not available)-(S)-fluoromethyl 6α,9α-difluoro-11β-hydroxy-16α-methyl-17α-propionyloxy-3-oxoandrosta-1,4-diene-17β-carbothioate (Fluticasone)
- **United states patent No.** 3007923 (the '923 patent; Indian reference not available)-16α-methyl-9α-fluoro-1,4-pregnadiene-11β,17α,21-triol-3,20-dione (Dexamethasone)
- **United states patent No** 3929768 (the '768 patent; Indian reference not available) exemplifies (11β,16α)-16,17-[Butylidenebis(oxy)]-11,21-dihydroxypregna-1,4-diene-3,20-dione (Budesonide)
- **United states patent No** 4472393 (the '393 patent; Indian reference not available) exemplifies (11β,16α)-9,21-Dichloro-17-[(2-furanylcarbonyl)oxy]-11-hydroxy-16-methylpregna-1,4-diene-3,20-dione (Mometasone furoate)

The above mentioned drugs are potent glucocorticoids which are already in clinical use. However, at high multiples of efficacy dose these drugs have the potential to cause systemic side effects. GB 2079755 discloses anti-inflammatory agents based on a glucocortio-dersid structure.

PCT publication WO 99/01467 describes therapeutically active steroidal compounds or salts or solvates having lactone group connected to the cyclopentane ring of the steroid nucleus (as given below). These compounds have been described to have reduced potential for systemic activity due to the relative instability of the lactone system in plasma. The compounds of the present invention do not possess a lactone group connected to the cyclopentane ring.

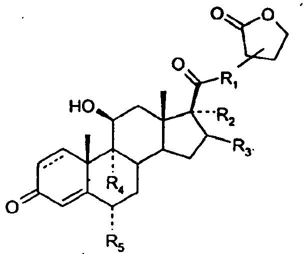

Our interest to develop compounds which act at the specific site of inflammation with insignificant or no noteworthy side effects has led us to the discovery of novel, safe 11β-hydroxyandrosta-4-ene-3-one compounds described in this invention. These compounds possess useful anti-inflammatory activity whilst having insignificant or no noteworthy systemic effects at multiples of efficacious dose.

### DESCRIPTION OF THE INVENTION

The present invention provides 11β-hydroxyandrosta-4ene-3-one, compound of formula I, and physiologically acceptable salt thereof, 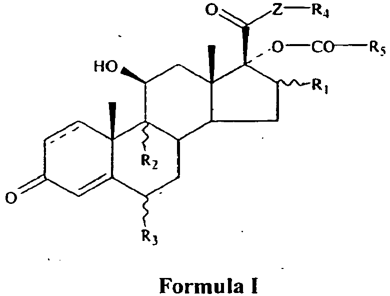 -̅-̅-̅-̅-̅-̅ represents a single or double bond and 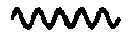 represents α or β-configuration.
wherein
Z represents O or S;
R₁ represents hydrogen or methyl which may be either in α or β-configuration, or methylene,
R₂ and R₃ are the same or different and each independently represents hydrogen, halogen or a methyl group;
R₅ represents a group selected from (C₁-C₁₀)-alkyl, (C₃-C₁₃)-cycloalkyl,
-O-(C₁-C₁₀)-alkyl, aryl or heterocyclic ring wherein the ring or ring system is unsubstituted or substituted by one or more halogen, -OH, (C₁-C₃)-alkyl, -O-(C₁-C₃)-alkyl, (C₁-C₁₃)-cycloalkyl;
wherein the alkyl or cycloalkyl groups can optionally contain one or more unsaturations and/or can have one or more hetero atom incorporated therein and optionally in each case have one or more hydrogen atoms replaced by halogen, -OH, (C₁-C₃)-alkyl, -O-(C₁-C₃)-alkyl, (C₃-C₁₃)-cycloalkyl; and
R₄ represents moiety selected from a group consisting of (A), (B) and (C), with a proviso that when R₄ represents moiety (C), Z is S: 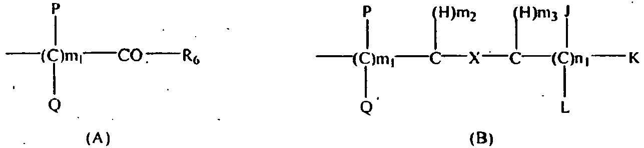 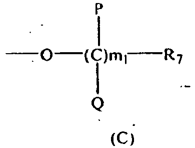 wherein
m, is 1, 2 or 3;
m₂ is 0 or 1;
m₃ is 0 or 1;
n₁ is 0, 1 or 2;
R₆ represents a group selected from (C₁-C₈)-alkyl, (C₃-C₁₃)-cycloalkyl, aryl and a heterocyclic radical, where the ring or ring system is unsubstituted or substituted by one or more substituents selected from (C₁-C₈)-alkyl, (C₃-C₁₃)-cycloalkyl, halogen, O-(C₁-C₈)-alkyl, O-(C₃-C₁₃)-cycloalkyl, OCO-(C₁-C₃)-alkyl, S(O)₀₋₂(C₁-C₈)-alkyl, COO-(C₁-C₈)-alkyl, -OCO-O-(C₁-C₃)-alkyl, -OCO-CO-O-(C₁-C₃)-alkyl, CONH₂, CONH-(C₁-C₈)-alkyl, CON-[(C₁-C₈)-alkyl]₂, - NHCO-(C₁-C₈)-alkyl, N-(C₁-C₈)-alkyl-CO-(C₁-C₈)-alkyl, -NHCO-O-(C₁-C₈)-alkyl, -N-(C₁-C₈)-alkyl-CO-O-(C₁-C₈)-alkyl, -NHCONH-(C₁-C₈)-alkyl, -N-(C₁-C₈)-alkyl-CONH-(C₁-C₈)-alkyl, - NHCONH-SO₂-(C₁-C₈)-alkyl,
-N-(C₁-C₈)-alkyl-CONH-SO₂-C₁-C₈)-alkyl, -NO₂, -CN; wherein the alkyl or cycloalkyl groups can optionally contain one or more unsaturations and/or can have one or more hetero atom incorporated therein and optionally, in each case have one or more hydrogen atoms replaced by halogen, -OH, (C₁-C₃)-alkyl, -OCO-(C₁-C₃)-alkyl or (C₃-C₁₃)-cycloalkyl;
with a proviso that when R₄ represents moiety (A) wherein R₆ represents (C₃-C₁₃)-cycloalkyl optionally containing one or more hetero atom incorporated therein, the hetero atom is not nitrogen;
P and Q are independently selected from hydrogen and C₁ to C₃ alkyl;
or P and Q can be joined together with the carbon atom to which they are attached to form (C₃-C₈)-cycloalkyl as represented by moiety (A-1): 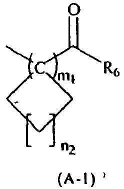 wherein m₁ is 1 and n₂ is 0,1, 2, 3, 4 or 5 and R₆ is an aryl as defined above;
or P and R₆ can be joined together to form a cyclic system as represented by moiety (A-2): 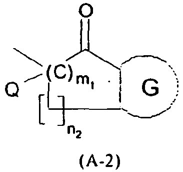 wherein m₁ is 1 and n₂ is 0, 1, 2, 3 or 4 and ring G is an aryl as defined above;
X represents either a double bond or a triple bond; and
J, K and L are independently selected from a group consisting of hydrogen, halogen, (C₁-C₁₀)-alkyl, (C₃-C₁₃)-cycloalkyl, -OH, -O-(C₁-C₁₀)-alkyl, -O-(C₃-C₁₃)-cycloalkyl, -OCO-(C₁-C₁₀)-alkyl,-OCO-(C₃-C₁₃)-cycloalkyl,-OCO-CO-O-(C₁-C₁₀)-alkyl, -OCO-CO-O-(C₃-C₁₃)-cycloalkyl, -OCO-O-(C₁-C₁₀)-alkyl, -OCO-O-(C₃-C₁₃)-cycloalkyl, -OCO-NH-(C₁-C₁₀)-alkyl, -OCO-NH-(C₃-C₁₃)-cycloalkyl, -OCO-N-[(C₁-C₁₀)-alkyl]₂, -OCO-N-[(C₃-C₁₃)-cycloalkyl]₂, -OCO-NHSO₂-(C₁-C₁₀)-alkyl, -OCO-NHSO₂-(C₃-C₁₃)-cycloalkyl, -NH₂, -NH-(C₁-C₈)-alkyl, -N-[(C₁-C₈)-alkyl]₂, -NO₂ and -CN;
wherein the alkyl or cycloalkyl groups can optionally contain one or more unsaturations and/or can have one or more hetero atom incorporated therein and optionally, in each case have one or more hydrogen atoms replaced by halogen, -OH, (C₁-C₃)-alkyl, -O-(C₁-C₃)-alkyl, -OCO-(C₁-C₃)-alkyl, -COOH, -COO-(C₁-C₅)-alkyl, -COO-(C₁-C₅)-haloalkyl, -NHCO-(C₁-C₈)-alkyl, -ONO₂, -NH-(C₁-C₈)-alkyl, -N-[(C₁-C₈)-alkyl]₂, -NO₂, -CN, (C₃-C₁₃)-cycloalkyl, aryl or heterocyclic radical;
or J and K can be joined together with the carbon atom to which they are attached to represent a (C₃-C₁₃)-cycloalkyl or -CO- group and L is as defined above;
or J, K and L are absent when n₁ is 0;
R₇ represents a group selected from hydrogen, halogen, aryl and CO-aryl, wherein the ring or ring system is unsubstituted or substituted as defined above;
Preferably,
11β-hydroxyandrosta-4-ene-3-one, compound of formula I-B, and physiologically acceptable salt thereof: 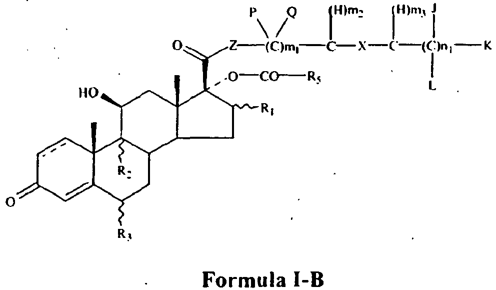 wherein
Z represents O or S;
R₁ represents hydrogen or methyl which may be either in α or β-configuration, or methylene;
R₁ and R₃ are the same or different and each independently represents hydrogen, halogen or a methyl group;
R₅ represents a group selected from (C₁-C₁₀)-alkyl, (C₃-C₁₃)-cycloalkyl, -O-(C₁-C₁₀)-alkyl, aryl or heterocyclic ring wherein the ring or ring system is unsubstituted or substituted by one or more halogen, -OH, (C₁-C₃)-alkyl, -O-(C₁-C₃)-alkyl, (C₃-C₁₃)-cycloalkyl; wherein the alkyl or cycloalkyl groups can optionally contain one or more unsaturations and/or can have one or more hetero atom incorporated therein and optionally in each case have one or more hydrogen atoms replaced by halogen, -OH, (C₁-C₃-alkyl, -O-(C₁-C₃)-alkyl, (C₃-C₁₃)-cycloalkyl; and
m₁ is 1;
m₂ is 0 or 1;
m₃ is 0 or 1;
n₁ is 0, 1 or 2;
P and Q are hydrogen;
X represents either a double bond or a triple bond; and
J, K and L are independently selected from a group consisting of hydrogen, halogen, (C₁-C₁₀)-alkyl, (C₃-C₁₃)-cycloalkyl, -OH, -O-(C₁-C₁₀)-alkyl, -O-(C₁-C₁₃)-cycloalkyl, -OCO-(C₁-C₁₀)-alkyl, -OCO-(C₃-C₁₃)-cycloalkyl, -OCO-CO-O-(C₁-C₁₀)-alkyl, -OCO-CO-O-(C₃-C₁₃)-cycloalkyl. -OCO-O-(C₁-C₁₀)-alkyl, -OCO-O-(C₃-C₁₃)-cycloalkyl, -OCO-NH-(C₁-C₁₀)-alkyl, -OCO-NH-(C₃-C₁₃)-cycloalkyl, -OCO-N-[(C₁-C₁₀)-alkyl]₂, -OCO-N-[(C₃-C₁₃)-cycloalkyl]₂, -OCO-NHSO₂-(C₁-C₁₀)-alkyl, -OCO-NHSO₂-(C₃-C₁₃)-cycloalkyl, -NH₂, -NH-(C₁-C₈)-alkyl, -N-[(C₁-C₈)-alkyl]₂, -NO₂ and -CN.
Most preferably,
11β-hydroxyandrosta-4-ene-3-one, compound of formula I-B, and physiologically acceptable salt thereof: 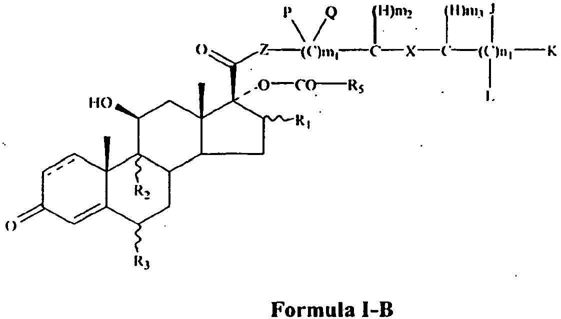 wherein
Z represents S;
R₁ represents hydrogen or methyl which may be either in α or β-configuration, or methylene;
R₂ and R₃ are the same or different and each independently represents hydrogen, halogen or a methyl group;
R₅ represents a group selected from (C₁-C₁₀)-alkyl, (C₃-C₁₃)-cycloalkyl,
-O-(C₁-C₁₀)-alkyl, aryl or heterocyclic ring wherein the ring or ring system is unsubstituted or substituted by one or more halogen, -OH, (C₁-C₃)-alkyl, -O-(C₁-C₃-alkyl, (C₃-C₁₃)-cycloalkyl; wherein the alkyl or cycloalkyl groups can optionally contain one or more unsaturations and/or can have one or more hetero atom incorporated therein and optionally in each case have one or more hydrogen atoms replaced by halogen, -OH, (C₁-C₃)-alkyl, -O-(C₁-C₃)-alkyl, (C₃-C₁₃)-cycloalkyl; and
m₁ is 1;
m₂ is 0;
m₃ is 0;
n₁ is 0, 1 or 2;
P and Q are hydrogen;
X represents a triple bond; and
J, K and L are independently selected from a group consisting of hydrogen, halogen, (C₁-C₁₀)-alkyl, (C₃-C₁₃)-cycloalkyl, -OH, -O-(C₁-C₁₀)-alkyl, -O-(C₃-C₁₃)-cycloalkyl, -OCO-(C₁-C₁₀)-alkyl, -OCO-(C₃-C₁₃)-cycloalkyl, -OCO-CO-O-(C₁-C₁₀)-alkyl, -OCO-CO-O-(C₃-C₁₃)-cycloalkyl, -OCO-O-(C₁-C₁₀)-alkyl, -OCO-O-(C₃-C₁₃)-cycloalkyl, -OCO-NH-(C₁-C₁₀)-alkyl, -OCO-NH-(C₃-C₁₃)-cycloalkyl, -OCO-N-[(C₁-C₁₀)-alkyl]₂, -OCO-N-[(C₃-C₁₃)-cycloalkyl]₂, -OCO-NHSO₂-(C₁-C₁₀)-alkyl, -OCO-NHSO₂-(C₃-C₁₃)-cycloalkyl, -NH₂, -NH-(C₁-C₈)-alkyl, -N-[(C₁-C₈)-alkyl]₂, -NO₂ and -CN.

### DETAILED DESCRIPTION OF THE INVENTION

The present invention relates to novel, safe 11β-hydroxyandrosta-4-ene-3-one, compound of formula I, and physiologically acceptable salt thereof.

Compound of formula I, may be in the form of diasteoromers and mixtures thereof. As used herein, any stereoisomeric form includes diastereomers and mixtures thereof.

Reference to compound of formula I hereinafter includes a compound of formula I and physiologically acceptable salt thereof.

With respect to the various groups encompassed by the generic terms used here and throughout this specification, the following definitions and explanations are applicable:

As used herein 'alkyl' can be straight-chain or branched and can optionally contain one or more unsaturations and/or can have one or more hetero atom incorporated therein and optionally, in each case have one or more hydrogen atoms replaced by -F, -Cl, -Br, -I;
-OH, -OCO-(C₁-C₃)-alkyl, (C₃-C₁₃)-cycloalkyl, aryl or heterocyclic radical.
As used herein 'alkyl including one or more unsaturations' is to be understood as meaning 'alkenyl' and/or 'alkynyl'. Exemplary alkyl groups include methyl, ethyl, *n*-propyl, *i*-propyl. *n-*butyl, *t*-butyl, *n*-pentyl, 3-pentyl, 2-octyl and the like. Exemplary alkenyl groups include ethenyl, propenyl, 1-butenyl, (*Z*)-2-butenyl, (*E*)-3-methylbut-2-enyl,
(*E*)-2,4-pentadienyl, (*Z*)-3-heptenyl and the like. Exemplary alkynyl groups include ethynyl, propynyl, 1-butynyl, 2-butynyl, 4-methyl-2-pentynyl, 2,4-hexadiynyl and the like.

As used herein 'cycloalkyl' is to be understood as meaning monocyclic, bicyclic, tricyclic and polycyclic ring systems such as norbornyl, adamantly and the like. The term 'cycloalkyl' as used herein can optionally contain one or more unsaturations and/or can have one or more hetero atom incorporated therein -F, -Cl, -Br, -I, -OH, -OCO-(C₁-C₃)-alkyl, (C₃-C₁₃)-cycloalkyl, aryl or heterocyclic radical.

As used herein 'halogen' or 'halo group' refers to fluorine, chlorine, bromine or iodine.

As used herein aryl is to be understood as meaning ring systems such as phenyl, naphthyl. anthracenyl, phenanthryl, preferably aryl is phenyl.

As used herein heterocyle or heterocyclic ring is to be understood as meaning ring systems which, in addition to carbon, also contain hetero atoms, such as, for example, nitrogen, oxygen or sulfur. This definition furthermore includes ring systems in which the heterocycle or heterocyclic radical is fused with benzene rings. Examples of heterocycles or heterocyclic rings are:
heteroaryls, such as
benzimidazolyl,
1-[(C₁-C₆)-alkyl]benzimidazolyl,
imidazolyl,
2- or 3-thienyl,
2- or 3-furyl,
benzoxazolyl,
benzothiazolyl,
2-, 3- or 4-pyridyl,
pyrimidinyl,
4-, 5- or 6-pyridazin-2*H*-yl-3-one,
4-, 5- or 6-pyridazin-2-(C₁-C₈)-alkyl-2*H*-yl-3-one,
2-benzyl-4-, -5- or -6-pyridazin-2*H*-yl-3-one,
3- or 4-pyridazinyl,
2-, 3-, 4- or 8-quinolinyl,
1-, 3- or 4-isoquinolinyl,
1-phthalazinyl,
3- or 4-cinnolinyl,
2- or 4-quinazolinyl,
2-pyrazinyl,
2-quinoxalinyl,
2-, 4- or 5-oxazolyl,
3-, 4- or 5-isoxazolyl,
2-, 4- or 5-thiazolyl,
3-, 4- or 5-isothiazolyl,
1-[(C₁-C₆)-alkyl]-2-, -4- or -5-imidazolyl,
3-, 4- or 5-pyrazolyl,
1-[(C₁-C₆)-alkyl]-3-, -4- or -5-pyrazolyl,
1- or 4-[1,2,4]-triazolyl,
4- or 5-[1,2,3]-triazolyl,
1-[(C₁-C₆)-alkyl]-4- or -5-[1,2,3]triazolyl,
3-, 4- or 7-indolyl,
*N*-[(C₁-C₆)-alkyl]-3-, -4- or -7-indolyl
2-[(C₁-C₆)-alkyl]-3(2*H*)-indazolyl,
1-[(C₁-C₆)-alkyl]-3(1*H*)-indazolyl,
5-tetrazolyl,
1-[(C₁-C₆)-alkyl]-1*H*-tetrazolyl,
2-[(C₁-C₆)-alkyl]-2*H*-tetrazolyl.

In one embodiment the preferred compounds of formula I are selected from the compounds wherein m₁ is I

In one embodiment the preferred compounds of formula I are selected from the compounds wherein m₁ is I and P and Q are hydrogen.

In one embodiment the compounds of formula I are selected from the compounds wherein R represents moiety (A).

In one embodiment the compounds of formula I are selected from the compounds wherein R₄ represents moiety (B).

In one embodiment the compounds of formula I are selected from the compounds wherein R₄ represents moiety (C).

In one embodiment the compounds of formula I are selected from the compounds wherein m₁ is 1; P and Q are both hydrogen and R₄ represents moiety (A).

In one embodiment the compounds of formula I are selected from the compounds wherein m₁ is 1; P and Q are both hydrogen and R₄ represents moiety (B).

In one embodiment the compounds of formula I are selected from the compounds wherein m₁ is 1; P and Q are both hydrogen and R₄ represents moiety (C).

In one embodiment the compounds of formula I are selected from the compounds wherein R₆ is selected from a group consisting of (C₁-C₈)-alkyl, aryl and a heterocyclic radical wherein the ring or ring system is unsubstituted or substituted as defined above.

In one embodiment the compounds of formula I are selected from the compounds wherein R₅ represents a group selected from R₆, wherein R₆ is selected from a group consisting of (C₁-C₈)-alkyl and a heterocyclic radical wherein the ring or ring system is unsubstituted or substituted as defined above.

In one embodiment the present invention provides 11β-hydroxyandrosta-4-ene-3-one, a compound of formula 1-A and physiologically acceptable salt, thereof: 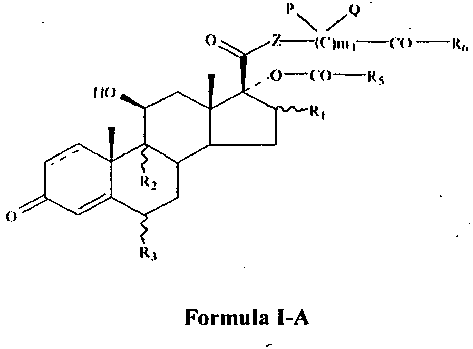 -̅-̅-̅-̅-̅-̅ represents a single or double bond and 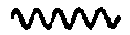 represents α or β-configuration.
wherein
Z represents O, S;
R₁ represents hydrogen or methyl which may be either in α or β-configuration, or methylene;
R₂ and R₃ are the same or different and each independently represents hydrogen, halogen or a methyl group;
m₁ is 1, 2 or 3;
R₆ represents a group selected from (C₁-C₈)-alkyl, (C₃-C₁₃)-cycloalkyl, aryl and a heterocyclic radical, where the ring or ring system is unsubstituted or substituted by one or more substituents selected from.
(C₁-C₈)-alkyl, (C₃-C₁₃)-cycloalkyl, halogen, O-(C₁-C₈)-alkyl, O-(C₃-C₁₃)-cycloalkyl, OCO-(C₁-C₃)-alkyl, S(O)₀₋₂(C₁-C₈)-alkyl, COO-(C₁-C₈)-alkyl, -OCO-O-(C₁-C₃)-alkyl, -OCO-CO-O-(C₁-C₃)-alkyl, CONH₂, CONH-(C₁-C₈)-alkyl, CON-C(C₁-C₈)-alkyl]₂, -NHCO-(C₁-C₈)-alkyl, N-(C₁-C₈)-alkyl-CO-(C₁-C₈)-alkyl, -NHCO-O-(C₁-C₈)-alkyl, -N-(C₁-C₈)-alkyl-CO-O-(C₁-C₈)-alkyl, -NHCONH-(C₁-C₈)-alkyl, -N-(C₁-C₈)-alkyl-CONH-(C₁-C₈)-alkyl, -NHCONH-SO₂-(C₁-C₈)-alkyl, -N-(C₁-C₈)-alkyl-CONH-SO₂-(C₁-C₈)-alkyl, -NO₂, -CN;
wherein the alkyl or cycloalkyl groups can optionally contain one or more unsaturations and/or can have one or more hetero atom incorporated therein and optionally, in each case have one or more hydrogen atoms replaced by -F, -Cl, -Br, -I, -OH, -OCO-(C₁-C₃)-alkyl, (C₃-C₁₃)-cycloalkyl, aryl or heterocyclic radical; with a proviso that when R₄ represents moiety A wherein R₆ represents (C₃-C₁₃)-cycloalkyl optionally containing one or more hetero atom incorporated therein, the hetero atom is not nitrogen;
P and Q are independently selected from hydrogen, halogen and C₁ to C₃ alkyl;
or P and Q can be joined together with the carbon atom to which they are attached to form (C₃-C₈)-cycloalkyl as represented by moiety (A-1): 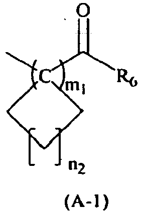 wherein m₁ is 1 and n₂ is 0,1, 2,3,4 or 5;
or P and R₆ can be joined together to form a cyclic system as represented by moiety (A-2): 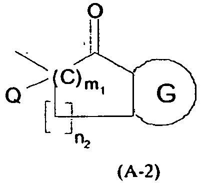 wherein m₁ is 1 and n₂ is 0, 1, 2, 3 or 4 and ring G is optionally present, wherein G represents a (C₃-C₈)-cycloalkyl, aryl or a heterocyclic radical as defined above;
R₅ represents a group selected from R₆, -O-R₆ or-N(R₈)R₆, wherein R₆ is as defined above and R₈ represents hydrogen, (C₁-C₆)-alkyl or (C₃-C₆)-cycloalkyl;

In one embodiment the compounds of formula I-A are selected from the compounds wherein
m₁ is 1; P and Q are both hydrogen; R₆ is selected from group consisting of (C₁-C₈)-alkyl, aryl and a heterocyclic radical; or
wherein m₁ is 1; P and Q are both hydrogen; R₆ is aryl wherein the ring or ring system is unsubstituted or substituted as defined above; or
wherein R₆ is phenyl wherein the ring or ring system is unsubstituted or substituted as defined above.

In one embodiment the present invention provides 11β-hydroxyandrosta-4-ene-3-one, a compound of formula I-B and physiologically acceptable salt thereof: 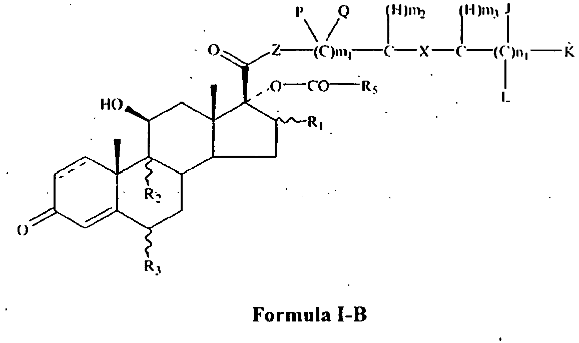 -̅-̅-̅-̅-̅-̅ represents a single bond or double bond and
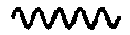 represents α or β-configuration.
wherein
Z represents O, S;
R₁ represents hydrogen or methyl which may be either in α or β-configuration, or methylene;
R₂ and R₃ are the same or different and each independently represents hydrogen, halogen or a methyl group;
m₁ is 1, 2 or 3; ,
m₂ is 0 or 1;
m₃ is 0 or 1;
n₁ is 0, 1 or 2;
P and Q are independently selected from hydrogen, halogen and C₁ to C₃ alkyl;
X represents either a double bond or a triple bond; and
J, K and L are independently selected from a group consisting of hydrogen, halogen, (C₁-C₁₀)-alkyl, (C₃-C₁₃)-cycloalkyl, -OH, -O-(C₁-C₁₀)-alkyl, -O-(C₃-C₁₃)-cycloalkyl, -OCO-(C₁-C₁₀)-alkyl, -OCO-(C₃-C₁₃)-cycloalkyl, -OCO-CO-O-(C₁-C₁₀)-alkyl, -OCO-CO-O-(C₃-C₁₃)-cycloalkyl, -OCO-O-(C₁-C₁₀)-alkyl, -OCO-O-(C₃-C₁₃)-cycloalkyl, -OCO-NH-(C₁-C₁₀)-alkyl, -OCO-NH-(C₃-C₁₃)-cycloalkyl, -OCO-N-[(C₁-C₁₀)-alkyl]₂, -OCO-N-[(C₃-C₁₃)-cycloalkyl]₂, -OCO-NHSO₂-(C₁-C₁₀)-alkyl, -OCO-NHSO₂-(C₃-C₁₃)-cycloalkyl, -CO-[(C₃-C₁₀)N-cyclo], -OCO-[(C₃-C₁₀))N-cyclo], -NH₂, -NH-(C₁-C₈)-alkyl, -N-[(C₁-C₈)-alkyl]₂, -NO₂, -CN, aryl and a heterocyclic radical as defined above;
or J and K can be joined together with the carbon atom to which they are attached to represent a (C₃-C₁₃)-cycloalkyl or -CO- group and L is as defined above;
R₅ represents a group selected from R₆, -O-R₆ or -N(R₈)R₆, wherein R₆, is as defined above and R₈ represents hydrogen, (C₁-C₆)-alkyl or (C₃-C₆)-cycloalkyl ;
In one embodiment the compounds of formula I-B are selected from the compounds wherein, m₁ is I; P and Q are both hydrogen; X represents either a double bond or a triple bond; m₂ is 0; m₃ is 0; n₁ is I and J, K and L are independently selected from hydrogen, halogen, -OH, -OCO-(C₁-C₁₀)-alkyl and -OCO-O-(C₁-C₁₀)-alkyl.

### Preferably,

### 11β-hydroxyandrosta-4-ene-3-one, compound of formula I-B, and physiologically acceptable salt thereof:

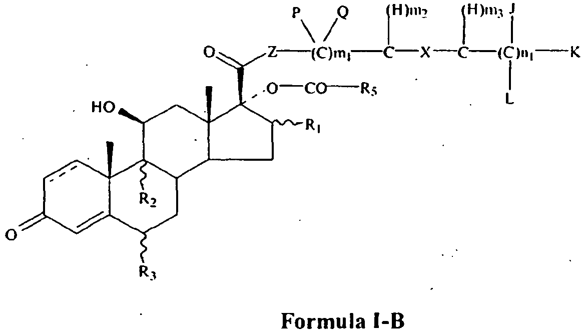

wherein
Z represents O or S;
R₁ represents hydrogen or methyl which may be either in α or β-configuration, or methylene;
R₂ and R₃ are the same or different and each independently represents hydrogen, halogen or a methyl group;
R₅ represents a group selected from (C₁-C₁₀)-alkyl, (C₃-C₁₃)-cycloalkyl,
-O-(C₁-C₁₀)-alkyl, aryl or heterocyclic ring wherein the ring or ring system is unsubstituted or substituted by one or more halogen, -OH, (C₁-C₃)-alkyl, -O-(C₁-C₃)-alkyl, (C₃-C₁₃)-cycloalkyl; wherein the alkyl or cycloalkyl groups can optionally contain one or more unsaturations and/or can have one or more hetero atom incorporated therein and optionally in each case have one or more hydrogen atoms replaced by halogen, -OH, (C₁-C₃)-alkyl. -O-(C₁-C₃)-alkyl, (C₃-C₁₃)-cycloalkyl; and
m₁ is 1;
m₂ is 0 or 1;
m₃ is 0 or 1;
n₁ is 0, 1 or 2;
P and Q are hydrogen;
X represents either a double bond or a triple bond; and
J, K and L are independently selected from a group consisting of hydrogen, halogen (C₁-C₁₀)-alkyl, (C₃-C₁₃)-cycloalkyl, -OH, -O-(C₁-C₁₀)-alkyl, -O-(C₃-C₁₃)-cycloalkyl, -OCO-(C₁-C₁₀)-alkyl, -OCO-(C₃-C₁₃)-cycloalkyl, -OCO-CO-O-(C₁-C₁₀)-alkyl, -OCO-CO-O-(C₃-C₁₃)-cycloalkyl, -OCO-O-(C₁-C₁₀)-alkyl, -OCO-O-(C₃-C₁₃)-cycloalkyl, -OCO-NH-(C₁-C₁₀)-alkyl, -OCO-NH-(C₃-C₁₃)-cycloalkyl, -OCO-N-[(C₁-C₁₀)-alkyl]₂, -OCO-N-[(C₃-C₁₃)-cycloalkyl]₂ - OCO-NHSO₂-(C₁-C₁₀)-alkyl, -OCO-NHSO₂-(C₃-C₁₃)-cycloalkyl, -NH₂, -NH-(C₁-C₈)-alkyl. - N-[(C₁-C₈)-alkyl]₂, -NO₂ and -CN.

### Most preferably,

### 11β-hydroxyandrosta-4-ene-3-one, compound of formula I-B, and physiologically acceptable salt thereof:

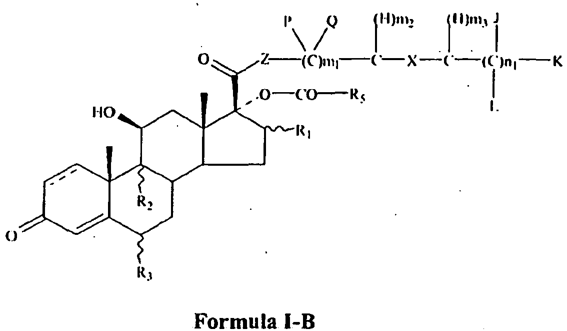

wherein
Z represents S;
R₁ represents hydrogen or methyl which may be either in α or β-configuration, or methylene; R₂ and R₃ are the same or different and each independently represents hydrogen, halogen or a methyl group;
R₅ represents a group selected from (C₁-C₁₀)-alkyl, (C₃-C₁₃)-cycloalkyl,
-O-(C₁-C₁₀)-alkyl, aryl or heterocyclic ring wherein the ring or ring system is unsubstituted or substituted by one or more halogen, -OH, (C₁-C₃)-alkyl, -O-(C₁-C₃)-alkyl, (C₃-C₁₃)-cycloalkyl; wherein the alkyl or cycloalkyl groups can optionally contain one or more unsaturations and/or can have one or more hetero atom incorporated therein and optionally in each case have one or more hydrogen atoms replaced by halogen, -OH, (C₁-C₃)-alkyl, -O-(C₁-C₃)-alkyl, (C₃-C₁₃)-cycloalkyl; and
m₁ is 1;
m₂ is 0;
m₃ is 0;
n₁ is 0, 1 or 2;
P and Q are hydrogen;
X represents a triple bond; and
J, K and L are independently selected from a group consisting of hydrogen, halogen, (C₁-C₁₀)-alkyl, (C₃-C₁₃)-cycloalkyl, -OH, -O-(C₁-C₁₀)-alkyl, -O-(C₃-C₁₃)-cycloalkyl, -OCO-(C₁-C₁₀)-alkyl, -OCO-(C₃-C₁₃)-cycloalkyl, -OCO-CO-O-(C₁-C₁₀)-alkyl, -OCO-CO-O-(C₃-C₁₃)-cycloalkyl, -OCO-O-(C₁-C₁₀)-alkyl, -OCO-O-(C₃-C₁₃)-cycloalkyl, -OCO-NH-(C₁-C₁₀)-alkyl. -OCO-NH-(C₃-C₁₃)-cycloalkyl, -OCO-N-[(C₁-C₁₀)-alkyl]₂, -OCO-N-[(C₃-C₁₃)-cycloalkyl]₂, - OCO-NHSO₂-(C₁-C₁₀)-alkyl, -OCO-NHSO₂-(C₃-C₁₃)-cycloalkyl, -NH₂, -NH-(C₁-C₈)-alkyl, - N-[(C₁-C₈)-alkyl]₂, -NO₂ and -CN.

In one embodiment the present invention provides 11β-hydroxyandrosta-4-ene-3-one, a compound of formula I-C and physiologically acceptable salt thereof: 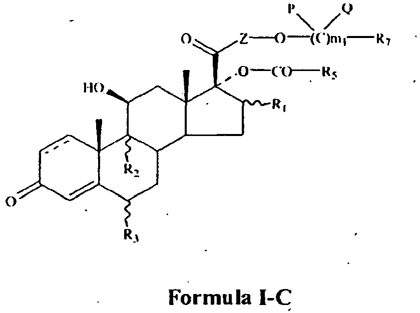 -̅-̅-̅-̅-̅-̅ represents a single or double bond and
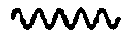 represents α or β-configuration.
wherein
Z represents S;
R₁ represents hydrogen or methyl which may be either in α or β-configuration, or methylene;
R₂ and R₃ are the same or different and each independently represents hydrogen, halogen or a methyl group;
m₁ is 1, 2 or 3;
P and Q are independently selected from hydrogen, halogen and C₁ to C₃ alkyl;
R₇ represents a group selected from halogen, CO-aryl and R₆, wherein the aryl ring of CO-aryl is unsubstituted or substituted by one or more substituents as defined above for the ring or ring system;
R₆ represents a group selected from (C₁-C₈)-alkyl, (C₃-C₁₃)-cycloalkyl, aryl and a heterocyclic radical, where the ring or ring system is unsubstituted or substituted by one or more substituents selected from
(C₁-C₈)-alkyl, (C₃-C₁₃)-cycloalkyl, halogen, O-(C₁-C₈)-alkyl, O-(C₁-C₁₃)-cycloalkyl, OCO-(C₁-C₃)-alkyl, S(O)₀₋₂(C₁-C₈)-alkyl, COO-(C₁-C₈)-alkyl, -OCO-O-(C₁-C₃)-alkyl, -OCO-CO-O-(C₁-C₃)-alkyl, CONH₂, CONH-(C₁-C₈)-alkyl, CON-[(C₁-C₈)-alkyl]₂, -NHCO-(C₁-C₈)-alkyl, N-(C₁-C₈)-alkyl-CO-(C₁-C₈)-alkyl, -NHCO-O-(C₁-C₈)-alkyl, -N-(C₁-C₈-alkyl-CO-O-(C₁-C₈)-alkyl, -NHCONH-(C₁-C₈)-alkyl, -N-(C₁-C₈)-alkyl-CONH-(C₁-C₈)-alkyl, -NHCONH-SO₂-(C₁-C₈)-alkyl, -N-(C₁-C₈)-alkyl-CONH-SO₂(C₁-C₈)-alkyl, -NO₂, -CN;
wherein the alkyl or cycloalkyl groups can optionally contain one or more unsaturations and/or can have one or more hetero atom incorporated therein and optionally, in each case have one or more hydrogen atoms replaced by -F, -Cl, -Br, -I, -OH, -OCO-(C₁-C₃)-alkyl, (C₁-C₁₃)-cycloalkyl, aryl or heterocyclic radical;
R₅ represents a group selected from R₆, -O-R₆ or -N(R₈)R₆, wherein R₆ and R₈ are as defined above.
In one embodiment the compounds of formula I-C are selected from the compounds wherein m₁ is I ; P and Q are both hydrogen and R₇ represents R₆ wherein, R₆ is selected from group consisting of (C₁-C₈)-alkyl or aryl wherein the ring or ring system is unsubstituted or substituted as defined above.

Physiologically acceptable salts are particularly suitable for medical applications, due to their greater solubility in water compared with the starting or base compounds. Suitable physiologically acceptable acid addition salts of the compounds of the invention may be salts of inorganic acids such as hydrochloric acid, hydrobromic acid, phosphoric acid, and the like or of organic acids such as, for example, acetic acid, benzenesulfonic acid, benzoic acid, citric acid and the like. Examples of suitable physiologically acceptable basic salts are ammonium salts, alkali metal salts such as sodium salts and potassium salts and alkaline earth metal salts such as magnesium salts and calcium salts.

The compounds of formula I are useful in human or veterinary medicine, in particular as anti-inflammatory and antiallergic agents.

The compounds of formula I have potentially beneficial anti-inflammatory or antiallergic effects, which are demonstrated by, for example, their ability to bind to the glucocorticoid receptor *in vitro*; and inhibit croton oil induced ear edema, cotton pellet induced granuloma, sephadex induced lung edema *in vivo* models, without causing systemic side effects like involution of thymus, enhanced deposition of glycogen in the liver, or appreciable change in body weight.

All references to compound(s) of the formula I herein refer to a compound/compounds of the formula (I) and to salt thereof.

The present invention provides process for preparation of the compounds of the formula I, comprising reacting the compound of formula II, with R₄-Y, a compound of formula III (Scheme-I): 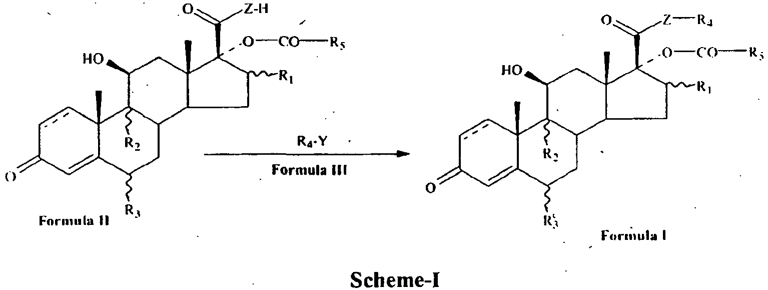 -̅-̅-̅-̅-̅-̅ represents a single or double bond and 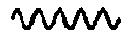 represents α or β-configuration.
wherein Z represents O or S;
R₁, R₂, R₃, R₄ and R₅ are as defined above; Y represents a leaving group;

In the compound of formula III, Y represents a suitable leaving group. A suitable leaving group is any group which upon reaction of a compound of formula II with the compound of formula III, facilitates formation of a compound of formula I. For example, Y may be halogen, alkylsulfonate or arylsulfonate group, like methanesulfonate, *p*-toluenesulfonate or trifluoromethanesulfonate group.

In one embodiment the present invention also provides a compound of formula I-A and a process for preparation of compound of formula IA comprising reacting a carboxylic acid compound of formula II-A (a compound of formula II, wherein Z is O) or a carbothioic acid compound of formula II-B (a compound of formula II, wherein Z is S) with a compound of formula III-A (Scheme-II): 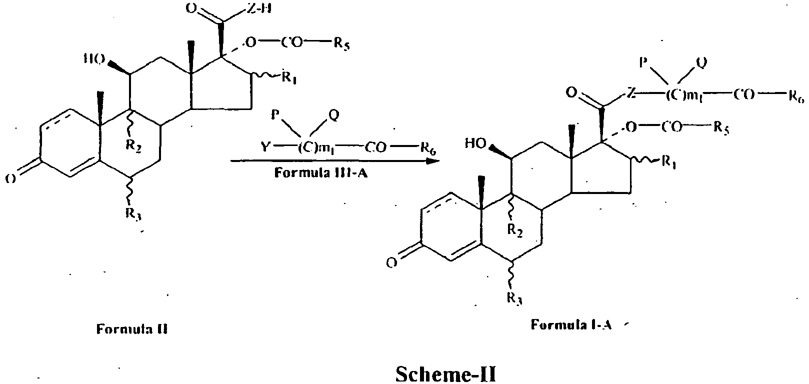 -̅-̅-̅-̅-̅-̅ represents a single or double bond and 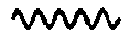 represents α or β-configuration.
wherein Z represents O, S;
R₁, R₂, R₃, R₅, R₆, Y, P, Q and m₁ are as defined above;
The reactions represented by Schemes I or II can be advantageously carried out in a suitable inert organic polar solvent or solvent system, for example, acetone, acetonitrile, *N,N-*dimethylformamide, *N,N-* dimethylacetamide, dimethyl sulfoxide and the like or mixture thereof, in presence of a suitable base, for example, alkali or alkaline earth metal carbonates like potassium carbonate, sodium carbonate, sodium bicarbonate and the like.

The starting compounds of formula II are either known in the art or can be prepared by following known methods, for example, Gordon J. Phillips et al., J. Med. Chem., 37, 3717-3729 (1994); United States Patent No. 4335121; PCT publication No. WO 04/001369 or WO 04/039827.

Scheme-III represents a reaction sequence following which compound of formula II can be prepared.

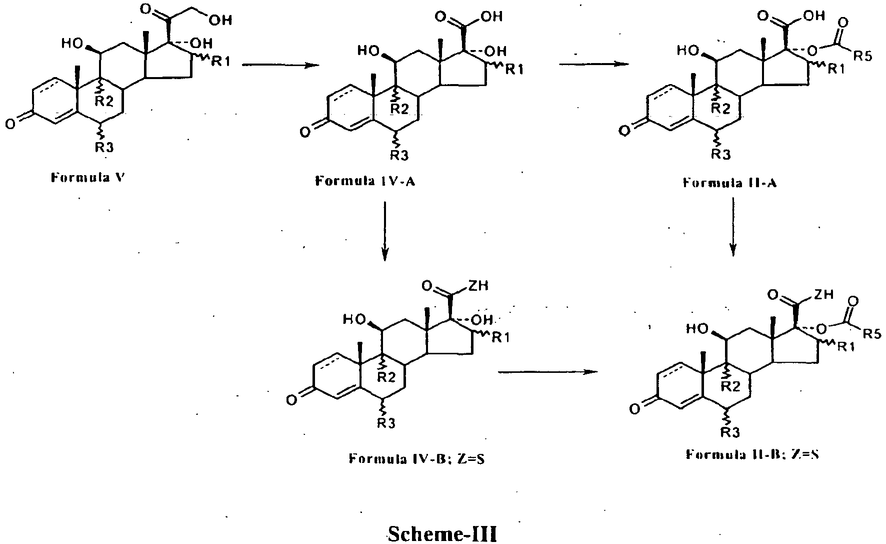

Preferably, the compounds of formula II-A (compound of formula II, wherein Z is O) may be prepared by treating a compound of formula IV-A with compounds of formula R₅COOH or an active derivative thereof such as an activated ester, anhydride or halide thereof, especially an acid halide, for example, an acid chloride, in the presence of an organic base like triethylamide or an inorganic base like potassium carbonate. To obtain compounds of formula II-A, wherein R₅ represents -O-R₆ or -N(R₈)R₆, the compound of formula IV-A is reacted with corresponding R₆-O-CO-Y' or R₆(R₈)N-CO-Y' where Y' represents a leaving group such as a halide, 4-nitrophenolate etc, in the presence of an organic base like triethylamine or an inorganic base like potassium carbonate. To obtain compounds of formula II-A, where R₅ represents -N(R₈)R₆, and R₈ is hydrogen, the compound of formula IV-A can be reacted with the corresponding isocyanate OCN-R₆. The compounds of formula IV-A may be prepared by oxidation of compounds of formula V in presence of of a chemical oxidizing agent like periodic acid or sodium periodate.

Preferably, the compounds of formula II-B (compound of formula II, wherein Z is S) may be prepared from compound formula II-A by initial reaction with *N,N*-dimethylthiocarbamoyl chloride, followed by aminolysis of the resulting rearranged mixed anhydride with diethylamine, or methanolysis by methanol and potassium carbonate, or reaction with sodium hydrosulfide in a suitable solvent, for example, *N,N*-dimethylformamide.

The compounds of formula II-B, where Z is S may also be prepared by treating a compound of formula IV-B with compounds of formula R₅COOH or an active derivative thereof such as an activated ester, anhydride or halide thereof, especially an acid halide, for example, an acid chloride, in the presence of an organic base like triethylamine or an inorganic abse like potassium carbonate. In compounds of formula II-B wherein R₅ represents -O-R₆ or -N(R₈)R₆, the compound of formula IV-B is reacted with corresponding R₆-O-CO-Y' or R₆(R₈)N-CO-Y' where Y' represents a leaving group such as a halide, 4-nitrophenolate etc, in the in the presence of an organic base like triethylamine or an inorganic base like potassium carbonate.

The compounds of formula IV-B, where Z is S may be prepared from compound formula IV-A by carbonyl activation with 1,1'-carbonyldiimidazole and reaction with hydrogen sulfide or sodium hydrosulfide in a suitable solvent, for example, *N,N*-dimethylformamide.

The compounds of formula II may be conveniently used as acid addition salts thereof, preferably in crystalline form. Any suitable base addition salts can be used, for example, sodium or potassium salt.

The compound of formula III-A are known or may be prepared by methods known in the art.

In one embodiment the present invention provides a compound of formula I-B and a process for preparation thereof comprising reacting a carboxylic acid compound of formula II-A (a compound of formula II, wherein Z is O) or carbothioic acid compound of formula II-B (a compound of formula II, wherein Z is S) with a compound of formula III-B (Scheme-IV): 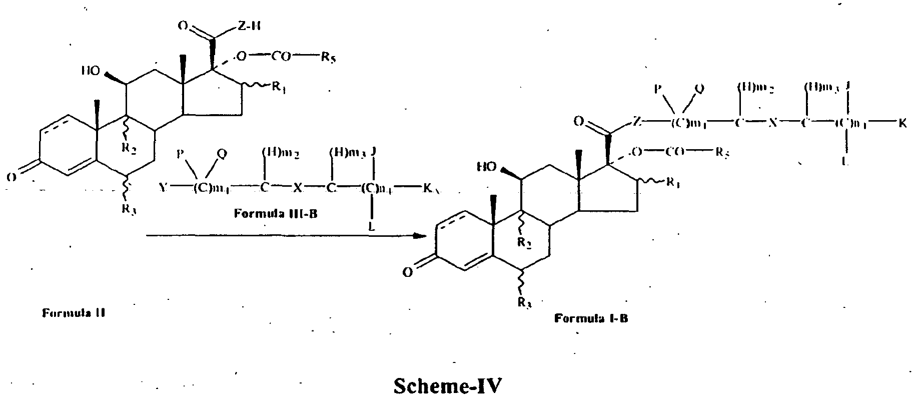 -̅-̅-̅-̅-̅-̅ represents a single or double bond and 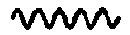 represents α or β-configuration.
wherein Z represents O or S;
R₁, R₂, R₃, R₅, Y, P, Q, J, K, L, m₁ and n₁ are as defined above, X represents either a double bond or a triple bond, m₂ is 0 or 1 and m₃ is 0 or I;

The compound of formula 1-B can be prepared by alkylation of compound of formula II with compound of formula III-B, advantageously carried out in an inert organic polar solvent, for example, acetone, acetonitrile, *N,N*-dimethylformamide, *N,N*-dimethylacetamide, dimethyl sulfoxide and the like in presence of a suitable base, for example, alkali or alkaline earth metal carbonates like potassium carbonate, sodium carbonate, sodium bicarbonate and the like. The compound of formula III-B are known or can be prepared by methods known.in the art.

In one preferred embodiment, the present invention provides a compound of formula I-C and a process for preparation thereof comprising reacting a compound of formula V1 with a compound of formula III-C (Scheme-V): 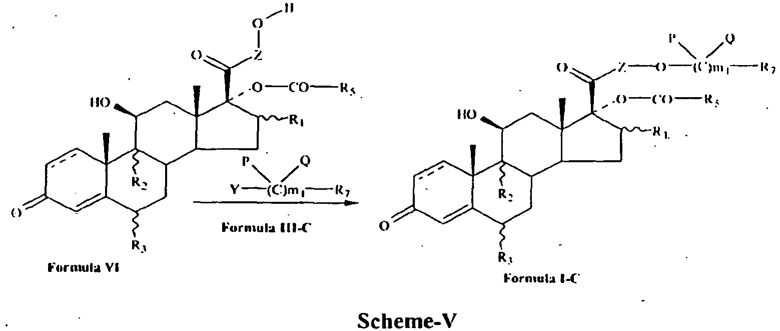 -̅-̅-̅-̅-̅-̅ represents a single or double bond and 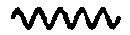 represents α or β-configuration.
wherein Z represents S;
R₁, R₂, R₃, R₅, R₇, Y, P, Q and m₁ are as defined above;

The compound of formula I-C can be prepared by the alkylation of compound of formula V1 with compound of formula III-C, advantageously carried out in an inert organic polar solvent, for example, acetone, acetonitrile, *N,N*-dimethylformamide, *N,N*-dimethylacetamide, dimethyl sulfoxide and the like in presence of a suitable base, for example, alkali or alkaline earth metal carbonates like potassium carbonate, sodium carbonate, sodium bicarbonate and the like. The compound of formula III-C are known or can be prepared by methods known in the art.

The compound of formula VI may be prepared by oxidation of compound of formula II-B with oxidizing agent, for example, hydrogen peroxide, urea-hydrogen peroxide; peracetic acid, *m-*chloroperbenzoic acid, sodium periodate and the like in a polar organic solvent such as methanol, ethanol, 2-propanol, acetic acid and the like.

The compound of formula I and physiologically acceptable salt thereof can also be used as intermediates for preparation of derivatives thereof, for example, the -OH group can be converted to halo group or acetylated, sulfonated etc.

The present invention also relates to the dosage forms for local application depends on the precise type of formulation to be prepared but generally may be within the range of 0.001 to 10% by weight. Generally, however for most types of preparations advantageously the proportion used will be within the range of 0.005 to 1% and preferably 0.01 to 0.5%. However, in powders for inhalation or insufflation the proportion used may be within the range of 0.1 to 5%. The daily dose may vary and would be dependent on the condition being treated, and the duration of treatment desired.

In one embodiment, the amount of a compound according to formula I used to attain the desired biological effect depends on a number of factors, for example the specific compound selected, the intended use, the type of administration and the clinical state of the patient. In general, the daily dose is in the range of 0.01 mg to 500mg (typically from 0.1 mg to 10mg). Orally administrable individual dose formulations such as, for example, tablets or capsules can contain, for example, from 0.1 mg to 500mg, typically from 1 mg to 100 mg.

In the case of physiologically acceptable salts, the above mentioned masses.relate to the mass of the free compound on which the addition salt is based. The compounds used for the prophylaxis or therapy may be the compounds according to formula I themselves, or, in one embodiment, they are present in the form of a pharmaceutical composition together with an acceptable carrier. In one embodiment, the carrier must be naturally acceptable, in the sense that it is compatible with the other ingredients of said composition and is not harmful to the patient's health. The carrier may be a solid or a liquid or both and is preferably formulated with the compound as an individual dose, for example as a tablet that may contain from 0.05% to 95% by weight of the active compound. The pharmaceutical compositions of the invention may be prepared according to any of the known pharmaceutical methods which essentially comprise mixing the ingredients with pharmacologically acceptable carriers and/or excipients.

Another aspect of the present invention relates to formulation of compounds of formula I in a suitable form for administration to a subject.

Pharmaceutical compositions of the invention are those which are suitable for oral, rectal, topical, peroral (e.g. sublingual) and parenteral (e.g. subcutaneous, intradermal or intraarticular) administration, although the most suitable manner of administration depends in each individual case on the nature and severity of the condition to be treated and on the nature of the compound according to formula I used in each case. Sugar-coated formulations and sugar-coated delayed-release formulations, too, are included within the scope of the invention. In one embodiment, preference is given to acid-resistant and enteric formulations. Suitable enteric coatings include cellulose acetate phthalate, polyvinyl acetate phthalate, hydroxypropylmethylcellulose phthalate and anionic polymers of methacrylic acid and methyl methacrylate.

Suitable pharmaceutical compounds for oral administration may be present in separate units as, for example, capsules, cachets, lozenges or tablets, which in each case contain a particular amount of the compound according to formula I; as powders or granules; as solution or suspension in an aqueous or non-aqueous liquid; or as an oil-in-water or water-in-oil emulsion. These compositions can be prepared according to any suitable pharmaceutical method which includes a step in which the active compound and the carrier (which may comprise one or more additional components) are contacted. In general, the compositions are prepared by uniform and homogeneous mixing of the active compound with a liquid and/or finely dispersed solid carrier. after which the product is shaped, if necessary. Thus a tablet, for example, may be prepared by pressing or shaping a powder or granules of the compound, where appropriate with one or more additional components. Pressed tablets can be prepared by tableting the compound in free-flowing forum, for example a powder or granules, mixed, where appropriate, with a binder. lubricant, inert diluent and/or one or more surface active/dispersing agents in a suitable machine. Shaped tablets can be prepared by shaping the pulverulent compound, moistened with an inert liquid diluent, in a suitable machine.

Pharmaceutical compositions which are suitable for peroral (sublingual) administration include lozenges which contain a compound according to formula I with a flavoring agent, usually sucrose and gum arabic or tragacanth, and pastilles which comprise the compound in an inert base such as gelatin and glycerol or sucrose and gum arabic.

Suitable pharmaceutical compositions for parenteral administration may, for example, comprise sterile preparations of a compound according to formula I. These preparations may be administered intradermally, although they may also be administered subcutaneously, intramuscularly or intraarticularly as an injection. These preparations may be prepared by mixing the compound with water and rendering the obtained solution sterile. Injectable compositions of the invention generally contain from 0.01 to 5% by weight of the active compound.

Suitable pharmaceutical compositions for rectal administration are present as individual dose suppositories. These may be prepared by mixing a compound according to formula I with one or more conventional solid carriers, for example cocoa butter, and shaping the resulting mixture.

Suitable pharmaceutical compositions for topical application to the skin may be present as ointment, cream, lotion, paste, spray, aerosol or oil. The carriers which may be used include, for example, petroleum jelly, lanolin, polyethylene glycols, alcohols and combinations of two or more of these substances. In one embodiment, the active compound is present at a concentration ranging from 0.1 to 15%, for example from 0.5 to 2%, by weight of the composition.

Transdermal administration is also possible. Suitable pharmaceutical compositions for transdermal administration may be present as individual patches, which are suitable for long-term close contact with the epidermis of the patient. Such patches suitably contain the active compound in an optionally buffered aqueous solution, dissolved and/or dispersed in an adhesive or dispersed in a polymer. A suitable active compound concentration is from approx. 0.1% to 35%, preferably approx. 1 to 15%. A particular possibility is the release of the active compound by electro-transport or ionophoresis, as described, for example, in Pharmaceutical Research. 2(6): 318 (1986).

In one preferred embodiment the compounds of formula I are adapted for local administration. Local administration as used herein includes administration by insufflations and inhalation. The various types of dosage forms useful for local administration include ointments, lotions, creams, gels, foams, preparations for transdermal delivery by patches, powders, sprays, aerosols, capsules or cartridges for use in inhaler or insufflator or drops (for example, eye,or nose drops), solution/suspensions for nebulisation, suppositories, pessaries, retention enemas and chewable or suckable tablets or pellets or microencapsulation preparations.

Ointments, creams and gels, may for example, be formulated with an aqueous or oily base with the addition of suitable thickening and/or gelling agent and/or solvents. Such bases may thus, for example, include water and/or oil such as liquid paraffin or a vegetable oil such as arachis oil or caster oil, or a solvent such as polyethylene glycol. Thickening agents and gelling agents which may be used according to the nature of the base include soft paraffin, aluminum stearate, cetostearyl alcohol, polyethylene glycols, woolfat, beewax, carboxypolymethylene and cellulose derivatives, and/or glyceryl monosterate and non-ionic emulsifying agents.

Powders for external application may be formed with the aid of any suitable powder base, for example, talc, lactose or starch. Drops may be formulated with an aqueous or non aqueous base also comprising agents, solubilizing agents, suspending agents or preservatives.

Spray compositions may for example be formulated as aqueous solution or suspensions or as aerosols delivered from pressurized packs, such as a metered dose inhaler, with the use of a suitable liquefied propellant. Aerosol compositions suitable for inhalation can be either a suspension or a solution and generally contain a compound of formula I and suitable propellant such as a fluorocarbon or hydrogen containing chlorofluorocarbon or mixtures thereof, particularly hydrofluoroalkanes, especially 1,1,1,2-tetrafluoroethane, 1,1,1,2,3,3,3-heptafluoro-*n-*propane or a mixture thereof. The aerosol composition may optionally contain additional formulation excipients well known in the art such as surfactants e.g. oleic acid or lecithin and co solvents e.g. ethanol.

Advantageously, the formulations of the invention may be buffered by the addition of suitable buffering agents.

Capsules and cartridges for use in an inhaler or insufflator, for example gelatine, may be formulated containing a powder mix or for inhalation of a compound of the invention suitable powder base such as lactose or starch. Each capsule or cartridge may generally contain between 20µg-1000µg of the compound of formula I. Alternatively, the compound of the invention may be presented without excipients such as lactose.

The proportion of the active compound of formula I in the local compositions according to the invention depends on the precise type of formulation to be prepared but will generally be within the range of form 0.001 to 10% by weight. Generally, however for most types of preparations advantageously the proportion used will be within the range of form 0.005 to 1% and preferably 0.01 to 0.5%. However, in powders for inhalation or insufflation the proportion used will be within the range of from 0.1 to 5%.

Aerosol formulations are preferably arranged so that each metered dose or "puff" of aerosol contains 20µg-2000µg, preferably about 20µg-500µg of a compound of formula I. Administration may be once daily or several times daily, for example 2, 3, 4 or 8 times, giving for example 1, 2 or 3 doses each time. The overall daily dose with an aerosol will be within the range 100µg-1000µg preferably, 200µg-2000µg. The overall daily dose and the metered dose delivered by capsules and cartridges in an inhaler or insufflator will generally be double those with aerosol formulations.

Topical preparation may be administrated by one or more applications per day to the effected area, occlusive dressing may advantageously be used. A continuous or prolonged delivery may be achieved by an adhesive reservoir system.

A pharmaceutical composition may be a solution or suspension comprising a tlterapeutically effective concentration of compound of formula I, with or without other bioactives in pharmaceutically effective concentrations. The composition may additionally comprise of various pharmaceutically acceptable excipients such as chelating agents, pH modifiers, buffers, viscosity enhancers, isotonicity agents, solubilizers, preservatives, antioxidants and the like.

A compound of formula I may be formulated in the form of drug delivery systems such as gel forming solutions.

For internal administration the compounds according to the invention may, for example, be formulated in conventional manner for oral, parenteral or rectal administration. Formulation for oral administration include syrups, elixirs, powders, granules, tablets and capsules which typically contain conventional excipients such as binding agents, fillers, lubricants, disintegrants, wetting agents, suspending agents, emulsifying agents, preservatives, buffer salts, flavoring, coloring and/or sweetening agents as appropriate. Dosage unit forms are, however, preferred as described below.

Slow release or enteric coated formulations may be advantageous, particularly for inflammatory bowel disorders.

Preferred forms of preparation for internal administration are dosage unit forms i.e. tablets and capsules: Such dosage unit forms contain from 0.01mg to 20mg preferably from 2.5mg to 10mg of the compounds of the invention.

The present invention provides a pharmaceutical composition comprising 11β-hydroxyandrosta-4-ene-3-one, a compound of formula I and physiologically acceptable salt thereof.

The present invention in one embodiment provides a pharmaceutical composition comprising 11β-hydroxyandrosta-4-ene-3-one,- a compound of formula I-A, and physiologically acceptable salt thereof.

The present invention in one embodiment provides a pharmaceutical composition comprising 11β-hydroxy androsta-4-ene-3-one, a compound of formula I-B, physiologically acceptable salt thereof.

The present invention in one embodiment provides a pharmaceutical composition comprising 11β-hydroxyandrosta-4-ene-3-one, a compound of formula I-C, and physiologically acceptable salt thereof.

The invention is illustrated but not restricted by the description in the following examples.

### EXAMPLES

### Example 1

### 9,11-Epoxy-17α-hydroxy-16α-methyl-3-oxandrosta-1,4-diene-17β-carboxylic acid

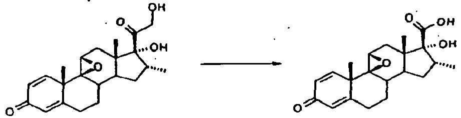

A solution of periodic acid (4.59g, 0.0201mol) in water (25ml) (prepared by heating to 50-55°C and then cooling to 30-35°C) was added dropwise to a stirred suspension of 17α,21-dihydroxy-9,11-epoxy-16α-methylpregna-1,4-diene-3,20-dione (5.0g, 0.0134mol) in tetrahydrofuran (25ml) at 0-5°. The reaction mixture was stirred at 0-5°C for 2hrs, water was added to the reaction mixture at 0-5°C. The precipitated product was filtered, washed with water, and dried to yield the title compound as white solid (4.75g, 98.7%).
¹H NMR (400 MHz in CDCl₃), δ: 0.92(d, *J*=6.99Hz, 3H), 1.04(s, 3H), 1.29-1.36(m, 1H), 1.44(s, 3H), 1.41-1.82(m, 4H), 2.12-2.94(m, 6H), 3.19(s, 1H), 3.55-3.65(m, 1H), 6.11(s, 1H), 6.14(dd, *J*₁=10.09Hz_{;} *J*₂=1.68Hz, 1H), 6.60(d, *J*=10.06Hz, 1H).

### Example 2

### 9,11-Epoxy-17α-[(2-furanylcarbonyl)oxy]16α-methyl-3-oxoandrosta-1,4-diene-17β-carboxylic acid

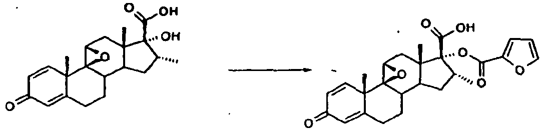

To suspension of 9,11-epoxy-17α-hydroxy-16α-methyl-3-oxoandrosta-1,4-diene-17β-carboxylic acid.(4.5g, 0.012mol) in acetone (25ml) at 10-15°C was added sequentially triethylamine (5.3ml, 0.038mol) and 2-furoyl chloride (4.96g, 0.038mol). After stirring for 4 hrs at 25-30°C, diethylamine (2.63ml, 0.025mol) was added dropwise at 10-15°C and then stirred at ambient temperature for 1hr. Thereafter the reaction mixture was acidified to pH 1.0-1.5 at 0-5°C, the precipitated product was filtered, washed with water, and dried to yield the title compound as white solid (5.96g, 99%). ¹H NMR (400 MHz in CDCl₃+DMSO-d6), δ: 0.98(d, *J*=7.09Hz, 3H), 1.07(s, 3H), 1.25-1.48(m, 2H), 1.46(s, 3H), 1.66-1.81(m, 3H), 2.02-2.74(m, 6H), 3.26(s, 1H), 3.26-3.32
(m, 1H), 6.19(s, 1H), 6.22(dd, *J₁*=10.13Hz, *J₂*=1.76Hz, 1H), 6.53(dd, *J1*=3.44Hz, *J₂*=1.61Hz, 1H), 6.64(d, *J*=10.04Hz, 1H), 7.15(d, *J*=3.37Hz, 1H), 7.62(d, *J*=0.81Hz, 1H).

### Example 3

### 17β-[(N,N-Dimethylcarbamoyl)thio]carbonyl-9,11-epoxy-17α-[(2-furanylcarbonyl)oxyl-16α-methyl-3-oxoandrosta-1,4-diene

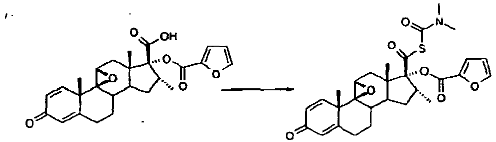

A solution of 9,11-epoxy-17α-[(2-furanylcarbonyl)oxy] 16α-methyl-3-oxoandrosta-1,4-diene-17β-carboxylic acid (7.0g, 0.0129mol) and *N,N*-dimethylthiocarbamoyl chloride (2.74g, 0.0259mol) in tetrahydrofuran (35ml) was cooled to 10 to 15°C and treated with triethylamine (2.86g, 0.0283mol) and tetrabutulammonium iodide (0.5g). The reaction mixture was then warmed to ambient temperature, stirred for 4hrs and then added *N,N*-dimethylacetamide (21 ml) followed by water (140ml). The resultant mixture was cooled to 0°C, the product filtered. washed with water and dried to yield the title compound as light yellow solid. (6.6g, 79.1%).
¹H NMR (400 MHz in CDCl₃+DMSO-d6), δ: 1.01(d, *J*=7.11Hz, 3H), 1.15(s, 3H), 1.41-1.49(m, 2H), 1.46(s, 3H), 1.72-1.87(m, 3H), 2.19-2.73(m, 6H), 3.06(s, 3H), 3.16(s, 3H), 3.30(s, 1H), 3.30-3.32(m, 1H), 6.19(s, 1H), 6.22(dd, *J₁*=10.02Hz, *J₂*=1.74Hz, 1H), 6.58(dd, *J*₁=3.51Hz, *J*₂=1.68Hz, 1H), 6.64(d, *J*=10.09Hz, 1H), 7.22(d, *J*=3.51Hz), 7.67(d, *J*=0.90Hz, 1H).

### Example 4

### 9,11-Epoxy-17α-[(2-furanylcarbonyl)oxy]-17α-hydroxy-16α-methyl-3-oxoandrosta-1,4-diene-17β-carbothioic acid

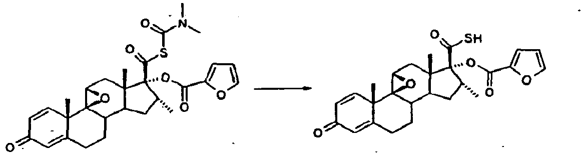

A suspension of 17β-[(*N,N*-dimethylcarbamoyl)thio]carbonyl-9,11-epoxy-17α-[(2-furanylcarbonyl)oxy]-16α-methyl-3-oxoandrosta-1,4-diene (5.0g, 0.0106 mol) and potassium carbonate (2.94g, 0.0213mol) in methanol (25ml) was stirred at ambient temperature for 5hrs under a blanket of nitrogen. Thereafter, water was added to the reaction mixture and the resultant clear solution was washed with toluene . The aqueous layer containing the product was charcoalized and then acidified with 2N HCl until pH was 1.5 to 2.0. The precipitated product was filtered, washed with water, and dried to yield the title compound as an off-white solid (3.6g, 83%).
¹H NMR (400 MHz in CDCl₃), δ: 1.01(d, *J*=7.11Hz, 3H), 1.12(s, 3H), 1.36-1.48(m, 2H), 1.46(s, 3H), 1.72-1.81(m, 3H), 2.18-2.69(m, 6H), 3.28-3.31(m, 1H), 3.31(s, 1H), 6.21(s, 1H), 6.25(dd, *J₁*=10.08Hz, *J₂*=1.76Hz, 1H), 6.55(dd, *J*₁=3.48Hz, *J*₂=1.61Hz, 1H), 6.63(d, *J*=10.06Hz, 1H), 7.23(dd, *J₁*=3.45Hz, *J₂*=0.58Hz, 1H), 7.64(d, *J*=0.97Hz, 1H).

### Example 5

### 17β-[(N,N-dimethylcarbamoyl)thio]carbonyl-6α,9α-difluoro-17α-[(2-furanyl-carbonyl)oxy]-11β-hydroxy-16α-methyl-3-oxoandrosta-1,4-diene

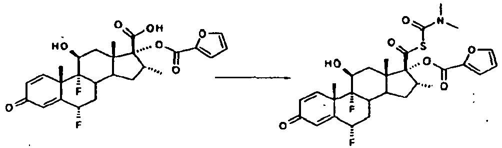

To a solution of 6α,9α-difluoro-17α-[(2-furanylcarbonyl)oxy]-11β-hydroxy-16α-methyl-3-oxoandrosta-1,4-diene-17β-carboxylic acid (12.3g, 0.025mol) and *N*,*N*-dimethylthiocarbamoyl chloride (9.29g, 0.075mol) in tetrahydrofuran (60ml) was added triethylamine (10.48ml, 0.075mol) at 10-15°C and tetrabutylammonium iodide (0.92g) and the mixture stirred for 4hrs at ambient temperature. Dimethylacetamide (37ml) and water (310ml) were added to the reaction mixture and stirred for 2hrs at 0°C. The solid obtained was filtered, washed with water and dried to yield the title compound as a white solid (14.3g, 99%).
¹H NMR (400 MHz in CDCl₃+DMSO-d6), δ: 1.00(d, *J*=7.10Hz, 3H), 1.19(s, 3H), 1.30-1.36(m, 1H), 1.54(s, 3H), 1.63-2.00(m, 3H), 2.22-2.60(m, 4H), 3.03(s, 3H), 3.16(s, 3H), 3.31-3.36(m, 1H), 4.29(d, *J*= 8.91 Hz, 1H), 5.39(br-s, 1H), 5.43-5.59(ni, 1H), 6.22(s, 1H), 6.27(dd, *J*₁=10.15Hz, *J*₂=1.75Hz, 1H), 6.60(dd, *J*₁=3.49Hz, *J*₂=1.70Hz, 1H), 7.15(d, *J*=3.46Hz, 1H ), 7.26(d, *J*=9.85Hz, 1H), 7.80(d, *J*=0.92Hz, 1H).

### Example 6

### 6α,9α-Difluoro-11β-hydroxy-16β-methyl-3-oxo-17α-propionyloxyandrosta-1,4-diene-17β-carbothioic acid

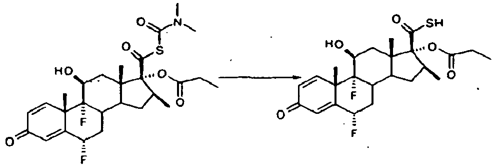

A suspension of 17β-[(*N*,*N*-dimethylcarbamoyl)thio]carbonyl-6α,9α-difluoro-11β-hydroxy-16β-methyl-3-oxo-17α-propionyloxyandrosta-1,4-diene (5.0g, 9.27 mmol) in 75ml diethylamine was heated under reflux for 8hrs. The cooled reaction mixture was poured in to ice-water acidified with 2N HCl to pH 2 and extracted with ethyl acetate. The ethyl acetate extract was washed with water, dried, and concentrated *in vacuo.* The residue obtained was crystallized from ethyl acetate to give the title compound as light yellow solid (3.2g, 73.7%)
¹H NMR (400 MHz in CDCl₃), δ: 1.06(s, 3H), 1.16(t, *J*=7.52Hz, 3H), 1.20-1.25(m, 1H), 1.39(d. *J*=7.23Hz, 3H), 1.54(s, 3H), 1.67-2.05(m, 4H), 2.22-2.80(m, 4H), 2.39(q, *J*=7.69Hz, 2H). 4.45(br-d, *J*=7.69Hz, 1H),5.30-5.50(m, 1H), 6.38(d, *J*=9.93Hz, 1H), 6.43(s, 1H), 7.15(dd. *J*=10.17Hz, 1H).
In an analogous manner was prepared 6α,9α-difluoro-17α-[(2-furanylcarbonyl)oxy]-11β-hydroxy-16β-methyl-3-oxoandrosta-1,4-diene-17β-carbothioic acid.

### Example 7

### 6α,9α-Difluoro-17α-[(2-furanylcarbonyl)oxy]-11β-hydroxy-16α-methyl-3-oxoandrosta-1,4-diene-17β-carbothioic acid

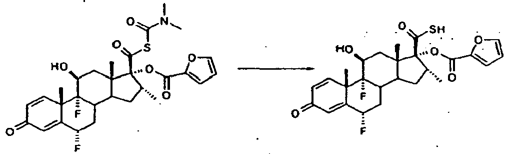

A suspension of 17β-[(*N*,*N*-dimethylcarbamoyl)thio]carbonyl-6α,9α-difluoro-17α-[(2-furanylcarbonyl)oxy]-11β-hydroxy-16α-methyl-3-oxoandrosta-1,4-diene (14.2g, 0.024mol) and potassium carbonate (6.78g, 0.0491mol) in methanol (70ml) was stirred at ambient temperature for 6hrs under a blanket of nitrogen. Thereafter, water was added to the reaction mixture and the resultant clear solution was washed with toluene. The aqueous layer containing the product was charcoalized and then acidified with 2N HCl until pH was 1.5 to 2.0. The precipitated product was filtered, washed with water, and dried to yield the title compound as white solid (11.8g 94.8%).
¹H NMR (400 MHz in CDCl₃+DMSO-d6), δ: 1.03(d,*J*=7.17Hz, 3H), 1.20(s, 3H), 1.31-1.37(m. 1H), 1.56(s, 3H), 1.73-2.03(m, 3H), 2.28-2.59(m, 4H), 3.37-3.42(m, 1H), 4.35-4.38(br-d, 1H), 4.89(d, *J*=1.22Hz, 1H), 5.34-5.51(m, 1H), 6.34(dd, *J*₁=10.06H_{Z}, *J*₂=1.88Hz, 1H), 6.39(s, 1H), 6.53(dd, *J*₁=3.45Hz, *J*₂=1.73Hz, 1H), 7.14(dd, *J*₁=3.28Hz, *J*₂=0.49Hz, 1H), 7.25(dd, *J*₁=10.12Hz, *J*₂=1.30Hz, 1H), 7.63(dd, *J*₁=1.73Hz, *J*₂=0.63Hz, 1H).

The following 17β-carbothioic acids were prepared by an analogous method to example 7:
9α-Fluoro-17α-[(2-furanylcarbonyl)oxy]-11β-hydroxy-16α-methyl-3-oxoandrosta-1,4-diene-17β-carbothioic acid.
6α,9α-Difluoro-11β-hydroxy-16a-methyl-17α-[[(3-methyl-2-furanyl)carbonyl]oxy]-3-oxoandrosta-1,4-diene-17β-carbothioic acid.
6α,9α-Difluoro-17α-[(3-furanylcarbonyl)oxy]-11β-hydroxy-16α-methyl-3-oxoandrosta-1,4-diene-17β-carbothioic acid.
6α,9α-Difluoro-11β-hydroxy-16α-methyl-3-oxo-17α-[(2-thienylcarbonyl)oxy]-androsta-1,4-diene-17β-carbothioic acid.
17α-[[(5-Chloro-2-thioenyl)carbonyl]oxy]-6α,9α-difluoro-11β-hydroxy-16β-methyl-3-oxoandrosta-1,4-dine-17β-carbothioic acid.
17α-Cyclopropylcarbonyloxy-6α,9α-difluoro-11β-hydroxy-16α-methyl-3-oxoandrosta-1,4-diene-17β-carbothioic acid.
17α-Cyclobutylcarbonyloxy-6α,9α-difluoro-11β-hydroxy-16α-methyl-3-oxoandrosta-1,4-diene-17β-carbothioic acid.
17α-Cyclopentylcarbonyloxy-6α,9α-difluoro-11β-hydroxy-16α-methyl-3-oxoandrosta-1,4-diene-17β-carbothioic acid.
17α-Cyclohexylcarbonyloxy-6α,9α-difluoro-11β-hydroxy-16α-methyl-3-oxoandrosta-1,4-diene-17β-carbothioic acid.
17α-Cyclohexylmethylcarbonyloxy-6α,9α-difluoro-11β-hydroxy-16α-methyl-3-oxoandrosta-1,4-diene-17β-carbothioic acid.
9α-Fluoro-11β-hydroxy-16α-methyl-3-oxo-17α-propionyloxyandrosta-1,4-diene-17β-carbothioic acid.
9α-Fluoro-11β-hydroxy-16β-methyl-3-oxo-17α-propionyloxyandrosta-1,4-diene-17β-carbothioic acid.
6α,9α-Difluoro-11β-hydroxy-16α-methyl-3-oxo-17α-propionyloxyandrosta-1,4-diene-17β-carbothioic acid.
6α,9α-Difluoro-11β-hydroxy-16β-methyl-3-oxo-17α-propionyloxyandrosta-1,4-diene-17β-carbothioic acid.
17α-Butryloxy-6α,9α-difluoro-11β-hydroxy-16α-methyl-3-oxoandrosta-1,4-diene-17β-carbothioic acid.
6α,9α-difluoro-11β-hydroxy-16α-methyl-3-oxo-17α-valeryloxyandrosta-1,4-diene-17β-carbothioic acid.
6α,9α-Difluoro-11β-hydroxy-16α-methyl-17α-(2-methylpropionyl)oxy-3-xoandrosta-1,4-diene-17β-carbothioic acid.
17α-Dichloroacetoxy-6α,9α-difluoro-11β-hydroxy-16α-methyl-3-oxoandrosta-1,4-diene-17β-carbothioic acid.
6α,9α-Difluoro-11β-hydroxy-17α-(methoxymethylcarbonyl)oxy-16α-methyl-3-oxoandrosta-1,4-diene-17β-carbothioic acid.
6α,9α-Difluoro-17α-(ethoxymethylcarbonyl)oxy-11β-hydroxy-16α-methyl-3-oxoandrosta-1,4-diene-17β-carbothioic acid.
17α(Ethoxycarbonyl)oxy-11β-hydroxy-16α-methyl-3-oxoandrosta-1,4-diene-17β-carhothioic acid.
6α,9α*-*Difluoro-17α-(ethoxycarbonyl)oxy-11β-hydroxy-16α-methyl-3-oxoandrosta-1,4-diene-17β-carbothioic acid.

### Example 8- Compound IA.44

### 2-Phenyl-2-oxoethyl 6α,9α-difluoro-11β-hydroxy-16α-methyl-3-oxo-17α-propionyloxyandrosta-1,4-diene-17β-carboxylate

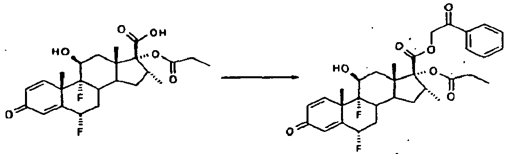

2-Bromo-1-phenylethanone (0.438g, 2.2mmol) and sodium iodide (50mg) were added to a stirred mixture of 6α,9α-difluoro-11β-hydroxy-16α-methyl-3-oxo-17α-propionyloxy-androsta-1,4-diene-17β-carboxylic acid (1.0g, 2.20mmol) and anhydrous potassium carbonate (0.360g, 2.6mmol) in acetone (15ml). The mixture was stirred under a blanket of nitrogen at 25 to 30°C for 8hrs, then poured into water and extracted with ethyl acetate. The organic layer was washed twice with water, dried, and concentrated *in vacuo.* The residue was purified by column chromatography (40% ethyl acetate in hexane) to yield the title compound as a white solid.

The following compounds were prepared in a manner similar to example 8 using an appropriate carboxylic acid of formula II and compound of formula III-A:
IA.1, IA.2, IA.4, IA.5, IA.7 to IA.10, IA.12, IA.15 to IA.19, IA.21, IA.23 to 25, IA.28 to 30, IA.32, IA.33, IA.36, IA.37, IA.42, IA.46, IA.48, IA50, IA.52, IA.54, IA.57, IA.60, IA.G2 to IA.67, IA.71, IA-72, IA-74, IA.76, IA.77, IA.80, IA.82, IA.84, IA.87, IA.91, IA.103, IA.115, IA.118, IA.121, IA.133.

### Example 9- Compound IA.38

### S-[2-Oxo-2-(4-trifluoromethylphenyl)ethyl] 6α,9α-difluoro-11β-hydroxy-16α-methyl-3-oxo-17α-propionyloxyandrosta-1,4-diene-17β-carbothioate

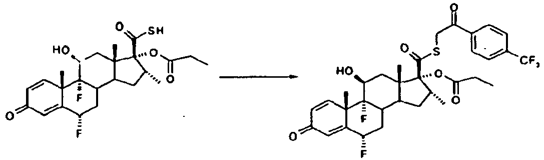

A mixture of 2-bromo-1-(4-trifluoromethylphenyl)ethan-1-one (0.569g, 2.13mmol), 6α,9α-difluoro-11β-hydroxy-16α-methyl-3-oxo-17α-propionyloxyandrosta-1,4-diene-17β-carbothioic acid (1.0g, 2.13mmol) and anhydrous potassium carbonate (0.295g, 2.13mmol) in acetone (25ml) was stirred under a blanket of nitrogen at 25 to 30°C for 3hrs. The reaction mixture was then poured into water and extracted with ethyl acetate. The organic layer was washed with water, dried, and concentrated *in vacuo.* The residue was purified by column chromatography (40% ethyl acetate in hexane) to yield the title compound as a white solid.
The following compounds were prepared in a manner similar to example 9 using an appropriate thioic acid of formula II and compound of formula III-A:
IA.3, IA.6, IA.11, IA.13, IA.14, IA.20, IA.22, IA.26, IA.27, IA.31, IA.34, IA.35, IA.39, IA.40, IA.43, IA.45, IA.47, IA.49, IA.51, IA.53, IA.55, IA.56, IA.58, IA.59, IA.61, IA.63, IA.68 to IA.70, IA.73, IA.75, IA.78, IA.79, IA.81, IA.83, IA.85, IA.86, IA.88 to IA.90, IA.92 to IA.102, IA.104 to IA.108, IA.110 to IA.114, IA.II6, IA.117, IA.119, IA.120, IA.122 to IA.124, IA.126 to IA.132, IA.134 to IA.186, IA.198.

### Example 10- Compound IA.59

### S-[1,1-Dimethyl-2-oxo-2-phenylethyl] 6α,9α-difluoro-11β-hydroxy-16α-methyl-3-oxo-17α-propionyloxyandrosta-1,4-diene-17β-carbothioate

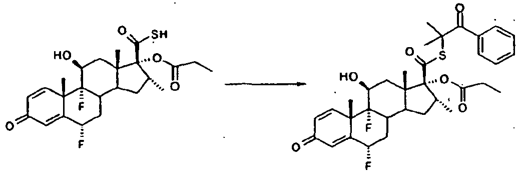

2-Bromo-2-methyl-1-phenyl-propan-1-one (0.483g, 2.13mmol) was added to a stirred mixture of 6α,9α-difluoro-11β-hydroxy-16α-methyl-3-oxo-17α-propionyloxyandrosta-1,4-diene-17β-carbothioic acid (1.0g, 2.13mmol) and anhydrous potassium carbonate (0.295g, 2.13mmol) in acetone (20ml) and the mixture was stirred under a blanket of nitrogen at 25 to 30°C for 3hrs. The reaction mixture was then poured into water and extracted with ethyl acetate. The organic layer was washed with water, dried, and concentrated *in vacuo.* The residue was purified by column chromatography (40% ethyl acetate in hexane) to yield the title compound as a white solid.

The following compounds were prepared in a manner similar to example 10 using an appropriate thioic acid of formula II and compound of formula III-A:
IA.109, IA.187 to IA.197.

### Example 11- Compound IA-1.2

### S-(1-Benzoylcyclopentyl) 6α,9α-difluoro-11β-hydroxy-16α-methyl-3-oxo-17α-propionyloxyandrosta-1,4-diene-17β-carbothioate

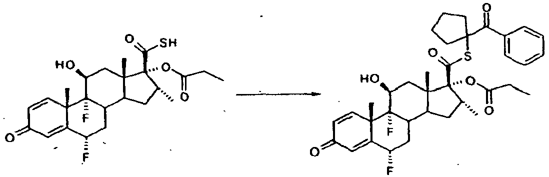

(1-Bromocyclopentyl) phenyl methanone (0.539g, 2.13mmol) was added to a stirred mixture of 6α,9α-difluoro-11β-hydroxy-16α-methyl-3-oxo-17α-propionyloxyandrosta-1,4-diene- 17β-carbothioic acid (1.0g, 2.13mmol) and anhydrous potassium carbonate (0.295g, 2.13mmol) in acetone (25ml), and the mixture was stirred under a blanket of nitrogen at 25 to 30°C for 3hrs. The reaction mixture was then poured into water and extracted with ethyl acetate. The organic layer was washed with water, dried, and concentrated *in vacuo.* The residue was purified by column chromatography (35% ethyl acetate in hexane) to yield the title compound as a white solid.
The compound IA-1.1 was prepared in a manner similar to example 11.

### Example 12- Compound IA-2.1

### S-(1-Oxo-1,2,3,4-tetrahydronapthalen-2-yl) 6α,9α-difluoro-11β-hydroxy-16α-methyl-17α-propionyloxy-3-oxoandrosta-1,4-diene-17β-carbothioate

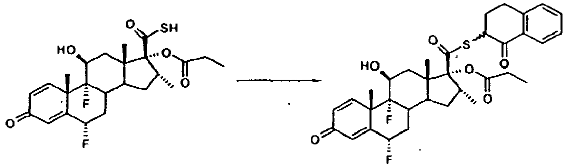

2-Bromo-1-tetralone (0.395g, 1.75mmol) was added to a stirred mixture of 6α,9α-difluoro-11β-hydroxy-16α-methyl-3-oxo-17α-propionyloxyandrosta-1,4-diene-17β-carbothioic acid (0.500g, 1.06mmol) and anhydrous potassium carbonate (0.161g, 1.17mmol) in acetone (10ml) and the mixture was stirred under a blanket of nitrogen at 25 to 30°C for 3hrs. The reaction mixture was then poured into water and extracted with ethyl acetate. The organic layer was washed with water, dried, and concentrated *in vacuo.* The residue was purified by column chromatography (40% ethyl acetate in hexane) to yield the title compound as a white solid.

### Example 13- Compound IA.41

### S-[2-Oxo-2-(2,4,6-Trimethylphenyl)ethyl] 9α-chloro-17α-1(2-furanylcarbonyl)oxy]-11β-hydroxy-16α-methyl-3-oxoandrosta-1,4-diene-17β-carbothioate

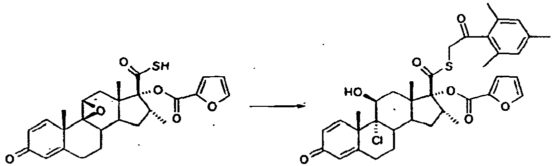

2-Bromo-1-(2,4,6-trimethylphenyl)ethanone (0.287g, 1.13mmol) was added to a stirred mixture of 9, 11-epoxy-17α-[(2-furanylcarbonyl)oxy]-16α-methyl-3-oxoandrosta-1,4-diene-17β-carbothioic acid (0.600g, 1.28mmol) and anhydrous potassium carbonate (0.194g, 1.40mmol) in acetone (25ml) and the mixture was stirred under a blanket of nitrogen at 25 to 30°C for 2.5hrs. The reaction mixture was then poured into water and extracted with ethyl acetate. The organic layer was washed with water, dried, and concentrated *in vacuo.* The crude solid was dissolved in glacial acetic acid (10ml) and concentrated hydrochloric acid (1ml) was added at 10-15°C. The mixture was stirred at 15-20°C for 30 min, poured into ice-water (400ml), the product filtered. washed with water, and finally dried in air oven. The crude solid was purified by column chromatography (35% ethyl acetate in hexane) to yield the title compound as a white solid.

### Example 14- Compound IB.45

### S-[4-Hydroxy-but-2-ynyl] 9α-chloro-17α-[(2-furanylcarbonyl)oxy]-11β-hydroxy-16α-methyl-3-oxoandrosta-1,4-diene-17β-carbothioate

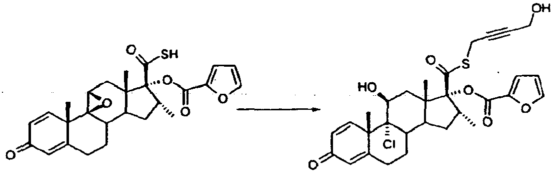

4-(Tetrahydropyran-2-yloxy)-but-2-ynyl toluene-4-sulfonic acid ester (0.727g, 2.24mmol) was added to a stirred mixture of 9,11-epoxy-17α-[(2-furanylcarbonyl)oxy]-17α-hydroxy-16α-methyl-3-oxoandrosta-1,4-diene-17β-carbothioic acid (1.0g, 2.13 mmol) and anhydrous potassium carbonate (0.300g, 2.34mmol) in acetone (20ml) and the mixture was stirred under a blanket of nitrogen at 25 to 30°C for 4hrs. The reaction mixture was then poured into water and extracted with ethyl acetate. The organic layer was washed with water, dried, and concentrated *in vacuo*. The crude solid was dissolve in methanol (15ml) and concentrated hydrochloric acid (4ml) was added at 10-15°C. The mixture was stirred at 25-30 for 30min and then heated at 60°C for 4hrs. The mixture was then cooled and poured in to ice-water. The solid was filtered, washed with water, and dried. The crude solid was purified by column chromatography (3% methanol in dichloromethane) to yield the title compound as a white solid.

### Example 15- Compound IB.2

### S-(4-Hydroxy-but-2-ynyl) 6α,9α-difluoro-11β-hydroxy-16α-methyl-3-oxo-17α-propionyloxyandrosta-1,4-diene-17β-carbothioate

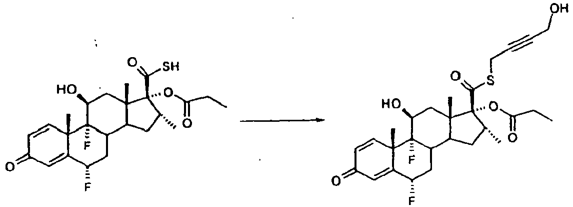

4-Chloro-but-2-yne-1-ol (0.169g, 1.62mmol) was added to a stirred mixture of 6α,9α-difluoro-11β-hydroxy-16α-methyl-3-oxo-17α-propionyloxyandrosta-1,4-diene-17β-carbothioic acid (0:800g, 1.54 mmol) and anhydrous potassium carbonate (0.234g, 1.70 mmol) in acetone (20ml) and the mixture was stirred under a blanket of nitrogen at 25 to 30°C for 3hrs. The reaction mixture was then poured into water and extracted with ethyl acetate. The organic layer was washed with water, dried, and concentrated *in vacuo.* The residue was purified by column chromatography (3% methanol in dichloromethane) to yield the title compound as a white solid.

The following compounds were prepared in a manner similar to example 15 using an appropriate thioic acid of formula II and compound of formula III-B:
IB.1, IB.4, IB.5, IB.7 to IB.11, IB.20 to IB.24, IB.26 to IB.29, IB.38, IB.39, IB.61, IB.62, IB:132.

### Example 16- Compound IB. 25

### S-[3-(1-Hydroxycyclohexyl)prop-2-ynyl] 6α,9α-difluoro-17α-[(2-furanylcarbonyl)oxy]-11β-hydroxy-16α-methyl-3-oxoandrosta-1,4-diene-17β-carbothioate

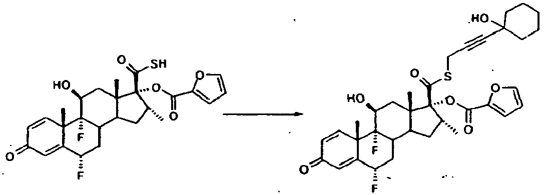

4-Methyl-[3-(hydroxycyclohexyl)prop-2-ynyl]benzenesulfonate (0.222g, 0.72mmol) was added to a stirred mixture of 6α,9α-difluoro-17α-[(2-furanylcarbonyl)oxy]-11β-hydroxy-16α-methyl-3-oxoandrosta-1,4-diene-17β-carbothloic acid (0.350g, 0.688mmol) and anhydrous potassium carbonate (0.104g, 0.756mmol) in acetone (20ml) and the mixture was stirred under a blanket of nitrogen at 25 to 30°C for 3hrs. The reaction mixture was then poured into water and extracted with ethyl acetate. The organic layer was washed with water, dried, and concentrated *in vacuo*. The residue was purified by column chromatography (35% ethyl acetate in hexane) to yield the title compound as a white solid.
Compound IB.40 was prepared in a manner similar to example 16.

### Example 17- Compound IB.35

### S-[4-n-Butylcarbonyloxy-but-2-ynyl] 6α,9α-difluoro-11β-hydroxy-16α-methyl-3-oxo-17α-propionyloxyandrosta-1,4-diene-17β-carbothioate

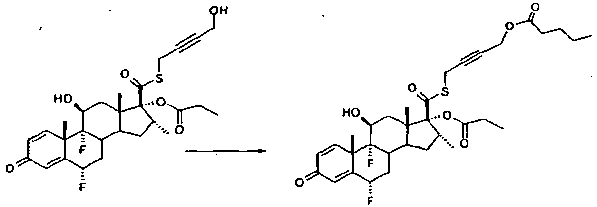

Valeryl chloride (0.337g, 2.79mmol) was added at 10-15°C in to a stirred mixture of S-(4-hydroxy-but-2-ynyl)6α,9α-difluoro-11β-hydroxy-16α-methyl-3-oxo-17α-propionyloxyandrosta-1,4-diene-1,7β-carbothioate (0.5g, 0.93mmol) and triethylamine (0.282g, 2.79mmol) in acetone (20ml) and the mixture was stirred under a blanket of nitrogen at 25-30°C for 8hrs. The reaction mixture was then poured into water and extracted with ethyl acetate. The organic layer was washed with water, dried, and concentrated *in vacuo*. The residue was purified by column chromatography (35% ethyl acetate in hexane) to yield the title compound as a white solid.

The following compounds were prepared with similar manner to example 17:
IB.31 to IB.37, IB.48 to IB.53, IB.55 to IB.60, IB.67 to IB.73, IB.75 to IB.86, IB.88, IB.92-IB.102, IB.104 to IB.122, IB.124, IB.133, IB.135, IB.148, IB.155, IB.I57.

### Example 18- Compound IB.3

### S-(4-Fluorobut-2-ynyl) 6α,9α-difluoro-11β-hydroxy-16α-methyl-3-oxo-17α-propionyl-oxyandrosta-1,4-diene-17β-carbothioate

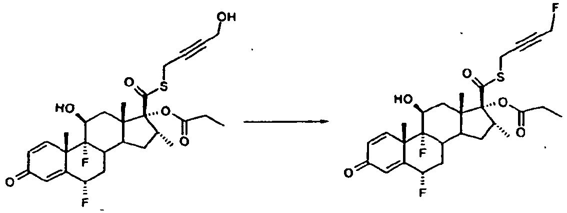

Diethylaminosulfur trifluoride (DAST) (0.151g, 0.93mmol) was added to a stirred solution of *S-*(4-hydroxy-but-2-ynyl) 6α,9α-difluoro-11β-hydroxy-16α-methyl-3-oxo-17α-propionyloxyandrosta-1,4-diene-17β-carbothioate (0.450g, 0.783mmol) in dry dichloromethane (20ml) at 25-30°C and the mixture was stirred under a blanket of nitrogen at 25-30°C for 2hrs. The reaction mixture was washed with sodium bicarbonate solution (25ml), water, dried, and concentrated *in vacuo.* The residue was purified by column chromatography (40% ethyl acetate in hexane) to yield the title compound as a white solid.

The compound IB.6 was prepared in a manner similar to example 18.

### Example 19- Compound IB. 15

### S-(4-Hydroxy-(Z)-but-2-enyl) 6α,9α-difluoro-17α-[(2-furanylcarbonyl)oxy]-11β-hydroxy-16α-methyl-3-oxoandrosta-1,4-diene-17β-carbothioate

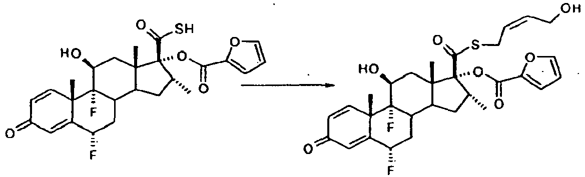

(*Z*)-4-Chloro-but-2-en-1-ol (0.691g, 6.48mmol) was added to a stirred mixture of 6α,9α-difluoro-17α-[(2-furanylcarbonyl)oxy]-11β-hydroxy-16α-methyl-3-oxoandrosta-1,4-diene-17β-carbothioic acid (3.0g, 5.87mmol) and anhydrous potassium carbonate (0.895g, 6.48 mmol) in acetone (60ml) and the mixture was stirred under a blanket of nitrogen at 25 to 30°C for 3hrs. The reaction mixture was then poured into water and extracted with ethyl acetate. The organic layer was washed with water, dried, and concentrated *in vacuo.* The residue was purified by column chromatography (1% methanol in dichloromethane) to yield the title compound as a white solid.
The compounds IB.16, IB.30 were prepared with similar manner to example 19. The compounds IB.13, IB.14, IB.17 were prepared by similar manner using (*E*)-4-bromo-bul-2-en-1-ol and an appropriate thioic acid of formula II.

### Example 20- Compound IB.44

### S-[4-(2-Ethoxy-1,2-dioxoethyl)oxy-(E)-but-2-enyl] 6α,9α-difluoro-11β-hydroxy-16α-methyl-3-oxo-17α-propionyloxyandrosta-1,4-diene-17β-carbothioate

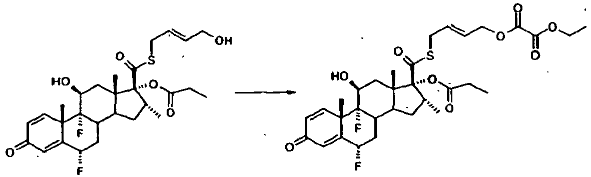

Ethyl oxalyl chloride (0.352g, 2.58mmol) was added at 10-15°C in to a stirred mixture of *S*-(4-hvdroxy-(*E*)-but-2-enyl)-6α,9α-difluoro-11β-hydroxy-16α-methyl-3-oxo-17α-propionyloxyandrosta-1,4-diene-17β-carbothioate (0.7g, 1.29mmol) and triethylamine (0.260g, 2.58mmol) in acetone (20ml) and the mixture was stirred under a blanket of nitrogen at 25-30°C for 8hrs. The reaction mixture was then poured into water and extracted with ethyl acetate. The organic layer was washed with water, dried, and concentrated *in vacuo.* The residue was purified by column chromatography (35% ethyl acetate in hexane) to yield the title compound as a white solid.

The compound IB.46 was prepared in a similar manner to example 20. The compounds IB.47 and IB.54 were prepared from IB.15 and IB.4 respectively, in a similar manner to example 20.

### Example 21- Compound IB.42

### 4-Methoxy-4-oxo-(E)-but-2-enyl 6α,9α-difluoro-11β-hydroxy-16α-methyl-3-oxo-17α-propionyloxyandrosta-1,4 diene-17β-carbothioate

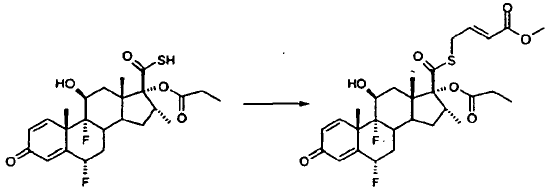

Methyl 4-bromocrotonate (0.191g, 1.06mmol) was added at 25-30°C in to a stirred mixture of 6α,9α-difluoro-11β-hydroxy-16α-methyl-3-oxo-17α-propionyloxyandrosta-1,4-diene-17β-carbothioic acid (0.5g, 1.06mmol) and anhydrous potassium carbonate (0.146g, 1.06mmol) in *N*,*N*-dimethylformamide (10ml) and the mixture was stirred under a blanket-of nitrogen at 25-30°C for 1.5hrs. The reaction mixture was then poured into water and extracted with ethyl acetate. The organic layer was washed with water, dried, and concentrated *in vacuo.* The residue was purified by column chromatography (40% ethyl acetate in hexane) to yield the title compound as a white solid.

Compounds IB.41 and IB.43 were prepared in a manner similar to example 21.

### Example 22

### 6α,9α-Difluoro-17α-[(2-furanylcarbonyl)oxy]-11β-hydroxy-16α-methyl-3-oxoandrosta-1,4-diene-17β-carbonylsulfenic acid

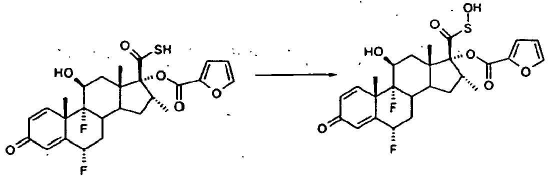

A solution of urea-hydrogen peroxide (2.41g, 0.0256mol) in *N*,*N*-dimethylformamide was added at 10 to 15°C into a clear solution of 6α,9α-difluoro-17α-[(2-furanylcarbonyl)oxy]-11β-hydroxy-16α-methyl-3-oxoandrosta-1,4-diene-17β-carbothioic acid (13.0g, 0.0256mol) in *N.N-*dimethylformamide (40ml). The reaction mixture was stirred at 10-15°C for 30min, then poured into ice-water. The solid was filtered, washed with water, and dried at 50-60°C to yield the title compound as white solid (12.0g, 89.48%).
¹H NMR (400 MHz in CDCl3+DMSO-d6), δ : 1.06(d. *J*=7.09Hz, 3H), 1.19(s, 3H), 1.33-1.38(m, 1H), 1.55(s, 3H), 1.58-1.98(m, 4H), 2.26-2.52(m, 4H), 3.02-3.06(m, 1H), 4,32-(br-d, *J*=8.76Hz, 1H), 5.14-5.19(br-s, 1H), 5.38-5.54(m, 1H), 6.29(s, 1H), 6.30(d, *J*=8.71Hz, 1H), 6.54(dd, *J*₁=3.32Hz, *J*₂=1.53Hz, 1H), 7.08(d, *J*=3.37Hz, 1H), 7.27(d, *J*=10.30Hz, 1H), 7.67(s, 1H).

### Example 23

### 6α,9α-Difluoro-11β-hydroxy-16α-methyl-3-oxo-17α-propionyloxyandrosta-1,4-diene-17β-carbonylsulfenic acid

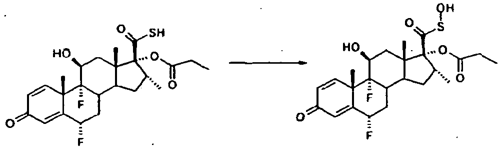

Hydrogen peroxide (30vol, 60ml) was added to a suspension of 6α,9α-difluoro-11β-hydroxy-16α-methyl-3-oxo-17α-propionyloxyandrosta-1,4-diene-17β-carbothioic acid (20.0g, 42.68mmol) in methanol (120ml) at 10 to 15°C . The reaction mixture was stirred at ambient temperature for 1hr. Excess peroxide was neutralized with 10% sodium metabisulfite solution, the product was filtered, washed with water, and dried to yield the title compound as white solid (19.9g, 96.22%).
¹H NMR (400 MHz in CDCl3+DMSO-d6), δ: 0.98(d, *J*=7.10Hz, 3H), 1.08(t, *J*=7.55Hz, 3H), 1.12(s, 3H), 1.25-1.33(m, 1H), 1.53(s, 3H), 1.58-1.93(m, 4H), 2.12-2.47(m, 4H), 2.33(q, *J*=7.57Hz, 2H), 2.95-2.99(m, 1H), 4.24-4.27(br-d, 1H), 5.15(br-s, 1H), 5.36-5.53(m, 1H), 6.26(s, 1H), 6.27(dd, *J*₁=10.21Hz, *J*₂=1.68Hz, 1H), 7.24(d, *J*=9.73Hz, 1H).

The following 17β-carbonylsulfenic acids were prepared by an analogous method to example 23:
9α-Fluoro-17α-[(2-furanylcarbonyl)oxy]-11β-hydroxy-16α-methyl-3-oxoandrosta-1,4-diene-17β-carbonylsulphenic acid.
6α,9α-Difluoro-11β-hydroxy-16α-methyl-17α-penatanoyloxy-3-oxoandrosta-1,4-diene-17β-carbonylsulphenic acid.

### Example 24- Compound IC.3

### 6α,9α-Difluoro-11β-hydroxy-16α-methyl-3-oxo-17α-propionyloxyandrosta-1,4-diene-17β-carbonylsulfenic acid fluoromethyl ester

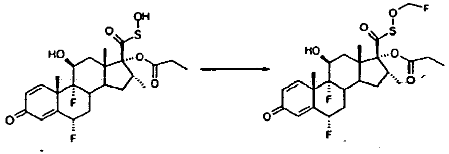

Bromofluoromethane (1.5ml) was added at 0°C in to a stirred mixture of 6α,9α-difluoro-11β-hydroxy-16α-methyl-17α-propionyloxy-3-oxoandrosta-1,4-diene-17β-carbonylsulphenic acid (1.5g, 3.1mmol) and anhydrous potassium carbonate (0.427g, 3.1mmol) in acetone (50ml). The mixture was stirred under a blanket of nitrogen at 5 to 10°C for 1hr. The reaction mixture was then poured into water and extracted with ethyl acetate. The organic layer was washed with water, dried, and concentrated *in vacuo.* The residue was purified by preparative HPLC to yield the title compound as a white solid.

Structure of compound IC.3 was confirmed by single crystal X-Ray as shown below:

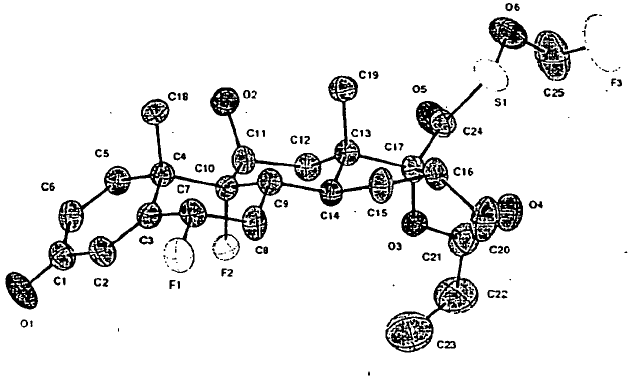

The compounds IC.4, IC.11 to IC.14 were prepared in a similar manner to example 24 using appropriate carbonyl sulfenic acid of formula VI and compound of III-C.

### Example 25- Compound IC.5

### 6α,9α-Difluoro-17α-[(2-furanylcarbonyl)oxy]-11β-hydroxy-16α-methyl-3-oxoandrosta-1,4-diene-17β-carbonylsulfenic acid (4-fluorophenyl)methyl ester

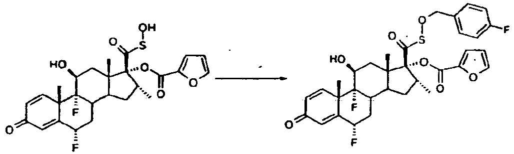

4-Fluorobenzyl chloride (0.277g, 1.91mmol) was added at 25-30°C in to a stirred mixture of 6α,9α-difluoro-17α-[(2-furanylcarbonyl)oxy]-11β-hydroxy-16α-methyl-3-oxoandrosta-1,4-diene-17β-carbonylsulfenic acid (1.0g, 1.91mmol) and anhydrous potassium carbonate (0.344g, 2.49mmol) in *N*,*N*-dimethylformamide (15ml), and the mixture was stirred under a blankei of nitrogen at 25-30°C for 2hrs. The reaction mixture was then poured into water and extracted with ethyl acetate. The organic layer was washed with water, and concentrated *in vacuo.* The residue was purified by crystallization from acetone to yield the title compound as a white solid.
The compounds IC.1, IC.2, IC.6 to IC.9, IC.15, IC.16, IC.18 to IC.23 were prepared in a manner similar to example 25.

### Example 26- Compound IC.10

### 6α,9α-Difluoro-11β-hydroxy-16α-methyl-3-oxo-17α-propionyloxyandrosta-1,4-diene-17β-carbonylsulfenic acid [2-(4-chlorophenyl)-2-oxoethyl] ester

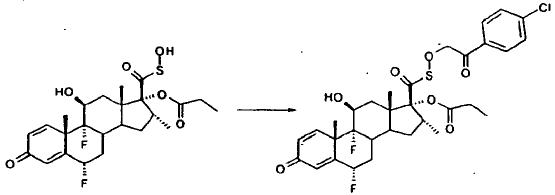

2-Bromo-1-(4-chlorophenyl)ethanone (0.120g, 0.51mmol) was added at 25-30°C in to a stirred mixture of 6α,9α-difluoro-11β-hydroxy-16α-methyl-3-oxo-17α-propionyloxyandrosta-1,4-diene-17β-carbonylsulfenic acid (0.25g, 0.51mmol) and anhydrous potassium carbonate (0.070g, 0.51mmol) in acetone (20ml) and the mixture was stirred under a blanket of nitrogen at 25-30°C for 2hrs. The reaction mixture was then poured into water and extracted with ethyl acetate. The organic layer was washed with water, dried, and concentrated *in vacuo*. The residue was purified by column chromatography (35% ethyl acetate in hexane) to yield the title compound as a white solid.
Compound IC.17 was prepared in a similar manner to example 26.

### Example 27-Compound IB.63

### S-(4-Methoxycarbonyloxy-but-2-ynyl) 6α,9α-difluoro-17α-[(2-furanylcarbonyl)-oxy]-11β-hydroxy-16α-methyl-3-oxoandrosta-1,4-diene-17β-carbothioate

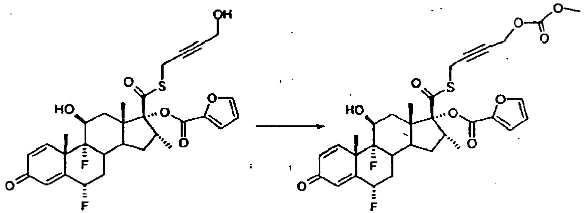

Methyl chloroformate(0.33g, 3.48mmol) was added at 25-30°C in to a stirred mixture of S-(4-hydroxy-but-2-ynyl) 6α,9α-difluoro-17α-[(2-furanylcarbonyl)oxy]-11β-hydroxy-16α-methyl-3-oxoandrosta-1,4-diene-17β-carbothioate (1.0g, 1.74mmol) and triethylamine (0.70g, 6.96mmol) in dry dichloromethane (30ml). The mixture was stirred at 25 to 30°C for 6hrs, then washed with water and concentrated *in vacuo.* The residue was purified by column chromatography (30% ethyl acetate in hexane) to yield the title compound as a white solid.
The compounds IB.12, IB.18, IB.19, IB.64 to IB.66, IB.89, IB.91, IB.123, IB.125 to IB.129, IB.131, IB.134, IB.137 to IB.147, IB.149, IB.150, IB_{.}153, IB.154, IB.156 were prepared in a manner similar to example 27.

### Example 28-Compound IB.103

### S-[4-(N,N-Dimethylaminocarbonyloxy)-but-2-ynyl] 6α,9α-difluoro-17α-[(2-furanyl-carbonyl)oxy]-11β3-hydroxy-16α-methyl-3-oxoandrosta-1,4-diene-17β-carbothioate

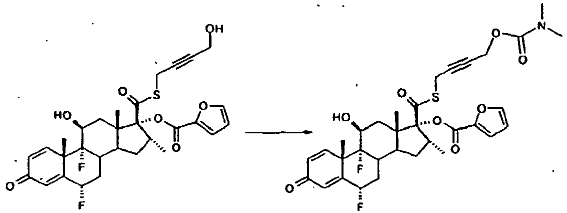

*N,N*-Dimethylcarbamoyl chloride (0.23g, 2.0mmol) was added at 25-30°C to a stirred mixture of *S*-(4-hydroxy-but-2-ynyl) 6α,9α-difluoro-17α-[(2-furanylcarbonyl)oxy]-11β-hydroxy-16α-methyl-3-oxoandrosta-1,4-diene-17β-carbothioate (1.0g, 1.74mmol) and triethylamine (0.70g. 6.96mmol) in dichloromethane (30ml). The mixture was stirred at 25 to 30°C for 4hrs, then washed with water and concentrated *in vacuo*. The residue was purified by column chromatography (40% ethyl acetate in hexane) to yield the title compound as a white solid.
The compounds IB. 129 to IB.131 were prepared in a manner similar to example 28.

### Example 29- Compound IB. 158

### S-[4-Methoxybut-2-ynyl] 6α,9α-difluoro-17α-[(2-furanylcarbonyl)oxy]-11β-hydroxy-16α-methyl-3-oxoandrosta-1,4-diene-17β-carbothioate.

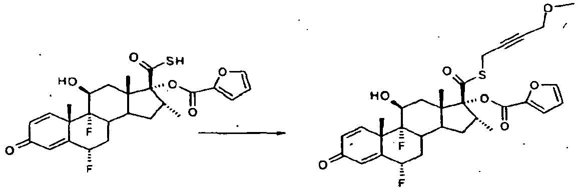

1-Chloro-4-methoxybut-2-yne (0.086g, 0.72mmol) was added to a stirred mixture of 6α,9α-difluoro-17α-[(2-furanylcarbonyl)oxy]-11β-hydroxy-16α-methyl-3-oxoandrosta-1,4-diene-17β-carbothioic acid (0.350g, 0.688mmol) and anhydrous potassium carbonate (0.104g, 0.756mmol) in acetone (20ml). The mixture was stirred under a blanket of nitrogen at 25 to 30°C for 3hrs.. then poured into water and extracted with ethyl acetate. The organic layer was washed with water, dried, and concentrated *in vacuo*. The residue was purified by column chromatography (35% ethyl acetate in hexane) to yield the title compound as a white solid.

Compounds IB. 159, IB.160 were prepared in a manner similar to example 29.

### Table 1- Lists the compound of formula I-A which have been synthesized

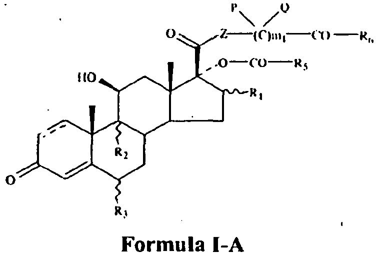

**Table-1**

| **No.** | **R₁** | **R₁** | **R₃** | **R₅** | **R₆** | **P** | **Q** | **m₁** | **Z** | **Chemical Name** |
|---|---|---|---|---|---|---|---|---|---|---|
| IA.1 | H | H | H | Ethoxy | 4-Chlorophenyl | H | H | 1 | O | 2-(4-Chlorophenyl)-2-oxoethyl 17*α*-ethoxycarbonyloxy-11*β-*hydroxy-3-oxoandrosta-1,4-diene-17*β*-carboxylate. |
| IA.2 | *α-*CH₃ | F | F | Ethyl | 4-Chlorophenyl | H | H | 1 | O | 2-(4-Chlorophenyl)-2-oxoethyl 6*α*,9*α*-difluoro-11*β*-hydroxy-16*α-*methyl-3-oxo-17*α*-pronionyloxyandrosta-1,4-diene-17*β*-carboxylate |
| IA-3 | *α-*CH₃ | F | F | Ethyl | 4-Chlorophenyl | H | H | 1 | S | *S*-[2-(4-Chlorophenyl)-2-oxoethyl]6*α*,9*α*-difluoro-11*β*-hydroxy-16*α*-methyl-3-oxo-17*α*-propionyloxyandrosta-1,4-diene-17*β-*carbothioate. |
| IA.4 | H | H | H | Ethoxy | 4-Fluorophenyl | H | H | 1 | O | 2-(4-Fluorophenyl)-2-oxoethyl 17α-ethoxycarbonyloxy-11*β-*hydroxy-3-oxoandrosta-1,4-diene-17*β*-carboxylate. |
| IA.5 | *α-*CH₃ | F | F | Ethyl | 4-Fluorophenyl | H | H | 1 | O | 2-(4-Fluorophenyl)-2-oxoethyl 6*α*,9*α*-difluoro-11*β*-hydroxy-16*α-*methyl-3-oxo-17*α*-propionyloxyandrosta-1,4-diene-17*β-*carboxylate. |
| IA.6 | *α-*CH₃ | F | F | Ethyl | 4-Fluorophenyl | H | H | 1 | S | *S*-[2-(4-Fluorophenyl)-2-oxoethyl] 6*α*,9*α*-difluoro-11*β*-hydroxy-16α*-*methyl-3-oxo-17*α*-propionyloxyandrosta-1,4-diene-17*β-*carbothioate. |
| IA.7 | H | H | H | Ethoxy | 2,4-Difluorophenyl | H | H | 1 | O | 2-(2,4-Difluorophenyl)-2-oxoethyl 17*α*-(ethoxycarbonyloxy-11*β-*hydrory-3-oxoandrosta-1,4-diene-17*β*- carboxylate. |
| IA.8 | H | H | H | Ethoxy | 2,4-Dichlorophenyl | H | H | 1 | O | 2-(2,4-Dichlorophenyl)-2-oxoethyl 17*α*-(ethoxycarbonyl)oxy-11*β-*hydroxy-3-oxoandrosta-1,4-diene-17*β*-carboxylate. |
| IA.9 | *α-*CH₃ | F | F | Ethyl | 2,4-Difluorophenyl | H | H | 1 | O | 2-(2,4-Difluorophenyl)-2-oxoethyl 6*α*,9*α*-difluoro-1*β*-hydroxy-16*α*-methyl-3-oxo-17*α*-propionyloxandrosta-1,4 diene-17*β-*carboxylate. |
| IA.10 | *α-*CH₃ | F | F | Ethyl | 2,4-Dichloro phenyl | H | H | 1 | O | 2-(2,4-Dichlorophenyl)-2-oxoethyl 6*α*,9*α*-difluoro-11*β*-hydroxy-16*α*-methyl-3-oxo-17*α*-propionyloxyandrosta-1,4-diene-17*β-*carboxylate. |
| IA.11 | *α-*CH₃ | F | F | Ethyl | 2,4-Difluorophenyl | H | H | 1 | S | *S*-2-[(2,4-Difluorophenyl)-2-oxoethyl] 6*α*,9*α*-difluoro-11*β-*hydroxy-16*α*-methyl-3-oxo-17*α*-propionyloxyandrosta-1,4-diene-17*β*-carbothioate. |
| IA.12 | H | H | H | Ethoxy | 3,4-Dichlorophenyl | H | H | 1 | O | 2-(3,4-Dichlorophenyl)-2-oxoethyl 17*α*-ethoxycarbonyloxy-11*β-*hydroxy-3-oxoandrosta-1,4-diene-17*β*-carboxylate. |
| IA.13 | *α-*CH₃ | F | F | Ethyl | 2,4-Dichlorophenyl | H | H | 1 | S | *S*-[2-(2,4-Dichlorophenyl)-2-oxoethyl] 6*α*,9*α*-difluoro-11*β-*hydroxy-16*α*-methyl-3-oxo-17*α*-propionyloxyandrosta-1,4-diene-17*β*-carbothioate. |
| IA.14 | *α-*CH₃ | F | F | Ethyl | 3,4-Dichlorophenyl | H | H | 1 | S | *S*-[2-(3,4-Dichlorophenyl)-2-oxoethyl] 6*α*,9*α*-difluoro-11*β-*hydroxy-16*α*-methyl-3-oxo-17*α*-propionyloxyandrosta-1,4diene-17*β*-carbothioate. |
| IA.15 | *α-*CH₃ | F | F | Ethyl | 3,4-Dichloro-phenyl | H | H | 1 | O | 2-(3,4-Dichlorophenyl)-2-oxoethyl 6*α*,9*α*-difluoro-11*β*-hydroxy-16*α*-methyl-3-oxo-17*α-*propionyloxyandrosta-1,4 diene-17*β*-carboxylate. |
| IA.16 | H | H | H | Ethoxy | 2-Thienyl | H | H | 1 | O | 2-Oxo-2-(thiophen-2-yl)ethyl 17*α-*(ethoxycarbonyl)oxy-11*β*-hydroxy-3-oxoandrosta-1,4-diene-17*β*-carboxylate. |
| IA.17 | H | H | H | Ethoxy | 5-Chloro-2-thienyl | H | H | 1 | O | 2-(5-Chlorothiophen-2-yl)-2-oxoethyl 17α-(ethoxycarbonyl)oxy-11β-hydroxy-3-oxoandrosta-1,4-diene-17β-carboxylate. |
| IA.18 | *α-*CH₃ | F | F | Ethyl | 5-Chloro-2-thienyl | H | H | 1 | O | 2-(5-Chlorothiophen-2-yl)-2-oxoethyl 6*α*, 9*α*-difluoro-11*β*-hydroxy-16*α*-methyl-3-oxo-17*α-*propionyloxyandrosta-1,4-diene-17*β*-carboxylate. |
| IA.19 | H | H | H | Ethoxy | 5-Methyl-2-furyl | H | H | 1 | O | 2-(5-Methylfuran-2-yl)-2-oxoethyl 17*α-*(ethoxycarbonyl)oxy-11*β*-hydroxy-3-oxoandrosta-1,4-diene-17*β*-carboxylate. |
| IA.20 | *α-*CH₃ | F | F | Ethyl | 5-Chloro-2-thienyl | H | H | 1 | S | *S*-[2-(5-Chlorothiophen-2-yl)-2-oxoethyl] 6*α*,9*α-*difluoro-11*β*-hydroxy-16*α*-methyl-3-oxo-17*α-*propionyloxyandrosta-1,4-diene-17*β*-carbothiate. |
| IA.21 | *α-*CH₃ | F | F | Ethyl | 5-Methyl-2-furyl | H | H | 1 | O | 2-(5-Methylfuran-2-yl)-2-oxoethyl 6*α*,9*α*-difluoro-11*β-*hydroxy-16*α*-methyl-3-oxo-17*α*-propionyloxyandrosta-1,4-diene-17*β*-carboxylate. |
| IA.22 | *α-*CH₃ | F | F | Ethyl | 5-Methyl-2-furyl | H | H | 1 | S | *S*-[2-(5-Methylfuran-2-yl)-2-oxoethyl] 6*α*,9*α*-difluoro-11*β*-hydroxy-16*α*-methyl-3-oxo-17*α-*propionyloxyandrosta-1,4-diene-17*β*-carbothioate. |
| IA.23 | H | H | H | Ethoxy | *t*-Butyl | H | H | 1 | O | 3,3-Dimethyl-2-oxobutyl 17*α*-(ethoxycarbonyl)oxy-11*β*-hydroxy-3-oxoandrosta-1,4-diene-17*β*-carboxylate. |
| IA.24 | H | H | H | Ethoxy | Methyl | H | H | 1 | O | 2-Oxopropyl 17*α*-(ethoxycarbonyl)oxy-11*β*-hydroxy-3-oxoandrosta-1,4-diene-17*β*-carboxylate. |
| IA.25 | *α-*CH₃ | F | F | Ethyl | *t*-Butyl | H | H | 1 | O | 3,3-Dimethyl-2-oxobutyl 6*α*,9*α*-difluoro-11*β*-hydroxy-16*α*-methyl-3-oxo-17*α*-propionyloxyandrosta-1,4-diene-17*β*-carboxylate. |
| IA.26 | *α-*CH₃ | F | F | Ethyl | *t*-Butyl | H | H | 1 | S | *S*-(3,3-Dimethyl-2-oxobutyl) 6*α*,9*α*-difluoro-11*β-*hydroxy-16*α*-methyl-3-oxo-17*α*-propionyloxyandrosta-1,4-diene-17*β*-carbothioate. |
| IA.27 | *α-*CH₃ | F | F | Ethyl | Methyl | H | H | 1 | S | *S*-[2-Oxopropyl] 6*α*,9*α*-difluoro-11*β*-hydroxy-16*α-*methyl-3-oxo-17*α*-propionyloxyandrosta-1,4-diene-17*β*-carbothioate. |
| IA.28 | *α-*CH₃ | F | F | Ethyl | Methyl | H | H | 1 | O | 2-Oxopropyl 6*α*,9*α*-difluoro-11*β*-hydroxy-16*α*-methyl-3-oxo-17*α*-propionyloxyandrosta-1,4-dien-17*β-*carboxylate. |
| IA.29 | H | H | H | Ethoxy | 4-Methoxy-phenyl | H | H | 1 | O | 2-(4-Methoxyphenyl)-2-oxoethyl 17*α-*(ethoxycarbonyl)oxy-11*β*-hydroxy-3-oxyandrosta-1,4-dien-17*β*-carboxylate. |
| IA.30 | *α-*CH₃ | F | F | Ethyl | 4-Methoxy-phenyl | H | H | 1 | O | 2-(4-Methoxyphenyl)-2-oxoethyl 6*α*,9*α*-difluoro-11*β-*hydroxy-16*α*-methyl-3-oxo-17*α*-propionyloxyandrosta-1,4-diene-17*β*-carboxylate. |
| IA.31 | *α-*CH₃ | F | F | Ethyl | 4-Methoxy-phenyl | H | H | 1 | S | *S*-[2-(4-Methoxyphenyl)-2-oxoethyl] 6*α*,9*α*-difluoro-11*β*-hydroxy-16*α*-methyl-3-oxo-17*α-*propionyloxyandrosta-1,4-diene-17*β*-carbothioate. |
| IA.32 | H | H | H | Ethoxy | 1-Adamantyl | H | H | 1 | O | 2-(Adamantan-1-yl)-2-oxoethyl 17*α-*(ethoxycarbonyl)oxy-11*β*-hydroxy-3-oxoandrosta-1,4-diene-17*β*-carboxylate. |
| IA.33 | *α-*CH₃ | F | F | Ethyl | 1-Adamantyl | H | H | 1 | O | 2-(Adamantan-1-yl)-2-oxoethyl 6*α*,9*α*-difluoro-11*β-*hydroxy-16*α*-methyl-3-oxo-17*α*-propionyloxyandrosta-1,4-diene-17*β*-carboxylate. |
| IA.34 | *α-*CH₃ | F | F | Methyl | 1-Adamantyl | H | H | 1 | S | *S*-[2-(Adamantan-1-yl)-2-oxoethyl] 6*α*,9*α*-difluoro-11*β*-hydroxy-16*α*-methyl-3-oxo-17*α-*propionyloxyandrosta-1,4-diene-17*β*-carbothioate. |
| IA.35 | *α-*CH₃ | F | F | Ethyl | 4-Trifluoro methyloxyphen yl | H | H | 1 | S | *S*-[2*-*Oxo-2*-*(4-trifluoromethyloxyphenyl)ethyl] 6*α*,9*α-*difluoro-11*β*-hydroxy-16*α*-methyl-3-oxo-17*α-*propionyloxyandrosta-1,4-diene-17*β*-carbothiate. |
| IA.36 | *α-*CH₃ | r | F | Ethyl | 4-Trifluoro-methyloxy-phenyl | H | H | 1 | O | 2-Oxo-2-(4-trifluoromethyloxyphenyl)ethyl 6*α*,9*α-*difluoro-11*β*-hydroxy-16*α*-methyl-3-oxo-17*α-*propionyloxyandrosta-1,4-diene-17*β*-carboxylate. |
| IA.37 | H | H | H | Ethoxy | 4-Trifluoro-methyloxy-phenyl | H | H | 1 | O | 2-Oxo-2-(4-trifluoromethyloxyphenyl)ethyl 17*α-*(ethoxycarbonyl)oxy-11*β*-hydroxy-3-oxoandrosta-1,4-diene-17*β*-carboxylate. |
| IA.38 | *α-*CH₃ | F | F | Ethyl | 4-Trifluoro methylphenyl | H | H | 1 | S | *S*-[2-Oxo-2-(4-trifluoromethylphenyl)ethyl] 6*α*,9*α-*difluoro-11*β*-hydroxy-16*α*-methyl-3-oxo-17*α-*propionyloxyandrosta-1,4-diene-17*β*-carbothioate. |
| IA.39 | *α-*CH₃ | F | F | 5-Chloro-2-thienyl | 4-Trifluoromethylphenyl | H | H | 1 | S | *S*-[2-Oxo-2-(4-trifluoromethylphenyl)ethyl] 17*α*-[[(5-chloro2-thienyl)carbonyl]oxy]-6*α*,9*α*-difluoro-11*β-*hydroxy-16*α*-methyl-3-oxoandrosta-1,4-diene-17*β-*carbothioate. |
| IA.40 | *α-*CH₃ | F | F | 5-Chloro-2-thienyl | 2,4,6-Trimethylphenyl | H | H | 1 | S | *S*-[2-Oxo-2-(2,4,6-trimethylphenyl)ethyl] 17*α*-[[(5-chloro2-thienyl)carbonyl]oxy]-6*α*,9*α*-difluoro-11*β-*hydroxy-16*α*-methyl-3-oxoandrosta-14-diene17*β-*carbothioate. |
| IA.41 | *α-*CH₃ | Cl | H | 2-Furyl | 2,4,6-Trimethylphenyl | H | H | 1 | S | *S*-[2-Oxo-2-(2,4,6-trimethylphenyl)ethyl] 9*α*-chloro-17*α*-[(2-furanylcarbonyl)oxy]-11*β*-hydroxy-16*α-*methyl-3-oxoandrosta-1,4-diene-17*β*-carbothioate. |
| IA.42 | *α-*CH₃ | F | F | Ethyl | 4-Methane-sulfonyl-phenyl | H | H | 1 | O | 2-(4-Methanesulphonylphenyl)-2-oxoethyl 6*α*,9*α-*difluoro-11*β*-hydroxy-16*α*-methyl-3-oxo-17*α-*propionyloxyandrosta-1,4-diene-17*β*-carboxylate. |
| IA.43 | *α-*CH₃ | F | F | Ethyl | 4-Methane sulfonyl-phenyl | H | H | 1 | S | *S*-[2-(4-Methanesulphonylphenyl)-2-oxoethyl] 6*α*,9*α-*difluoro-11*β*-hydroxy-16*α*-methyl-3-oxo-17*α-*propionyloxyandrosta-1,4-diene-17*β*-carbothioate. |
| IA.44 | *α-*CH₃ | F | F | Ethyl | Phenyl | H | H | 1 | O | 2-Oxo-2-phenylethyl 6*α*,9*α*-difluoro-11*β*-hydroxy-16*α*-methyl-3-oxo-17*α*-propionyloxyandrosta-1,4-diene-17*β*-carboxylate. |
| IA.45 | *α-*CH₃ | F | F | Ethyl | Phenyl | H | H | 1 | S | *S*-[2-Oxo-2-phenylethyl] 6*α*,9*α*-difluoro-11*β*-hydroxy-16*α*-methyl-3-oxo-17*α*-propionyloxyandrosta-1,4-diene-17*β*-carbothioate. |
| IA.46 | *α-*CH₃ | F | F | Ethyl | 4-Methylphenyl | H | H | 1 | O | 2-(4-Methylphenyl)-2-oxoethyl 6α,9*α*-difluoro-11*β-*hydroxy-16*α*-methyl-3-oxo-17*α*-propionyloxyandrosta-1,4-diene-17*β*-carboxylate. |
| IA.47 | *α* CH₃ | F | F | Ethyl | 4-Methylphenyl | H | H | 1 | S | *S*-[2-(4-Methylphenyl)-2-oxoethyl] 6*α*,9*α*-difluoro-11*β*-hydroxy-16*α*-methyl-3-oxo-17*α-*propionyloxyandrosta-1,4-diene-17*β*-carbothioate. |
| IA.48 | *α-*CH₃ | F | F | Ethyl | 2,6-Dichlorophenyl | H | H | 1 | O | 2-(2,6-Dichlorophenyl)-2-oxoethyl 6*α*,9*α*-difluoro-11*β*-hydroxy-16*α*-methyl-3-oxo-17*α-*propionyloxyandrosta-1,4-diene-17*β*-carboxylate. |
| IA.49 | *α-*CH₃ | F | F | Ethyl | 2,6-Dichlorophenyl | H | H | 1 | S | *S*-[2-(2,6-Dichlorophenyl)-2-oxoethyl] 6*α*,9*α*-difluoro-11*β*-hydroxy-16*α*-methyl-3-oxo-17*α-*propionyloxyandrosta-1,4-diene-17*β*-carbothioate. |
| IA.50 | *α-*CH₃ | F | F | Ethyl | 2,3-Dichlorophenyl | H | H | 1 | O | 2-(2,3-Dichlorophenyl)-2-oxoethyl 6*α*,9*α*-difluoro-11*β*-hydroxy-16*α*-methyl-3-oxo-17α-propionyloxyandrosta-1,4-diene-17*β*-carboxylate. |
| IA.51 | *α-*CH₃ | F | F | Ethyl | 2,3-Dichlorophenyl | H | H | 1 | S | *S*-[2-(2,3-Dichlorophenyl)-2-oxoethyl] 6*α*,9*α*-difluoro-11*β*-hydroxy-16*α*-methyl-3-oxo-17*α-*propionyloxyandrosta-1,4-diene-17*β*-carbothioate. |
| IA.52 | *α-*CH₃ | F | F | Ethyl | 2,6-Difluorophenyl | H | H | 1 | O | 2-(2,6-Difluorophenyl)-2-oxoethyl 6*α*,9*α*-difluoro-11*β-*hydroxy-16*α*-methyl-3-oxo-17*α*-propionyloxyandrosta-1,4-diene-17*β*-carboxylate. |
| IA.53 | *α-*CH₃ | F | F | Ethyl | 2,6-Difluorophenyl | H | H | 1 | S | *S*-[2-(2-6-Difluorophenyl)-2-oxoethyl] 6*α*,9*α*-difluoro-11*β*-hydroxy-16*α*-methyl-3-oxo-17*α-*propionyloxyandrosta 1,4-diene-17*β*-carbothioate. |
| IA.54 | *α-*CH₃ | F | F | Ethyl | 2,3-Difluorophenyl | H | H | 1 | O | 2-(2,3-Difluorophenyl)-2-oxoethyl 6*α*,9*α*-difluoro-11*β-*hydroxy-16*α*-methyl-3-oxo-17*α*-propionyloxyandrosta-1,4-diene-17*β*-carboxylate. |
| IA.55 | *α-*CH₃ | F | F | Ethyl | 2,3-Difluorophenyl | H | H | 1 | S | *S*-[2-(2,3-Difluorophenyl)-2-oxoethyl] 6*α*,9*α*-difluoro-11β-hydroxy-16α-methyl-3-oxo-17α-propionyloxyandrosta-1,4-diene-17β-carbothioate. |
| IA.56 | *α-*CH₃ | F | F | 5-Chloro-2-thienyl | 4-Chlorophenyl | H | H | 1 | S | *S-*[2-(4-Chlorophenyl)-2-oxoethyl] 17*α*-[[(5-chloro-2-thienyl)carbonyl]oxy]-6*α*,9*α*-difluoro-11*β-*hydroxy-16*α*-methyl-3-oxoandrosta-1,4-diene-17*β-*carbothioate. |
| IA.57 | *α-*CH₃ | F | F | Ethyl | 2,4,6-Trimethylphenyl | H | H | 1 | O | 2-Oxo-2-(2,4,6-trimethylphenyl)ethyl 6*α*,9*α-*difluoro-11*β*-hydroxy-16*α*-methyl-3-oxo-17*α-*propionyloxyandrosta-1,4-diene-17*β*-carboxylate. |
| IA.58 | *α-*CH₃ | F | F | Ethyl | 2,4,6-Trimethylphenyl | H | H | 1 | S | *S*-[2-Oxo-2-(2,4,6-trimethylphenyl)ethyl] 6*α*,9*α-*difluoro-11*β*-hydroxy-16*α*-methyl-3-oxo-17*α-*propionyloxyandrosta-1,4-diene-17*β*-carbothioate. |
| IA.59 | *α-*CH₃ | F | F | Ethyl | Phenyl | CH₃ | CH₃ | 1 | S | *S*-[1,1-Dimethyl-2-oxo-2-phenylethyl] 6*α*,9*α-*difluoro-11*β*-hydroxy-16*α*-methyl-3-oxo-17*α-*propionyloxyandrosta-1,4-diene-17*β*-carbothiate. |
| IA.60 | *α-*CH₃ | F | F | Ethyl | 2-Thienyl | H | H | 1 | O | 2-Oxo-2-thiophen-2-yl)ethyl 6*α*,9*α*-difluoro-11*β-*hydroxy-16*α*-methyl-3-oxo-17*α*-propionyloxyandrosta-1,4-diene-17*β*-carboxylate. |
| IA.61 | *α-*CH₃ | F | F | Ethyl | 2-Thienyl | H | H | 1 | S | *S*-[2-Oxo-2-(thiophen-2-yl)ethyl] 6*α*,9*α*-difluoro-11*β*-hydroxy-16*α*-methyl-3-oxo-17*α-*propionyloxyandrosta-1,4-diene-17*β*-carbothioate. |
| IA.62 | *α-*CH₃ | F | F | Ethyl | 3,4-Difluorophenyl | H | H | 1 | O | 2-(3,4-Difluorophenyl)-2-oxoethyl 6*α*,9*α*-difluoro-11*β*-hydroxy-16*α*-methyl-3-oxo-17*α-*propionyloxyandrosta-1,4-diene-17*β*-carboxylate. |
| IA.63 | *α-*CH₃ | F | F | Ethyl | 3,4-Difluorophenyl | H | H | 1 | S | *S*-[2-(3,4-Difluorophenyl)-2-oxoethyl] 6*α*,9*α-*difluoro-11*β*-hydroxy-16*α*-methyl-3-oxo-17*α-*propionyloxyandrosta-1,4-diene-17*β*-carbothioate. |
| IA.64 | *α-*CH₃ | F | F | Ethyl | 2-Furyl | H | H | 1 | O | 2-(Furan-2-yl)-2-oxoethyl 6*α*,9*α*-difluoro-11*β-*hydroxy-16*α*-methyl-3-oxo-17*α*-propionyloxyandrosta-1,4-diene-17*β*-carboxylate. |
| IA.65 | *α-*CH₃ | F | F | Dichloromethyl | 4-Fluorophenyl | H | H | 1 | O | 2-(4-Fluorophenyl)-2-oxoethyl 17*α*-dichloromethyl-carbonyloxy-6*α*,9*α*-difluoro-11*β*-hydroxy-16*α-*methyl-3-oxoandrosta-1,4-diene-17*β*-carboxylate. |
| IA.66 | *α-*CH₃ | F | F | Dichloromethyl | 2,4-Difluorophenyl | H | H | 1 | O | 2-(2,4-Difluorophenyl)-2-oxoethyl 17*α-*dichloromethylcarbonyloxy-6*α*,9*α*-difluoro-11*β-*hydroxy-16*α*-methyl-3-oxoandrosta-1,4-diene-17*β-*carboxylate. |
| IA.67 | *α-*CH₃ | F | F | Ethyl | 2-Benzofuryl | H | H | 1 | O | 2-(Benzofuran-2-yl)-2-oxoethyl 6*α*,9*α*-difluoro-11*β-*hydroxy-16*α*-methyl-3-oxo-17*α-*propionyloxyandrosta-1,4-diene-17*β*-carboxylate. |
| IA.68 | *α-*CH₃ | F | F | Ethyl | 2-Furyl | H | H | 1 | S | *S*-[2-(Furan-2-yl)-2-oxoethyl] 6*α*,9*α*-difluoro-11*β-*hydroxy-16*α*-methyl-3-oxo-17*α-*propionyloxyandrosta-1,4-diene-17*β*-carbothioate. |
| IA.69 | *α-*CH₃ | F | F | Ethyl | 2-Benzofuryl | H | H | 1 | S | *S*-[2-(Benzofuran-2-yl)-2-oxoethyl] 6*α*,9*α*-difluoro-11*β*-hydroxy-16*α*-methyl-3-oxo-17*α-*propionyloxyandrosta-1,4-diene-17*β*-carbothioate. |
| IA.70 | *α-*CH₃ | F | F | Ethyl | 4-*t*-Butylphenyl | H | H | 1 | S | *S*-[2-(4-*t*-Butylphenyl)-2-oxoethyl] 6*α*,9*α*-difluoro-11*β*-hydroxy-16*α*-methyl-3-oxo-17*α-*propionyloxyandrosta-1,4-diene-17*β*-carbothioate. |
| IA.71 | *α-*CH₃ | F | F | Ethyl | 7-Methoxy-2-benzofuryl | H | H | 1 | O | 2-(7-Methoxybenzofuran-2-yl)-2-oxoethyl 6*α*,9*α-*difluoro-11*β*-hydroxy-16*α*-methyl-3-oxo-17*α-*propionyloxyandrosta-1,4-diene-17*β*-carboxylate. |
| IA.72 | *α-*CH₃ | F | F | Ethyl | 4-*t*-Butylphenyl | H | H | 1 | O | 2-(4-t-Butylphenyl)-2-oxoethyl 6*α*,9*α*-difluoro-11*β-*hydroxy-16*α*-methyl-3-oxo-17*α-*propionyloxyandrosta-1,4-diene-17*β*-carboxylate. |
| IA.73 | *α-*CH₃ | F | F | Ethyl | 7-Methoxy-2-benzofuryl | H | H | 1 | S | *S*-[2-(7-Methoxybenzofuran-2-yl)-2-oxoethyl] 6*α*,9*α*-difluoro-11*β*-hydroxy-16*α*-methyl-3-oxo-17*α*-propionyloxyandrosta-1,4-diene-17*β-*carbothioate. |
| IA74 | *α-*CH₃ | F | F | Ethyl | 2-Fluoro-4-methoxyphenyl | H | H | 1 | O | 2-(2-Fluoro-4-methoxyphenyl)-2-oxoethyl 6*α*,9*α-*difluoro-11*β*-hydroxy-16*α*-methyl-3-oxo-17*α-*propionyloxy-androsta-1,4-diene-17*β*-carboxylate. |
| IA.75 | *α-*CH₃ | F | F | Ethyl | 2-Fluoro-4-methoxyphenyl | H | H | 1 | S | S-[2-(2-Fluoro-4-methoxyphenyl)-2-oxoethyl] (6*α*.9*α*-difluoro-11*β*-hydroxy-16*α*-methyl-3-oxo-17*α*-propionyloxyandrosta-1,4-diene-17*β-*carbothioate. |
| IA.76 | *α-*CH₃ | F | F | Ethyl | 2-Methoxyphenyl | H | H | 1 | O | 2-(2-Methoxyphenyl)-2-oxoethyl 6*α*,9*α*-difluoro-11*β*-hydroxy-16*α*-methyl-3-oxo-17*α*-propionyloxyandrosta-1,4-diene-17*β*-carboxylate. |
| IA.77 | *α-*CH₃ | F | F | Ethyl | 2,4-Dimethoxyphenyl | H | H | 1 | O | 2-(2,4-Dimethoxyphenyl)-2-)oxoethyl 6*α*,9*α-*difluoro-11*β*-hydroxy-16*α*-methyl-3-oxo-17α-propionyloxyandrosta-1,4-diene-17*β*-carboxylate. |
| IA.78 | *α-*CH₃ | F | F | Ethyl | 2,4-Dimethoxyphenyl | H | H | 1 | S | *S-*[2-(2,4-Dimethoxyphenyl)-2-oxoethyl]6*α*,9*α-*difluoro-11*β*-hydroxy-16*α*-methyl-3-oxo-17*α-*propionyloxyandrosta-1,4-diene-17*β*-carbothioate. |
| IA.79 | *α-*CH₃ | F | F | Ethyl | 2-Methoxyphenyl | H | H | 1 | S | *S*-[2-(2-Methoxyphenyl)-2-oxoethyl) 6*α*,9*α-*difluoro-11*β*-hydroxy-16*a*-methyl-3-oxo-17*α-*propionyloxyandrosta-1,4-diene-17*β*-carbothioate. |
| IA.80 | *α-*CH₃ | F | F | Ethyl | 2-Tritluoromethylphenyl | H | H | 1 | O | 2-Oxy-2-(2-trifluoromethylphenyl)ethyl 6*α*,9*α-*difluoro-11*β*-hydroxy-16*α*-methyl-3-oxo-17*α-*propionyloxy-androsta-1,4-diene-17*β*-carboxylate. |
| IA.81 | *α-*CH₃ | F | F | Ethyl | 2-Trifluoromethylphenyl | H | H | 1 | S | *S*-[2-Oxy-2-(2-trifluoromethylphenyl)ethyl] 6*α*,9*α-*difluoro-11*β*-hydroxy-16*α*-methyl-3-oxo-17*α-*propionyloxyandrosta-1,4-diene-17*β*-carbothioate. |
| IA.82 | *α-*CH₃ | F | F | 2-Furyl | 4-Fluorophenyl | H | H | 1 | O | 2-(4-Fluorophenyl)-2-oxoethyl]6*α*,9*α*-difluoro-17*α-*[(2-furanylcarbonyl)oxy]-11*β*-hydroxy-16*α*-methyl-3-oxoandrosta-1,4-diene-17*β*-carbothioate. |
| IA.83 | *α-*CH₃ | F | F | 2-Furyl | 4-Fluorophenyl | H | H | 1 | S | *S*-[2-(4-Fluorophenyl)-2-oxoethyl]6*α*,9*α*-difluoro-17*α*-[(2-furanylcarbonyl)oxy]-11*β*-hydroxy-16*α-*methyl-3-oxoandrosta-1,4-diene-17*β*-carbothioate. |
| IA.84 | *α-*CH₃ | F | F | 2-Furyl | 2,4-Difluorophenyl | H | H | 1 | O | 2-(2,4-Difluorophenyl)-2-oxoethyl 6*α*,9*α*-difluoro-17*α*-[(2-furunylcarbonyl)oxy]-11*β*-hydroxy-16*α-*methyl-3-oxo-androsta-1,4-diene-17*β*-carboxylate. |
| IA.85 | *α-*CH₃ | F | F | 2-Furyl | 2,4-Difluorophenyl | H | H | 1 | S | *S*-[2-(2,4-Difluorophenyl)-2-oxoethyl]6*α*,9*α-*difluoro-17*α*-[(2-furanylcarbonyl)oxy]-11*β*-hydroxy-16*α*-methyl-3-oxoandrosta-1,4-diene-17*β-*carbothioate. |
| IA.86 | *α-*CH₃ | F | H | Ethyl | 4-Fluorophenyl | H | H | 1 | S | *S*-[2-(4-Fluorophenyl)-2-oxoethyl]9*α*-fluoro-11*β-*hydroxy-16*α*-methyl-3-oxo-17*α*-propionyloxy-androsta-1,4-diene-17*β*-carbothioate |
| IA.87 | *α-*CH₃ | F | H | Ethyl | 4-Fluorophenyl | H | H | 1 | O | 2-(4-Fluorophenyl)-2-oxoethyl 9*α*-fluoro-11*β-*hydroxy-16*α*-methyl-3-oxo-17*α*-propionyloxy-androsta-1,4-diene-17*β*-carboxylate. |
| IA.88 | *α-*CH₃ | F | F | Ethyl | 3,5-Bis(trifluoromethyl)phenyl | H | H | 1 | S | *S*-[2-[3,5-Bis(trifluoromethyl)phenyl]-2-oxoethyl] 6*α*,9*α*-difluoro-11*β*-hydroxy-16*α*-methyl-3-oxo-17*α*-propionyloxyandrosta-1,4-diene-17*β-*carbothioate. |
| IA.89 | *α-*CH₃ | F | F | Ethyl | 3-Trifluoromethyl phenyl | H | H | 1 | S | *S*-[2-Oxo-2-(3-trifluomethyl)phenylethyl]6*α*,9*α-*difluoro-11*β*-hydroxy-16*α*-methyl-3-oxo-17*α-*propionyloxyandrosta-1,4-diene-17*β*-carbothioate. |
| IA.90 | *α-*CH₃ | F | F | Ethyl | 4-Isobutylphenyl | H | H | 1 | S | *S-*[2-(4-Isobutylphenyl)-2-oxoethyl]6*α*,9*α*-difluoro-11*β*-hydroxy-16*α*-methyl-3-oxo-17*α*-propionyloxy-androsta-1,4-diene-17*β*-carbothioate. |
| IA.91 | *β-*CH₃ | F | H | Ethyl | 4-Fluorophenyl | H | H | 1 | O | 2-(4-Fluorophenyl)-2-oxoethyl 9*α*-fluoro-11*β-*hydroxy-16*β*-methyl-3-oxo-17*α*-propionyloxy-androsta-1,4-diene-17*β*-carboxylate. |
| IA.92 | *α-*CH₃ | F | F | Ethyl | 4-Ethyl-phenyl | H | H | 1 | S | *S*-[2-(4-Ethylphenyl)-2-oxoethyl]6*α*,9*α*-difluoro-11*β*-hydroxy-16*α*-methyl-3-oxo-17*α*-propionyloxy-androsta-1,4-diene-17*β*-carbothiate. |
| IA.93 | α-CH₃ | F | F | Ethyl | 4-Cyclohexylphenyl | H | H | 1 | S | *S*-[2-(4-Cyclohexylphenyl)-2-oxoethyl]6α,9α-difluoro-11β-hydroxy-16α-methyl-3-oxo-17α-propionyloxyandrosta-1,4-diene-17β-carbothioate. |
| IA.94 | β-CH₃ | F | H | Ethyl | 2,4-Difluorophenyl | H | H | 1 | S | *S*-[2-{2,4-Difluorophenyl)-2-oxoethyl 9α-fluoro-11β-hydroxy-16β-methyl-3-oxo-17α-propionyloxy-androsta-1,4-diene-17β-carbothioate. |
| IA.95 | *α-*CH₃ | F | F | Ethyl | 4-Isopropylphenyl | H | H | 1 | S | *S*-[2-(4-Isopropylphenyl)-2-oxoethyl]6*α*,9*α-*difluoro-11*β*-hydroxy-16*α*-methyl-3-oxo-17*α-*propionyloxyandrosta-1,4-diene-17*β*-carbothioate. |
| IA.96 | *α-*CH₃ | F | F | Ethyl | 4-Cyclopentyloxyphenyl | H | H | 1 | S | *S*-[2-(4-Cyclopentyloxyphenyl)-2-oxoethyl]6*α*,9*α-*difluoro-11*β*-hydroxy-16*α*-methyl-3-oxo-17*α-*propionyloxyandrosta-1,4-diene-17*β*-carbothioate |
| IA.97 | *α-*CH₃ | F | H | Ethyl | 4-Methylsulfonylphenyl | H | H | 1 | S | *S*-[2-(4-Methylsolfonylphenyl)-2-oxoethyl] 9*α-*fluoro-11*β*-hydroxy-16*α*-methyl-3-oxo-17*α-*propionyloxyandrosta-1,4-diene-17*β*-carbothioate. |
| IA.98 | *α-*CH₃ | F | H | Ethyl | 2,4-Difluorophenyl | H | H | 1 | S | *S*-[2-(2,4-Difluorophenyl)-2-oxoethyl] 9*α*-fluoro-11*β*-hydroxy-16*α*-methyl-3-oxo-17*α*-propionyloxy-androsta-1,4-diene-17*β*-carbothioate. |
| IA.99 | *β-*CH₃ | F | H | Ethyl | 4-Methylsulfonylphenyl | H | H | 1 | S | *S*-[2-(4-Methylsolfonylphenyl)-2-oxoethyl] 9*α-*fluoro-11*β*-hydroxy-16*β*-methyl-3-oxo-17*α-*propionyloxyandrosta-1,4-diene-17*β*-carbothioate. |
| IA.100 | *α-*CH₃ | F | H | Ethyl | 4-Chlorophenyl | H | H | 1 | S | *S*-[2-(4-Chlorophenyl)-2-oxoethyl] 9*α*-fluoro-11*β-*hydroxy-16*α*-methyl-3-oxo-17*α-*propionyloxyandrosta-1,4-diene-17*β*-carbothioate. |
| IA.101 | *β-*CH₃ | F | H | Ethyl | 4-Chlorophenyl | H | H | 1 | S | *S*-[2-(4-Chlorophenyl)-2-oxoethyl] 9*α*-fluoro-11*β-*hydroxy-16*β*-methyl-3-oxo-17*α-*propionyloxyandrosta-1,4-diene-17*β*-carbothioate. |
| IA.102 | *α-*CH₃ | F | F | Ethoxy | 4-Trifluoromethylphenyl | H | H | 1 | S | *S*-[2-Oxo-2-(4-trifluoromethylphenyl)ethyl]6*α*,9*α-*difluoro-17*α*-(ethoxycarbonyl)oxy-11*β*-hydroxy-16*α*-methyl-3-oxoandrosta-1,4-diene-17*β-*carbothioate. |
| IA.103 | *α-*CH₃ | F | F | Cyclopropyl | 4-Fluorophenyl | H | H | 1 | O | 2-(4-Fluorophenyl)-2-methyl17*α-*cyclopropylcarbonyloxy-6*α*,9*α*-difluoro-11*β-*hydroxy-16*α*-methyl-3-oxoandrosta-1,4-diene-17*β-*carboxylate. |
| IA.104 | *α-*CH₃ | F | F | Cyclopropyl | 2,4-Difluorophenyl | H | H | 1 | S | *S*-[2-(2,4-Difluorophenyl)-2-oxoethyl] 17*α-*cyclopropylcarbonyloxy-6*α*,9*α*-difluoro-11*β-*hydroxy-16*α*-methyl-3-oxoandrosta-1,4-diene-17*β-*carbothioate. |
| IA.105 | *α-*CH₃ | F | F | Ethoxy | 4-Chlorophenyl | H | H | 1 | S | *S*-[2-(4-Chlorophenyl)-2-oxoethyl] 6*α*,9*α*-difluoro-17*α*-(ethoxycarbonyl)oxy-11*β*-hydroxy-16*α*-methyl-3-oxoandrosta-1,4-diene-17*β*-carbothioate. |
| IA.106 | *α-*CH₃ | F | F | Cyclopropyl | 4-Chlorophenyl | H | H | 1 | S | *S*-[2-(4-Chlorophenyl)-2-oxoethyl] 17*α*-cyclopropylcarbonyloxy-6*α*,9*α*-difluoro-11*β*-hydroxy-16*α-*methyl-3-oxoandrosta-1,4-diene-17*β*-carbothioate. |
| IA.107 | *β-*CH₃ | F | H | Ethyl | 4-Methoxyphenyl | H | H | 1 | S | *S*-[2-(4-Methoxyphenyl)-2-oxoethyl]9α-fluoro-11*β-*hydroxy-16*β*-methyl-3-oxo-17*α*-propionyloxy-androsta-1,4-diene-17*β*-carbothioate. |
| IA.108 | *α-*CH₃ | F | H | Ethyl | 4-Trifluoromethylphenyl | H | H | 1 | S | *S*-[2-Oxo-2-(4-trifluoromethylphenyl)-2-oxoethyl] 9*α*-fluoro-11*β*-hydroxy-16*α*-methyl-3-oxo-17*α-*propionyloxyandrosta-1,4-diene-17*β*-carbothioate. |
| IA.109 | *α-*CH₃ | F | F | Ethyl | 4-Methoxyphenyl | CH₃ | CH₃ | 1 | S | *S*-[1,1-Dimethyl-2-(4-methoxyphenyl)-2-oxoethyl] 6*α*,9*α*-difluoro-11*β*-hydroxy-16*α*-methyl-3-oxo-17*α*-propionyloxyandrosta-1,4-diene-17*β-*carbothioate. |
| IA.110 | *α-*CH₃ | F | F | Ethoxy | 4-Fluorophenyl | H | H | 1 | S | *S*-[2-(4-Fluorophenyl)-2-oxoethyl] 6*α*,9*α*-difluoro 17*α*-(ethoxycarbonyl)oxy-11*β*-hydroxy-16*α*-methyl-3-oxoandrosta-1,4-diene-17*β*-carbothioate. |
| IA.111 | *β-*CH₃ | F | H | Ethyl | 4-Trifluoromethylphenyl | H | H | 1 | S | *S*-[2-Oxo-2-(4-trifluoromethylphenyl)-2-oxoethyl] 9*α*-fluoro-11*β*-hydroxy-16*β*-methyl-3-oxo-17*α-*propionyloxyandrosta-1,4-diene-17*β*-carbothioate. |
| IA.112 | *α-*CH₃ | F | F | Cyclohexyl | 4-Trifluoromethylphenyl | H | H | 1 - | S | *S*-[2-Oxo-2-(4-trifluoromethylphenyl)ethyl] 17*α-*cyclohexylcarbonyloxy-6*α*,9*α*-difluoro-11*β-*hydroxy-16*α*-methyl-3-oxoandrosta-1,4-diene-17*β-*carbothioate. |
| IA.113 | *α-*CH₃ | F | F | Cyclohexyl | 4-Chlorophenyl | H | | 1 | S | *S*-[2-(4-Chlorophenyl)-2-oxoethyl] 17*α-*cyclohexylcarbonyloxy-6*α*,9*α*-difluoro-11*β-*hydroxy-16*α*-methyl-3-oxoandrosta-1,4-diene-17*β-*carbothioate. |
| IA.114 14 | *α-*CH₃ | F | F | Cyclohexyl | 2,4-Difluorophenyl | H | H | 1 | S | *S*-[2-(2,4-Difluorophenyl)-2-oxoethyl] 17*α-*cyclohexylcarbonyloxy-6*α*,9*α*-difluoro-11*β-*hydroxy-16*α*-methyl-3-oxoandrosta-1,4-diene-17*β-*carbothioate. |
| IA.115 | *α-*CH₃ | F | F | Cyclohexyl | 4-Fluorophenyl | H | H | 1 | O | 2-(4-Fluorophenyl)-2-oxoethyl 17*α-*cyclohexylcarbonyloxy-6*α*,9*α*-difluoro-11*β-*hydroxy-16*α*-methyl-3-oxoandrosta-1,4-diene-17*β-*carboxylate. |
| IA.116 | *α-*CH₃ | F | F | Cyclohexyl | 4-Methoxyphenyl | H | H | 1 | S | *S*-[2-(4-Methoxyphenyl)-2-oxoethyl] 17*α-*cyclohexylcarbonyloxy-6*α*,9*α*-difluoro-11*β-*hydroxy-16*α*-methyl-3-oxo-androsta-1,4-diene-17*β-*carbothioate. |
| IA.117 | *α-*CH₃ | F | F | Cyclopentyl | 4-Chlorophenyl | H | H | 1 | S | *S*-[2-(4-Chlorophenyl)-2-oxoethyl] 17*α-*cyclopentylcarbonyloxy-6*α*,9*α*-difluoro-11*β-*hydroxy-16*α*-methyl-3-oxoandrosta-1,4-diene-17*β-*carbothioate. |
| IA.118 | *α-*CH₃ | F | F | Cyclopentyl | 4-Fluorophenyl | H | H | 1 | O | *S*-[2-(4-Fluorophenyl)-2-oxoethyl] 17*α-*cyclopentylcarbonyloxy-6*α*,9*α*-difluoro-11*β-*hydroxy-16*α*-methyl-3-oxoandrosta-1,4-diene-17*β-*carboxylate. |
| IA.119 | *α-*CH₃ | F | F | Isopropyl | 4-Trifluoromethylphenyl | H | H | 1 | S | *S*-[2-Oxo-2-(4-trifluoromethylphenyl)ethyl] 6*α*,9*α-*difluoro-11*β*-hydroxy-16*α*-methyl-17*α*-(2-methylpropionyloxy)-3-oxoandrosta-1,4-diene-17*β-*carbothioate. |
| IA.120 | *α-*CH₃ | F | F | Cyclopentyl | 2,4-Difluorophenyl | H | H | 1 | S | *S*-[2-(2,4-Difluorophenyl)-2-oxoethyl]17*α-*cyclopentylcarbonyloxy-6*α*,9*α*-difluoro-11*β-*hydroxy-16*α*-methyl-3-oxoandrosta-1,4-diene-17*β-*carbothioate. |
| IA.121 | *α-*CH₃ | F | F | Isopropyl | 4-Fluorophenyl | H | H | 1 | O | 2-(4-Fluorophenyl)-2-oxoethyl 6*α*,9*α*-difluoro-11*β-*hydroxy-16*α*-methyl-17*α*-(2-methylpropionyloxy)-3-oxoandrosta-1,4-diene-17*β*-carboxylate. |
| IA.122 | *α-*CH₃ | F | F | Isopropyl | 4-Methoxyphenyl | H | H | 1 | S | *S*-[2-(4-Methoxyphenyl)-2-oxoethyl] 6*α*,9*α-*difluoro-11*β*-hydroxy-16*α*-methyl-17*α*-(2-methylpropionyloxy)-3-oxoandrosta-1,4-diene-17*β-*carbothioate. |
| IA.123 | *α-*CH₃ | F | F | Isopropyl | 2,4-Difluorophenyl | H | H | 1 | S | *S*-[2-(2-4-Difluorophenyl)-2-oxoethyl] 6*α*,9*α-*difluoro-11*β*-hydroxy-16α-methyl-17*α*-(2-methylpropionyloxy)-3-oxoandrostat-1,4-diene-17*β-*carbothioate. |
| IA.124 | *α-*CH₃ | F | F | 2-Furyl | 4-Trifluoromethylphenyl | H | H | 1 | S | *S*-[2-Oxo-2-(4-trifluoromethylphenyl)ethyl] 6*α*,9*α-*difluoro-17*α*-[(2-furanylcarbonyl)oxy]-11*β*-hydroxy-16*α*-methyl-3-oxoandrosta-1,4-diene-17*β-*carbothioate. |
| IA.125 | *α-*CH₃ | F | F | Ethyl | 4-Chlorophenyl | CH₃ | CH₃ | 1 | S | *S*-[1,1-Dimethyl-2-(4-chorophenyl)-2-oxoethyl] 6*α*,9*α*-difluoro-11*β*-hydroxy-16*α*-methyl-3-oxo-17*α*-propionyloxyandrosta-1,4-diene-17*β-*carbothioate. |
| IA.126 | *α-*CH₃ | F | F | Methoxy | 4-Methoxyphenyl | H | H | 1 | S | *S*-[2-(4-Methoxyphenyl)-2-oxoethyl] 6*α*,9*α-*difluoro-17*α*-(ethoxycarbonyloxy-11*β*-hydroxy-16*α*-methyl-3-oxoandrosta-1,4-diene-17*β-*carbothioate. |
| IA.127 | *α-*CH₃ | F | F | 2-Furyl | 4-Methoxyphenyl | H | H | 1 | S | *S*-[2-(4-Methoxyphenyl)-2-oxoethyl] 6*α*,9*α*-difluoro-17*α*[(2-furanylcarbonyl)oxy]-11*β*-hydroxy-16*α-*methyl-3-oxoandrosta-1,4-diene-17*β*-carbothioate. |
| IA.128 | *α-*CH₃ | F | F | *n*-Butyl | 4-trifluoromethylphenyl | H | H | 1 | S | *S*-[2-Oxo-2-(4-trifluoromethylphenyl)ethyl] 17*α*-*n-*butylcarbonyloxy-6*α*,9*α*-difluoro-11*β*-hydroxy-16*α-*methyl-3-oxoandrosta-1,4-diene-17*β*-carbothioate. |
| IA.129 | *α-*CH₃ | F | F | *n*-Butyl | 4-Chlorophenyl | H | H | 1 | S | *S*-[2-(4-Chlorophenyl)-2-oxoethyl] 17*α*-*n-*butylcarbonyloxy-6*α*,9*α*-difluoro-11*β*-6hydroxy-16*α-*methyl-3-oxoandrosta-1,4-diene-17*β*-carbothioate. |
| IA.130 | *α-*CH₃ | F | F | *n*-Butyl | 2,4-Difluorophenyl | H | H | 1 | S | *S*-[2-(2,4-Difluorophenyl)-2-oxoethyl] 17*α*-*n-*butylcarbonyloxy-6*α*,9*α*-difluoro-11*β*-hydroxy-16*α-*methyl-3-oxoandrosta-1,4-diene-17*β*-carbothioate. |
| IA.131 | *α-*CH₃ | F | F | *n*-Butyl | 4-Fluorophenyl | H | H | 1 | S | *S*-[2-(4-Fluorophenyl)-2-oxoethyl] 17*α*-*n*-butylcarbonyloxy-6*α*,9*α*-difluoro-11*β*-hydroxy-16*α-*methyl-3-oxoandrosta-1,4-diene-17*β*-carbothioate. |
| IA.132 | *α-*CH₃ | F | F | Isopropyl | 4-Chlorophenyl | H | H | 1 | S | *S*-[2-(4-Chlorophenyl)-2-oxoethyl] 6*α*,9*α*-difluoro-11*β*-hydroxy-16*α*-methyl-17*α*-(2-methylpropionyloxy)-3-oxoandrosta-1,4-diene-17*β*-carbothioate. |
| IA.133 | *α-*CH₃ | F | F | Cyclobutyl | 4-Fluorophenyl | H | H | 1 | O | *S*-[2-(4-Fluorophenyl)-2-oxoethyl] 17*α*-cyclobutylcarbonyloxy-6*α*,9*α*-difluoro-11*β*-hydroxy-16*α-*methyl-3-oxoandrosta-1,4-diene-17*β*-carboxylate. |
| IA.134 | *α-*CH₃ | F | F | Cyclopenlyl | 4-Trifluoromethylphenyl | H | H | 1 | S | *S*-[2-Oxo-2(4-trifluoromethylphenyl)ethyl] 17*α-*cyclopentylcarbonyloxy-6*α*,9*α*-difluoro-11*β-*hydroxy-16*α*-methyl-3-oxoandrosta-1,4-diene-17*β-*carbothioate. |
| IA.135 | *α-*CH₃ | F | F | Cyclobutyl | 4-Chlorophenyl | H | H | 1 | S | *S*-[2-(4-Chlorophenyl)-2-oxoethyl] 17*α-*cyclobutylcarbonyloxy-6*α*,9*α*-difluoro-11*β-*hydroxy-16*α*-methyl-3-oxoandrosta-1,4-diene-17*β-*carbothioate. |
| IA.136 | *α-*CH₃ | F | F | Cyclobutyl | 4-Trifluoromethylphenyl | H | H | 1 | S | *S*-[2-Oxo2-(4-trifluoromethylphenyl)ethyl] 17*α-*cyclobutylcarbonyloxy-6*α*,9*α*-difluoro-11*β-*hydroxy-16*α*-methyl-3-oxoandrosta-1,4-diene-17*β-*carbothioate. |
| IA.137 | *α-*CH₃ | F | F | Cyclobutyl | 2,4-Difluorophenyl | H | H | 1 | S | *S*-[2-(2-4-Difluorophenyl)-2-oxoethyl] 17*α-*cyclobutylcarbonyloxy-6*α*,9*α*-difluoro-11*β-*hydroxy-16*α*-methyl-3-oxoandrosta-1,4-diene-17*β-*carbothioate. |
| IA.138 | *α-*CH₃ | F | F | Cyclobutyl | 4-Methoxyphenyl | H | H | 1 | S | *S*-[2-(4-Methoxyphenyl)-2-oxoethyl] 17*α-*cyclobutylcarbonyloxy-6*α*,9*α*-difluoro-11*β-*hydroxy-16*α*-methyl-3-oxonandrosta-1,4-diene-17*β-*carbothioate. |
| IA.139 | *α-*CH₃ | F | H | 2-Furyl | 4-Tritluoromethylphenyl | H | H | 1 | S | *S*-[2-Oxo-2-(4-trifluoromethylphenyl)ethyl] 9*α-*fluoro-17*α*-[(2-furanylcarbonyl)oxy]-11*β*-hydroxy-16*α*-methyl-3-oxoandrosta-1,4-diene-17*β-*carbothioate. |
| IA.140 | *α-*CH₃ | F | H | 2-Furyl | 2,4-Difluorophenyl | H | H | 1 | S | *S*-[2-(2,4-Difluorophenyl)-2-oxoethyl] 9*α*-fluoro-17*α*-[(2-furanylcarbonyl)oxy]-11*β*-hydroxy-16*α-*methyl-3-oxoandrosta-1,4-diene-17*β*-carbothioate. |
| IA.141 | *α-*CH₃ | F | H | 2-Furyl | 4-Chlorophenyl | H | H | 1 | S | *S*-[2-(4-Chlorophenyl)-2-oxoethyl] 9*α*-fluoro-17*α-*[(2-furanylcarbonyl)oxy]-11*β*-hydroxy-16*α*-methyl-3-oxoandrosta-1,4-diene-17*β*-carbothioate. |
| IA.142 | *α-*CH₃ | F | H | 2-Furyl | 4-Methoxyphenyl | H | H | 1 | S | *S*-[2-(4-Methoxyphenyl)-2-ethyl] 9*α*-fluoro-17*α-*[(2-furanylcarbonyl)oxy-11*β*-hydroxy-16*α*-methyl-3-oxoandrosta-1,4-diene-17*β*-carbothioate. |
| IA.143 | *α-*CH₃ | F | H | 2-Furyl | 4-Fluorophenyl | H | H | 1 | S | *S*-[2-(4-Fluorophenyl)-2-oxoethyl] 9*α*-fluoro-17*α-*[(2-furanylcarbonyl)oxy]-11*β*-hydroxy-16*α*-methyl-3-oxoandrosta-1,4-diene-17*β*-carbothioate. |
| IA.144 | *α-*CH₃ | F | F | Isopropyl | 2,4,6-Trimethylphenyl | H | H | 1 | S | *S*-[2-Oxo-2-(2,4,6-trimethylphenyl)ethyl] 6*α*,9*α-*difluoro-11*β*-hydroxy-16*α*-methyl-17*α*-(2-methyl-propionyloxy)-3-oxoandrosta-1,4-diene-17*β-*carbothioate. |
| IA.145 | *α-*CH₃ | F | F | Cyclohexyl | 2,4,6-Trimethylphenyl | H | H | 1 | S | *S*-[2-Oxo-2-(2,4,6-trimethylphenyl)ethyl] 17*α-*cyclohexylcarbonyloxy-6*α*,9*α*-difluoro-11*β*-hydroxy-16*α*-methyl-oxoanadrosta-1,4-diene-17*β*-carbonothioate |
| IA.146 | *α-*Ch₃ | F | F | Cyclopentyl | 2,4,6-Trimethylphenyl | H | H | 1 | S | *S*-[2-Oxo-2-(2,4,6-trimethylphenyl)ethyl] 17*α-*cyclopenthylcarbonyloxy-6*α*,9*α*-difluoro-11*β*-hydroxy-16*α*-methyl-3-oxoandrosta-1,4-diene-17*β*-carbothioate |
| IA.147 | *α-*Ch₃ | F | F | Cyclobutyl | 2,4,6-Trimethylphenyl | H | H | 1 | S | *S*-[2-Oxo-2-(2,4,6-trimethylphenyl)ethyl] 17*α-*cyclobutylcarbonyloxy-6*α*,9*α*-difluoro-11*β*-hydroxy-16*α*-methyl-3-oxoandrosta-1,4-diene-17*β*-carbothioate. |
| IA.148 | *α-*CH₃ | F | F | Cyclohexylmethyl | 2,4,6-Trimethyl-phenyl | H | H | 1 | S | *S*-[2-Oxo-2-(2,4,6-trimethylphenyl)ethyl] 17*α-*cyclohexylmethylcarbonyloxy-6*α*,9*α*-difluoro-11*β-*hydroxy-16*α*-methyl-3-oxoandrosta-1,4-diene-17*β-*Carbothioate. |
| IA.149 | *α-*CH₃ | F | F | 2-Thienyl | 2,4,6-Trimethylphenyl | H | H | 1 | S | *S*-[2-Oxo-2-(2,4,6-trimethylphenyl)ethyl] 6*α*,9*α* difluoro-11*β*-hydroxy-16*α*-methyl-3-oxo-17*α*[(2-thienylcarbonyl)oxyandrosta-1,4-diene-17*β-*carbothioate. |
| IA.150 | *α-*CH₃ | F | F | Cyclohexylmethyl | 2,4-Difluorophenyl | H | H | 1 | S | *S*-[2-(2,4-Difluorophenyl)-2-oxoethyl] 17*α-*cyclohexylmethylcarbonyloxy-6*α*,9*α*-difluoro-11*β-*hydroxy-16*α*-methyl-3-oxoandrosta-1,4-diene-17*β-*carbothioate. |
| IA.151 | *α-*CH₃ | F | F | Cyclohexylmethyl | 4-Chlorophenyl | H | H | 1 | S | *S*-[2-(4-Chlorophenyl)-2-oxomethyl] 17*α-*cyclohexylmethylcarbonyloxy-6*α*,9*α*-difluoro-11*β-*hydroxy-16*α*-methyl-3-oxoandrosta-1,4-diene-17*β-*carbothioate. |
| IA.152 | *α-*CH₃ | F | F | Cyclohexylmethyl | 4-Trifluoro-methyl-phenyl | H | H | 1 | S | *S*-[2-Oxo-2-(4-trifluoromethyl)ethyl] 17*α-*cyclohexylmethylcarbonyloxy-6*α*,9*α*-difluoro-11*β-*hydroxy-16*α*-methyl-3-oxoandrosta-1,4-diene-17*β-*carbothioate. |
| IA.153 | *α-*CH₃ | F | F | Cyclohexylmethyl | 4-methoxyphenyl | H | H | 1 | S | *S*-[2-(4-Methoxyphyl)-2-oxoethyl] 17*α-*cyclohexylmethylcarbonyloxy-6*α*,9*α*-difluoro-11*β-*hydroxy-16*α*-methyl-3-oxoandrosta-1,4-diene-17*β-*carbothioate. |
| IA.154 | *α-*CH₃ | F | F | *n*-Butyl | 2,4,6-Trimethyl-phenyl | H | H | 1 | S | *S*-[2-Oxo-2-(2,4,6-trimethylphenyl)ethyl] 17*α*-*n-*butylcarbonyloxy-6*α*,9*α*-difluoro-11*β*-hydroxy-16*α-*methyl-3-oxoandrosta-1,4-diene-17*β*-carbothioate. |
| IA.155 | *α-*CH₃ | F | F | Ethoxy | 2,4,6-Trimethylphenyl | H | H | 1 | S | *S*-[2-Oxo-2-(2,4,6-trimethylphenyl)ethyl] 6*α*,9*α-*difluoro-17*α*-(ethoxycarbonyl)oxy-11*β*-hydroxy-16*α-*methyl-3-oxo-androsta-1,4-diene-17*β*-carbothioate. |
| IA.156 | *α-*CH₃ | F | F | 2-Furyl | 2,4,6-Trimethylphenyl | H | H | 1 | S | *S*-[2-Oxo-2-(2.4.6-trimethylphenyl)ethyl] 6*α*,9*α-*difluoro-17*α*-[(2-furanylcarbonyl)oxy]-11*β*-hydroxy-16*α*-methyl-3-oxoandrosta-1,4-diene-17*β*-carbothioate. |
| IA.157 | *α-*CH₃ | F | F | 2-Furyl | 4-Chloro-phenyl | H | H | 1 | S | *S*-[2-(4-Chlorophenyl)-2-oxoethyl] 6*α*,9*α*-difluoro-17*α*-[(2-furanylcarbonyl)oxy]-11*β*-hydroxy-16*α-*methyl-3-oxoandrosta-1,4-diene-17*β*-carbothioate. |
| IA.158 | *β-*CH₃ | F | F | Ethyl | 4-Chlorophenyl | H | H | 1 | S | *S*-[2-(4-Chlorophenyl)-2-oxoethyl] 6*α*,9*α*-difluoro-11*β*-hydroxy-16*β*-methyl-3-oxo-17*α-*propionyloxyandrosta-1,4-diene-17*β*-carbothioate. |
| IA.159 | *α-*CH₃ | F | F | 2-Thienyl | 4-Trifluoromethylphen yl | H | H | 1 | S | *S*-[2-Oxo-2-(4-trifluoromethylphenyl)ethyl] 6*α*,9*α-*difluoro-17*α*-[(2-thienylcarbonyl)oxy]-11*β*-hydroxy-16*α*-methyl-1-3-oxoandrosta-1,4-diene-17*β*-carbothioate. |
| IA.160 | *α-*CH₃ | F | F | Ethyl | 3-Nitrophenyl | H | H | 1 | S | *S*-[2-(3-Nitrophenyl)-2-oxoethyl] 6*α*,9*α*-difluoro-11*β-*hydroxy-16*α*-methyl-3-oxo-17*α*-propionyloxyandrosta-1,4-diene-17*β*-carbothioate. |
| IA.161 | *α-*CH₃ | F | F | Ethyl | 3,4-Dimethylphenyl | H | H | 1 | s | *S*-[2-(3,4-Dimethylphenyl)-2-oxoethyl] 6*α*,9*α-*difluoro-11*β*-hydroy-16*α*-methyl-3-oxo-17*α-*propionyloxyandrosta-1,4-diene-17*β*-carbothioate. |
| IA.162 | *β-*CH₃ | F | F | Ethyl | 4-Trifluoromethylphenyl | H | H | 1 | S | *S*-[2-Oxo-2-(4-trifluoromethylphenyl)ethyl] 6*α*,9*α-*difluoro-11*β*-hydroy-16*β*-methyl-3-oxo-17*α-*propionyloxyandrosta-1,4-diene-17*β*-carbothioate |
| IA.163 | *α-*CH₃ | F | F | *n*-Propyl | 4-Trifluoromethylphenyl | H | H | 1 | S | *S*-[2-Oxo-2-(4-trifluoromethylphenyl)ethyl] 6*α*,9*α-*difluoro-11*β*-hydroxy-16*α*-methyl-3-oxo-17*α*-*n-*propylcarbonyloxyandrosta-1,4-diene-17*β-*carbothioate. |
| IA.164 | *α-*CH₃ | F | F | *n*-Propyl | 4-Chlorophenyl | H | H | 1 | S | *S*-[2-(4-Chlorophenyl)-2-oxoethyl] 6*α*,9*α*-difluoro-11*β*-hydroxy-16*α*-methyl-3-oxo-17*α*-*n-*propylcarbonyloxyandrosta-1,4-diene-17*β-*carbothioate. |
| IA.165 | *α-*CH₃ | F | F | *n*-Propyl | 2,4-Difluorophenyl | H | H | 1 | S | *S*-[2-(2,4-Difluorophenyl)-2-oxoethyl] 6*α*,9*α-*difluoro-11*β*-hydroxy-16*α*-methyl-3-oxo-17*α*-*n-*propylcarbonyloxyandrosta-1,4-diene-17*β-*carbothioate. |
| IA.166 | *α-*CH₃ | F | F | *n*-Propyl | 4-Fluorophenyl | H | H | 1 | S | *S*-[2-(4-Fluorophenyl)-2-oxoethyl] 6*α*,9*α*-difluoro-11*β*-hydroxy-16*α*-methyl-3-oxo-17*α*-*n-*propylcarbonyloxyandrostat-1,4-diene-17*β-*carbothioate. |
| IA.167 | *α-*CH₃ | F | F | *n*-Propyl | 4-Methoxy-phenyl | H | H | 1 | S | *S*-[2-(4-Methoxyphenyl)-2-oxoethyl] 6*α*,9*α-*difluoro-11*β*-hydroxy-16*α*-methyl-3-oxo-17*α*-*n-*propylcarbonyloxyandrosta-1,4-diene-17*β-*carbothioate. |
| IA.168 | *α-*CH₃ | F | F | 2-Furyl | 3-Chloro-phenyl | H | H | 1 | S | *S*-[2-(3-Chlorophenyl)-2-oxoethyl] 6*α*,9*α*-difluoro-17*α*-[(2-furanylcarbonyl)oxy]-11*β*-hydroxy-16*α-*methyl-3-oxoandrosta-1,4-diene-17*β*-carbothioate. |
| IA.169 | *β-*CH₃ | F | F | 2-Furyl | 4-Trifluoro-methylphenyl | H | H | 1 | S | *S*-[2-Oxo-2-(4-trifluoromethylphenyl)ethyl] 6*α*,9*α-*difluoro-17*α*-[(2-furanylcarbonyl)oxy]-11*β*-hydroxy-16*β*-methyl-3-oxoandrosta-1,4-diene-17*β-*carbothioate. |
| IA.170 | *α-*CH₃ | F | F | 2-Furyl | 3.5-Dimethoxy-phenyl | H | H | 1 | S | *S*-[2-(3,5-Dimethyloxyphenyl)-2-oxoethyl] 6*α*,9*α-*difluoro-17*α*-[(2-furanylcarbonyl)oxy]-11*β*-hydroxy-16*α*-methyl-3-oxoandrosta-1,4-diene-17*β-*carbothioate. |
| IA.171 | *α-*CH₃ | F | F | 2-Furyl | 4-Nitrophenyl | H | H | 1 | S | *S*-[3-(4-Nitrophenyl)-2oxoethyl] 6*α*,9*α*-difluoro-17*α*-[(2-furanylcarbonyl)oxy]-11*β*-hydroxy-16*α-*methyl-3-oxoandrosta-1,4-diene-17*β*-carbothioate |
| IA.172 | *α-*CH₃ | F | F | Methoxy-methyl | 4-Trifluoromethylphenyl | H | H | 1 | S | *S*-[2-Oxo-2-(4-trifluoromethylphenyl)ethyl] 6*α*,9*α-*difluoro-11*β*-hydroxy-17*α*-methoxymethyl-carbonyl)oxy-16*α*-methyl-3-oxoandrosta-1,4-diene-17*β*-carbothioate. |
| IA.173 | *α-*CH₃ | F | F | Methoxymethyl | 2,4,6-Trimethyl-phenyl | H | H | 1 | S | *S*-(2-Oxo-2-(2,4,6-trimethylphenyl)ethyl] 6*α*,9*α-*difluoro-11*β*-hydroxy-17*α*-(methoxymethyl-carbonyl)oxy-16*α*-methyl-3-oxoandrosta-1,4-diene-17*β*-carbothioate. |
| IA.174 | *α-*CH₃ | F | F | Ethyl | 3-Chloro-4-fluorophenyl | H | H | 1 | S | *S-*[2-(3-Chloro-4-fluorophenyl)-2-oxoethyl] 6*α*,9*α-*difluoro-11*β*-hydroxy-16*α*-methyl-oxo-17*α-*propionyloxy-androsta-1,4-diene-17*β*-carbothioate. |
| IA.175 | *β-*CH₃ | F | F | 2-Furyl | 2,4,6-Trimethyl-phenyl | H | H | 1 | S | *S*-[2-Oxo-2-(2,4,6-trimetylphenyl)ethyl] 6*α*,9*α-*difluoro-17*α*-[(2-furanylcarbonyl)oxy]-11*β*-hydroxy-16*β*-methyl-3-oxoandrosta-1,4-diene-17*β-*carbothioate. |
| IA.176 | *α-*CH₃ | F | F | Ethoxy-methyl | 2,4,6-Trimethyl-phenyl | H | H | 1 | S | *S*-[2-Oxo-2-(2,4,6-trimethylphenyl)ethyl] 6*α*,9*α-*difluoro-17*α*-(ethoxymethylcarbonyl)oxy-11*β-*hydroxy-16*α*-methyl-3-oxoandrosta-1,4-diene-17*β-*carbothioate. |
| IA.177 | *α-*CH₃ | F | F | Ethoxy-methyl | 4-Trifluoro-methylphenyl | H | H | 1 | S | *S*-[2-Oxo-2-(4-trifluoromethylphenyl)ethyl] 6*α*,9*α-*difluoro-17*α*-(ethoxymethylcarbonyl)oxy-11*β-*hydroxy-16*α*-methyl-3-oxoandrosta-1,4-diene-17*β-*carbothioate. |
| IA.179 | *α-*CH₃ | F | F | 2-Furyl | 2-Naphthyl | H | H | 1 | S | *S*-[2-(Naphtalen-2-yl)-2-oxoethyl] 6*α*,9*α*-difluoro-17*α*-[(2-furanylcarbonyl)oxy]-11*β*-hydroxy-16*α-*methyl-3-oxoandrosta-1,4-diene-17*β*-carbothioate. |
| IA.180 | *α-*CH₃ | F | F | 2-Furyl | 1-Naphthyl | H | H | 1 | S | *S*-[2-(Naphtalen-2-yl)-2-oxoethyl] 6*α*,9*α*-difluoro-17*α*-[(2-furanylcarbonyl)oxy]-11*β*-hydroxy-16*α-*methyl-3-oxoandrosta-1,4-diene-17*β*-carbothioate. |
| IA.181 | *α-*CH₃ | F | F | 3-Methyl-2-furyl | 4-Trifluoro-methylphenyl | H | H | 1 | S | *S*-[2-Oxo-2-(4-trifluoromethylphenyl)ethyl] 6*α*,9*α-*difluoro-11*β*-hydroxy-17*α*[[(3-methyl2-furanyl)-carbonyl]oxy]-16α-methyl-3-oxoandrosta-1,4-diene-17*β*-carbothioate. |
| IA.182 | *α-*CH₃ | F | F | 3-Methyl-2-furyl | 2,4,6-Trimethyl-phenyl | H | H | 1 | S | *S*-[2-Oxo-2-(2,4,6-trifluromethylphenyl)ethyl] 6*α*,9*α-*difluoro-11*β*-hydroxy-17*α*-[[(3-methyl2-furanyl)-carbonyl]oxy]-16*α*-methyl-3-oxoandrosta-1,4-diene-17*β*-carbothioate. |
| IA.183 | *α-*CH₃ | F | F | 2-Furyl | 3-Nitro-4-methoxyphenyl | H | H | 1 | S | *S*-[2-(3-Nitro-4-methoxyphenyl)-2-oxoethyl] 6*α*,9*α-*difluoro-17*α*-[(2-furanylcarbonyl)oxy]-11*β*-hydroxy-16*α*-methyl-3-oxoandrosta-1,4-diene-17*β-*carbothioate. |
| IA.184 | *α-*CH₃ | F | F | 2-Furyl | 3,4-dimethylphenyl | H | H | 1 | S | *S*-[2-(3,4-Dimethylphenyl)-2-oxoethyl] 6*α*,9*α-*difluoro-17*α*-[(2-furanylcarbonyl)oxy]-11*β*-hydroxy-16*α*-methyl-3-oxoandrosta-1,4-diene-17*β-*carbothioate. |
| IA.185 | *α-*CH₃ | F | F | 3-Furyl | 4-Trifluoromethylphenyl | H | H | 1 | S | *S*-[2-Oxo-2-(4-trifluoromethylphenyl)ethyl] 6*α*,9*α-*difluoro-17*α*-[(3-furanylcarbonyl)oxy]-11*β*-hydroxy-16*α*-methyl-3-oxoandrosta-1,4-diene-17*β-*carbothioate. |
| IA.186 | *α-*CH₃ | F | F | 3-Furyl | 2,4,6-Trimethyl-phenyl | H | H | 1 | S | *S*-[2-Oxo-2-(2,4,6-trimethylphenyl)ethyl] 6*α*,9*α-*difluoro-17*α*-[(3-furanylcarbonyl)oxy]-11*β*-hydroxy-16*α*-methyl-3-oxoandrosta-1,4-diene-17*β-*carbothioate. |
| IA.187 | *α-*CH₃ | F | F | Ethyl | 4-Trifluoro-methylphenyl | CH₃ | CH₃ | 1 | S | *S*-[1,1-Dimethyl-2-oxo-2-(4-trifluoromethylphenyl)-ethyl] 6*α*,9*α*-difluoro-11*β*-hydroxy-16*α*-methyl-3-oxo-17*α*-propionyloxyandrosta-1,4-diene-17*β-*carbothioate. |
| IA.188 | *α-*CH₃ | F | F | Ethyl | 4-Fluoro-phenyl | CH₃ | CH₃ | 1 | S | *S*-[1,1-Dimethyl-2-(4-fluorophenyl)-2-oxoethyl] 6*α*,9*α*-difluoro-11*β*-hydroxy-16*α*-methyl-3-oxo-carbothioate. |
| IA.189 | *α-*CH₃ | F | H | Ethyl | Phenyl | CH₃ | CH₃ | 1 | S | *S*-[1,1-Dimethyl-2-phenyl-2-oxoethyl] 9*α*-fluoro-11*β*-hydroxy-16*α*-methyl-3-oxo-17*α*-propionyloxy-androsta-1,4-diene-17*β*-carbothioate. |
| IA.190 | *α-*CH₃ | F | F | Cyclohexyl | Phenyl | CH₃ | CH₃ | 1 | S | *S*-[1,1-Dimethyl-2-phenyl-2-oxoethyl] 17*α-*cyclohexylcarbonyloxy-6*α*,9*α*-difluoro-11*β-*hydroxy-16*α*-methyl-3-oxoandrosta-1,4-diene-17*β-*carbothioate. |
| IA.191 | *α-*CH₃ | F | F | Cyclopentyl | Phenyl | CH₃ | CH₃ | 1 | Q | *S*-[1,1-Dimethyl-2-phenyl-2-oxomethyl] 17*α-*cyclopentylcarbonyloxy-6*α*,9*α*-difluoro-11*β-*hydroxy-16*α*-methyl-3-oxoandrosta-1,4-diene-17*β-*carbothioate. |
| IA.192 | *α-*CH₃ | F | F | Isopropyl | Phenyl | CH₃ | CH₃ | 1 | S | *S*-[1,1-Dimethyl-2-phenyl-2-oxoethyl] 6*α*,9*α-*difluoro-11*β*-hydroxy-16*α*-methyl-17*α*-(2-methylpropionyloxy)-3-oxoandrosta-1,4-diene-17*β-*carbothioate. |
| IA.193 | *α-*CH₃ | F | F | Cyclo-butyl | Phenyl | CH₃ | CH₃ | 1 | S | *S*-[1,1-Dimethyl-2-phenyl-2-oxoethyl] 17*α-*cyclobutylcarbonyloxy-6*α*,9*α*-difluoro-11*β*-hydroxy-16*α*-methyl-3-oxoandrosta-1,4-diene-17*β-*carbothioate. |
| IA.194 | *α-*CH₃ | F | H | 2-Furyl | Phenyl | CH₃ | CH₃ | 1 | S | *S*-[1,1-Dimethyl-2-phenyl-2-oxoethyl] 9*α*-fluoro-17*α*-[(2-furanylcarbonyl)oxy]-11*β*-hydroxy-16*α-*methyl-3-oxoandrosta-1,4-diene-17*β*-carbothioate. |
| IA.195 | *α-*CH₃ | F | F | Cyclohexylmethyl | Phenyl | CH₃ | CH₃ | 1 | S | *S*-[1,1Dimethyl-2-phenyl-2-oxoethyl] 17*α-*cyclohexylmethylcarbonyloxy-6*α*,9*α*-difluoro-11*β-*hydroxy-16*α*-methyl-3-oxoandrosta-1,4-diene-17*β-*carbothioate. |
| IA.196 | *α-*CH₃ | F | F | 2-Furyl | 4-Fluorophenyl | CH₃ | CH₃ | 1 | S | *S*-[1,1-Dimethyl-2-(4-fluorophenyl)-2-oxoethyl] 6*α*,9*α*-difluoro-17*α*[(2-furanylcarbonyl)oxy]-11*β-*hydroxy-16*α*-methyl-3-oxoandrosta-1,6-diene-17*β-*carbothioate. |
| IA.197 | *α-*CH₃ | F | F | 2-Furyl | 4-Trifluoro-methylphenyl | CH₃ | CH₃ | 1 | S | *S*-[1,1Dimethyl-2oxo-2-(4-trifluoromethylphenyl)ethyl] 6*α*,9*α*-difluoro-17*α-*[(2-furanylcarbonyl)oxy]-11*β*-hydroxy-16*α*-methyl-3-oxoandrosta-1,4-diene-17*β*-carbothioate. |
| IA.198 | *α-*CH₃ | F | F | 5-Chloro-2-thienyl | 3,4-Dimethyl-phenyl | H | H | 1 | S | *S*-[2(3,4-Dimethylphenyl)-2-oxomethyl] 17*α*-[[(5-chloro-2-thienyl)carbonyl]oxy]-6*α*,9*α*-difluoro-11*β-*hydroxy-16*α*-methyl-3-oxoandrosta-1,4-diene-17*β-*carbothioate. |

### Table 2- Lists the compound of formula I-A-1 and Table-3 lists the compound of formula I-A-2 which have been synthesized

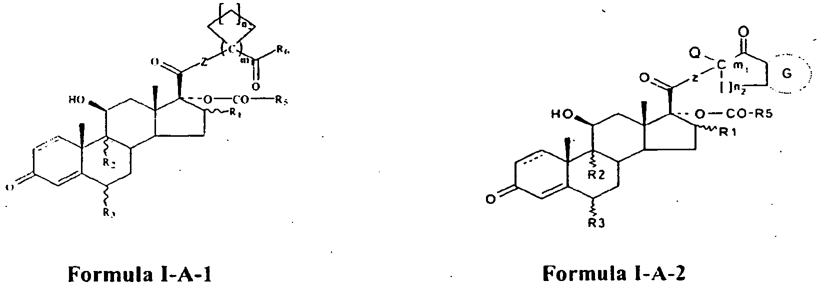

**Table 2**

| **Sr. No** | **R₁** | **R₂** | **R₃** | **R₅** | **R₆** | **n₂** | **m₁** | **Z** | **Chemical Name** |
|---|---|---|---|---|---|---|---|---|---|
| IA-1.1 | *α-*CH₃ | F | F | Ethyl | 4-Fluorophenyl | 2 | I | *S* | *S*-[1-(4-Fluorobenzoyl)cyclopentyl] 6*α*,9*α*-difluoro-11*β*-hydroxy-16*α*-methyl-3-oxo-17*α*-propionyloxy-androsta-1,4-diene-17*β*-carbothioate. |
| IA-1.2 | *α-*CH₃ | F | F | Ethyl | Phenyl | 2 | I | *S* | *S*-[1-Benzoylcyclopentyl] 6*α*,9*α*-difluoro-11*β*-hydroxy-16*α*-methyl-3-oxo-17*α-*propionyloxyandrosta-1,4-diene-17*β*-carbothioate. |

**Table 3**

| **Sr. No** | **R₁** | **R₂** | **R₃** | **R₅** | **n₂** | **Q** | **G** | **m₁** | **Z** | **Chemical Name** |
|---|---|---|---|---|---|---|---|---|---|---|
| IA- 2.1 | *α-*CH₃ | F | F | Ethyl | 2 | H | Phenyl | 1 | *S* | *S*-(1-oxo-1,2,3,4-tetrahydronapthalen-2-yl) 6*α*,9*α*-difluoro-11*β*-hydroxy-16*α-*methyl-3-oxo-17*α*-propionyloxyandrosta-1,4-diene-17*β*-carbothioate. |

### Table 4- Lists the compound of formula I-B which have been synthesized

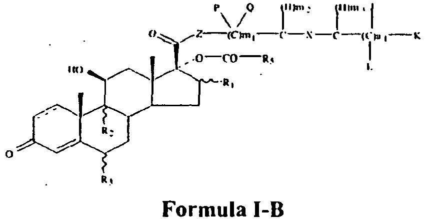

**Table-4**

| **Sr. No** | **R₁** | **R₂** | **R₃** | **R₅** | **P** | **Q** | **J** | **K** | **Z** | **X** | **m₁** | **m₂** | **m₃** | **n₁** | **L** | **Chemical Name** |
|---|---|---|---|---|---|---|---|---|---|---|---|---|---|---|---|---|
| IB.1 | *α-*CH₃ | F | F | Ethyl | H | H | H | H | O | ≡ | 1 | 0 | 0 | 1 | OH | 4-Hydroxy-but-2-ynyl 6*α*,9*α*-difluoro-11*β-*hydroxy-16*α*-methyl-3-oxo-17*α-*propionyloxyandrosta-1,4-diene-17*β-*carboxylate. |
| IB.2 | *α-*CH₃ | F | F | Ethyl | H | H | H | H | S | ≡ | 1 | 0 | 0 | 1 | OH | *S*-(4-Hydroxy-but-2-ynyl) 6*α*,9*α*-difluoro-11*β*-hydroxy-16*α*-methyl-3-oxo-17*α-*propionyloxyandrosta-1,4-diene-17*β-*carbothioate. |
| IB.3 | *α-*CH₃ | F | F | Ethyl | H | H | H | H | S | ≡ | 1 | 0 | 0 | 1 | F | *S*-(4-Fluoro-but-2-ynyl) 6*α*,9*α*-difluoro-11*β*-hydroxy-16*α*-methyl-3-oxo-17*α-*propionyloxyandrosta-1,4-diene-17*β-*carbothioate. |
| IB.4 | *α-*CH₃ | F | F | 2-Furyl | H | H | H | H | S | ≡ | 1 | 0 | 0 | 1 | OH | *S*-(4-Hydroxy-but-2-ynyl) 6*α*,9*α*-difluoro-17α-[(2-furanylcarbonyl)oxy]-11*β-*hydroxy-16*α*-methyl-3-oxoandrosta-1,4-diene-17*β*-carbothioate. |
| IB.5 | *α-*CH₃ | F | H | Ethyl | H | H | H | H | S | ≡ | 1 | 0 | 0 | 1 | OH | *S*-(4-Hydroxy-but-2-ynyl) 9*α*-fluoro-11*β-*hydroxy-16*α*-methyl-3-oxo-17*α-*propionyloxyandrosta-1,4-diene-17*β-*carbothioate. |
| IB.6 | *α-*CH₃ | F | F | 2-Furyl | H | H | H | H | S | ≡ | 1 | 0 | 0 | 1 | F | *S*-(4-Fluoro-but-2-ynyl) 6*α*,9*α*-difluoro-17α-[(2-furanylcarbonyl)oxy]-11*β-*hydroxy-16*α*-methyl-3-oxoandrosta-1.4-diene-17*β*-carbothioate. |
| IB.7 | *α-*CH₃ | F | F | Isopropyl | H | H | H | H | S | ≡ | 1 | 0 | 0 | 1 | OH | S-(4-Hydroxy-but-2-ynyl) 6*α*,9*α*-difluoro-11*β*-hydroxy-16*α*-methyl-17*α-*(2-methyl-propionyloxy)-3-oxoandrosta-1,4-diene-17*β*-carbothioate. |
| IB.8 | *α-*CH₃ | F | F | Cyclobutyl | H | H | H | H | S | ≡ | 1 | 0 | 0 | 1 | OH | *S*-(4-Hydroxy-but-2-ynyl) 17*α-*cyclobutylcarbonyloxy-6*α*,9*α*-difluoro-11*β*-hydroxy-16*α*-methyl-3-oxoandrosta-1,4-diene-17*β*-carbothioate. |
| IB.9 | *α-*CH, | F | H | 2-Furyl | H | H | H | H | S | ≡ | 1 | 0 | 0 | 1 | OH | *S*-(4-Hydroxy-but-2-ynyl) 9*α*-fluoro-17*α-*[(2-furanylcarbonyl)oxy]-11*β*-hydroxy-16*α*-methyl-3-oxoandrosta-1,4-diene-17*β-*carbothioate. |
| IB.10 | *α-*CH₃ | F | F | 2-Thienyl | H | H | H | H. | S | ≡ | 1 | 0 | 0 | 1 | OH | *S*-(4-Hydroxy-but-2-ynyl) 6*α*,9*α*-difluoro-11*β*-hydroxy-16*α*-methyl-3-oxo-17*α*-[(2-thienylcarbonyl)oxy]-androsta-1,4-diene-17*β*-carbothioate. |
| IB.11 | *α-*CH₃ | F | F | Ethyl | H | H | -- | -- | S | ≡ | 1 | 0 | 1 | 0 | - | *S*-(prop-2-ynyl) 6*α*,9*α*-difluoro-11*β-*hydroxy-16*α*-methyl-3-oxo-17*α*-propionyloxyandrosta-1,4-diene-17*β*-carbothioate. |
| IB.12 | *α-*CH₃ | F | F | 2-Furyl | H | H | H | H | S | ≡ | 1 | 0 | 0 | 1 | 1,4-dioxa-spiro[4,5] decan-2-ylmethoxy carbonyloxy | *S*-[4-(1,4-Dioxa-spiro)4,5]decan-2-ylmethoxycarbonyloxy)-but-2-ynyl] 6*α,*9*α-*difluoro-17*α*-[(2-furanyl-carbonyl)oxy]-11*β*-hydroxy-16*α*-methyl-3-oxoandrosta-1,4-diene-17*β*-carbothioate. |
| IB.13 | *α-*Ch₃ | F | F | Ethyl | H | H | H | H | S | = (*E*) | 1 | 1 | 1 | 1 | OH | *S*-[4-Hydroxy-*(E)*-but-2-enyl] 6*α*,9*α-*difluoro-11*β*-hydroxy-16*α*-methyl-3-oxo-17*α*-propionyloxyandrosta-1,4-diene-17*β-*carbothioate. |
| IB.14 | *α-*CH₃ | F | F | 2-Furyl | H | H | H | H | S | = (*E*) | 1 | 1 | 1 | 1 | OH | *S*-[4-Hydroxy-*(E)*-but-2-enyl] 6*α*,9*α-*difluoro-17*α*-[(2-furanylcarbonyl)oxy]-11*β-*hydroxy-16*α*-methyl-3-oxoandrosta-1,4-diene-17*β*-carbothioate. |
| IB.15 | *α-*CH₃ | F | F | 2-Furyl | H | H | H | H | S | (*Z*) | 1 | 1 | 1 | 1 | OH | *S*-[4-Hydroxy-(*Z)*-but-2-enyl] 6*α*,9*α-*difluoro-17*α*-[(2-furanylcarbonyl)oxy]-11*β-*hydroxy-16*α*-methyl-3-oxoandrosta-1,4-diene-17*β*-carbothioate. |
| IB.16 | *α-*CH₃ | F | F | 2-Thienyl | H | H | H | H | S | = (*Z*) | 1 | 1 | 1 | 1 | OH | *S*-[4-Hydroxy-*(Z)*-but-2-enyl] 6*α,*9*α-*difluoro-11*β*-hydroxy-16*α*-methyl-3-oxo-17*α-*[(2-thienylcarbonyl)oxy-]androsta-1,4-diene-17*β*-carbothioate. |
| IB.17 | *α-*CH₃ | F | F | 2-Thienyl | H | H | H | H | S | = (*E*) | 1 | 1 | 1 | 1 | OH | *S*-[4-Hydroxy-*(E)*-but-2-enyl] 6*α*,9*α-*difluoro-11*β*-hydroxy-16*α*-methyl-3-oxo-17*α*-[(2-thienylcarlbonyl)oxy]-androsta-1,4-diene-17*β*-carbothioate. |
| IB.18 | *α-*CH₃ | F | F | 2-Furyl | H | H | H | H | S | ≡ | 1 | 0 | 0 | 1 | 1,4-Dioxa-spiro[4,4] nonan-2-ylmethoxy carbonyloxy | *S*-[4-(1,4-Dioxa-spiro[4,4]nonan-2-ylmethoxycarbonyloxy)-but-2-ynyl] 6*α*,9*α-*difluoro-17*α*-[(2-furanyl-carbonyl)oxy]-11*β*-hydroxy-16*α*- methyl-3-oxoandrosta-1,4-diene-17*β*-carbothioate. |
| IB.19 | *α-*CH₃ | F | F | 2-Furyl | H | H | H | H | S | ≡ | 1 | 0 | 0 | 1 | OCO-O(CH₂)₂-ONO₂ | *S*-(4-(4-Nitroxybutyloxycarbonyloxy)-but-2-ynyl) 6*α*,9*α*-difluoro-17*α*[(2-furanylcarbonyl)oxy]-11*β*-hydroxy-16*α-*methyl-3-oxoandrosta-1,4-diene-17*β-*carbothioate. |
| IB.20 | *α-*CH₃ | F | F | 5-Chloro 2-thienyl | H | H | H | H | S | ≡ | 1 | 0 | 0 | 1 | OH | *S*-[4-Hydroxy-but-2-ynyl] 17*α*-[[(5-chloro2-thienyl)carbonyl]oxy]-6*α*,9*α-*difluoro-11*β*-hydroxy-16*α*- methyl-3-oxoandrosta-1,4-diene-17*β*-carbothioate. |
| IB.21 | *α-*CH₃ | F | F | 2-Furyl | H | H | H | H | S | ≡ | 1 | 0 | 0 | 1 | H | *S*-[But-2-ynyl] 6*α*,9*α*-difluoro-17*α*-[(2-furanylcarbonyl)oxy]-11*β*-hydroxy-16*α-*methyl-3-oxoandrosta-1,4-diene-17*β-*carbothioate. |
| IB.22 | *α-*CH₃ | F | F | 3-Methyl-2-Furyl | H | H | H | H | S | ≡ | 1 | 0 | 0 | 1 | OH | *S*-[4-Hydroxy-but-2ynyl] 6*α*,9*α*-difluoro-11*β*-hydroxy-17*α*-[[(3-methyl2-furanyl)-carbonyl]oxy]-16*α*-methyl-3-oxoandrosta-1,4-diene-17*β*-carbothioate. |
| IB.23 | *α-*CH₃ | F | F | Ethoxmethyl | H | H | H | H | S | ≡ | 1 | 0 | 0 | 1 | OH | *S*-[4-Hydroxy-but-2-ynyl] 6*α*,9*α*-difluoro-17*α*-(ethosymethylcarbonyl)oxy-11*β-*hydroxy-16*α*-methyl-3-oxoandrosta-1,4-diene-17*β*-carbothioate. |
| IB.14 | *α-*CH₃ | F | F | Methoxymethyl | H | H | H | H | S | ≡ | 1 | 0 | 0 | 1 | OH | S-[4-Hydroxy-but-2-ynyl] 6*α*,9*α*-difluoro-11*β*-hydroxy-17*α*(metoxymethylcarbonyl)oxy-16*α*-methyl-3-oxoandrosta-1,4-diene-17*β*-carbothioate. |
| IB. 25 | *α-*CH₃ | F | F | 2-Furyl | H | H | (CH₂)₅ | | S | ≡ | 1 | 0 | 0 | 1 | OH | *S*-[3-1-Hydroxycyclohexyl)prop-2-ynyl] 6*α*,9*α*-difluoro-17*α*-[(2-furanylcarbonyl)-oxy]-11*β*-hydroxy-16*α*-methyl-3-oxoandrosta-1,4-diene-17*β*-carbothioate. |
| IB.26 | *α-*CH₃ | F | F | CycloHexylmethyl | H | H | H | H | S | ≡ | 1 | 0 | 0 | 1 | OH | *S*-[4-Hydroxy-but-2-ynyl] 17*α-*cyclohexylmethylcarbonyloxy-6*α*,9*α-*difluoro-11*β*-hydroxy-16*α*-methyl-3-oxoandrosta-1,4-diene-17*β*-carbothioate. |
| IB.27 | *α-*CH₃ | F | F | *n*-Butyl | H | H | H | H | S | ≡ | 1 | 0 | 0 | 1 | OH | *S*-[4-Hydroxy-but-2-ynyl] 17*α*-*n-*butylcarbonyloxy-6*α*,9*α*-difluoro-11*β-*hydroxy-16*α*-methyl-3-oxoandrosta-1,4-diene-17*β*-carbothioate. |
| IB.28 | *α-*CH₃ | F | F | Ethyl | H | H | - | -- | O | ≡ | 1 | 0 | 1 | 0 | -- | Prop-2-ynyl 6*α*,9*α*-difluoro-11*β*-hydroxy-16*α*-methyl-3-oxo-17*α*-propionyloxy-androsta-1,4-diene-17*β*-carboxylate. |
| IB.29 | *α-*CH₃ | F | F | Cyclopentyl | H | H | H | H | S | ≡ | 1 | 0 | 0 | 1 | OH | *S*-[4-Hydroxy-but-2-ynyl] 17*α-*cyclopentylcarbonyloxy-6*α*,9*α*-difluoro-11*β*-hydroxy-16*α*-methyl-3-oxoandrosta-1,4-diene-17*β*-carbothioate. |
| IB.30 | *α-*CH₃ | F | F | Ethyl | H | H | H | H | S | = (*Z*) | 1 | 1 | 1 | 1 | OH | *S*-[4-Hydroxy-*(Z)*-but-2-enyl] 6*α*,9*α-*difluoro-11*β*-hydroxy-16*α*-methyl-3-oxo-17*α*-propionyloxyandrosta-1,4-diene-17*β-*carbothioate. |
| IB.31 | *α-*CH₃ | F | F | Ethyl | H | H | H | H | S | ≡ | 1 | 0 | 0 | 1 | OCOC₂H₅ | *S*-[4-Propionyloxy-but-2-ynyl] 6*α,*9*α-*difluoro-11*β*-hydroxy-16*α*-methyl-3-oxo-17*α*-propionyloxyandrosta-1,4-diene-17*β-*carbothioate. |
| IB.32 | *α-*CH₃ | F | F | Ethyl | H | H | H | H | S | ≡ | 1 | 1 | 0 | 1 | OCOCH₃ | *S*-[4-Acetoxy-but-2-ynyl] 6*α*,9*α*-difluoro-11*β*-hydroxy-16*α*-methyl-3-oxo-17*α-*propionyloxyandrosta-1,4-diene-17*β-*carbothioate. |
| IB.33 | *α-*CH₃ | F | F | Ethyl | H | H | H | H | S | ≡ | 1 | 0 | 0 | 1 | OCO-*n*-C₃H₂ | *S*-[4-*n*-Propylcarbonyloxy-but-2-ynyl] 6*α*,9*α*-difluoro-11*β*-hydroxy-16*α*-methyl-3-oxo-17*α*-propionyloxyandrosta-1,4-diene-17*β*-carbothioate. |
| IB.34 | *α-*CH₃ | F | F | Ethyl | H | H | H | H | S | ≡ | 1 | 0 | 0 | 1 | OCO-CH(CH₃)₂ | *S*-[4-(2-Methylpropionyloxy)-but-2-ynyl] 6*α*,9*α*-difluoro-11*β*-hydroxy-16*α*-methyl-3-oxo-17*α*-propionyloxyandrosta-1,4-diene-17*β*-carbothioate. |
| IB.35 | *α-*CH₃ | F | F | Ethyl | H | H | H | H | S | ≡ | 1 | 0 | 0 | 1 | OCO-*n*-C₄H₉ | *S*-[4-*n*-Butylcarbonyloxy-but-2-ynyl] 6*α*,9*α*-difluoro-11*β*-hydroxy-16*α*-methyl-3-oxo-17*α*-propionyloxyandrosta-1,4-diene-17*β*-carbothioate. |
| IB.36 | *α-*CH₃ | F | F | Ethyl | H | H | H | H | S | ≡ | 1 | 0 | 0 | 1 | OCO-cyclobutyl | *S*-[4-Cyclobutylcarbonyloxy-but-2-ynyl] 6*α*,9*α*-difluoro-11*β*-hydroxy-16*α*-methyl-3-oxo-17*α*-propionyloxyandrosta-1,4-diene-17*β*-carbothioate. |
| IB.37 | *α-*CH₃ | F | F | Ethyl | H | H | H | H | S | ≡ | 1 | 0 | 0 | 1 | OCO-*n*-C₅H₁₁ | *S*-[4-n-Pentylcarbonyloxy-but-2-ynyl] 6*α*,9*α*-difluoro-11*β*-fidroxy-16*α*-methyl-3-oxo-17*α*-propionyloxyandrosta-1,4-diene-17*β*-carbothioate. |
| IB.38 | *β-*CH₃ | F | H | Ethyl | H | H | H | H | S | ≡ | 1 | 0 | 0 | 1 | OH | *S*-[4-Hydroxy-but-2-ynyl] 9*α*-fluoro-11*β-*hydroxy-16*β*-methyl-3-oxo-17*α-*propionyloxyandrosta-1,4-diene-17*β-*carbothioate. |
| IB.39 | *β-*CH₃ | F | F | Ethyl | H | H | H | H | S | ≡ | 1 | 0 | 0 | 1 | OH | *S*-[4-Hydroxy-but-2-ynyl] 6*α*,9*α*-difluoro-11*β*-hydroxy-16*β*-methyl-3-oxo-17*α-*propionyloxyandrosta-1,4-diene-17*β-*carbothioate. |
| IB.40 | *α-*CH₃ | F | F | 2-Furyl | H | H | CH₂ | CH₃ | S | ≡ | 1 | 0 | 0 | 1 | OH | *S*-[4-Hydroxy-4-methyl-pent-2-ynyl] 6*α*,9*α*-difluoro-17*α*-[(2-furanylcarbonyl)oxy]-11*β*-hydroxy-16*α*-methyl-3-oxoandrosta-1,4-diene-17*β*-carbothioate. |
| IB.41 | *α-*CH₃ | F | F | Ethyl | H | H | O | | O | = (*E*) | 1 | 1 | 1 | 1 | OCH₃ | 4-Methoxy-4-oxo-*(E)*-but-2-enyl 6*α*,9*α-*difluoro-11*β*-hydroxy-16*α*-methyl-3-oxo-17*α*-propionyloxyandrosta-1,4-diene-17*β-*carboxylate. |
| IB.42 | *α-*CH₃ | F | F | Ethyl | H | H | O | | S | = (*E*) | 1 | 1 | 1 | 1 | OCH₃ | *S*-[4-methoxy-4-oxo-*(E)*-but-2-enyl] 6*α*,9*α*-difluoro-11*β*-hydroxy-16*α*-methyl-3-oxo-17*α*-propionyloxyandrosta-1,4- diene-17*β*-carbothioate. |
| IB.43 | - | H | H | Ethoxy | H | H | O | | O | = (*E*) | 1 | 1 | 1 | 1 | OCH₃ | 4-Methoxy-4-oxo-*(E)*-but-2-enyl 17*α-*(etoxycarbonyl)oxy-11*β*-hydroxy-3-oxoandrosta-1,4-diene-17*β*-carboxylate. |
| IB.44 | *α-*CH₃ | F | F | Ethyl | H | H | H | H | S | = (*E*) | 1 | 1 | 1 | 1 | OCOCO-OC₂H₅ | *S*-[4-(2-Ethoxy-1,2-dioxoethyly-*(E)*-but-2-enyl] 6*α*,9*α*-difluoro-11*β*-hydroxy-16*α-*methyl-3-oxo-17*α*-propionyloxyandrosta-1,4-diene-17*β*-carbothioate. |
| IB.45 | *α-*CH₃ | Cl | H | 2-Furyl | H | H | H | H | S | ≡ | 1 | 0 | 0 | 1 | OH | *S*-[4-Hydroxy-but-2-ynyl] 9*α*-chloro-17*α-*[(2-furanylcarbonyl)oxy]-11*β*-hydroxy-16*α*-methyl-3-oxoandrosta-1,4-diene-17*β-*carbothioate. |
| IB.46 | *α-*CH₃ | F | F | 2-Furyl | H | H | H | H | S | = *(E)* | 1 | 1 | 1 | 1 | OCOCO-OC₂H₃ | *S*-[4-(2-Ethoxy-1,2-dioxoethyl)oxy-*(E)*-but-2-enyl] 6*α*,9*α*-difluoro-17*α*-[(2-furanylcarbonyl)oxy]-11*β*- hydroxy-16*α-*methyl-3-oxoandrosta-1,4-diene-17*β-*carbothioate. |
| IB.47 | *α-*CH₃ | F | F | 2-Furyl | H | H | H | H | S | = *(Z)* | I | 1 | 1 | 1 | OCOCO-OC₂H₅ | *S*-[4-(2-Ethoxy-1,2-dioxoethyl)oxy-*(Z)*-but-2-enyl] 6*α*,9*α*-difluoro-17*α*-[(2-furanylcarbonyl)oxy]-11*β*-hydroxy-16*α-*methyl-3-oxoandrosta-1,4-diene-17*β-*carbothioate. |
| IB.48 | *α-*CH₃ | F | F | 2-Furyl | H | H | H | H | S | ≡ | 1 | 0 | 0 | 1 | (*S*)-(-)-2-Acetoxypropionyloxy | *S*-[4-((s)-(-)2-Acetoxypropionyloxy) but-2-ynyl] 6*α*,9*α*-difluoro-17*α*-[(2-furanylcarbonyl)oxy]-11*β*-hydroxy-16*α*-methyl-3-oxoandrosta-1,4-diene-17*β*-carbothioate. |
| IB.49 | *α-*CH₃ | F | F | 2-Furyl | H | H | H | H | S | ≡ | 1 | 0 | 0 | 1 | OCO-*n*-C₃H₃ | *S*-(4-n-Propylcarbonyloxy-but-2-ynyl] 6*α*,9*α*-difluoro-17*α*-[(2-furanylcarbonyl)-oxy]-11*β*-hydroxy-16*α*-methyl-3-oxoandrosta-1,4-diene-17*β*-carbothioate. |
| IB.50 | *α-*CH₃ | F | F | 2-Furyl | H | H | H | H | S | ≡ | 1 | 0 | 0 | 1 | OCO-CH(CH₃)₂ | *S*-[4-(2-Methylpropionyloxy)but-2-ynyl] 6*α*,9*α*-difluoro-17*α*-[(2-furanylcarbonyl)-oxy]-11*β*-hydroxy-16*α*-methyl-3-oxoandrosta-1,4-diene-17*β*-carbothioate. |
| IB.51 | *α-*CH₃ | F | F | 2-Furyl | H | H | H | H | S | ≡ | 1 | 0 | 0 | 1 | OCO-C(CH₃)₂ | *S*-[4-(2,2-Dimethylpropionyloxy)-but-2-ynyl] 6*α*,9*α*-difluoro-17*α*-[(12-furanyl-carhonyl)oxy)-11*β*-hydroxy-16*α*-methyl-3-oxoandrosta-1,4-diene-17*β*-carbothioate. |
| IB.52 | *α-*CH₃ | F | F | Ethyl | H | H | H | H | S | ≡ | 1 | 0 | 0 | 1 | OCO-C(CH₃)₃ | *S*-[4-(2,2-Dimethylpropionyloxy)-but-2-ynyl] 6*α*,9*α*-difluoro-11*β*-hydroxy-16*α-*methyl-3-oxo-17*α*-propionyloxyandrosta-1,4-diene-17*β*-carbothioate. |
| IB.53 | *α-*CH₃ | F | F | 2-Furyl | H | H | H | H | S | = *(Z)* | 1 | 1 | 1 | 1 | OCO-*n*-C₃H₇ | *S*-[4*-n*-Prypylcarbonyloxy-(*Z*)-but-2-enyl) 6*α*,9*α*-difluoro-17*α*-[(2-furanylcarbonyl)-oxy]-11*β*-hydroxy-16*α*-methyl-3-oxoandrosta-1,4-diene-17*β*-carbothioate. |
| IB.54 | *α-*CH₃ | F | F | 2-Furyl | H | H | H | H | S | ≡ | 1 | 0 | 0 | 1 | OCOCO-OC₂H₅ | *S*-[4-(2-Ethoxy-1,2-dioxoethyl)oxy-but-2-ynyl] 6*α*,9*α*-difluoro-17*α*-[(2-furanyl-carbonyl)oxy]-11*β*-hydroxy-16*α*-methyl-3-oxoandrosta-1,4-diene-17*β*-carbothioate. |
| IB.55 | *α-*CH₃ | F | F | 2- Thienyl | H | H | H | H | S | ≡ | 1 | 0 | 0 | 1 | OCO-*n*-C₃H₅ | *S*-[4-*n*-Propylcarbonyloxy-but-ynyl] 6*α*,9*α*-difluoro-11*β*-hydroxy-16*α*-methyl-3-oxo-17*α*-[(2-thoenylcarbonyl)oxy]androsta-1,4-diene-17*β*-carbothioate. |
| IB.56 | *α-*CH₃ | F | F | 5-Cl-2-Thienyl | H | H | H | H | S | ≡ | 1 | 0 | 0 | 1 | OCO-*n*-C₃H₇ | *S*-[4-*n*-Propylcarbonyloxy-but-2-ynyl] 17α-[[(5-chloro-2-thienyl)carbonyl]-oxy]-6*α*,9*α*-difluoro-11*β*-hydroxy-16*α*-methyl-3-oxo-androsta-1,4-diene-17*β*-carbothioate. |
| IB.57 | *α-*CH₃ | F | F | 2-Furyl | H | H | H | H | S | ≡ | 1 | 0 | 0 | 1 | OCO-cyclopropyl | *S*-[(4-cyclopropycarbonyloxy)-but-2-ynyl] 6*α*,9*α*-difluoro-17*α-*[(2-furanylcarbonyl)-oxy]-11*β*-hydroxy-16*α*-methyl-3-oxoandrosta-1,4-diene-17*β*-carbothioate. |
| IB.58 | *α-*CH₃ | F | F | 2-Furyl | H | H | H | H | S | ≡ | 1 | 0 | 0 | 1 | OCO-cyclobutyl | *S*-[(4-cyclobutycarbonyloxy)-but-2-ynyl] 6*α*,9*α*-difluoro-17*α*-[(2-furanylcarbonyl)-oxy]-11*β*-hydroxy-16*α*-methyl-3-oxoandrosta-1,4-diene-17*β*-carbothiate. |
| IB.59 | *α-*CH3 | F | F | 2-Furyl | H | H | H | H | S | ≡ | 1 | 0 | 0 | 1 | OCO-cyclopentyl | *S*-[(4-cyclopentylcarbonyloxy)-but-2-ynyl] 6*α*,9*α*-difluoro-17*α-*[(2-furanylcarbonyl)-oxy]-11*β*-hydroxy-16*α*-methyl-3-oxoandrosta-1,4-diene-17*β*-carbothioate. |
| IB.60 | α-CH₃ | F | F | 2-Furyl | H | H | H | H | S | ≡ *(E)* | 1 | 1 | 1 | 1 | OCO-*n*-C₃H₇ | *S*-[4-*n*-Propylcarbonyloxy-*(E)*-but-2-enyl] 6*α*,9*α*-difluoro-17*α*-[(2-furanylcarbonyl)-oxy]-11*β*-hydroxy-16*α*-methyl-3-oxoandrosta-1,4-diene-17*β*-carbothioate. |
| IB.61 | *α-*CH₃ | F | F | 4-Cl-phenyl | H | H | H | H | S | ≡ | 1 | 0 | 0 | 1 | OH | *S*-[4-Hydroxy-but-2-ynyl] 17*α*-(4-chlorophenyl)carbonyloxy-6*α*,9*α*-difluoro-11*β-*hydroxy-16*α*-methyl-3-oxoandrosta-1,4-diene-17*β*-carbothioate. |
| IB.62 | *α-*CH₃ | F | F | 3-Cl-phenyl | H | H | H | H | S | ≡ | 1 | 0 | 0 | 1 | OH | *S*-[4-Hydroxy-but-2-ynyl] 17*α*-(3-chlorophenyl)carbonyl-6*α*,9*α*-difluoro-11*β*-hydroxy-16*α*-methyl-3-oxocandrosta-1,4-diene-17*β*-carbothiate. |
| IB.63 | *α-*CH₃ | F | F | 2-Furyl | H | H | H | H | S | ≡ | 1 | 0 | 0 | 1 | OCO-OCH₃ | *S*-[(4-Methoxycarbonyloxy)-but-2-ynyl] 6*α*,9*α*-difluoro-17*α*-[(2-furanylcarbonyl)-oxy]-11*β*-hydroxy-16*α*-methyl-3-oxoandrosta-1,4-diene-17*β*-carbothioate. |
| IB.64 | *α-*CH₃ | F | F | 2-Furyl | H | H | H | H | S | ≡ | 1 | 0 | 0 | 1 | OCO-OC₂H₃ | *S*-[(4-Ethoxycarbonyloxyl-but-2-ynyl] 6*α*,9*α*-difluoro-17*α*-[(2-furanylcarbonyl)-oxy]-11*β*-hydroxy-16*α*-methyl-3-oxoandrosta-1,4-diene-17*β*-carbothioate. |
| IB.65 | *α-*CH₃ | F | F | 2-Furyl | H | H | H | H | S | ≡ | 1 | 0 | 0 | 1 | OCO-OC(CH₃)₃ | *S*-[4-2,2-Dimethylethyloxycarbonyloxy)-but-2-ynyl] 6*α*,9*α*-difluoro-17*α*-[(2-furanylcarbonyl)oxy]-11*β*-hydroxy-16*α-*methyl-3-oxoandrosta-1,4-diene-17*β-*carbothioate. |
| IB.66 | *α-*CH₃ | F | F | 2-Furyl | H | H | H | H | S | ≡ | 1 | 0 | 0 | 1 | OCO-OCH₂CH-(CH₃)₂ | *S*-[4-(2-Methylpropyloxycarbonyloxy)-but-2-ynyl] 6*α*,9*α*-difluoro-17*α*-[(2-furanylcarbonyl)oxy]-11*β*-hydroxy-16*α-*methyl-3-oxoandrosta-1,4-diene-17*β-*carbothioate. |
| IB.67 | *α-*CH₃ | F | F | 2-Furyl | H | H | H | H | S | ≡ | 1 | 0 | 0 | 1 | OCO-(CH₂)₂CHH₂Cl | *S*-[4-(3-Chloropropylcarbonyloxy)-but-2-ynyl] 6*α*,9*α*-difluoro-17*β*-[(2-furanyl-carbonyl)oxy]-11*β*-hydroxy-16*α*-methyl-3-oxoandrosta-1,4-diene-17*β*-carbothioate. |
| IB.68 | *α-*CH₃ | F | F | 2-Furyl | H | H | H | H | S | = *(Z)* | 1 | 1 | 1 | 1 | OCO-C(CH₃)₃ | *S*-[4-(2,2-Dimethylpropionyloxy)-(*Z*)-but-2-enyl] 6*α*,9*α*-difluoro-17*α*-[(2-furanyl-carbonyl)oxy]-11*β*-hydroxy-16*α*-methyl-3-oxoandrosta-1,4-diene-17*β*-carbothioate. |
| IB.69 | *α-*CH₃ | F | F | 2-Furyl | H | H | H | H | S | = *(E)* | 1 | 1 | 1 | 1 | OCO-C(CH₃)₃ | *S*-[4-(2,2-Dimethylpropionyloxy)-(*E*)-but-2-enyl] 6*α*,9*α-*difluoro-17*α*-[(2-furanyl-carbonyl)oxy]-11*β*-hydroxy-16*α*-methyl-3-oxoandrosta-1,4-diene-17*β*-carbothioate. |
| IB.70 | *α-*CH₃ | F | F | 2-Furyl | H | H | H | H | S | ≡ | 1 | 0 | 0 | 1 | OCOCHCl₂ | *S*-[4-Dichloroacetoxy-but-2-ynyl] 6*α*,9*α-*difluoro-17*α*-[(2-furanylcarbonyl)oxy]-11*β*-hydroxy-16*α*-methyl-3-oxandrosta-1,4-diene-17*β*-carbothioate. |
| IB.71 | *α-*CH₃ | F | F | 2-Furyl | H | H | H | H | S | ≡ | 1 | 0 | 0 | 1 | 2-methyl propyl-carbonyloxy | *S*-[4-(2-methylpropylcarbonyloxy)-but-2-ynyl] 6*α*,9*α*-difluoro-17*α*-[(2-furanyl-carbonyl)oxy]-11*β*-hydroxy-16*α*-methyl-3-oxoandrosta-1,4-diene-17*β*-carbothioate. |
| IB.72 | *α-*CH₃ | F | F | 2-Furyl | H | H | H | H | S | ≡ | 1 | 0 | 0 | 1 | OCOCH₃-C(CH₃)₃ | *S*-[4-(3,3-dimethylpropylcarbonyloxy)-but-2-ynyl] 6*α*,9*α*-difluoro-17*α*-[(2-furanylcarbonyl)oxy]-11*β*-hydroxy-16*α-*methyl-3-oxoandrosta-1,4-diene-17*β-*carbothioate. |
| IB.73 | *α-*CH₃ | F | F | 2-Furyl | H | H | H | H | S | ≡ | 1 | 0 | 0 | 1 | OCO-CH₂CH₃ | *S*-[4-Propionyloxy-but-2-ynyl] 6*α,*9*α-*difluoro-11*β*-hydroxy-17*α*-[(2-furanyl-carbonyl)oxy]-16*α*-methyl-3-oxoandrosta-1,4-diene-17*β*-carbothiate. |
| IB.74 | *α-*CH₃ | F | F | Cyclobutyl | H | H | H | H | S | ≡ | 1 | 0 | 0 | 1 | OCO-COOCH₂CH₃ | *S*-[4-(2-Ethoxy-1,2-dioxoethyl)oxy-but-2-ynyl] 17*α*-cyclobutylcarbonyl-oxy-6*α*,9*α-*difluoro-11*β*-hydroxy-16*α*-methyl-3-oxoandrosta-1,4-diene-17*β*-carbothioate. |
| IB.75 | *α-*CH₃ | F | F | Cyclo-butyl | H | H | H | H | S | ≡ | 1 | 0 | 0 | 1 | OCO-CH(CH₃)₂ | *S*-(4-(2-Methylpropionyloxy-but-2-ynyl) 17*α*-cyclobutylcarbonyloxy-6*α*,9*α-*difluoro-11*β*-hydroxy-16*α*-methyl-3-oxoandrosta-1,4-diene-17*β*-carbothioate. |
| IB.76 | *α-*CH₃ | F | F | 2-Furyl | H | H | H | H | S | ≡ | 1 | 0 | 0 | 1 | Adamantyl-carbonyloxy | *S*-[4-(Adamantylcarbonyloxy)-but-2-ynyl] 6*α*-9*α*-difluoro-17*α*-[(2-furanyl-carbonyl)oxy]-1*β*-hydroxy-16*α*-methyl-3-oxoandrosta-1,4-diene-17*β*-carbothioate. |
| IB.77 | *α-*CH₃ | F | F | 2-Furyl | H | H | H | H | S | ≡ | 1 | 0 | 0 | 1 | OCOCH₂-CF₃ | *S*-(4-(3,3,3-Trifluoropropionyloxy)-but-2-ynyl) 6*α*,9*α*-difluoro-17α-[(2-furanylcarbonyl)oxy]-11*β*-hydroxy-16*α*-methyl-3-oxo-androsta-1,4-diene-17*β-*carbothioate. |
| IB.78 | *α-*CH₃ | F | F | 2-Furyl | H | H | H | H | S | ≡ | 1 | 0 | 0 | 1 | OCOCH₂-OCH₃ | *S*-(4-(Methoxyacetyloxy)-but-2-ynyl) 6*α*,9*α*-difluoro-17*α*-[(2-furanyl-carbonyl)oxy]-11*β*-hydroxy-16*α*-methyl-3-oxoandrosta-1,4-diene-17*β*-carbothioate |
| IB.79 | *α-*CH₃ | F | F | 2-Furyl | H | H | H | H | S | ≡ | 1 | 0 | 0 | 1 | OCOCH₂-OCH₂CH₃ | *S*-(-4-(Ethoxyacetyloxy)-but-2-ynyl) 6*α*,9*α-*difluoro-17*α*-[(2-furanyl-carbonyl)oxy]-11*β*-hydroxy-16*α*-methyl-3-oxoandrosta-1,4-diene-17*β*-carbothioate. |
| IB.80 | *α-*CH₃ | F | F | 2-Furyl | H | H | H | H | S | ≡ | 1 | 0 | 0 | 1 | OCOCH₂-CH₂COOH | *S*-[4-(4-Hydroxybutyl-1,4-dione)oxy-but-2-ynyl] 6*α*,9*α*-difluoro-11*β*-hydroxy-16*α-*methyl-17*α*-[(2-furanylcarbonyl)oxy]-3-oxoandrosta-1,4-diene-17*β*-carbothiote. |
| IB.81 | *α-*CH₃ | F | F | 2-Furyl | H | H | H | H | S | ≡ | 1 | 0 | 0 | 1 | OCOCH₃ | *S*-[4-Acetoxy-but-2-ynyl] 6*α*,9*α*-difluoro-17*α*-[(2-furanylcarbonyl)-oxy]-11*β-*hydroxy-16*α*-methyl-3-oxoandrosta-1,4-diene-17*β*-carbothioate. |
| IB.82 | *α-*CH₃ | F | F | 2-Furyl | H | H | H | H | S | = (*E*) | 1 | 1 | 1 | 1 | OCO-CH(CH₃)₂ | *S*-[4-(2-Methylpropionyloxy)-(*E*)-but-2-enyl] 6*α*,9*α*-difluoro-17*α*-[(2-furanylcarbonyl)oxy]-11*β*-hydroxy-16*α-*methyl-3-oxoandrosta-1,4-diene-17*β*-carbothioate. |
| IB.83 | *α-*CH₃ | F | F | 2-Furyl | H | H | H | H | S | = (*Z*) | 1 | 1 | 1 | 1 | OCO-CH(CH₃)₂ | *S*-[4-(2-Methylpropionyloxy-(*Z*)-but-2-enyl] 6*α*,9*α*-difluoro-17*α*-[(2-furanylcarbony)oxy]-11*β*-hydroxy-16*α-*methyl-3-oxoandrosta-1,4-diene-17*β-*carbothioate. |
| IB.84 | *α-*CH₃ | F | F | Cyclobutyl | H | H | H | H | S | ≡ | 1 | 0 | 0 | 1 | OCO-C(CH₃)₃ | *S*-[4-(2,2-Dimethylpropionyloxy)-but-2-ynyl] 17*α*-cyclobutylcarbonyloxy-6*α*,9*α-*difluoro-11*β*-hydroxy-16*α*-methyl-3-oxoandrosta-1,4-diene-17*β*-carbothioate. |
| IB.85 | *α-*CH₃ | F | F | 2-Furyl | H | H | H | H | S | ≡ | 1 | 0 | 0 | 1 | 2,2-Dimethyl-butylcarbony loxy | *S*-[4-(2,2-Dimethylbutylcarbonyloxy)-but-2-ynyl] 6*α*,9*α*-difluoro-17*α*-[(2-furanyl-carbonyl)oxy]-11*β*-hydroxy-16*α*-methyl-3-oxoandrosta-1,4-diene-17*β*-carbothioate. |
| IB.86 | *α-*CH3 | F | F | 2-Furyl | H | H | H | H | S | ≡ | 1 | 0 | 0 | 1 | (1*S*)-Camphanyl-carbonyloxy | *S*-[4-(1*S*)-Camphanylcarbonyloxy-but-2-ynyl](6*α*,9*α*-difluoro-17*α*-[(2-furanyl-carbonyl)oxy]-11*β*-hydroxy-16*α*-methyl-3-oxoandrosta-1,4-diene-17*β*-carbothioate. |
| IB.87 | *α-*CH₃ | F | F | 2-Furyl | H | H | H | H | S | ≡ | 1 | 0 | 0 | 1 | OCONH-C(CH₃)₃ | *S*-[4-(1,1-Dimethylethylcarbamoyloxy)-but-2-ynyl] 6*α*,9*α*-difluoro-17*α*-[(2-furanyl-carbonyl)oxy]-11*β*-hydroxy-16*α*-methyl-3-oxoandrosta-1,4-diene-17*β*-carbothiate. |
| IB.88 | *α-*CH₃ | F | F | 2-Furyl | H | H | H | H | S | ≡ | 1 | 0 | 0 | 1 | OCO(CH₂)₂-COOC₂H₅ | *S*-[4-(4-Ethoxybutyl-1,4-dione)oxy-but-2-ynyl] 6*α*,9*α*-difluoro-17*α*-[(2-furanyl-carbonyl)oxy]-11*β*-hydroxy-16*α*-methyl-3-oxoandrosta-1,4-diene-17*β*-carbothioate. |
| IB.89 | *α-*CH₃ | F | F | 2-Furyl | H | H | H | H | S | ≡ | 1 | 0 | 0 | 1 | Menthyl-carbonyloxy | *S*-[4-Menthylcarbonyloxy-but-2-ynyl] 6*α*,9*α*-difluoro-17*α*-[(2-furanylcarbonyl)-oxy]-11*β*-hydroxy-16*α*-methyl-3-oxoandrosta-1,4-diene-17*β*-carbothioate. |
| IB.90 | *α-*CH₃ | F | F | 2-Furyl | H | H | H | H | S | ≡ | 1 | 0 | 0 | 1 | OCOCH₂NH -COCH₃ | *S*-[4-Acetamidoacetoxy-but-2-ynyl] 6α,9α-difluoro-17*α*-[(2-furanyl-carbonyl)oxy]-11*β*-hydroxy -16α-methyl-3-oxoandrosta-1,4-diene-17β-carbothioate. |
| IB.91 | *α-*CH₃ | F | F | 5-Chloro-2-Thienyl | H | H | H | H | S | ≡ | 1 | 0 | 0 | 1 | OCOOCH₂-CH(CH₃)₂ | *S*-[4-(2-Methylpropyloxycarbonyloxy)-but-2-ynyl]17*α*-[[(5-chloro2-thienyl])carbonyl]-oxy]-6*α*,9*α*-difluoro-11*β*-hydroxy-16*α*-methyl-3-oxoandrosta-1,4-diene-17*β-*methyl-3-oxoandrosta-1,4-diene-17*β-*carbothioate. |
| IB.92 | *α-*CH₃ | F | F | 5-Chloro-2-Thienyl | H | H | H | H | S | ≡ | 1 | 0 | 0 | 1 | OCO-CH(CH₃)₂ | *S*-[4-(2-Methylpropionyloxy)-but-2-ynyl) 17*α*-[[(5-chloro2-thienyl)-carbonyl]oxy]-oxy-6*α*,9*a*-difluoro-11*β*-hydroxy-16*α-*methyl-3-oxoandrosta-1,4-diene-17*β-*carbothioate. |
| IB.93 | *α-*CH₃ | F | F | 2-Furyl | H | H | H | H | S | ≡ | 1 | 0 | 0 | 1 | OCOCH=CH-COOC₂H₃ | *S*-[4-(4-Ethyloxy-*(Z)*-but-2-en-1,4-dione)oxy-but-2-ynyl]6*α*,9*α*-difluoro-17*α-*[(2-furanylcarbonyl])oxy]-11*β*-hydroxy-16*α*-methyl-3-oxoandrosta-1,4-diene-17*β-*carbothione. |
| IB.94 | *α-*CH₃ | F | F | 2-Furyl | H | H | H | H | S | ≡ | 1 | 0 | 0 | 1 | OCO(CH₂)₂-COOCH₃ | *S*-[4-(4-Methoxybutyl-1,4-dione)oxy-but-2-ynyl] 6*α*,9*α*-difluoro-17*α*-[(2-furanyl-carbonyl)oxy]-11*β*-hydroxy-16*α*-methyl-3-oxoandrosta-1,4-diene-17*β*-carbothioate. |
| IB.95 | *α-*CH₃ | F | F | 2-Furyl | H | H | H | H | S | ≡ | 1 | 0 | 0 | 1 | OCO(CH₂)₂-COOCH-(CH₃)₂ | *S*-[4-(4-Isopropyloxybutyl-1,4-dione)oxy-but-2-ynyl]6*α*,9*α*-difluoro-17*α*-[(2-furanyl-carbonyl)oxy]-11*β*-hydroxy-16*α*-methyl-3-oxoandrosta-1,4-diene-17*β*-carbothioate. |
| IB.96 | *α-*CH₃ | F | F | 2-Furyl | H | H | H | H | S | ≡ | 1 | 0 | 0 | 1 | OCO(CH₂)₂-COOCH₂CH -(CH₃)₂ | -*S*-[4-(4-Isobutyloxybutyl-1,4-dione)oxy-but-2-ynyl]6*α*,9*α*-difluoro-17*α*-[(2-furanyl-carbonyl)oxy]-11*β*-hydroxy-16*α*-methyl-3-oxoandrosta-1,4-diene-17*β*-carbothioate. |
| IB.97 | *α-*CH₃ | F | F | 2-Furyl | H | H | H | H | S | ≡ | 1 | 0 | 0 | 1 | OCO(CH₂)₂-COO(CH₂)₃-CH₃ | *S*-[4-(4-*n*-Butyloxybutyl-1,4-but-2-ynyl] 6*α*,9*α*-difluoro-17*α*-[(2-furanyl-carbonyl)oxy]-11*β*-hydroxy-16*α*-methyl-3-oxoandrosta-1,4-diene-17*β*-carbothioate. |
| IB.98 | *α-*CH₃ | F | F | 2-Furyl | H | H | H | H | S | ≡ | 1 | 0 | 0 | 1 | 3-Chloro-2.2-dimethyl-propionyloxy | *S*-[4-(3-Chloro-2,2-dimethylpropionyloxy)-but-2-ynyl] 6*α*,9*α*-difluoro-17*α*-[(2-furanylcarbonyl)oxy]-11*β*-hydroxy-16*α*-methyl-3-oxoandrosa-1,4-diene-17*β*- carbothioate. |
| IB.99 | *α-*CH₃ | F | F | 2-Furyl | H | H | H | H | S | ≡ | 1 | 0 | 0 | 1 | OCOCH=CH-(CH₃)₂ | *S*-[4-(4-Isobutyloxy-(*Z*)-but-2-en-1,4-dione)oxy-but-2-ynyl] 6*α*,9*α*-difluoro-17*α-*[(2-furanylcarbony)oxy]-11*β*-hydroxy-16*α-*methyl-3-oxoandrosta-1,4-diene-17*β-*carbothioate. |
| IB.100 | *α-*CH₃ | F | F | 2-Furyl | H | H | H | H | S | ≡ | 1 | 0 | 0 | 1 | OCOCH=CH-COOCH₃ | *S*-[4-(4-Methoxy-(*E*)-but-2-en-1,4-dione)oxy-but-2-ynyl 6*α*,9*α*-difluoro-17*α-*[(2-furanylcarbony)oxy]-11*β*-hydroxy-16*α-*methyl-3-oxoandrosta-1,4-diene-17*β-*carbothioate. |
| IB.101 | *α-*CH3 | F | F | 2-Furyl | H | | H | H | S | ≡ | 1 | 0 | 0 | 1 | OCO-CH₂CH(CH₃)₂ | *S*-[4-(3-Methyl-1-oxobutyloxy)-but-2-ynyl] 6*α*,9*α*-difluoro-17*α*-[(2-furanylcarbonyl)-oxy]-11*β*-hydroxy-16*α*-methyl-3-oxoandrosta-1,4-diene-17*β*-carbothiote. |
| IB.102 | *α-*CH₃ | F | F | 2-Furyl | H | H | H | H | S | ≡ | 1 | 0 | 0 | 1 | OCO-CH(C₂H₅)₂ | *S*-[4-(2-Ethyl-1-oxobutyloxy)-but-2-ynyl] 6*α*,9*α*-difluoro-17*α*-[(2-furanylcarbonyl)-oxy]-11*β*-hydroxy-16*α*-methyl-3-oxoandrosta-1,4-diene-17*β*-carbothioate. |
| IB.103 | *α-*CH₃ | F | F | 2-Furyl | H | H | H | H | S | ≡ | 1 | 0 | 0 | 1 | OCON(CH₃)₂ | *S*-[4-(*N,N*-Dimethylaminocarbonyl-oxy)-but-2-ynyl] 6*α*,9*α*-difluoro-17*α*-[(2-furanylcarbonyl)oxy]-11*β*-hydroxy-16*α-*methyl-3-oxoandrosta-1,4-diene-17*β-*carbothioate. |
| IB.104 | *α-*CH₃ | F | F | 2-Furyl | H | H | H | H | S | ≡ | 1 | 0 | 0 | 1 | OCO-CCl(CH₃)₂ | *S*-[4-(2-Chloro-2-methylpropionyl -oxy)-but-2-ynyl] 6*α*,9*α*-difluoro-17*α*-[(2-furanylcarbonyl)oxy]-11*β*-hydroxy-16*α-*methyl-3-oxoandrosta-1,4-diene-17*β-*carbothioate. |
| IB.105 | *α-*CH₃ | F | F | 2-Furyl | H | H | H | H | S | ≡ | 1 | 0 | 0 | 1 | OCO(CH₂)₂-CH(CH₃)₂ | *S*-[4-(4-Methyl-1-oxopentyloxy)-but-2-ynyl] 6*α*,9*α*-difluoro-17*α*-[(2-furanylcarbonyl)oxy]-11*β*-hydroxy-16*α-*methyl-3-oxandrosta-1,4-diene-17*β-*carbothioate. |
| IB.106 | *α-*CH₃ | F | F | 2-Furyl | H | H | H | H | S | ≡ | 1 | 0 | 0 | 1 | OCO(CH₂)₂-COOCH₂F | *S*-[4-(4-Fluoromethoxy-1,4-dioxo-butyl)oxy-but-2-ynyl] 6*α*,9*α*-difluoro-17*α-*[(2-furanylcarbonyl)oxy]-11*β*-hydroxy-16*α*-methyl-3-oxoandrodrosta-1,4-diene-17*β*-carbothioate. |
| IB.107 | *α-*CH₃ | F | F | 2-Furyl | H | H | H | H | S | ≡ | 1 | 0 | 0 | 1 | OCOCH=CH-COOCH₂F | *S*-[4-(4-Fluoromethyloxy-1,4-dioxo-(*Z*)-but-2-enhoxy-but-2-ynyl] 6*α*,9*α*-difluoro-17*α-*[(2-furanylcarbonyl)oxy]-11*β*-hydroxy-16*α-*methyl-3-oxoandrosta-1,4-diene-17*β-*carbothioate. |
| IB.108 | *α-*CH₃ | F | F | 2-Furyl | H | H | H | H | S | ≡ | 1 | 0 | 0 | 1 | OCOCH₂-COOCH₃ | *S*-[4-(3-Methoxypropyl-1,3-dione)oxy-but-2-ynyl] 6*α*,9*α*-difluoro-17*α*-[(2-furanylcarbonyl)oxy]-11*β*-hydroxy-16*α-*methyl-3-oxoandrosta-1,4-diene-17*β-*carbothioate. |
| IB.109 | *α-*CH₃ | F | F | 2-Furyl | H | H | H | H | S | ≡ | 1 | 0 | 0 | 1 | OCOC(CH₃)₂-COOCH₃ | *S*-[4-(3-Methoxy-2,2-dimethylpropyl-1,3-dione)oxy-but-2-ynyl]6*α*,9*α*-difluoro-17*α*-[(2-furanylcarbonyl)-oxy]-11*β*-hydroxy-16*α*-methyl-3-oxoandrosta-1,4-diene-17*β-*carbothioate. |
| IB.110 | *α-*CH₃ | F | F | 2-Furyl | H | H | H | H | S | ≡ | 1 | 0 | 0 | 1 | OCOC(OH)-(CH₃)₂ | *S*-[4-(2-Hydroxy-2-methylpropionyloxy-but-2-ynyl] 6*α*,9*α*-difluoro-17*α*-[(2-furanyl-carbonyl)oxy]-11*β*-hydroxy-16*α*-methyl-3-oxoandrosta-1,4-diene-17*β*-carbothioate. |
| IB.111 | *α-*CH₃ | F | F | 2-Furyl | H | H | H | H | S | ≡ | 1 | 0 | 0 | 1 | OCOCF(CH₃)₂ | *S*-[4-(2-Fluoro-2-methylpropionyloxy)-but-2-ynyl] 6*α*,9*α-*carbonyl)oxy]-11*β*-hydroxy-16*α*-methyl-3-oxoandrosta-1,4-diene-17*β*-carbothioate. |
| IB.112 | *α-*CH₃ | F | F | 2-Furyl | H | H | H | H | S | ≡ | 1 | 0 | 0 | 1 | OCOCH=CH-COOCH₃ | *S*-[4-(4-Methoxy-*(Z)*-but-2-en-1,4-dione)-oxy-but-2-ynyl]6*α*,9*α*-difluoro-17*α*[(2-furanylcarbonyl)oxy]-11*β*-hydroxy-16*α-*methyl-3-oxoandrosta-1,4-dione-17*β-*carbothioate. |
| IB.113 | *α-*CH₃ | F | F | 2-Furyl | H | H | H | H | S | ≡ | 1 | 0 | 0 | 1 | OCOCH=CH-COOC₂H₃ | *S*-[4-(4-Ethoxy-*(E)*-but-2-en-1,4-dione)oxy-but-2-ynyl]6*α*,9*α*-difluoro-17*α*-[(2-furanyl-carbonyl)oxy]-11*β*-hydroxy-16*α*-methyl-3-oxoandrosta-1,4-diene-17*β*-carbothioate. |
| IB.114 | *α-*CH₃ | F | F | 2-Furyl | H | H | H | H | S | ≡ | 1 | 0 | 0 | 1 | OCOCH=CH-COOCH₃-CH(CH₃)₂ | *S*-[4-(4-Isobutyloxy-1,4-dioxo-*(E)*-but-2-en)oxy-but-2-ynyl]6*α*,9*α*-difluoro-17*α*-[(2-furanylcarbonyl)-oxy]-11*β*-hydroxy-16*α-*methyl-3-oxoandrosta-1,4-diene-17*β-*carbothioate. |
| IB.115 | *α-*CH₃ | F | F | 2-Furyl | H | H | H | H | S | ≡ | 1 | 0 | 0 | 1 | OCOCH=CH-COO(CH₂)₃-CH₃ | *S*-[4-(4-*n*-butyloxy-1-4-dioxo-*(E)*-but-2-en)oxy-but-2-ynyl]6*α*,9*α*-difluoro-17*α*[(2-furanylcarbonyl)oxy]-11*β*-hydroxy-16*α-*methyl-3-oxoandrosta-1,4-diene-17*β-*carbothioate. |
| IB.116 | *α-*CH₃ | Cl | H | 2-Furyl | H | H | H | H | S | ≡ | 1 | 0 | 0 | 1 | OCOCH-(CH₃)₂ | *S*-[4-(2-Methylpropionyloxy)-but-2-ynyl] 9*α*-chloro-17*α*-[(2-furanylcarbonyl)oxy]-11*β*-hydroxy-16*α*-methyl-3-oxoandrosta-1,4-diene-17*β*-carbothioate. |
| IB.117 | *α-*CH₃ | F | F | 2-Furyl | H | H | H | H | S | ≡ | 1 | 0 | 0 | 1 | 2-(*S*)-Methyl-butyryloxy | *S*-[4-(2-(*S*)-methylbutyryloxy)-but-2-ynyl] 6*α*,9*α*-difluoro-17*α*-[(2-furanylcarbonyl)-oxy]-11*β*-hydroxy-16*α*-methyl-3-oxoandrosta-1,4-diene-17*β*-carbothioate. |
| IB.118 | *α-*CH₃ | F | F | 2-Furyl | H | H | H | H | S | ≡ | 1 | 0 | 0 | 1 | OCO-(2-Furyl) | *S*-[4-((furan-2-yl)carbonyloxy)-but-2-ynyl] 6*α*,9*α*-difluoro-17*α*-[(2-furanylcarbonyl)-oxy]-11*β*-hydroxy-16*α*-methyl-3-oxoandrosta-1,4-diene-17*β*-carbothioate. |
| IB.119 | *α-*CH₃ | F | F | 2-Furyl | H | H | H | H | S | ≡ | 1 | 0 | 0 | 1 | OCOCH=CH-COOCH-(CH₃)₂ | *S*-[4-(4-Isopropyloxy-1,4-dioxo-*(E)*-but-2-en)oxy-but-2-ynyl]6*α*,9*α*-difluoro-17*α*-[(2-furanylcarbonyl)-oxy]-11*β*-hydroxy-16*α-*methyl-3-oxoandrosta-1,4-diene-17*β-*carbothioate. |
| IB.120 | *α-*CH₃ | F | F | 2-Furyl | H | H | H | H | S | ≡ | 1 | 0 | 0 | 1 | 3-Hydroxy-2,2-dimethyl-Propionyl-oxy | *S*-[4-(3-Hydroxy-2,2-dimethyl-propionyl]-oxy)-but-2-ynyl] 6*α*,9*α*-difluoro-17*α*-[(2-furanylcarbonyl)oxy]-11*β*-hydroxy-16*α-*methyl-3-oxoandrosta-1,4-diene-17*β-*carbothioate. |
| IB.121 | *α-*CH₃ | F | F | 2-Furyl | H | H | H | H | S | ≡ | 1 | 0 | 0 | 1 | OCO-CH=CH₂ | *S*-(4-Acryloyloxy-but-2-ynyl)6*α*,9*α-*difluoro-17*α*-[(2-furanylcarbonyl)oxy]-11*β-*hydroxy-16*α*-methyl-3-oxoandrosta-1,4-diene-17*β*-carbothioate. |
| IB.122 | *α-*CH₃ | F | F | 2-Furyl | H | H | H | H | S | ≡ | 1 | 0 | 0 | 1 | OCOC(OH)-(C₂H₅)₃ | *S*-[4-(1-Ethyl-1-hydroxypropylcarbonyl-oxy)-but-2-ynyl] 6*α*,9*α*-difluoro-17*α*-[(2-furanylcarbonyl)oxy]-11*β*-hydroxy-16*α-*methyl-3-oxoandrosta-1,4-diene-17*β-*carbothioate. |
| IB.123 | *α-*CH₃ | F | F | 2-Furyl | H | H | H | H | S | ≡ | I | 0 | 0 | 1 | OCOOCH₂-cyclopropyl | *S*-[4-(Cyclopropylmethyloxycarbonyl-oxy)-but-2-ynyl]6*α*,9*α*-difluoro-17*α*-[(2-furanyl-carbonyl)oxy]-11*β*-hydroxy-16*α*-methyl-3-oxoandrosta-1,4-diene-17*β*-carbothioate. |
| IB.124 | *α-*CH₃ | F | F | 2-Furyl | H | H | H | H | S | ≡ | 1 | 0 | 0 | 1 | OCOC=C-COOCH₃ | *S*-[4-(4-Methoxybut-2-yne-1,4-dione)oxy-but-2-ynyl] 6*α*,9*α*-difluoro-17*α*[(2-furanyl-carbonyl)oxy]-11*β*-hydroxy-16*α*-methyl-3-oxoandrosta-1,4-diene-17*β*-carbothioate. |
| IB.125 | *α-*CH₃ | F | F | 2-Furyl | H | H | H | H | S | ≡ | 1 | 0 | 0 | 1 | OCOO-cyclopentyl | *S*-[4-(Cyclopentyloxycarbonyloxy)-but-2-ynyl]6*α*,9*α*-difluoro-17*α*-[(2-furanyl-carbonyl)oxy]-11*β*-hydroxy-16*α*-methyl-3-oxoandrosta-1,4-diene-17*β*-carbothioate. |
| IB.126 | *α-*CH₃ | F | F | 2-Furyl | H | H | H | H | S | ≡ | 1 | 0 | 0 | 1 | OCOO-CH₂ cyclopentyl | *S*-[4-(Cyclopentylmethyloxycarbonyl-oxy)-but-2-ynyl] 6*α*,9*α*-difluoro-17*α*-[(2-furanyl-carbonyl)oxy]-11*β*-hydroxy-16*α*-methyl-3-oxoandrosta-1,4-diene-17*β*-carbothioate. |
| IB.127 | *α-*CH₃ | Cl | H | 2-Furyl | H | H | H | H | S | ≡ | 1 | 0 | 0 | 1 | OCOO-CH₂-CH(CH₃)₂ | *S*-[4-(2-Methylpropyloxycarbonyloxy)-but-2-ynyl]9*α*-chloro-17*α*-[(2-furanyl-carbonyl)oxy]-11*β*-hydroxy-16*β*-methyl-3-oxoandrosta-1,4-diene-17*β*-carbothioate. |
| IB.128 | *α-*CH₃ | F | F | 2-Furyl | H | H | H | H | S | ≡ | 1 | 0 | 0 | 1 | 2,2-Dimethyl-1,3-dioxolan-4-yl-methoxy-carbonyloxy | *S*-[4-(2,2-Dimethyl-1,3-dioxolan-4-yl-methoxycarbonyloxy)-but-2-ynyl] 6*α*,9*α-*difluoro-17*α*-[(2-furanyl-carbonyl)oxy]-11*β*-hydroxy-16*α*-methyl-3-oxoandrosta-1,4-diene-17*β*-carbothioate. |
| IB.129 | *α-*CH₃ | F | F | 2-Furyl | H | H | H | H | S | ≡ | 1 | 0 | 0 | 1 | OCON(C₂H₃)₂ | *S*-[4-(N,N-Diethylaminocarbonyloxy)-but-2-ynyl] 6*α*,9*α*-difluoro-17*α*-[(2-furanyl-carbonyl)oxy]-11*β*-hydroxy-16*α*-methyl-3-oxoandrosta-1,4-diene-17*β*-carbothioate. |
| IB.130 | *α-*CH₃ | F | F | 2-Furyl | H | H | H | H | S | ≡ | 1 | 0 | 0 | 1 | Piperidin-1-ylcarbonyl-oxy | *S*-[4-(Piperidin-1-ylcarbonyloxy)-but-2-ynyl] 6*α*,9*α*-difluoro-17*α*-[(2-furanyl-carbonyl)oxy]-11*β*-hydroxy-16*α*-methyl-3-oxoandrosta-1,4-diene-17*β*-carbothioate. |
| IB.131 | *α-*CH₃ | F | F | 2-Furyl | H | H | H | H | S | ≡ | 1 | 0 | 0 | 1 | 4-Methylpiperazine-1-yl-carbonyloxy | *S*-[4-(4-Methyl-piperazine-1-yl-carbonyloxy)-but-2-ynyl] 6*α*,9*α*-difluoro-17*α*-[(2-furanylcarbonyl)-oxy]-11*β*-hydroxy-16*α-*methyl-3-oxoandrosta-1,4-diene-17*β-*carbothioate. |
| IB.132 | *β-*CH₃ | F | F | 2-Furyl | H | H | H | H | S | ≡ | 1 | 0 | 0 | 1 | OH | *S*-(4-Hydroxy-but-2-ynyl) 6*α*,9*α*-difluoro-17*α*-[(2-furanylcarbonyl)oxy]-11*β-*hydroxy-16*β*-methyl-3-oxoandrosta-1,4-diene-17*β*-carbothioate. |
| IB.133 | *β-*CH₃ | F | F | 2-Furyl | H | H | H | H | S | ≡ | 1 | 0 | 0 | 1 | OCOCH-(CH₃)₂ | *S*-[4-(2-Methylpropionyloxy)-but-2-ynyl] 6*α*,9*α*-difluoro-17*α*-[(2-furanylcarbonyl)-oxy]-11*β*-hydroxy-16*β*-methyl-3-oxoandrosta-1,4-diene-17*β*-carbothioate. |
| IB.134 | *β-*CH₃ | F | F | 2-Furyl | H | H | H | H | S | ≡ | 1 | 0 | 0 | 1 | OCOOCH₂-CH(CH₃)₂ | *S*-[4-(2-Methylpropyloxycarbonyloxy)-but-2-ynyl] 6*α*,9*α*-difluoro-17*α*-[(2-furanyl-carbonyl)oxy]-11*β*-hydroxy-16*β*-methyl-3-oxoandrosta-1,4-diene-17*β*-carbothioate. |
| IB.135 | *α-*CH₃ | F | F | 2-Furyl | H | H | H | H | S | ≡ | 1 | 0 | 0 | 1 | OCOCCl₂-CH₃ | *S*-[4-(2,2-Dichloropropionyloxy)-but-2-ynyl] 6*α*,9*α*-difluoro-17*α*-[(2-furanyl-carbonyl)oxy]-11*β*-hydroxy-16*α*-methyl-3-oxoandrosta-1,4-diene-17*β*-carbothioate. |
| IB.136 | *α-*C H₃ | F | F | 2-Furyl | H | H | H | H | S | ≡ | 1 | 0 | 0 | 1 | N(C₂H₅)₂ | *S*-[4-(*N*,*N*-Diethylamino)-but-2-ynyl] 6*α*,9*α*-difluoro-17*α*-[(2-furanylcarbonyl)-oxy]-11*β*-hydroxy-16*α*-methyl-3-oxoandrosta-1,4-diene-17*β*-carbothioate. |
| IB.137 | *α-*CH₃ | F | F | 2-Furyl | H | H | H | H | S | ≡ | 1 | 0 | 0 | 1 | OCOOCH-(CH₃)₂ | *S*-[4-(Isopropyloxycarbonyloxy)-but-2-ynyl] 6*α*,9*α*-difluoro-17*α*-[(2-furanyl-carbonyl)oxy]-11*β*-hydroxy-16*α*-methyl-3-oxoandrosta-1,4-diene-17*β*-carbothioate. |
| IB.138 | *α-*CH₃ | F | F | 2-Furyl | H | H | H | H | S | ≡ | 1 | 0 | 0 | 1 | OCOOCH₂ CH=C(CH₃)₂ | *S*-[4-(3-Methylbut-2-enoxycarbonyl-oxy)-but-2-ynyl]6*α*,9*α*-difluoro-17*α*-[(2-furanyl-carbonyl)oxy]-11*β*-hydroxy-16*α*-methyl-3-oxoandrosta-1,4-diene-17*β*-carbothioate. |
| IB.139 | *α-*CH₃ | F | F | 2-Furyl | H | H | H | H | S | ≡ | 1 | 0 | 0 | 1 | OCOO-(CH₂)₅CH₃ | *S*-[4-(*n*-Hexyloxycarbonyloxy)-but-2-ynyl] 6*α*,9*α*-difluoro-17*α*-[(2-furanylcarbonyl)-oxy]-11*β*-hydroxy-16*α*-methyl-3-oxoandrosta-1,4-diene-17*β*-carbothioate. |
| IB.140 | *α-*CH₃ | F | F | 2-Furyl | H | H | H | H | S | ≡ | 1 | 0 | 0 | 1 | OCOOCH₂-cyclohexyl | *S*-[4-(Cyclohexylmethyloxycarbonyl-oxy)-but-2-ynyl]6*α*,9*α*-difluoro-17*α*-[(2-furanyl-carbonyl)oxy]-11*β*-hydroxy-16*α*-methyl-3-oxoandrosta-1,4-diene-17*β*-carbothioate. |
| IB.141 | *α-*CH₃ | F | F | 2-Furyl | H | H | H | H | S | ≡ | 1 | 0 | 0 | 1 | OCOO-cyclohexyl | *S*-[4-(Cyclohexyloxycarbonyloxy)-but-2-ynyl] 6*α*,9*α*-difluoro-17*α*-[(2-furanyl-carbonyl)oxy]-11*β*-hydroxy-16*α*-methyl-3-oxoandrosta-1,4-diene-17*β*-carbothioate. |
| IB.142 | *α-*CH₃ | F | F | 2-Furyl | H | H | H | H | S | ≡ | 1 | 0 | 0 | 1 | OCOO-(CH₂)₆CH₃ | *S*-[4-(*n*-Nonanyloxycarbonyloxy]-but-2-ynyl] 6*α*,9*α*-difluoro-17*α*-[(2-furanyl-carbonyl)oxy]-11*β*-hydroxy-16*α*-methyl-3-oxoandosta-1,4-diene-17*β*-carbothioate. |
| IB.143 | *α-*CH₃ | F | F | 2-Furyl | H | H | H | H | S | ≡ | 1 | 0 | 0 | 1 | OCOOCH₂-C(CH₃)₃ | *S*-[4-(2,2-Dimethylpropyloxycarbonyl-oxy)-but-2-ynyl] 6*α*,9*α*-dfluoro-17*α*-[(2-furanylcarbonyl)oxy]-11*β*-hydroxy-16*α-*methyl-3-oxoandrosta-1,4-diene-17*β-*carbothioate. |
| IB.144 | *α-*CH₃ | F | F | 2-Furyl | H | H | H | H | S | ≡ | 1 | 0 | 0 | 1 | OCOOCH₂-cyclobutyl | *S*-[4-(Cyclobutylmethyoxycarbonyloxy)-but-2-ynyl] 6*α*,9*α*-difluoro-17*α*-[(2-furanylcarbonyl)oxy]-11*β*-hydroxy-16*α-*methyl-3-oxoandrosta-1,4-diene-17*β-*carbothioate. |
| IB.145 | *α-*CH3 | F | F | 2-Furyl | H | H | H | H | S | ≡ | 1 | 0 | 0 | 1 | OCOOCH₂-(furan-2-yl) | *S*-[4-(2-Methylfuryloxycarbonyloxy)-but-2-ynyl] 6*α*,9*α*-difluoro-17*α*-[(2-furanyl-carbonyl)oxy]-11*β*-hydroxy-16*α*-methyl-3-oxoandrosta-1,4-diene-17*β*-carbothioate. |
| IB.146 | *α-*CH₃ | F | F | 2-Furyl | H | H | H | H | S | ≡ | 1 | 0 | 0 | 1 | OCOO-(CH₂)₃CH₃ | *S*-[4-(*n*-Butyloxycarbonyloxy)-but-2-ynyl] 6*α*,9*α*-difluoro-17*α*-[(2-furanylcarbonyl)-oxy]-11*β*-hydroxy-16*α*-methyl-3-oxoandrosta-1,4-diene-17*β*-carbothioate. |
| IB.147 | *α-*CH₃ | F | F | 2-Furyl | H | H | H | H | S | ≡ | 1 | 0 | 0 | I | OCOO-(CH₂)₄CH₃ | *S*-[4-(*n*-Pentyloxycarbonyloxy)-but-2-ynyl] 6*α*,9*α*-difluoro-17*α*-[(2-furanyl-carbonyl)oxy]-11*β*-hydroxy-16*α*-methyl-3-oxoandrosta-1,4-diene-17*β*-carbothioate. |
| IB.148 | *α-*CH₃ | F | F | 2-Furyl | H | H | H | H | S | ≡ | 1 | 0 | 0 | 1 | 2.2-Dimethyl-3-fluoro-propionyl-oxy | *S*-[4-(2,2-Dimethyl-3-fluoro-propionyloxy)-but-2-ynyl]6*α*,9*α*-difluoro-17*α*-[(2-furanylcarbonyl)oxy]-11*β*-hydroxy-16*α-*methyl-3-oxoandrosta-1,4-diene-17*β-*carbothioate. |
| IB149 | *α-*CH₃ | F | F | 2-Furyl | H | H | H | H | S | ≡ | 1 | 0 | 0 | 1 | (*S*)-2,2-Dimethyl-1,3-dioxolan-4-yl-methoxy-carbonyloxy | *S*-[4-((*S*)-2,2-Dimethyl-1,3-dioxolan-4-yl-methoxycarbonyloxy)-but-2-ynyl] 6*α*,9*α-*difluoro-17*α*-[(2-furanylcarbonyl)oxy]-carbonyloxy-11*β*-hydroxy-16*α*-methyl-3-hydroxy-16*α*-methyl-3-oxoandrosta-1,4-diene-17*β*-carbothioate. |
| IB.150 | *α-*CH₃ | F | F | 2-Furyl | H | H | H | H | S | ≡ | 1 | 0 | 0 | 1 | (R)-2,2-Dimethyl-1,3-dioxolan-4-yl-methoxy-carbonyloxy | *S*-[4-((R)-2,2-Dimethyl-1,3-dioxolan-4-yl-methoxy-carbonyloxy)-but-2-ynyl] 6*α*,9*α-*difluoro-17*α*-[(2-furanylcarbonyl)oxy]-11*β-*hydroxy-16*α*-methyl-3-oxoandrosta-1,4-diene-17*β*-carbothioate. |
| IB.151 | *α-*CH₃ | F | F | 2-Furyl | H | FI | H | H | S | ≡ | 1 | 0 | 0 | 1 | (Morpholin-4-yl)-carbonyloxy | *S*-[4-(Morpholin-4-yl)carbonyloxy-but-2-ynyl]6*α*,9*α*-difluoro-17*α*-[(2-furanyl-carbonyl)oxy]-11*β*-hydroxy-16*α*-methyl-3-oxoandrosta-1,4-diene-17*β*-carbothioate. |
| IB.152 | *α-*CH₃ | F | F | 2-Furyl | H | H | H | H | S | ≡ | 1 | 0 | 0 | 1 | 2,2-Dimethyl-1,3-dioxolan-4-ylmethyl carbamoyl-oxy | *S*-[4-(2,2-Dimethyl-1,3-dioxolan-4-yl-methylcarbamoyloxy)-but-2-ynyl]6*α*,9*α-*difluoro-17*α*-[(2-furanylcarbonyl)oxy]-11*β-*hydroxy-16*α*-methyl-3-oxoandrosta-1,4-diene-17*β*-carbothioate. |
| IB.153 | *α-*CH₃ | F | F | 2-Furyl | H | H | H | H | S | ≡ | 1 | 0 | 0 | 1 | OCOO(CH₂)₂-cyclopentyl | *S*-[4-(2-Cyclopentylethoxycarbonyloxy)-but-2-ynyl] 6*α*,9*α*-difluoro-17*α*-[(2-furanylcarbonyl)oxy]-11*β*-hydroxy-16*α-*methyl-3-oxoandrosta-1,4-diene-17*β-*carbothioate. |
| IB.154 | *α-*CH₃ | F | F | 2-Furyl | H | H | H | H | S | ≡ | 1 | 0 | 0 | 1 | OCOO-(CH₂)₂ CH(CH₃)₂ | *S*-[4-(Isoamyloxycarbonyloxy)-but-2-ynyl] 6*α*,9*α*-difluoro-17*α*-[(2-furanylcarbonyl)-oxy]-11*β*-hydroxy-16*α*-methyl-3-oxoandrosta-1,4-diene-17*β*-carbothioate. |
| IB.155 | *α-*CH₃ | F | F | 2-Furyl | H | H | H | H | S | ≡ | 1 | 0 | 0 | 1 | OCO(CH₂)₃-ONO₂ | *S*-[4-(3-Nitroxypropylcarbonyloxy)-but-2-ynyl]6*α*,9*α*-difluoro-17*α*-[(2-furanyl-carbonyl)oxy]-11*β*-hydroxy-16*α*-methyl-3-oxoandrosta-1,4-diene-17*β*-carbothioate. |
| IB.156 | *α-*CH₃ | F | F | 2-Furyl | H | H | H | H | S | ≡ | I | 0 | 0 | 1 | 2.2.7.7-Tetramethyl tetrahydro-bis [1,3]dioxolo] 4,5-b;4',5'-d)pyran-5-yl methoxy carbonyloxy | *S*-[4-(2,2,7,7-Tetramethyltetrahydro-bis[1,3]dioxolo[4,5-b;4',5'-d]pyran-5-yl methoxycarbonyloxy)-but-2-ynyl] 6*α*,9*α-*difluoro-17*α*-[(2-furanylcarbonyl)oxy]-11*β-*hydroxy-16*α*-methyl-3-oxoandrosta-1,4-diene-17*β*-carbothioate. |
| IB.157 | *α-*CH₃ | F | F | 2-Furyl | H | H | H | H | S | ≡ | 1 | 0 | 0 | 1 | OCO(CH₂)₄,-ONO₂ | *S*-[4-(4-Nitroxybutylcarbonyloxy)-but-2-ynyl] 6*α*,9*α*-difluoro-17*α*-[(2-furanyl-carbonyl)oxy]-11*β*-hydroxy-16*α*-methyl-3-oxoandrosta-1,4-diene-17*β*-carbothioate. |
| IB.158 | *α-*CH₃ | F | F | 2-Furyl | H | H | H | H | S | ≡ | 1 | 0 | 0 | 1 | OCH₃ | *S*-(4-Methoxy-but-2-ynyl) 6*α*,9*α*-difluoro-17*α*-[(2-furanylcarbonyl)oxy]-11*β*-hydroxy-16*α*-methyl-3-oxoandrosta-1,4-diene-17*β-*carbothioate. |
| IB.159 | *α-*CH₃ | F | F | 2-Furyl | H | H | H | H | S | ≡ | 1 | 0 | 0 | 1 | OCH₂CH₃ | *S*-(4-Ethoxy-but-2-ynyl) 6*α*,9*α*-difluoro-17*α*-[(2-furanylcarbonyl)oxy]-11*β*-hydroxy-16*α*-methyl-3-oxoandrosta-1,4-diene-17*β-*carbothioate. |
| IB.160 | *α-*CH₃ | F | F | 2-Furyl | H | H | H | H | S | ≡ | 1 | 0 | 0 | 1 | OCH₂CH-(CH₃)₂ | *S*-[4-Isobutoxy-but-2-ynyl] 6*α*,9*α*-difluoro-17*α*-[(2-furanylcarbonyl)oxy]-11*β*-hydroxy-16*α*-methyl-3-oxoandrosta-1,4-diene-17*β-*carbothioate. |

### Table 5- Lists the compound of formula I-C which have been synthesized

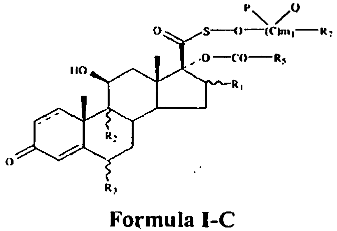

**Table-5**

| **Sr. No** | **R₁** | **R₂** | **R₃** | **R₅** | **m₁** | **R₁** | **P** | **Q** | **Chemical Name** |
|---|---|---|---|---|---|---|---|---|---|
| IC.1 | *α-*CH₃ | F | F | Ethyl | 1 | 4-Fluoro-phenyl | H | H | 6*α,*9*α*-Difluoro-11*β*-hydroxy-16*α*-methyl-3-oxo-17*α-*propionyloxyandrosta-1,4-diene-17*β*-carbonylsulfenic acid (4-fluorophenyl)methyl ester. |
| IC.2 | *α-*CH₃ | F | F | Ethyl | 1 | 3,4-Dichloro-phenyl | H | H | 6*α*,9*α*-Difluoro-11*β*-hydroxy-16*α*-methyl-3-oxo-17*α-*propionyloxyandrosta-1,4-diene-17*β*-carbonylsulfenic acid (3,4-dichlorophenyl)methyl ester. |
| IC.3 | *α-*CH₃ | F | F | Ethyl | 1 | F | H | H | 6*α*,9*α*-Difluoro-11*β*-hydroxy-16*α*-methyl-3-oxo-17*α-*propionyloxyandrosta-1,4-diene-17*β*-carbonylsulfenic acid fluoromethyl ester. |
| IC.4 | *α-*CH₃ | F | F | 2-Furyl | 1 | F | H | H | 6*α,*9*α*-Difluoro-17*α-*[(2-furanylcarbonyl)oxy]-11*β*-hydroxy-16*α-*methyl-3-oxoandrosta-1,4-diene-17*β*-carbonylsulfenic acid fluoromethyl ester. |
| IC.5 | *α-*CH₃ | F | F | 2-Furyl | 1 | 4-Fluoro-phenyl | H | H | 6*α*,9*α*-Difluoro-17*α*-[(2-furanylcarbonyl)oxy]-11*β*-hydroxy-16*α-*methyl-3-oxoandrosta-1,4-diene-17*β-*carbonylsulfenic acid (4-fluorophenyl)methyl ester. |
| IC.6 | *α-*CH, | F | F | 2- . Furyl | 1 | 4-Trifluoro-methylphenyl | H | H | 6*α*,9*α*-Difluoro-17*α*-[(2-furanylcarbonyl)oxy]-11*β*-hydroxy-16*α-*methyl-3-oxoandrosta-1,4-diene-17*β*-carbonylsulfenic acid (4. trifluoromethylphenyl)methyl ester. |
| IC.7 | *α-*CH₃ | F | F | 2-Furyl | 1 | 4-chloro phenyl | H | H | 6*α,*9*α*-Difluoro-17*α-*[(2-furanylcarbonyl)oxy]-11*β*-hydroxy-16*α-*methyl-3-oxoandrosta-1,4-diene-17*β*-carbonylsulfenic acid (4-chlorophenyl)methyl ester. |
| IC.8 | *α-*CH₃ | F | F | Ethyl | 1 | 4-Trifluoro-methylphenyl | H | H | 6*α*,9*α*-Difluoro-11*β*-hydroxy-16*α-*methyl-3-oxo-17*α-* propionyloxyandrosta-1,4-diene-17*β*-carbonylsulfenic acid (4-trifluoromethylphenyl)methyl ester. |
| IC.9 | *α-*CH₃ | F | H | 2-Furyl | 1 | 4-Fluorophenyl | H | H | 9*α-*Fluoro-17*α-*[(2-furanylcarbonyl)oxy]-1*β*-hydroxy-16*α-*methyl-3-oxoandrosta-1,4-diene-17*β*-carbonylsulfenic acid (4-fluorophenylmethyl ester. |
| IC.10 | *α-*CH₃ | F | F | Ethyl | 1 | 4-Chlorophenyl-carbonyl | H | H | 6*α*,9*α*-Difluoro-11*β*-hydroxy-16*α-*methyl-3-oxo-17*α-*propionyloxyandrosta-1,4-diene-17*β*-carbonylsulfenic acid [2-(4-chlorophenyl)-2-oxoethyl] ester. |
| IC.11 | *α-*CH₃ | F | H | 2-Furyl | 1 | F | H | H | 9*α-*Fluoro-17*α-*[(2-furanylcarbonyl)oxy]-11*β*-hydroxy-16*α-*methyl-3-oxoandrosta-1,4-diene-17*β*-carbonylsulfenic acid fluoromethyl ester. |
| IC.12 | *α-*CH₃ | F | F | *n-*C₄H₉ | 1 | F | H. | H | 17*α*-*n*-Butylcarbonyloxy-6*α-*9*α-*difluoro-11*β*-hydroxy-16*α*-methyl-3-oxoandrosta-1,4-diene-17*β*-carbonylsulfenic acid fluoromethyl ester |
| IC.13 | *α-*CH₃ | F | F | Ethyl | 1 | H | H | H | 6*α*,9*α-*Difluoro-11*β*-hydroxy-16*α-*methyl-3-oxo-17*α-*propionyloxyandrosta-1,4-diene-17*β*-carbonylsulfenic acid methyl ester. |
| IC.14 | *α-*CH₃ | F | F | 2-Furyl | 1 | H | H | H | 6*α*,9α*-*Difluoro-17*α-*[(2-furanylcarbonyl)oxy]-11*β*-hydroxy-16*α-*methyl-3-oxoandrosta-1,4-diene-17*β*-carbonylsulfenic acid methyl ester. |
| IC. 15 | *α-*CH₃ | F | F | Ethyl | 1 | Phenyl | H | H | 6*α,*9*α-*Difluoro-11*β*-hydroxy-16*α-*methyl-3-oxo-17*α-*propionyloxyandrosta-1,4-diene-17*β*-carbonylsulfenic acid phenylmethyl ester. |
| IC.16 | *α-*CH₃ | F | F | 2-Furyl | 1 | Phenyl | H | H | 6*α*,9*α*-Difluoro-17*α*-[(2-furanylcarbonyl)oxy]-11*β*-hydroxy-16*α-*methyl-3-oxomedrosta-1,4-diene-17*β*-carbonylsulfenic acid phenylmethyl ester. |
| IC. 17 | *α-*CH₃ | F | F | Ethyl | 1 | 2,4,4-Tri-methylphenyl-carbonyl | H | H | 6*α*,9*α*-Difluro-11*β*-hydroxy-16*α*-methyl-3-oxy-17*α-*propionyloxyandrosta-1,4-carbonylsulfenic acid [2-(2,4,6-trimethylphenyl)-2-oxoethyl ester. |
| IC.18 | *α-*CH₃ | F | F | Ethyl | 1 | 4-Chloro-phenyl | H | H | 6*α*,9*α*-Difluoro-11*β*-hydroxy-16*α*-methyl-3-oxo-17*α-*propionyloxyandrosta-1,4-diene-17*β*-carbonylsulfenic acid (4-chlorophenyl)methyl ester. |
| IC.19 | *α-*CH₃ | F | F | 2-Furyl | 1 | 3,4-Dichloro-phenyl | H | H | 6*α*,9*α*-Difluoro-17*α*-[(2-furanylcarbonyl)oxy]-11*β*-hydroxy-16*α-*methyl-3-oxoandrosta-1,4-diene-17*β*-carbonylsulfenic acid (3,4-dichlorophenyl)methyl ester. |
| IC.20 | *α-*CH₃ | F | F | 2-Furyl | 1 | 2,4-Dichloro-phenyl | H | H | 6*α*,9*α*-Difluro-17*α*-[(2-furanycarbonyl)oxy]-11*β*-hydroxy 16*α-*methyl-3-oxoandrosta-1,4-diene-17*β*-carbonylsulfenic acid (2,4-dichlorophenyl)methyl ester |
| IC.21 | *α-*CH₃ | F | F | 2-Furyl | 1 | 3-Chloro-phenyl | H | H | 6*α*,9*α*-Difluoro-17*α*-[(2-furanylcarbonyl)oxy]-11*β*-hydroxy-16*α*-methyl-3-oxoandrosta-1,4-diene-17*β*-carbonylsulfenic acid (3-chlorophenyl)methyl ester. |
| IC.2*2* | *α-*CH₃ | F | F | 2-Furyl | 1 | 2,4,6-Trifluoro-phenyl | H | H | 6*α,*9*α-*Dichloro-17*α-*[(2-furanylcarbonyl)-oxy]-11*β*-hydroxy-16*α-*methyl-3-oxoandrosta-1,4-diene-17*β*-carbonylsulfenic acid (2,4,6-trifluorophenyl)methyl ester. |
| IC.23 | *α-*CH₃ | F | F | 2-Furyl | 1 | 3-(Trifluoromethyl) phenyl | H | H | 6*α*,9*α-*Dichloro-17*α-*[(2-furanylcarbony)oxy]-11*β*-hydroxy-16*α-*methyl-3-oxoandrosta-1,4-diene-17*β*-carbonylsulfenic acid (3-trifluoromethylphenyl)methyl ester. |

### Table-6: Spectral data for the synthesized compounds

| **Comp. No** | **¹H-NMR (δ ppm), MASS (EI / ES+, *m*/*z*)** |
|---|---|
| IA.1 | 1.19 (s, 3H), 1.29 (t, 3H, *J*=7.1Hz), 1.45 (s, 3H), 1.5-1.54 (m, 1H), 1.66-1.73 (m, 3H), 1.81-1.84 (m, 1H), 1.95-2.24 (m, 6H), 1.32-2.36 (m, 1H), 2.56-2.62 (m, 1H), 2.9-3.0 (m, 1H), 4.1-4.2 (q, 2h, *J*=7.1), 4.51 (br, d, 1H, *J*=2.51 Hz), 5.07 (d, 1H *j*=16.4Hz). 5.62 (d, 1H. *J*=16.4Hz), 6.17 (s, 1H), 6.27 (dd, 1H, *J*₁=10.1Hz. *J*₂=1.8Hz); 7.27 (d, 1H, *J*=10.4Hz). 7.47 (d, 2H. *J*=6.9Hz), 7.84 (d, 2H, *J*=6,9Hz) **MASS (ES+):** 592.9(M+Na)⁺ |
| IA.2 | 0.96 (d, 3H, *J*=7.16Hz), 1.11-1.15 (m, 3H), 1.24 (s, 3H), 1.55 (s, 3H), 1.67 (s, 2H), 1.71-1.89 (m, 3H), 2.25 -2.40 (m, 6H), 3.3-3.4 (m, 1H), 4.42 (br, 1H), 4.95 (d. 1H, *J*=16.6Hz), 5.69 (d, 1H. *J*=16.6Hz), 5.3-5.37 & 5.43-5.49 (m, 1H, 6.38 (dd, 1H, *J*₁=10.14Hz. *J*₂=1.8Hz ). 6.45 (s, 1H), 7.16 (dd, 1H, *J*₁=10. 1Hz, *J*₂=1.8 Hz), 7.49 (d, 2H, *J* = 8.8Hz), 7.56 (d, 2H, *J*=8.6Hz), **MASS (ES+):** (627.0(M+Na) |
| IA.3 | 0.99 (d, 3H, *J*=7.14Hz), 1.11-1.14 (m, 6H), 1.53 (s, 3H), 1.62 (s, 2H), 1.7-1.91 (m, 4H), 2.21-2.29 (m, 2H), 2.34-2.38 (m, 4H 3.35-3.42 (m, 1H). 4.3 (d, 1H, *J*=16.5Hz), 4.41 (br, 1H), 4.48 (d. 1H, *J*=16.5Hz), 5.30-5.35 & 5.42-5.47 (m, 1H), (6.38 (dd, 1H, *J*₁=10.15Hz, *J*₂=175Hz), 6.43 (s, 1H), 7.12 (d, 1H, *J*=10.15Hz), 7.47 (d, 2H, *J*=8.5Hz), 7.9 (d. 2H, *J*=8.5Hz). MASS (ES+): 643.1(M+Na) |
| IA.4 | 1.16 (d, 1H, *J*=3.30Hz), 1.20 (s, 3H). 1.29 (t, 3H. *J=* 7.1 0Hz), 1.48 (s, 3H). 1.54 (m. 2H). 1.70 (m, 1H), 1.83 (m, 2H), 1.96-2.22 (m, 5*H*), 2.34 (m, 1H), 2.59 (m, 1H), 2.95 (m. 1H), 4.16 (q, 2H, *J*=7.00Hz), 4.52 (br, 1H), 5.08 (d, 1H, *J*=16.32Hz), 5.63 (d, 1H, *J*=16.32Hz), 6.02 (s, 1H), 6.27 (dd, 1H, *J*₁=10.59Hz, *J*₂=1.67Hz), 7.17 (dd, 2H, *J*₁=*J*₂=8.55Hz), 7.28 (1H, *J*=10.19Hz), 7.94 (dd, 2h, *J*₁=8.76. *J*₂=5.31). **MASS (EI):** 554 |
| IA.5 | 0.96 (s, 3H), 1.13 (t, 3H, *J*=7.55Hz), 1.24(s, 3H), 1.30 (m, 1H), 1.55 (s, 3H), 1.82 (m, 2H), 2.05 (m, 2H), 2.20 (br, 1H), 2.25-2.50 (m, 6H), 3.35 (m, 1H), 4.41 (d. 1H. *J*= 8.52Hz), 5.00 (d, 1h, *J*= 16.53), 5.33 & 5.46 (m, 1H), 5.69 (d, 1H, *J*=16.53Hz), 6.39 (1H, dd, *J*₁=10.15Hz, *J*₂=1.64Hz), 6.44 (s, 1H), 7.18 (dd, 2H, *J*₁=*J*₂=8.50Hz), 7.93 (dd, 2H, *J*₁=8.70Hz, J₂=5.30Hz), **MASS (ES+):** 611.1(M+Na) |
| IA.6 | 0.99 (d, 3H, *J*=7.15Hz), 1.13 (m, 6H), 1.25 (s, 1H), 1.33 (m, 1H). 1.53 (s, 3H), 1.86 (m, 3H), 2.26 (m, 2H), 2.39 (m, 4H), 3.39 (m, 1H), 4.32 (d, 1H, *J*=16.48Hz), 4.41 (d, 1H, *J*=8.92Hz), 4.48 (d, 1H, *J*=16.49Hz), 5.32 & 5.44 (m, 1H). 6.38 (dd, 1H, *J*₁=10.15Hz, *J*₂=1.69Hz), 6.44 (s, 1H), 7.12 (dd, 1H, *J*₁=10.19Hz, *J*₂=0.95Hz), 7.17 (dd, 2H, J₁=J₂=8.50Hz), 8.05 (dd, 2H,. *J*₁=10.26Hz. *J*₂=5.35Hz). **MASS (E1+):** 627.1(M+Na)⁺. |
| IA.7 | 1.18 (dd, 2H, *J*₁=11.44Hz, *J*₂=3.21Hz), 1.21 (s, 3H), 1.29 (t, 3H, *J*=7.11Hz), 1.48 (s, 3H), 1.52 (m, 1H), 1.66-1.84 (m, 2H), 1.95-2.22 (m, 6H), 2.32-2.36 (m, 1H), 2.91-2.98 (m, 1H), 4.16 (q, 2H, *J*=7.14Hz), 4.52 (s, 1H), 5.00 (dd, 1H. *J*₁=17.35Hz, *J*₂=Hz3.61). 5.48 (dd, 1H. *J*₁=17.35Hz, *J*₂=3.61 Hz), 6.02 (s, 1H), 6.22 (dd, 1H, *J*₁10.09Hz, *J*₂=1.73Hz), 6.92 (t, 1H, *J*=6.67Hz), 7.00 (1H, *J*=6.67Hz), 7.29 (1H. d, *J*=10.15H₂). 8.01 (dd, 1H, *J*₁=8.25Hz, *J*₂=6.75Hz), **MASS (ES+):** 595.1 (M+Na)⁺. |
| IA.8 | 1.13 (s, 3H), 1.16 (dd, 2H, *J*₁=11.37Hz, *J*₂=3.2Hz), 1.28 (t, 3H, *J*=7.13Hz), 1.47 (s, 3H), 1.50-1.52 (m, 1H), 1.64-168 (m, 1H) 1.80-1.83 (m, 1H), 1.91-2.20 (m, 6H), 2.33 (dd, 1H, *J*₁=13.36Hz, *J*₂=2.8Hz), 2.53-2.61 (m, 1H), 2.88-2.95 (m, 1H, 4.14 (q, 2H, *J*=7.12Hz, 4.51 (s, 1H), 5.00 (d, 1H, *J*=16.75Hz). 5.41 (d, 1H, *J*=16.75Hz, 6.02 (s, 1H), 6.26 (dd, 1H, *J*₁=10.01. *J*₂=1.78Hz), 7.27 (d, 1H, *J*=10.09Hz), 7.36 (dd, 1H, *J*₁=8.38Hz, *J*₂=1.93Hz), 7.47 (d, 1H, *J*=1.89Hz), 7.62 (d, 1H, *J*=8.37Hz), **MASS (ES+):** 627.0(M+Na)⁺. |
| IA.9 | 0.96 (d, 3H, *J*=7.14Hz), 1.14 (t, 3H, *J*=7.57Hz), 1.26 (s, 3H), 1.29-1.34 (m, 3H), 1.56 (s, 3H), 1.71-1.88 (m, 4H), 2.02-2.05 (m, 1H), 2.25-2,50 (m, 6H), 3.33-3.38 (m, 1H), 4.42 (d, 1H, *J*=8.87Hz), 4.92 (dd, 1H, *J*=17.59Hz, *J*₂=3.07Hz), 5.35 & 5.46 (m, 1H), 5.56 (dd, 1H, *J*=17.59Hz, *J*₂=3.07Hz), 6.38 ((dd, 1H, *J*₁=10.14Hz, *J*₂=1.70Hz), 6.44 (s, 1H), 6.90-7.04 (m, 1H), 7.15 (d, 1H, *J*=10.12Hz), 8.00 (dd, 1H, *J*₁=15.04Hz, J₂=8.43Hz), **MASS (ES+):** 629.0(M+Na)⁺. |
| IA.10 | 0.94 (d, 3H, *J*=6.91Hz), 1.12 (t, 3H, *J*=7.51Hz), 1.18 (s, 3H), 1.24-1.32 (m, 2H), 1.55 (s, 3H), 1.71-1.96 (m, 4H). 2.27-2.48 (m, 5H), 3.32 (s, 1H, 4.40 (d, 1H, *J*=7.18Hz), 4.90 (d, 1H, *J*=16.85Hz), 5.33 (m, 0.5H), 5.46 (d, 1.5H, *J*=16.87Hz), 6.38 (d, 1H, *J*=10.07Hz), 6.44 (s, 1H), 7.15 (d, 1H, *J*=9.95Hz), 7.37 (d, 1H, *J*=8.30Hz), 7.49 (s, 1H), 7.64 (d, 1H, *J*=8.33) **MASS (ES+):** 661.0 (M+Na)⁺. |
| IA.11 | 0.98 (d, 3H, *J*=7.13Hz), 1.10-1.13 (m, 6H), 1.26-1.35 (m, 2H), 1.54 (s, 3H), 1.72-2.01 (m, 4H), 2.23-2.46) (m, 5H), 3.37(m), 1H), 4.92 (dd, 1H, *J*₁=17.43Hz, *J*₂=2.69Hz), 4.43 (dd, 2H, *J*₁=17.44Hz_{,} *J*₂=2.76Hz), 5.33 & 5.45 (m, 1H), 6.37 (dd, 1H, *J*₁=10.13Hz, *J*₂=1.73Hz), 6.43(s, 1H), 6.89-701 (m, 2H), 7.16 (dd, 1H, *J*₁=10.14Hz, *J*₂=1.05Hz), 7.94 (dd, 1H, *J*₁=15.09hz, *J*₂=8.50Hz), **MASS (ES+):** 645.0 (M+Na)⁺ |
| IA.12 | 1.1.5 (d, 1H, *J*=3.26Hz), 1.17 (s, 4H), 1.29 (t, 3H, *J*=7.13Hz), 1.48 (s, 3H), 1.51-1.54 (m, 1H), 1.66-1.73 (m, 2H), 1.81-1.84 (m, 1H), 1.95-2.22 (m, 5H), 2.34 (dd, 1H, *J*₁=13.4 1Hz, *J*₂=2.71 Hz), 2.56-2.62 (m, 1H), 2.90-2.97 (m, 1H), 4.15 (q, 2H, *J*=7.15Hz), 4.52 (s, 1H), 5.06 (d, 1H, *J*=16.43Hz), 5.56 (d, 1H, *J*=16.43Hz), 6.02 (s, 1H), 6.27 (dd, 1H, *J*₁=10.08Hz, *J*₂=1.7 Hz), 7.29 (d, 1H, *J*=10-08Hz), 7.59 (d, 1H, *J*=8.34Hz), 7.73 (dd, 1H, *J*₁=8.35Hz, *J*₂=1.92Hz), 7.99(d, 1H, *J*=1.88Hz), **MASS (ES+)**: 627.0(M+Na) |
| IA.13 | 0.97 (d, 3H, *J*=7.16Hz), 1.05 (s, 3H), 1.12 (t, 3H, *J*=7.58Hz), 1.28-1.35 (m, 2H), 1.53 (s. 3H). 1.72-1.94 (m, 3H), 2.24-2.42 (m, 6H), 3.35 (m, 1H), 4.21 (d, 1H, *J*=16.84Hz), 4.34 (d, 1H, *J*=6.84Hz), 5.32 & 5.44 (m, 1 H), 6.38 (dd, 1H, *J*₁=10.14Hz, *J*₂=1.78Hz), 6.43 (s, 1H), 7.11 (dd, 1H, *J*₁=10.12Hz, *J*₂=1.22Hz), 7.35 (dd, 1H, *J*₁=8.34Hz, *J*₂=1.92Hz), 7.46 (d, 1H, *J*=8.33Hz), **MASS (ES+):** 677.0(M+Na)⁺. |
| IA.14 | 0.99 (3H, d, *J*=7.10Hz), 1.10 (s, 3H), 1.12 (t, 3H, *J*=7.57Hz), 1.33 (m, 1H), 1.53 (s, 3H), 1.73-1.97 (m, 5H), 2.20-2.46 (m, 5H), 3.37 (m, 1H), 4.28 (d, 2H, *J*=16.38Hz), 4.41 (d, 2H, *J*=15.93Hz), 5.32 & 5.44 (m, 1H), 6.36 (d, 1H, *J*=10.14Hz), Hz), 6.43 (s, 1H), 7.13(d, 1H, *J*=10.14HZ), 7.58 (d, 1H, *J*= 8.35Hz), 7.83 (dd, 1H, *J*₁=8.23Hz, *J*₂=1.66Hz), 8.08 (d, 1Hz), **MASS (ES+):** 6.77.0 (M+Na)⁺ |
| IA.15 | 0.96 (d, 3H, *J*=7.10Hz), 1.13 (t, 3H, *J*=7.56Hz), 1.22 (s, 3H), 1.26-1.33 (m, 2H), 1.55 (s, 3H), 1.71-1.90 (m, 3H), 2.00 (d, 1H, *J*=14.88Hz), 2.27-2.50 (m, 5H), 3.34 (m, 1H), 4.41 (d, 1H, *J*=8.73Hz), 4.96 (d, 1H, *J*=16.66 Hz), 5.33 & 5.45 (m, 1H), 5.64 (d, 1H, *J*=16.59Hz), 6.38 (d, 1H, *J*=10.14Hz), 6.44 (s, 1H), 7.14 (d, 1H, *J*=10.14Hz), 7.59 (d, 1H, *J*=8.35Hz), 8.01 (s, 1H), |
| IA.16 | 1.15 (m, 2H), 1.19 (s, 3H), 1.29 (t, 3H, *J*=7.13Hz), 1.61 (s, 3H), 1.66-1.84 (m, 2H). 1.95-2.22 (m, 6H), 2.32-2.36 (m, 1H), 2.55-2.63 (m, 2H), 2.92-2.98 (m, 1H), 4.15 (q, 2H, *J*=7.04Hz), 4.51 (br, 1H), 5.00 (d, 1H, *J*=16.00Hz), 5.58 (d, 1H, *J*=16.00Hz), 6.02 (s, 1H), 6.27 (dd, 1H. *J*₁=10.09Hz, *J*₂=1.74Hz), 7.1 8 (t, 1H, *J*=3.94Hz), 7.29 (s, 1H). 7.72 (d, 1H, *J*=4.92Hz), 7.75 (d, 1H, *J*=3.82Hz), **MASS (ES+):** 565.1 (M+Na)⁺. |
| IA.17 | 1.16 (s, 3H), 1.19 (m, 2H), 1.28 (t, 3H, *J*=7.13Hz), 1.45 (m, 2H), 1.47 (s, 3H), 1.67-2.38 (m, 6H) 2.51-2.67 (m, 1H), 2.88-3.00 (m, 1H), 4.14 (q, 2H, *J*₁,=14.24Hz, *J*₂=7.11Hz), 4.51 (br, 1H), 4.94 (d, 1H, *J*=15.95Hz), 5.49 (d, 1H, *J*=15.98Hz), 6.02 (s, 1H), 6.27 (dd, 1H, *J*₁=10.07Hz, *J*₂=1.72Hz), 7.00 (d, 1H, *J*=4.08Hz), 7.27 (d, 1H, *J*=9.96Hz), 7.54 (d, 1H, *J*=4.09Hz), **MASS (ES+):** 599.0(M+Na)⁺. |
| IA.18 | 0.95 (d, 3H, *J*=7.14Hz), 1.13 (t, 3H, *J*=7.56Hz), 1.20 (s, 3H), 1.26-1.36 (m, 1H, 1.54 (s, 3H), 1.77-2.04 (m, 4H), 2.25-2.43 (m, 6H), 3.30-3.37 (m, 1H), 4.40 (d, 1H, *J*=9.64Hz), 4.85 (d, 1H, *J*=16.19Hz), 5.28 & 5.51 (m, 1H), 5.56 (d, 1H, *J*=16.17Hz), 6.33-4.44 (m, 2H), 7.00 (d, 1H, *J*=4.09Hz), 7.14 (d, 1H, *J*= 10.02 Hz), 7.54 (d, 1H, *J*=4.08Hz), **MASS (ES+):** 633.0(M+Na)⁺. |
| IA.19 | 1.17(m, 2H), 1.18 (s, 3H), 1.28 (t, 3H, *J*=7.11Hz), 1.47 (s, 5H), 1.70-2.38 (m, 8H), 2.41 (s, 3H), 2.51-2.66 (m, 1H), 2.88-3.01 (m, 1H), 4.15 (q, 2H, *J*₁=14.21 Hz, *J*₂=7.09Hz), 4.49 (br, 1H), 4.89 (d. 1H, *J*= 16.20Hz), 5.49 (d, 1 H, *J*=16.21Hz), 6.02 (s, 1H), 6.21 (d, 1H, *J*=3.51Hz), 6.27 (dd, 1H, *J*₁=10.11Hz, *J*₂= 1.72Hz), 7.19 (d, 1H, *J*=3.46Hz), 7.28 (d, 1H, *J*=9.26Hz). **MASS (EI):** 540 |
| IA.20 | 0.99 (d, 3H, *J*=7.35Hz), 1.10-1.16 (m, 6H), 1.21-1.38 (m, 1H), 1.53 (s, 3H), 1.69-2.02 (m, 4H, 2.16-2.49 (m, 6H), 3.34-3.46 (m, 1H), 4.17 (d, 1H, *J*=16.03Hz), 4.35 (d, 1H, *J*=16.01Hz), 4.43 (br, 1H), 5.28 & 5.51 (m, 1H), 6.37 (d, 1H, *J*=10.33Hz), 6.99 (d, 1H, *J*=4.03Hz), 7.16 (d, 1H, *J*=(10.03hz), 7.65 (d, 1H, *J*=4.04Hz). **MASS (ES+):** 649.0 (M+Na)⁺. |
| IA.21 | 0.95 (d, 3H, *J*=7.13Hz), 1.13 (t, 3H, *J*=7.56Hz), 1.22 (s, 3H), 1.54 (s, 3H), 1.70-1.88 (m, 2H), 2.03-2.08 (m, 1H), 2.25-2.51 (m, 8H), 2.40 (s, 3H), 3.30-3.38 (m, 1H), 4.39 (d, 1H, *J*=8.28Hz), 4.80 (d, 1H, *J*=16.31Hz), 5.28 & 5.50 (m, 1H), 5.52 (d, 1H, *J*=16.33Hz), (6.21 (d, 1H, *J*=2.99Hz), 6.37 (dd, 1H, *J*₁=10.12 Hz, *J*₂=1.77Hz), 6.41 (s, 1H), 7.15-7.20 (m, 2H), **MASS (EI):** 574 |
| IA.22 | 0.98 (d. 3H, *J*=7.13Hz), 1.09-1.16 (m, 6H), 1.22-1.37 (m, 1H), 1.53 (s, 3H), 1.69-2.56 (m, 10H, 2.41 (s, 3H), 3.36-3.43 (m, 1H), 4.12 (d, 1H, *J*= 16.31Hz), 4.33 (d, 1H, *J*=16.33 Hz), 4.43 (br, 1H), 5.28 & 5.52 (m, 1H), 6.21 (d. 1H, *J*=3.12Hz), 6.34-6.43 (m, 2H), 7.18 (dd, 1H, *J*₁=10.04Hz, *J*₂=1.03Hz), 7.27 (d, 1H, *J*=3.60Hz), **MASS (ES+):** 613.1(M+Na)⁺. |
| IA.23 | 1.17 (s, 3H), 1.20 (s, 1H), 1.21 (s, 9H), 1.28 (t, 3H, *J*=7.12Hz), 1.43 (s, 1H), 1.47 (s, 1H), 163-2.97 (m. 12H), 4.14 (q. 2H. *J*₁=14.26Hz. *J*₁=7.12Hz), 4.49 (br, 1H), 4.63 (d, 1H. *J*=16.68Hz), 5.23 (d, 1H, *J*=16.69Hz), 6.01 (s, 1H), 6.27 (dd, 1H, *J*₁=10010Hz, *J*₂=1.84Hz), 7.27 (d, 1H, *J*=9.73Hz), **MASS (EI):** 516 |
| IA.24 | 1.13 (s, 3H), 1.18-1.25 (m, 3H), 1.28 (t, 3H, *J*=7.12Hz), 1.47 (s, 3H), 1.55-2.16 (m, 8H), 2.18 (s, 3H), 2.36-2.96 (m, 3H), 4.14 (q, 2H, *J*=7.13Hz), 4.50 (d, 1H, *J*=16.83Hz), 4.51 (m, 1H), 4.92 (d, 1H, *J*=16.84 Hz), 6.02 (s, 1H), 6.26 (dd. 1H, *J*₁=10.09Hz, *J*₂=1.82Hz), 7.28 (d, 1H, *J*=10.10Hz). **MASS (EI):** 474 |
| IA.25 | 0.94 (d, 3H, *J*=7.17Hz), 1.12 (t, 3H, *J*=7.56Hz), 1.21 (s, 12H), 1.26-1.29 (m, 1 H), 1.54 (s, 3H), 1.77-2.41 (m, 10H), 3.28-3.35 (m, 1H), 4.38 (br, 1H), 4.54 (d, 1H, *J*=16.98Hz), 5.28 & 5:52 (m, 1H), 5.30 (d, 1H, *J*=16.91Hz), 6.38 (dd, 1H, *J*₁=10.10Hz, *J*₂=1.73Hz), 6.43 (s, 1H). 7.17 (dd, 1H, *J*₁=10.05 Hz, J₂=1.10Hz), **MASS (ES+):** 573.2 (M+Na)⁺. |
| IA.26 | 0.98 (d, 3H, *J*=7.12Hz), 1.12 (s, 3H), 1.12 (t, 3H, *J*=7.54Hz), 1.24 (s, 9H), 1.23-1.34 (m, 1H), 1.54 (s, 3H), 1.75-250 (m, 10H), 3.34-3.42 (m, 1H), 3.89 (d, 1H, *J*=17.40Hz), 4.14 (d, 1H, *J*=17.38Hz), 4.38-4.42 (br, 1H), 5.28 & 5.52 (m, 1H), 6.37 (dd, 1H, *J*₁=10.20Hz. J₂=1.64Hz), 6.43 (s, 1H), 7.17 (d, 1H, *J*=9.96 Hz), **MASS (ES+):** 589.1 (M+Na)⁺. |
| IA.27 | 0.99 (d, 3H, *J*=7.13Hz), 1.10 (s, 3H), 1.12 (t, 3H, *J*=7.55Hz), 1.20-1.39 (m, 2H), 1.53 (s, 3H), 1.69-1.97 (m, 4H), 2.22-2.42 (m, 8H), 3.33-3.40 (m, 1H), 3.72 (d, 1H, *J*=16.52Hz), 3.83 (d, 1H, *J*=16.51Hz), 4.40 (br, 1H), 5.28 & 5.50 (m, 1H), 6.38 (dd, 1H, *J*₁=10.08Hz, *J*₂=1.28Hz), 6.43 (s, 1H), 7.13 (dd, 1H, *J*₁=10.08 Hz, *J*₂=1.37Hz), **MASS (ES+):** 547.1(M+Na)⁺. |
| IA.28 | 0.94 (d, 3H, *J*=7.12Hz), 1.12 (t, 3H, *J*=7.58Hz), 1.17 (s, 3H), 1.24-1.35 (m, 1H), 1.55 (s, 3H). 1.70-1.95 (m, 3H), 2.18 (s, 3H), 2.24-2.42 (m, 7H), 3.30 (m, 1H), 4.39 (br, 1H), 4.43 (d, 1H, *J*=17.04Hz), 4.96 (d, 1H, *J*=17.04Hz), 5.28 & 5.52 (m, 1H), 6.37 (dd, 1H, *J*₁=10.10Hz, *J*₂=1.79Hz), 6.42 (s, 1H), 7.17 (dd, 1H, *J*₁=10.07Hz, *J*₂=1.Hz). **MASS (ES+):** 531.1 (M+Na)*. |
| IA.29 | 1.15(d, *J*=3.312Hz, 3H), 1.21(s, 3H), 1.29(t, *J*=7.08, 3H), 1.48(s, 3H), 1.71-2.38(m, 6H), 2.58(m, 1H), 2.97(m, 1H), 3.89(s, 3H), 4.16(q, *J*=7.07Hz, 2H), 4.51(d, 1H), 5.05(d, *J*=16.2Hz, 1H), 5.60(d, *J*=16.2Hz, 1H), 6.02(s, 1H), 6.27(dd, *J*₁=10.11Hz, *J*₂=1.83Hz, 1H), 6.96(d, *J*=8.9Hz, 2H), 7.28(d, *J*=10.3Hz, 1H), 7.88(d, *J*=8.9Hz, 2H). |
| IA.30 | 0.96(d, *J*=7.1Hz 3H), 1.13(t, *J*=7.5Hz 3H), 1.25(s, 3H), 1.55(s, 3H), 1.70.1.88(m, 2H), 2.08(d, *J*=15.9Hz, 1H), 2.26-2.46(m, 7H), 2.56(m, 1H), 3.34(m, 1H), 3.38(s, 3H), 4.40(d, *J*=8.63Hz, 1H), 4.97(d, *J*=16.4Hz, 1H), 5.42(m, 1H), 5.70(d, *J*=16.42, Hz, 1H), 6.37(dd, *J*₁=10.1Hz *J*₂=1.76Hz, 1H), 6.43(s, 1H), 6.95(d, *J*=10.9Hz, 2H), 7.175(dd, J₁=1.03Hz, *J*₂=1.03Hz, 1H), 7.88(d *J*=8.89Hz, 1H). **MASS (ES+)**: 623.1(M+Na)⁺. |
| IA.31 | 0.98(d, *J*=6.2Hz, 3H), 1.11(s, 3H), 1.13(t, *J*=7.3Hz, 3H), 1.22-1.37(m, 2H), 1.53(s, 3H), 1.69-2.27(m, 3H), 2.37(q, *J*=7.52Hz, 2H), 2.15-2.35(m, 4H), 3.40(m, 1H), 3.88(s, 3H), 4.30(d, *J*=16.5Hz, 1H), 4.39(bd, 1H), 4.52(d, *J*=16.5Hz, 1H). 5.25-5.60(m, 1H), 6.38(dd. *J*₁=10.10Hz, *J*₂=1.7Hz, 1H), 6.42(s, 1H), 6.95(d, J=8.8Hz, 2H), 7.16(d, *J*=10Hz, 1H), 7.98(d, *J*=8.89Hz, 2H). **MASS (ES+)**: 639.1(M+Na)⁺. |
| IA.32 | 1.16(s, 6H), 1.26(t, *J*=7.07Hz, 3H), 1.47(s, 3H), 1.54-2.36(m, 24H), 2.57(m, 1H), 2.90(t, *J*=11.31Hz, 1H), 4.13(q, *J*=7.14Hz, 2H), 4.49)(d, *J*=1.39Hz, 1H), 4.60(d, *J*=16.61Hz, 1H), 5.21(d, *J*=16.70Hz, 1H), 6.01(s, 1H), 6.26(dd, *J*₁=1.64Hz, *J*₂=10.82Hz, 1H), 7.27(d, *J=*3.22Hz, 1H), **MASS (ES+)**: 617.2(M+Na)⁺. |
| IA.33 | 0.935(d, *J*=7.11Hz, 3H), 1.11(t, *J*=7.66Hz, 3H), 1.20-1.34(m, 3H), 1.54(s, 3H), 1.66-1.94(m, 14H), 2.05(d, *J*=4.87Hz, 6H), 2.18-2.51 (m, 6H). 3.30(m, 1H), 4.38(d, *J*=8, 13Hz, 1H), 4.51(d, *J*=16.91Hz, 1H), 5.25(d, *J*=17.23Hz, 1H), 5.40-5.60(m, 1H), 6.37(dd, *J*₁=1.73Hz, *J*₂=10.09Hz, 1H), 6.42(s 1H), 7.15(d, *J*=9.011Hz, 1H). **MASS (ES+)**: 651.2(M+Na)⁺. |
| IA.34 | 0.97(d, *J*=7.14Hz, 3H), 1.12(t, *J*=7.57Hz, 6H), 1.22-1.36(m, 2H), 1.52(s, 3H), 1.71(s, 6H), 1.82-1.91(m, 9H), 2.06(s, 3H), 2.21-2.26(m, 4H), 2.35(q, *J*=7.15Hz, 2H), 3.39(m, 1H), 3.84(d, *J*=17.48Hz, 1H), 4.11(d, *J*=17.49Hz, 1H), 4.40(d, *J*=7.69Hz, 1H), 5.40-3.60(m 1H), 6.35(d, *J*=1.81Hz, 1H), 6.41(d, *J*=6.48Hz, 1H), 7.16(dd *J*₁=1.21Hz, *J*₂=10.06Hz, 1H), **MASS (ES+)**: 667.1(M+Na)⁺. |
| IA.35 | 0.99(d, *J*=7.06Hz, 3H), 1.12(t, *J*=7.44Hz, 6H), 1.22-1.38(m, 2H), 1.53(s, 3H), 1.69-2.02(m, 3H), 2.16-2.38(m, 6H), 3.38(m, 1H), 4.30(d, *J*=16.50Hz, 1H), 4.39(s, 1H), 4.48(d, *J*=16.51Hz, 1H), 5.20-5.50(m, 1H), 6.345(d, *J*=1.69Hz, 1H), 6.41(d, *J*=4.53Hz, 1H), 7.16(d, *J*=10.03Hz, 1H), 7.31(d, *J*=8.31Hz, 2H), 8.06(d, *J*=8.87Hz, 2H), **MASS (ES+)**: 693.1(M+Na)⁺. |
| 1A.36 | 0.96(d, *J*=7.02Hz, 3H), 1.12(t, *J*=7.50Hz, 3H), 1.24-1.36(m, 4H), 1.55(s, 3H), 1.64-2.07(m, 3H), 2.26-2.58(m, 6H), 3.31-3.38(m, 1H), 4.41(d, *J*=8.22Hz, 1H), 5.03(d, *J*=16.58Hz, 1H), 5.40(m, 1H), 5.65(d, *J*=16.59Hz 1H), 6.35(d, *J*=1.62Hz, 1H), 6.42(d, *J*=5.32Hz, 1H). 7.17(d, *J*=9.99Hz, 1H), 7.32(d, *J*=8.23Hz, 2H), 7.97(d, *J*=8.76Hz, 2H), **MASS (ES+)**: 677.0 (M+Na)⁺. |
| IA.37 | 0.88-1.32(m, 10H), 1.48(s, 3H), 1.53-2.37(m, 7H), 2.51-3.01(m, 2H), 4.15(q, *J*=7.13Hz, 2H), 4.52(d, *J*=2.46Hz, 1H), 5.08(d, *J*=16.41Hz. 1H), 5.64(d, *J*=16.41Hz, 1H), 6.02(s, 1H), 6.28(dd, *J*₁=1.78Hz, *J*₂=10.07Hz, 1H), 7.26(s, 1H), 7,33(d, *J*=8.60Hz, 2H), 7.97(d, *J*=8.84Hz, 2H). **MASS (ES+)**: 643.1 (M+Na)⁺. |
| IA.38 | 1.00(d, *J*=7.18Hz, 3H), 1.05-1.50(m, 8H), 1.53(s, 3H), 1,69-2.42(m, 9H), 3.38(m, 1H), 4.32(d, *J*=16.14Hz, 1H), 4.40(s, 1H). 4.50(d, *J*=16.10Hz, 1H), 5.30(m, 1H), 6.38(dd, *J*₁=1.67Hz, *J*₂=10.12Hz, 1H), 6.43(s, 1H), 7.12(d, *J*=10.05Hz, 1H), 7.76(d, *J*=8.21Hz, 2H). 8.12(d, *J*=8.08Hz, 2H). **MASS (ES+)**: 677.1(M+Na)⁺. |
| IA.39 | 1.06(d, *J*=7.12Hz, 3H), 1.17(s, 3H), 1.36-1.41(m, 1H), 1.55(s, 3H), 1.72-2.01(m, 4H), 2.28-2.49(m, 4H), 3.43-3.48(m, 1H), 4.38(d, *J*=16.56Hz, 1H), 4.47(d, *J*=10.79Hz, 1H), 4.52(d, *J*=16.65Hz, 1H), 5.32-5.49(m, 1H), 6.41(d,d *J*₁=10.13Hz, *J*₂=1.79Hz, 1H), 6.46(s, 1H), 6.93(d, *J*=4.18Hz, 1H), 7.14(d,d *J*₁=10.19Hz, *J*₂=1.16Hz, 1H), 7.52(d, *J*=4.00Hz, 1H), 7.77(d, *J*=8.24Hz, 2H), 8.12(d, *J*=8.13Hz, 2H), **MASS (E5+)** : 743.5 |
| IA.40 | 1.05(d, *J*=7.15Hz, 3H), 1.11(s, 3H), 1.34-1.40(m, 1H), 1.54(s, 3H), 1.77-2.01(m, 4H), 2.25(s, 6H), 2.28(s, 3H), 2.28-2.50(m, 4H), 3.45-3.50(m, 1H), 4.09(d, *J*=17.76Hz, 1H), 4.29(d, *J*=17.83Hz, 1H), 4.43(d, *J*=8.40Hz, 1H), 5.32-5.48(m, 1H), 6.40(dd, *J*₁=10.15Hz, *J*₂=1.79Hz, 1H), 6.46(s,1H), 6.85(s, 2H), 6.93(d, *J*=4.09Hz, 1H), 7.14(dd, *J*₁=10.11Hz, *J*₂=1.00Hz, 1H), 7.52(d, *J*=4.07Hz, 1H). **MASS (ES+)**: 717.5 |
| IA.41 | 1.00(d, *J*=7.16Hz, 3H), 1.10(s, 3H), 1.29-1.35(m, 1H), 1.67(s, 3H), 1.77-1.92(m, 4H), 2.25(s, 6H), 2.28(s, 3H), 2.42-2.87(m, 5H), 3.45-3.48(m, 1H), 4.07(d, *J*=17.54Hz, 1H), 4.31(d, *J*=17.68Hz, 1H), 4.59(s, 1H), 6.11(s, 1H), 6.36(dd, *J*₁=10.09Hz, *J*₂=1.74Hz, 1H).6.49(dd, *J*₁=3.45Hz, *J*₂=1.67Hz, 1H), 6.85(s, 2H), 7.17(d, *J*=3.45Hz, 1H), 7.20(d, *J*=10.13Hz, 1H), 7.58(d, *J*=0.8Hz, 1H). **MASS (ES+)** : 687.5 (M+Na)⁺. |
| IA.42 | 0.96(d, *J*=7.10Hz, 3H), 1.13(t, *J*=7.35Hz, 2H), 1.22-1.34(m, 4H), 1.55(s, 3H), 1.64-2.04(m, 7H), 2.17.2.51(m, 4H), 3.10(s, 3H), 3.34(m, 1H), 4.41(d, *J*=8.44Hz, 1H), 5.02(d, *J*=16.63Hz, 1H), 5.44(m, 1H), 5.70(d, *J*=16.73Hz, 1H), 6.38(d, *J*=10.14Hz, 1H), 6.44(s, 1H), 7.14(d, *J*=10.14Hz, 1H), 9.09(s, 4H). **MASS (ES+)**: 671.0 (M+Na)⁺. |
| IA.43 | 0.99(d, *J*=7.11Hz, 3H 1.12(t, *J*=7.54Hz, 3H), 1.24-1.52(m, 4H), 1.72-1.90(m, 8H), 2.23-2.43(m, 6H), 3.10(s, 3H), 3.35(m, 1H), 4.36(d, *J*=16.36Hz, 1H), 4.40(d, 1H), 4.43(d, *J*=16.19Hz, 1H), 5.40(m, 1H), 6.37(d, *J*=10.14Hz, 1H), 6.43(s, 1H), 7.11(d, *J*=10.09Hz, 1H), 8.04(d, *J*=8.32Hz, 2H), 8.17(d, *J*=8.33Hz, 2H), **MASS (ES+)**: 687.0 (M+Na)⁺. |
| IA.44 | 0.96(d, *J*=7.14Hz, 3H), 1,13(t, *J*=7.56Hz, 3H), 1.24-1.33(m, 3H), 1.70-2.08(m, 8H), 2.25-2.42(m, 6H), 3.36(m, 1H), 4.41(d, *J*=8.98Hz, 1H), 5.02(d. *J*=16.63Hz 1H), 5.40(m, H), 5.75(d, *J*=16.64Hz, 1H), 6.38(dd, *J*₁=1.52Hz, *J*₂=10.13Hz, 1H), 6.44(s, 1H), 7.12(d, *J*=10.09Hz, 1H), 7.50(t, *J*=7.73Hz, 2H), 7.63(t, *J*=7.38Hz, 1H), 7.91(d, 7.47Hz, 2H), **MASS(ES+):** 593.1 (M+Na)⁺. |
| IA.45 | 1.00(d, *J*=7.12Hz, 3H), 1,13(t, *J*=7.67Hz, 6H), 1.14-1.35(m, 3H), 1.67-2.00(m, 6H), 2.23-2.44(m, 5H), 3.40(m, 1H), 4.35(d, *J*=16.72Hz, 1H), 4.40(d, *J*=9.27 Hz 1H), 4.53(d, *J*=16.82Hz, 1H), 5.40(m, 1H), 6.38(dd, *J*₁=1.22Hz, *J*₂=10.10Hz, 1H), 6.43(s, 1H), 7.12(d, *J*=10.05Hz, 1H), 7.49(t, *J*=7.71Hz, 2H), 7.61(t, *J*=7.43Hz, 1H), 8.01(d, J=Hz, 2H), **MASS (ES+)**: 609.1 (M+Na)⁺. |
| IA.46 | 0.96(d, *J*=7.11Hz, 3H), 1.13(t, *J*=7.55Hz, 3H), 1.24(s, 3H), 1.27-2.09(m, 8H), 2.24-2.40(m, 6H), 2.43(s, 3H), 3.36(m, 1H), 4.41(d, *J*=8.93Hz, 1H), 5.00(d, *J*=16.55Hz 1H), 5.40(m, 1H), 5.73(d, *J*=16.56Hz, 1H), 6.37(dd, J₁=10.18Hz, *J*₂=1.37Hz, 1H), 6.44(s, 1H), 7.15(d, *J*=10.15Hz, 1H), 7.29(d, *J*=8.0)Hz, 2H), 7.80(d, *J*=8.08Hz, 2H), **MASS (ES+)**: 607.1 (M+Na)⁺. |
| IA.47 | 0.99(d, *J*=7.15Hz, 3H), 1.12(t, *J*=7.49Hz, 6H), 1.29-2.00(m, 8H), 2.20-2.42(m, 6H), 2.42(s, 3H), 3.40(m, 1H), 4.34(d, *J*=16.63Hz, 1H), 4.40(d, *J*=8.60Hz 1H), 4.52(d, *J*=16.61Hz, 1H), 5.40(m, 1H), 6.37(dd, *J*₁=10.17Hz, *J*₂=Hz, 1H), 6.43(s, 1H), 7.13(d, *J*=10.15Hz 1H), 7.28(d, *J*=8.05Hz, 2H), 7.90(d, *J*=8.17Hz, 2H). **MASS (ES+)**: 623.1 (M+Na)⁺. |
| IA.48 | 0.93(d, *J*=7.14Hz, 3H), 1.11(t, *J*=7.65Hz, 3H), 1.15(s, 3H), 1.25-1.84(m, 8H), 2.21-2.38(m, 6H), 3.32(m, 1H), 4.34(d, J=8.93Hz, 1H). 4.81(d, *J*=17.90Hz, 1H), 5.40(m, 1H), 5.41(d, *J*=17.68Hz, 1H), 6.38(d,d *J*₁=10.17Hz, *J*₂=10.49Hz, 1H), 6.43(s, 1H), 7.11(d, *J*=10.07Hz, 1H), 7.36(d, *J*=2.95Hz, 3H). **MASS (ES+)**: 661.0 (M+Na)⁺. |
| IA.49 | 0.98(d, *J*=7.13Hz, 3H). 1.07(s, 3H), 1.12(t, *J*=7.554Hz, 3H), 1.25-2.01(m, 8H), 2.23-2.43(m, 6H), 3.40(m, 1H), 4.25(d, *J*=17.50Hz, 1H), 4.42(d, *J*=8.45Hz, 1H), 4.45(d, *J*=17.57Hz 1H), 5.40(m, 1H), 6.39(d, *J*=10.15Hz, 1H), 6.43(s, 1H), 7.13(d, *J*=10.11Hz, 1H), 7.26-7.36(m, 3H). **MASS (E1+)**: 677.0 (M+Na)⁺. |
| IA.50 | 0.93(d, *J*=7.141Hz, 3H), 1.08(s, 3H), 1.12(t, *J*=7.53Hz, 3H), 1.26-1.86(m, 8H), 2.24-2.38(m, 6H), 3.29(m, 1H), 4.39(d, *J*=8.69Hz, 1H), 4.85(d, *J*=19.97Hz, 1H), 5.36(d, *J*=17.05Hz 1H), 5.40(m, 1H), 6.38(d,d *J*₁=10.10Hz, *J*₂=1.50Hz, 1H), 6.43(s, 1H), 7.15(d, *J*=10.11Hz. 1H), 7.3.1(t, *J*=7.90Hz, 1H), 7.47(d,d *J*₁=7.60Hz, *J*₂=1.2Hz, 1H), 7.62(d,d *J*₁=7387Hz, *J*₂=1.11Hz, 1H), **MASS (ES+)**: 661.0 (M+Na)⁺. |
| IA.51 | 0.97(d, *J*=7.13Hz, 3H), 1.01(s, 3H), 1.12(t, *J*=7.55Hz, 3H), 1.29-1.95(m, 8H), 2.24-2.40(m, 6H), 3.35(m, 1H), 4.17(d, *J*=16.89Hz, 1H), 4.32(d, *J*=16.88Hz, 1H), 4.41(d, *J*=8.1Hz, 1H), 5.40(m, 1H), 6.38(dd, *J*₁=10.04Hz, *J*₂=1.50Hz, 1H), 6.43(s, 1H), 7.13(d, *J*=10.14Hz 1H), 7.31(d, *J*=7.89Hz, 1H), 7.53(dd, *J*₁=7.62Hz, *J*₂=1.15Hz, 1H), 7.58(dd, *J*₁=8.01Hz, *J*₂=1.19Hz, 1H). **MASS (ES+)**: 677.0 (M+Na)⁺. |
| IA.52 | 0.94(d, *J*=7016Hz, 3H), 1.12(t, *J*=7.55Hz, 3H), 1.19(s, 3H), 1.25-1.97(m, 8H), 2.17-2.41(m, 6H), 3.34(m, 1H), 4.39(d, *J*=8.73Hz, 1H). 4.81(d, *J*=17.22Hz, 1H), 5.38(m, 1H), 5.42(d, *J*=17.55Hz 1H), 6.38(d,d *J*₁=10.13Hz, *J*₂=1.37Hz, 1H), 6.43(s, 1H), 7.02(d, *J*=8.53Hz, 2H), 7.15(d, *J*=10.17Hz, 1H), 7.47-7.51(m, 1H), **MASS (ES+)**: 629.1 (M+Na)⁺. |
| IA.53 | 0.97(d, *J*=7.13Hz, 3H), 1.07(s, 3H), 1.10(t, *J*=7.54Hz, 3H), 1.16-1.95(m, 8H). 2.22-2.44(m, 6H). 3.36(m, 1H), 4.17(d, *J*=16.90Hz. 1H). 4.29(d, *J*=16.88Hz, 1H), 4.40(d, *J*=8.72Hz, 1H), 5.40(m, 1H), 6.37(dd, *J*₁=10.16Hz, *J*₂=1.46Hz, 1H), 6.43(s, 1H), 6.98(1, *J*=8.34Hz 2H), 7.13(d, *J*=9.90Hz, 1H, 7.44(d, *J*=7.62Hz, 1H). **MASS (ES+)**: 645.0 (M+Na)⁺. |
| IA.54 | 0.96(d, *J*=7.13Hz, 3H), 1.13(t, *J*=7.55Hz, 3H), 1.24(s, 3H), 1.25-1.99(m, 8H), 2.17-2.43(m, 6H), 3.35(m, 1H), 4.42(d, *J*=8.41Hz, 1H). 4.95(dd, *J*1=17.53Hz, *J*₂=2.75Hz 1H), 5.40(m, 1H), 5.57(dd, *J*₁=17.55Hz, *J*₂=2.80Hz, 1H), 6.38(d,d *J*₁=10.10Hz, *J*₂=1.06Hz, 1H), 6.44(s, 1H), 7.15(d, *J*=10.15Hz, 1H), 7.22(d,d *J*₁=10.05Hz, *J*₂=5.61Hz, 1H), 7.43(d,d *J*₁=16.25Hz, *J*₂=8.85Hz, 1H), 7.69(t, *J*=7.42Hz, 1H). **MASS (ES+)**: 629.1(M+Na)⁺. |
| IA.55 | 0.98(d, *J*=7.08Hz, 3H), 1.10-1.14(m, 6H), 1.24-1.99(m, 8H), 2.23-2.43(m, 6H), 3.36(m, 1H), 4.29(dd, *J*₁=17.37Hz, *J*₂=1.85Hz 1H), 4.43(dd, *J*₁=17.25Hz, *J*₂=2.03Hz 2H), 5.40(m, 1H), 6.38(d, *J*=10.14Hz, 1H), 6.43(s, 1H), 7.13(d, *J*=10.11Hz 1H), 721(dd, *J*₁=12.35Hz. *J*₂=7.931Hz, 1H), 7.40(d, *J*=8.21Hz, 1H), 7.63(t, *J*=6.40Hz, 1H). **MASS (ES+)**: 645.1 (M+Na)⁺. |
| IA.56 | 1.06(d, *J*=7.11Hz, 3H), 1.17(s, 3H), 1.35-1.41(m, 1H), 1.54(s, 3H), 1.77-2.03(m, 4H), 2.28-2.49(m, 4H), 3.43-3.49(m, 1H), 4.34(d, 1H, *J*=16.58Hz), 4.47(d, *J*=6.32Hz, 1H), 4.50(d, *J*=16.57Hz, 1H), 532-5.49(m, 1H), 6.40(dd, *J*₁=10.19Hz, *J*₂=1.84Hz, 1H), 6.46(s, 1H), 6.93(d, *J*=3.95Hz, 1H), 7.14(dd, *J*₁=10.12Hz, *J*₂=1.10Hz, 1H), 7.47(d, *J*=8.60Hz, 2H), 7.52(d, *J*=4.06Hz, 1H), 7.95(d, *J*=8.60Hz, 2H). **MASS (ES+)** : 731.5 (M+Na)⁺. |
| IA.57 | 0.93(d, *J*=7.11Hz, 3H), 1.07(s, 3H), 1.11(t, *J*=7.54Hz, 3H), 1.25-1.82(m, 8H), 2.25(s, 9H), 2.22-2.37(m, 6H), 3.31(m, 1H), 4.27(d, *J*=8.7Hz, 1H), 4.55(d, *J*=17.82Hz, 1H), 5.33(d, *J*=17.85Hz, 1H), 5.40(m, 1H), 6.38(d,d *J*₁=10.14Hz, *J*₂=1.44Hz, 1H), 6.43(s, 1H), 6.87(s, 2H), 7.11(d, *J*=9.94Hz, 1H), **MASS (ES-)**: 611.3 (M-H)⁻. |
| IA.58 | 0.98(d, *J*=7.07Hz, 3H), 1.03(s, 3H), 1.12(t, *J*=7.53Hz, 3H), 1.25-1.95(m, 8H), 2.25(s, 9H), 2.22-2.41(m, 6H), 3.40(m, 1H), 4.05(d, *J*=17.72Hz, 1H), 4.28(d, *J*=17.74Hz, 1H), 4.38(d, *J*=8.06Hz, 1H), 5.41(m, 1H), 6.38(d, *J*=10.17Hz, 1H), 6.43(s, 1H), 6.85(s, 2H), 7.12(d, *J*=10.13Hz 1H), **MASS (ES+)**: 651.1 (M+Na)⁺. |
| IA.59 | 0.44(s, 3H), 0.95(d, *J*=7.02Hz, 3H), 1.08(t, *J*=7.53Hz, 3H), 1.22-1.75(m, 15H), 2.17-2.32(m, 6H), 3.15(m, 1H), 3.29(d, *J*=6.8Hz, 1H), 5.40(m, 1H), 6.35(dd, *J*₁=10.11Hz, *J*₂=1.43Hz 1H), 6.40(s, 1H), 7.05(d, *J*=9.98Hz 1H), 7.36(t, *J*=7.70Hz, 2H), 7.46(t, *J*=7.20Hz, 1H). 7.95(d, *J*=7.43Hz, 2H). **MASS (ES+)**: 637.1 (M+Na)⁺. |
| IA.60 | 0.96(d, *J*=7.10Hz, 3H), 1.13(t, *J*=7.56Hz, 3H), 1.23-2.06(m, 11H), 2.17-2.49(m, 6H), 3.35(m, 1H), 4.40(d, *J*=8.55Hz, 1H), 4.93(d, *J*=16.21Hz, 1H), 5.39(m, 1H), 5.64(d, *J*=16.21Hz, 1H), 6.38(d,d *J*₁=10.18Hz, *J*₂=1.30Hz, 1H), 6.43(s, 1H), 7.15(d, *J*=10.22Hz, 1H), 7.18(t, *J*=4.56Hz, 1H), 7.73(d, *J*=4.96Hz, 1H), 7.75(d, *J*=3.66Hz, 1H), **MASS (ES+):** 599.2 (M+Na)⁻. |
| IA.61 | 0.99(d, *J*=7.11Hz, 3H), 1.11-1.14(m, 6H), 1.25-1.96(m, 8H), 2.17-2.41(m, 6H), 3.39(m, 1H), 4.27(d, *J*=16.28Hz, 1H), 4.41(d, *J*=13.26Hz, 1H), 4.44(d, *J*=16.48Hz, 1H), 5.42(m, 1H), 6.38(dd, *J*₁=10.16Hz, *J*₂=1.46Hz, 1H), 6.43(s, 1H), 7.13(d, *J*=9.66Hz 1H), 7.17(t, *J*=4.16Hz, 1H), 7.71(d, *J*=4.90Hz, 1H), 7.86(d, *J*=3.51Hz, 1H), **MASS (ES+)**: 615.2 (M+Na)⁺. |
| IA.62 | 0.96(d, *J*=7.11Hz, 3H), 1.13(t, *J*=7.54Hz, 3H), 1.23(s, 3H), 1.25-2.03(m, 8H), 2.24-2.42(m, 6H), 3.34(m, 1H), 4.40-4.42(m, 1H), 4.97(d, *J*=16.57Hz, 1H), 5.40(m, 1H), 5.64(d, *J*=16.56Hz, 1H), 6.37(d,d *J*=10.15Hz, 1H), 6.44(s, 1H), 7.14(d, *J*=10.11Hz, 1H), 7.33-7.26(m, 1H), 7.68-7.79(m, 2H). **MASS (ES+)**: 629.2 (M+Na)⁺. |
| IA.63 | 0.99(d, *J*=7.09Hz, 3H), 1.11(t, *J*=7.80Hz, 6H), 1.25-1.93(m, 8H), 2.17-2.44(m, 6H), 3.36(m, 1H), 4,18-4.38(m, 3H), 4.40(d. *J*=16.10Hz, 1H), 5.38(m, 1H), 6.33(d, *J*=10.07Hz, 1H), 6.39(s, 1H), 7.20(d, *J*=10.05Hz 1H). 7.28-7.32(m, 1H), 7.79-7.85(m, 1H), **MASS (ES+)**:615.1 (M+Na)⁺. |
| IA.64 | 0.95(d, *J*=7.13Hz, 3H), 1.13(t, *J*=7.56Hz, 3H), 1.22(s, 3H), 1.25-2.05(m, 8H), 2.24-2.49(m, 6H), 3.32-3.37(m, 1H), 4.40(d, *J*=8.68Hz, 1H), 4.86(d, *J*=16.63Hz, 1H), 5.32-5.48(m, 1H), 5.58(d, *J*=16.63Hz, 1H), 6.39(d,d *J*₁=10.13Hz, *J*₂=1.29Hz, 1H), 6.43(s, 1H), 6.60(d,d *J*₁=3.19Hz, *J*₂=1.29Hz, 1H), 7.14(d, *J*=10.16Hz, 1H), 7.27(d, *J*=3.65Hz, 1H), 7.63(s, 1H), **MASS (ES+):** 583.2 (M+Na)⁺. |
| IA.65 | 1.03(d, *J*=7.10Hz, 3H), 1.25-1.95(m, 10 H), 2.07(d, *J*=14.85Hz, 1H) 2.28-2.51(m, 4H), 3.41-3.44(m, 1H), 4.44(d, *J*=8.26Hz, 1H), 5.02(d, *J*=16.48Hz, 1H), 5.32-5.49(m, 1H), 5.73(d, *J*=16.48Hz, 1H), 5.97(s, 1H), 6.38(d, *J*=10.19Hz, 1H), 6.44(s, 1H), 7.13-7.21(m, 3H), 7.95(d,d *J*₁=8.18Hz, *J*₂=5.45Hz, 2H). **MASS (ES-)**: 641.2(M-H)⁺. |
| IA.66 | 1.03(d, *J*=7.12Hz, 3H), 1.25-2.07(m, 11H), 2.28-2.52(m, 4H), 3.41-3.44(m, 1H), 4.44 (d, *J*=8.40Hz, 1H), 4.93(d,d *J*₁=17.51Hz, *J*₂=3.47Hz, 1H), 5.32-5.49(m, 1H), 5.67(d,d *J*₁=17.49Hz, *J*₂=3.40Hz, 1H), 5.96(s, 1H), 6.38(d,d *J*₁=10.16Hz, *J*₂=1.38Hz, 1H), 6.44(s, 1H), 6.91-6.97(m, 1H), 7.01-7.05(m, 1H), 7.15(d, *J*=10.12Hz 1H), 8.01(d,d *J*₁=14.98Hz, *J*₂=8.29Hz, 1H). **MASS (ES+)**: 683.1 (M+Na⁻). |
| IA.67 | 0.97(d, *J*=7.10Hz, 3H), 1.14(t, *J*=7.53Hz, 3H), 1.24-2.05(m,8H), 2.27-2.44(m, 6H), 3.32-3.30(m, 1H). 4.40(d, *J*=9.07Hz, 1H), 5.00(d, *J*=16.85Hz, 1H), 5.20-5.60(m, 1H), 5.72(d, *J*=16.8Hz, 1H), 6.38(d,d *J*₁=10.11Hz, *J*₂=1.74Hz, 1H), 6.44(s, 1H), 7.15(d, *J*=10.06Hz, 1H), 7.31-7.39(m, 1H), 7.49-7.59(m, 3H), 7.74(d, *J*=7.85 Hz, 1H), **MASS (ES+)**: 633.2 (M+Na⁻). |
| 1.4.68 | 0.99(d, *J*=7.11Hz, 3H), 1.13(d, *J*=7.32Hz, 6H), 1.25-1.99(m, 8H), 2.23-2.45(m, 6H), 3.38(m, 1H), 4.39(d, *J*=16.55Hz, 1H), 4.36(d, *J*=16.90Hz, 1H), 4.40(d, *J*=13.09Hz, 1H), 5.39(m, 1H), 6.38(dd, *J*₁=10.15Hz, *J*₂=1.46Hz, 1H), 6.43(s, 1H), 7.13(d, *J*=10.16Hz 1H), 7.33(d, *J*=3.51Hz, 1H), 7.64(s, 1H), **MASS (ES+)**: 599.1 (M+Na)⁺. |
| IA.69 | 0.99(d, *J*=7.07Hz, 3H), 1.13(t, *J*=7.22Hz, 6H), 1.25-1.93(m, 8H), 2.00-2.48(m, 6H), 3.35-3.42(m, 1H), 4.27-4.52(m, 3H), 5.25-5.52(m, 1H), 6.34-640(m, 1H), 6.43(s, 1H), 7.13(d, *J*=9.96Hz 1H), 7.33(t, *J*=6.94Hz, 1H), 7.47-7.76(m, 4H). **MASS (ES+):** 649.1 (M+Na)⁺. |
| IA.70 | 0.95(d, *J*=7.18Hz, 3H), 1.09-1.16(m, 6H), 1.25-1.30(m, 2H), 1.34(s, 9H), 1.42-1.95(m, 6H), 2.17-2.43(m, 6H), 3.37-3.44(m, 1H), 4.28-4.49(m, 2H), 4.53(d, *J*=16.72Hz, 1H), 5.20-5.60(m, 1H), 6.37(dd, *J*₁=10.12Hz, *J*₂=1.73Hz, 1H), 6.43(s, 1H), 7.13(d, *J*=10.19Hz 1H). 7.50(d, *J*=8.52Hz, 2H), 7.95(d, *J*=8.52Hz, 2H), **MASS (ES+)**: 665.2 (M+Na)⁺. |
| IA.71 | 0.96(d, *J*=7.11Hz, 3H), 1.14(t, *J*=7.52Hz, 3H), 1.24-1.36(m, 5H), 1.55-2.07(m, 6H), 2.20-2.46(m, 6H), 3.32-3.39(m, 1H), 4.02(s, 3H), 4.40(d, *J*=9.33Hz, 1H), 5.02(d, *J*=16.95Hz, 1H), 5.20-5.60(m, 1H), 5.74(d, *J*=16.94Hz, 1H), 6.37(d,d *J*₁=10.13Hz, *J*₂=1.73Hz, 1H), 6.44(s, 1H), 6.97(d,d *J*₁=6.86Hz, *J*₂=1.97Hz, 1H), 7.13-7.28(m, 3H), 7.56(s, 1H), **MASS (ES+)**: 663.2 (M+Na)⁺. |
| IA.72 | 0.96(d, *J*=7.11Hz, 3H), 1.14(t, *J*=7.52Hz, 3H), 1.24-1.30(m, 6H), 1.34(s, 8H), 1.42-1.92(m, 6H), 2.11-2.54(m, 6H), 3.30-3.41(m, 1H), 4.41(d, *J*=9.37Hz, 1H), 5.00(d, *J*=16.55Hz, 1H), 5.26-5.53(m, 1H), 5.74(d, *J*=16.56Hz, 1H), 6.38(d,d *J*₁=10.12Hz, *J*₂=1.67Hz, 1H), 6.44(s, 1H), 7.15(d, *J*=10.03Hz, 1H), 7.50(d, *J*=8.48Hz, 2H), 7.85(d, *J*=8.47Hz, 2H). **MASS (ES+)**: 649.2 (M+Na)⁻. |
| IA.73 | 1.00(d, *J*=7.18Hz, 3H), 1.05-1.50(m, 8H), 1.53(s, 3H), 1.69-2.42(m, 9H), 3.38(m, 1H), 4.32(d, *J*=16.14Hz, 1H), 4.40(s, 1H), 4.50(d, *J*=16.10Hz, 1H), 5.3(m, 1H), 6.38(dd, *J*₁=1.67Hz, *J*₂=10.12Hz, 1H), 6.43(s, 1H), 7.12(d, *J*=10.05Hz, 1H), 7.76(d, *J*=8.21Hz, 2H), 8.12(d, *J*=8.08Hz, 2H), **MASS (ES+)**: 679.1(M+Na)⁻. |
| IA.74 | 0.96(d, *J*=7.10Hz, 3H), 1.13(t, *J*=7.54Hz, 3H), 1.25-1.99(m, 8H), 2.11-2.43(m, 6H), 3.30-3.50(m, 1H), 3.88(s, 3H), 4.40(d, *J*=9.28Hz, 1H), 4.85(d,d *J*₁=17.50Hz, *J*₂=3.72Hz, 1H), 5.20-5.60(m, 1H), 3.60(d,d *J*₁=17.50Hz, *J*₂=3.65Hz, 1H), 6.37(d,d *J*₁=10.11Hz, *J*₂=1.74Hz, 1H), 6.44(s, 1H), 6.65(d,d *J*₁=13, 13Hz, *J*₂=2.25Hz, 1H), 6.79(d,d *J*₁=8.90Hz, *J*₂=2.35Hz, 1H), 7.15(d,d *J*₁=10.14Hz, *J*₂=1.04Hz, 1H), 7.91(t, *J*=8.54Hz, 1H), **MASS (ES+)**: 641.2(M+Na)⁺. |
| IA.75 | 0.99(d, *J*=7.17Hz, 3H), 1.09-1.16(m, 6H), 1.25-1.97(m, 8H), 2.05-2.44(m, 6H), 3.30-3.50(m, 1H), 3.87(s, 3H), 4.25-4.54(m, 3H), 5.20-5.60(m, 1H), 6.38(dd, *J*₁=10.09Hz, *J*₂=1.73Hz, 1H), 6 43(s, 1H), 6.65(dd, *J*₁=13.18Hz, *J*₂=2.35Hz, 1H), 6.77(dd, *J*₁=8.40Hz, *J*₂=2.38Hz, 1H), 7.14(d, *J*=9.05Hz, 1H), 7.89(t, *J*=8.67Hz, 1H), **MASS (ES+)**: 657.1(M+Na)⁺. |
| IA.76 | 0.96(d, *J*=7.15Hz, 3H), 1.15(t, *J*=7.60Hz, 3H), 1.25-1.94(m, 8H), 2.04-2.43(m, 6H), 3.33-3.41(m, 1H), 3.95(s, 3H), 4.41(d, J=8.47Hz, 1H), 4.97(d, *J*=17.66Hz, 1H), 5.20-5.60(m, 1H), 5.65(d, *J*=17.71Hz, 1H), 6.34-6.44(m, 2H), 6.98-7.08(m, 2H), 7.15(d, *J*=10.01Hz, 1H), 7.520, *J*=6.82Hz, 1H), 7.89(dd, *J*₁=7.79Hz, J₂=1.66Hz, 1H). **MASS(ES+)**: 623.2(M+Na)⁺. |
| IA.77 | 0.96(d, *J*=7.13Hz, 3H), 1.13(t, *J*=7.55Hz, 3H), 1.24-2.43(m, 17H), 3.31-3.40(m, 1H), 3.87(s, 3H), 3.92(s, 3H), 4.41(d, *J*=9.87Hz, 1H), 4.88(d, *J*=17.64Hz, 1H), 5.20-5.68(m, 2H), 6.34-6.59(m, 4H), 7.17(d,d *J*₁=10.11Hz, *J*₂=0.97Hz, 1H), 7.92(d, *J*=8.80Hz, 1H). **MASS (ES+)**: 635.2(M+Na)⁺. |
| IA.78 | 0.98(d, *J*=7.16Hz, 3H), 1.09-1.16(m, 6H), 1.25-1.88(m, 8H), 2.05-2.41(m, 6H), 3.36-3.45(m, 1H), 3.86(s, 3H), 3.93(s, 3H), 4.30-4.61(m, 3H), 5.20-5.60(m, 1H), 6.34-6.57(m, 4H), 7.15(dd, *J*₁=10.05Hz, *J*₂=1.00Hz, 1H), 7.85(d, *J*=8.73Hz, 1H), **MASS (ES+)**:669.1(M+Na)⁺. |
| IA.79 | 0.97(d, *J*=7.16Hz, 3H), 1.09-1.25(m. 6H), 1.29-1.96(m, 8H), 2.03-2.41(m, 6H), 3.30-3.43(m, 1H), 3.95(s, 3H), 4.31-4.00(m, 3H), 5.20-5.6(m, 1H), 6.35-6.45(m, 2H), 6.97-7.05(m, 2H), 7.14(d, *J*=9.89Hz, 1H), 7.46-7.55(m, 1H), 7.77(dd, *J*₁=7.71Hz, *J*₂=1.58Hz, 1H). **MASS (ES+):** 639.1 (M+Na)⁺. |
| IA;80 | 0.95(d, *J*=7.16Hz, 3H), 1.08-1.18(m, 6H), 1.22-1.93(m, 8H), 2.19-2.41(m,6H), 3.28-3.37(m, 1H), 4.39(d, *J*=8.00Hz, 1H), 4.73(d, *J*=17.18Hz, 1H), 5.22-5.53(m, 2H), 6.38(d,d *J*₁=10.09Hz, *J*₂=1.78Hz, 1H), 6.43(s, 1H), 7.14(d,d *J*₁=10.07Hz, *J*₂=1.16Hz, 1H), 7.63-7.88(m, 4H). **MASS (ES+):** 661.1(M+Na)⁺. |
| IA.81 | 0.99(d, *J*=7.15Hz, 3H), 1.09-1.17(m, 6H), 1.25-2.02(m, 8H), 2.17-2.44(m, 6H), 3.34-3.41(m, 1H), 4.11(d, *J*=17.43Hz, 1H), 4.30(d, *J*=17.42Hz, 1H), 4.41(d, *J*=9.75Hz, 1H), 5.20-5.60(m, 1H), 6.38(dd, *J*₁=10.11Hz, *J*₂=1.74Hz, 1H), 6.43(s, 1H), 7.13(dd, *J*₁=10.13Hz, *J*₂=1.07Hz, 1H), 7.60-7:88(m, 4H). **MASS (ES+)**: 677.1(M+Na)⁺. |
| IA.82 | 1.03(d, *J*=7.07Hz, 3H), 1.25-1.79(m, 7H), 1.79-2.55(m, 8H), 3.41-3.45(m, 1H), 4.45(d, J=8.28Hz, 1H), 4.98(d, *J*=16.58Hz, 1H), 5.34-5.50(m, 1H), 5.79(d, *J*=16.48Hz, 1H), 6.40(d,d *J*₁=10.16Hz, *J*₂=1.38Hz, 1H), 6.46(s, 1H), 6.51(s, 1H), 7.14-7.19(m, 4H), 7.60(s, 1H). 7.93(d,d *J*₁=8.55Hz, *J*₂=5.35Hz, 2H). **MASS (ES+)**: 649.1(M+Na)⁺. |
| IA.83 | 1.07(d, *J*=6.86Hz, 3H), 1.17(s, 3H), 1.38-1.92(m, 6H), 1.95-2.54(m, 6H). 3.47(m, 1H), 4.31(d, *J*=16.60Hz, 1H), 4.45-67(m, 2H), 5.32-5.49(m, 1H), 6.39-6.51(m, 3H), 7.14-7.19(m, 4H), 759(s, 1H), 8.05(dd, *J*₁=8.14Hz, *J*₂=5.42Hz 2H), **MASS (ES+)**: 665.1(M+Na)⁺. |
| IA.84 | 1.03(d, *J*=7.08Hz, 3H), 1.25-1.66(m, 7H), 1.76-2.56(m, 8H), 3,41-3.45(m, 1H), 4.45(d, *J*=8.26Hz, 1H), 4.90(d,d *J*₁=17.59Hz, *J*₂=3.07Hz, 1H), 5.34-5.5(m, 1H), 5.65(d,d *J*₁=17.61Hz, *J*₂=3.17Hz, 1H), 6.41(d,d *J*₁=10.05Hz, *J*₂=1.50Hz, 1H), 6.40(s, 1H), 6.51(d,d *J*₁=3.20Hz, *J*₂=1.52Hz, 1H), 6.90-7.04(m, 2H), 7.13-7.19(m, 2H), 7.59(s, 1H), 7.99(d,d *J*₁=15.08Hz, *J*₂=8.28Hz, 1H). **MASS (ES+)**: 683.1(M+Na)⁺. |
| IA.85 | 1.000d, *J*=7.02Hz, 3H), 1.19(s, 3H), 1.36-1.66(m, 6H), 1.71-2.55(m, 6H), 3.44-3.48(m, 1H), 4.31(d, *J*=17.67Hz, 1H), 4.46-4.50(m, 2H), 5.33-5.66(m, 1H), 6.41(dd, *J*₁=10.18Hz, *J*₂=0.81Hz, 1H), 6.45(s, 1H), 6.50(d, *J*=1.54Hz, 1H), 6.89-7.01(m, 2H, 7.12-7.17(m, 2H, 7.59(s, 1H), 7.95(dd, *J*₁=15.16Hz, *J*₂=8.35Hz, 1H), **MASS (ES+)**: 683.1(M+Na)⁺. |
| IA.86 | 0.96(d, *J*=7.14Hz, 3H), 1.11(s, 3H), 1.12(t, *J*=7.53Hz, 3H), 1.24-1.34(m, 1H), 1.54(s, 3H), 1.59-1.64(m, 1H), 1.75-1.98(m, 4H), 2.13-2.61(m, 7H), 3.37(m, 1H), 4.36(d, *J*=11.18, 1H), 4.42(d, *J*=8.7.1H), 4.49(d, *J*=16.46,1H), 6.34(s, 1H), 6.34(d,d *J*₁=10.12Hz, *J*₂=1.79Hz, 1H), 7.11-7.23(m, 3H), 8.01(m, 2H), **MASS (ES+)**: 609.1(M+Na)⁺. |
| IA.87 | 0.94(d, *J*=7.09Hz, 3H), 1.12(t, *J*=7.54Hz, 3H), 1.24(s, 3H), 1.27-1.31(m, 1H), 1.57(s, 3H), 1.59-1.62(m, 1H), 1.81-1.87(m, 2H), 1.97-2.03(m, 2H), 2.15-2.23(m, 1H), 2.34-2.46(m, 6H), 2.60-2.66(m, 1H), 3.30-3.36(m, 1H), 4.41(d, *J*=8.85Hz, 1H), 4.98(d, *J*=10.51Hz, 1H), 5.69(d, *J*=16.54Hz, 1H), 6.13(s, 1H), 6.34(d,d *J*₁=9.98Hz, *J*₂=1.40Hz, 1H), 7.17(t, *J*=8.50Hz, 2H), 7.23(d, *J*=10.14Hz, 1H), 7.95(dd, *J*₁=8.58Hz, *J*₂=5.36Hz, 2H). **MASS (ES+)**: 593.2 (M+Na)⁺. |
| IA.88 | 0.98(d, *J*=6.7Hz, 3H); 1.09(m, 6H); 1.31-1.35(m, 2H); 1.54(s, 3H): 1.71-1.84(m, 1H); 1.87- 2.00(m, 2H); 2.20-2.33(m, 6H): 2.88(s, 1H): 3.36(m, 1H); 4.39(d, 1H, *J*=31.8Hz); 4.47(d, 1H, *J*=31.7Hz); 5.31-5.48(m, 1H); 6.36(d, 1H, *J*=10.1Hz); 6.41(s, 1H); 7.21(d, 1H, *J*=10.0); 8.10(s, 1H); 8.44(s, 2H), **MASS (ES+)**: 745.1(M+Na)⁻ |
| IA.89 | 0.99(d, *J*=6.91 Hz, 3H); 1.10(m, 5H); 1.32(m, 1H); 1.54(s, 3H); 1.80(m, 2H); 2.20-2.48(m, 7H); 3.21(m, 1H); 3.38(m, 1H); 4.36-4.47(m, 3H); 5.32-5.48(m, 1H); 6.36(d, 1H, *J*=10.0Hz); 6.41(s, 1H); 7.22(d, 1H, *J*=10.0Hz); 7.65(t, 1H, *J*=7.7Hz ): 7.85(d, 1H, *J*=7.6Hz), 8.19(d, 1H, *J*=7.8Hz), 8.24(s, 1H), **MASS (ES+)**: 677.1 (M+Na)⁺ |
| IA.90 | 0.90(d, *J*=6.4Hz, 6H), 0.99(d, 3H, *J*=6.7Hz), 1.12(s, 3H), 1.30-1.34(m, 1H), 1.52(s, 3H), 1.72-1.89(m, 3H), 1.91-2.19(m, 4H), 2.25-2.47(m, 7H), 2.53(m, 3H), 3.40(m, 1H), 4.30(d, 1H, *J*=17.0Hz), 4.51(d, 1H, *J*=16.5Hz), 5.30-5.50(m, 1H), 6.37(d, 1H, *J*=10.0Hz, 6.43(s, 1H). 7.17(d, 1H, *J*=10.0Hz), 7.25(d, 2H, *J*=7.8Hz), 7.91(d, 2H, *J*=7.8Hz). **MASS (ES+)**: 664.2(M+Na)⁻ |
| IA.91 | 1.11(t, *J*=7.5Hz, 3H), 1.18(s, 3H), 1.23-1.25(m, 1H), 1.43(d, *J*=7.34Hz, 3H), 1.58 (s, 3H), 1.61-1.64(m, 1H), 1.91-2.07(m, 4H), 2.13-2.22(m, 2H), 2.29-2.55(m, 7H), 2.61-2.66(m, 1H), 4.43(d.*J*=8.38Hz, 1H), 4.88(d, *J*=16.49Hz, 1H), 5.68(d, *J*=16.4Hz, 1H), 6.14(s, 1H), 6.35(d,d *J*₁=10.12Hz, *J*₂=1.44Hz, 1H), 7.17(t, *J*=8.52Hz, 2H), 7.25(d, *J*=9.93Hz, 1H), 7.92(dd, *J*₁=8.67H, *J*₂=5.3Hz, 2H). **MASS (ES+):** 593.2(M+Na)⁺ |
| IA.92 | 0.99(d, 3H, *J*=6.95Hz); 10.6-1.14(m, 6H); 1.26(1, 3H, *J*=7.52); 130-1.34(m, 1H); 1.52(s, 3H), 1.72-1.96(m, 4H), 2.0-2.28(m,2H), 2.34. 2.44(m, 4H), 2.72(q, 2H, *J*₁=15.05Hz, *J*₃=7.51Hz), 3.39-3.42(m, 1H), 4.33-4.41(m, 2H), 4.52(1H, d, *J*=16.60Hz), 5.25-5.50(m, 1H). 6.37(1H, d, *J*=10.09Hz), 6.43(s, 1H), 7.13(d, 1H, *J*=10.07Hz), 7.31(d, 2H, *J*=7.92Hz), 7.93(d, 2H, *J*=8.00Hz). **MASS (ES+):** 637.1(M+Na)⁺ |
| IA.93 | 0.99(d, *J*=7.0Hz, 3H); 1.H-1.50(m, 5H), 1.26-1.48(m, 7H); 1.52(s, 3H); 1.67(s, 3H); 1.70-1.80(m, 2H), 1.87-2.00(m, 6H); 2.20-2.45(m, 3H); 2.5(m, 1H); 3.38-3.42(m, 1H); 4.32(d, 1H, *J*=16.7Hz); 4.40(d, 1H, *J*=8.01Hz): 4.54(d, 1H, *J*=16.7Hz); 5.30-5.47(m, 1H); 6.38(dd, 1H, *J*₁=10.0Hz, *J*₁=1.09Hz); 6.43(s, m); 7.13(d, 1H, *J*=5.05Hz); 7.32(d, 2H, *J*=8.13Hz); 7.94(d, 2H, *J*=8.12Hz). **MASS (ES+):** 691.2(M+Na)⁺ |
| IA.94 | 0.99(s,3H), 1.14(t, *J*=7.53Hz, 3H), 1.22-1.2.9(m, 1H). 1.36(d, *J*=7.31 Hz, 3H), 1.56(s,3H), 1.63-2.11(m,6H), 2.25-2.65(m, 7H), 4.01(d,d, *J*₁=17.6Hz, *J*₂=3.11Hz, 1H), 4.47(d,d, *J*₁=17.62Hz, *J*₂=3.11Hz, 1H), 4.49(d, *J*=7.05Hz, 1H), 6.13(s, 1H), 6.34(d,d *J*₁=10.11Hz. *J*₂=1.8Hz, 1H), 6.86-7.04(m, 2H), 7.22(d, *J*=10.18Hz, 1H), 7.87-7.98(m,1H). **MASS(ES+):** 627.1(M+Na)⁺ |
| IA.95 | 0.99(d, *J*=08Hz, 6H), 1.11-1.15(m,5H), 1.27(d, 6H, *J*=6.8Hz), 1.52(s,3H), 1.67(s,3H), 1.72-1.89(m,3H). 1.99(d, 1H, *J*=14.47Hz), 2.37(q, 2H), 2.96-2.99(m, 1H), 3.39-3.42(m, 1H), 4.32(d; 1H, *J*=16.63Hz), 4.40(d, 1H, *J*=64Hz), 4.54(d, 1H, *J*=16.7Hz). 5.30-5.47(m, 1H), 6.37(d, 1H, *J*=10.0Hz), 6.43(s,1H), 7.14(d, 1H, *J*=10.0Hz), 7.34(d, 2H, *J*=8.17Hz), 7.94(d, 2H, *J*=8.1Hz). **MASS (ES+):** 651.1(M+Na)⁺ |
| IA.96 | 0.83-0.95(m, 1H), 0.99(d, 3H, *J*=7.0Hz), 1.11(d, 3H, *J*=4.13Hz), 1.26-1.34(m, 2H), 1.05-1.69(m, 2H), 1.72-2.08(m, 11H). 2.14-2.32(m, 2H), 2.36(q, 2H, *J*₁⁻ 15.04, *J*₂⁻7.58), 3.39-3.42(m, 1H), 4.32(d, 1H, *J*=16.43Hz), 4.40(1H, d, *J*=8.26Hz), 4.49(d, 1H, *J*=16.41Hz), 5.30-5.46(m, 1H), 6.37(d, 1H, *J*=9.92Hz), 6.43(s, 1H), 6.90(d, *J*=8.71Hz), 7.13(d, 1H, *J*=10.04Hz), 7.95(d, 2H, *J*=8.71Hz). **MASS(ES+)**: 693.1(M+Na)⁺ |
| IA.97 | 0.94(d, *J*=7.11Hz, 3H), 1.04(s,3H), 1.11(t, *J*=7.55Hz, 3H), 1.21-1.44(m, 1H), 1.54(s,3H), 1.75-2.04(m,4H), 2.12-2.41(m, 7H), 3.1(s, 3H), 3.29-3.33(m, 1H), 4.34(d, *J*=16.23, 1H), 4.44(d, *J*=16.35, 1H), 6.12(s, 1H), 6.34(d,d, *J*₁=10.12Hz, *J*₁=1.77Hz, 1H), 7.19(d, *J*=10.15Hz. 1H), 8.06(d, *J*=8.59Hz, 2H), 8.18(d, *J*=8.62Hz, 2H). **MASS (ES+):** 669.1(M+Na)⁺ |
| IA.98 | 0.97(d, *J*=7.14Hz, 3H), 1:11(t, *J*=7.54Hz, 3H), 1.13(s.3H), 1.21-1.45(m, 1H), 1.55(s,3H), 1.60-2.07(m,5H), 2.17-2.68(m, 7H), 3.31-3.41(m, 1H), 4.22(dd, *J*₁=17.96Hz, *J*₂=3.50Hz, 1H), 4.40-4.42(d, 1H), 4.43(dd, *J*₁=17.47Hz, *J*=2.98Hz, 1H), 6.13(s, 1H), 6.35(d,d *J*₁=10.13Hz, *J*₂=1.83Hz, 1H), 6.86-7.03(m, 2H), 7.21(d, *J*=10.16Hz, 1H), 7.89-8.01(m, 1H). **MASS(ES+):** 627.1(M+Na)⁺ |
| IA.90 | 0.94(s, 3H), 1.12(t, *J*=7.54Hz, 3H), 1.20-1.29(m, 1H), 1.37(d, *J*=728Hz, 3H), 1.55(s,3H), 1.60-1.95(m,6H), 2.25-2.46(m, 7H), 3.1(s, 3H), 4.00(d, *J*=16.59Hz, 1H), 4.42(d, *J*=9.05Hz, 1H), 4.53(d, *J*=16.61Hz, 1H), 6.12(s, 1H), 6.35(d,d *J*₁=10.12Hz, *J*₁=1.75Hz, 1H), 7.19(d, *J*=10.15Hz,1H), 8.07(d, *J*=6.69Hz, 2H), 8.13(d, *J*=8.73Hz, 2H).**MASS(ES+):** 669.0(M+ Na)⁺ |
| IA.100 | 0.98(d, *J*=7.10Hz, 3H), 1.11(s,3H), 1.11(t, *J*=7.46Hz, 3H), 1.27-1.33(m, 1H), 1.54(s, 3H), 1.57-1.65(m, 4H), 2.14-2.41(m, 7H), 2.61-2.63(m, 1H), 3.35-3.37(m, 1H), 4.30(d, 1H, *J*=16.48Hz), 4.40(d, 1H, *J*=8.83Hz), 4.47(d, 1H, *J*=16.47Hz), 6.13(s,1H), 6.34(d,d *J*₁=10.13Hz, *J*₂=1.45Hz, 1H), 7.20(d, *J*=10.15Hz, 1H), 7.47(d, *J*=8.5Hz, 2H), 7.95(d,*J*=8.5Hz, 2H). **MASS (ES+):** 625.1(M+Na)⁺ |
| IA.101 | 0.97(s,3H), 1.14(t, *J*=7.50Hz, 3H), 1.18-1.27(m, 1H), 1.38(d, *J*=7.26Hz, 3H), 1.56(s, 3H), 1.60-1.64(m. 1H), 1.89-2.29(m, 6H), 2.35-2.47(m, 5H), 2.59-2.64(m, 1H), 3.97(d, *J*=16.8Hz, 1H), 4.43(d, *J*=8.17Hz, 1H), 455(d, *J*=16.82Hz, 1H), 6.13(s, 1H), 6.35(d, *J*=10.18Hz, 1H), 7.22(d, *J*=10.13Hz, 1H), 7.47(d, *J*=8.37Hz,2H), 7.92(d, *J*=8.38Hz,2H). **MASS (ES+):** 625.1(M+Na)⁺ |
| IA.102 | 1.05-1.15(m, 6H), 1.22-152(m, 9H), 1.76-2.47(m, 8H), 3.34-3.41(m, 1H), 4.07-4.16(m, 2H), 4.16-4.56(m, 3H), 5.23-5.48(m, 1H), 6.36(d, *J*=11.36Hz, 1H), 6.42(s, 1H), 7.11(d, *J*=9.95Hz, 1H), 7.76(d, *J*=8.11Hz, 2H), 8.11(d, *J*=8.12Hz, 2H). **MASS (ES+):** 693.5(M+Na)⁺ |
| IA.103 | 0.87(m, 4H), 0.96(d, *J*=6.88Hz, 3H), 1.24(s,3H), 1.29-1.35(m, 1H), 1.55(s,3H), 1.61-1.66(m,1H), 1.81-2.09(m, 2H), 2.28-2.50(m, 6H), 3.31-3.36(m, 1H), 4.42(d, *J*=8.86Hz, 1H), 4.97(d, *J*=16.53Hz, 1H), 5.32-5.48(m, 1H), 5.69(d, *J*=16.53Hz, 1H), 6.38(d,d *J*₁=10.17Hz, *J*₁=1.46Hz, 1H), 6.44(s, 1H), 7.16(d, *J*=8.50Hz, 2H), 7.23(t, *J*=8.64Hz, 3H), 7.94(dd, *J*₁=8.60Hz, *J*₂=5.35Hz, 2H). **MASS (ES+):** 623.0 (M+Na)⁺ |
| IA.104 | 0.86-096(m,4H), 0.98(d, *J*=7.35Hz, 3H), 1.12(s, 3H), 1.31-1.36(m, 1H), 1.53(s,3H), 1.60-1.65(m,1H), 1.70-2.03(m, 5H), 2.22-2.29(m, 2H), 2.39-2.45(m, 2H), 3.35-3.39(m, 1H), 4.26(d, *J*=17.62Hz, 1H), 4.42(d, *J*=8.24Hz, 1H), 4.43(d,*J*=17.30Hz, 1H), 5.31-5.47(m, 1H), 6.38(d,d, *J*₁=10.11Hz, *J*₂=1.09Hz, 1H), 6.43(s, 1H), 6.89-6.94(m, 1H), 6.96-7.01(m, 1H), 7.14(d, *J*=10.14Hz, 1H), 7.95(dd, *J*₁=15.13Hz, *J*₂=8.38Hz, 1H). **MASS (ES+):** 657.5(M+Na)⁺ |
| IA.105 | 1.07(d, *J*=7.14Hz, 3H), 1.10(s, 3H), 1.27(t, *J*=7.60Hz, 3H), 1.32-1.97(m, 8H), 2.23-2.44(m, 4H), 3.36-3.42(m, 1H), 4.09-4.14(m, 2H), 4.30(d, *J*=16.57Hz, 1H), 4.40(d, *J*=8.43Hz, 1H), 4.51(d, *J*=16.58Hz, 1H), 5.20-5.60(m, 1H), 6.36(dd *J*₁=10.15Hz, *J*₂=1.20Hz, 1H), 6.42(s, 1H), 7.12(d, *J*=10.14Hz, 1H), 7.47(d, *J*=8.52Hz, 2H), 7.95(d, *J*=8.54Hz, 2H). **MASS (ES+):** 659.4(M + Na)⁺ |
| IA.106 | 0.87-0.97(m,4H), 0.99(d, *J*=7.12Hz, 3H), 1.00(s, 3H), 1.31-1.36(m, 1H), 1.53(s,3H), 1.62-1.66(m,1H), 1.77-2.01(m, 3H). 2.25-2.42(m, 4H), 3.36-3.39(m, 1H), 4.28(d, *J*=16.54Hz, 1H), 4.42(d, *J*=14.05Hz, 1H), 4.46(d, *J*=16.73Hz, 1H), 5.30-5.48(m, 1H), 6.38(d,d, *J*₁=9.63Hz, *J*₂=1.85Hz, 1H), 6.43(s, 1H), 7.14(d, *J*=10.10Hz, 1H), 7.46(d, *J*=8.51Hz, 2H), 7.94(d, *J*=8.53Hz,2H). **MASS (ES+):** 655.2(M+Na)⁺ |
| IA.107 | 0.98(s,3H), 1.14(t, *J*=7.56Hz, 3H), 1.20-1.27(m, 1H), 1.38(d, *J*=7.23Hz, 3H), 1.56(s, 3H), 1.60-1.65(m, 1H), 1.91-1.98(m,3H), 2.04-2.30(m, 2H), 2.36.2.66(m, 7H), 3.88(s, 3H), 3.97(d, 1H, *J*=16.74Hz), 4.44(d, 1H, *J*=8.41Hz), 4.58(d, 1H, *J*=16.74Hz), 6.13(s, 1H), 6.35(d, *J*=10.07Hz, 1H), 6.95(d, *J*=8.74Hz, 2H), 7.24(d. *J*=10.33Hz, 1H), 7.97(d, *J*=8.74Hz, 2H). **MASS (ES+):** 621.3 (M+Na)⁺. |
| IA.108 | 0.98(d, *J*=7.15Hz, 3H), 1.11(s,3H), 1.11(t, *J*=7.56Hz, 3H), 1.27-1.33(m, 1H), 1.54(s, 3H), 1.57-1.94(m, 5H), 2.13-2.17(m, 1H), 2.32-2.41(m, 5H), 2.62-2.63(m, 1H),3.33-3.38(m, 1H), 4.33(d, 1H, *J*=16.52Hz), 4.41(d,1H, *J*=9.09Hz), 4.49(d, 1H, *J*=16.49Hz), 6.13(s, 1H), 6.34(d,d *J*₁=10.12Hz, *J*₂=1.81Hz,1H), 7.21(d, *J*=10.12Hz, 1H), 7.76(d, *J*=8.25Hz, 2H), 8.12(d, *J*=8.14Hz,2H). **MASS(ES+):** 659.3 (M+Na)⁺. |
| IA.109 | 0.53(s,3H), 0.93(d, *J*=6.88Hz, 3H), 1.07(t, *J*=7.52Hz, 3H), 1.12-1.90(m, 14H), 2.07.2.42(m,6H), 3.14-3.17(m, 1H), 3.84(s,3H), 4.28(d, *J*=7.12Hz, 1H), 5.25-5.42(m, 1H), 6.34(dd, *J*₁=10.14Hz, *J*₁=1.19Hz 1H), 6.40(s, 1H), 6.87(d, *J*=8.84Hz, 2H), 7.03(d, *J*=10.0Hz, 1H), 80.2(d, *J*=8.80Hz, 2H). **MASS (ES+):** 667.3 (M+Na)⁺. |
| IA.110 | 1.07(d, *J*=7.20Hz, 3H), 1.11(s, 3H), 1.27(t, *J*=7.12Hz, 3H),1.33-1.39(m, 1H), 1.52(s, 3H), 1.74-1.98(m, 6H), 2.33-2.45(m, 4H), 3.36-3.42(m, 1H), 4.08-4.14(m, 2H), 4.31(d, *J*=16.55Hz, 1H), 4.41(d, *J*=8.80Hz, 1Hz), 4.52(d, *J*=16.55Hz, 1H), 5.29-5.46(m, 1H), 6.37(dd *J*₁=10.15Hz, *J*₂=1.75Hz, 1H), 6.24(s, 1H), 7.13(dd *J*₁=10.15Hz, *J*₂=1.05Hz, 1H), 7.17-7.19(m, 2H), 8.03-8.07(m, 2H). **MASS (ES+):** 643.2 (M+Na)⁺. |
| IA.111 | 0.97(s,3H), 1.13(t, *J*=7.55Hz, 3H), 1.15-1.29(m, 1H), 1.38(d, *J*=7.33Hz, 3H), 1.56(s, 3H). 1.58-1.66(m, 1H), 1.88-2.14(m, 5H), 2.17-2.30(m, 1H), 2.35-2.64(m,5H), 4.01(d, *J*=16.84Hz, 1H), 4.45(d, *J*=8.23Hz, 1H), 4.57(d, *J*=16.84Hz, 1H), 6.13(s, 1H), 6.35(d,d *J*₁=10.12Hz, *J*₂=1.77Hz, 1H), 7.22(d, *J*=10.13Hz, 1H), 7.77(d, *J*=8.24Hz, 2H), 8.09(d, *J*=8.14Hz, 2H). **MASS (ES+):** 659.2 (M+Na)⁺. |
| IA.112 | 0.97(d, *J*=7.05Hz, 3H), 1.10(s, 3H), 1.12-1.48(m, 7H), 1.53(s,3H), 1.71-2.01(m, 8H), 2.20-2.43(m, 5H), 3.36-1.39(m, 1H), 4.30(d, *J*=16.59Hz, 1H), 4.43(d, *J*=8.27Hz, 1H), 4.51(d, *J*=16.56Hz, 1H), 5.31-5.47(m, 1H), 6.38(d,d *J*₁=10.12Hz, *J*₂=1.33Hz, 1H), 6.44(s, 1H), 7.16(d, *J*=10.07Hz, 1H), 7.76(d, *J*=8.15Hz, 1H), 8.11(d, *J*=8.08Hz). **MASS (ES+):** 731.2 (M+Na)⁺. |
| IA.113 | 0.97(d, *J*=7.06Hz, 3H), 1.10(s,3H), 1.15-1.45(m,6H), 1.53(s,3H), 1.58-166(m,1H), 1.72-2.0(m, 8H), 2.20-2.44(m, 5H), 3.36-3.4(m, 1H), 4.28(d, *J*=16.54Hz, 1H), 4.43(d, *J*=8.81Hz, 1H), 4.48(d, *J*=16.56Hz, 1H), 5.31-5.47(m,1H), 6.38(d,d *J*₁=10.15Hz, *J*₂=1.46Hz, 1H), 6.44(s,1H), 7.14(d, *J*=10.06Hz, 1H), 7.46(d, *J*=8.53Hz, 2H), 7.94(d, *J*=8.54Hz, 2H). **MASS (ES+):** 697.2(M+Na)⁺. |
| IA.114 | 0.96(d, *J*=7.05Hz,3H), 1.12(s,3H), 1.17-1.48(m,6H), 1.53(s,H), 1.58-1.65(m, 1H), 1.71-2.06(m, 8H), 2.20-2.43(m, 5H), 3.36-3.39(m, 1H), 4.27(d, *J*=17.51 Hz, 1H), 4.24-4.45(m, 2H), 5.31-5.47(m, 1H), 6.38(dd, *J*₁=10.12Hz, *J*₂= 1.43Hz, 1H), (1.44(s,1H), 6.89-7.01(m, 2H), 7.14(d, *J*=10.11Hz, 1H), 7.94(dd, *J*₁=15.1Hz, *J*₂=8.41Hz, 1H), **MASS (ES+):** 699.2(M+Na)⁺. |
| IA.115 | 0.94(d, *J*=7.07Hz, 3H), 1.24(s,3H), 1.28-1.46(m, 5H), 1.55(s,3H), 1.61-2.06(m,10H), 2.24-2.50(m, 5H), 3.32-3.37(m, 1H), 4.42(d, *J*=8.32Hz, 1H), 4.96(d, *J*=16.52Hz, 1H), 5.32-5.48(m, 1H), 5.68(d, *J*=16.53Hz, 1H). 6.38(d,d *J*₁=10.12Hz, *J*₂=1.30Hz, 1H), 6.44(s, 1H), 7.17(d, *J*=9.9Hz, 1H), 7.16-7.19(m, 2H), 7.94(dd, *J*₁=8.62Hz, *J*₂=5.31Hz, 2H). **MASS (ES+):** 665.5 (M+Na)⁺. |
| IA.116 | 0.96(d, *J*=7.14Hz, 3H), 1.10(s,3H), 1.14-1.43(m, 6H), 1.53(s,3H), 1.62-2.06(m,10H), 2.23-2.45(m, 4H), 3.38-3.42(m, 1H), 3.88(s, 3H), 4.27(d, *J*=16.51Hz, 1H), 4.42(d, *J*=8.73Hz, 1H), 4.53(d, *J*=16.53Hz, 1H), 5.31-5.47(m, 1H), 6.38(d,d *J*₁= 10.11Hz, *J*₂=1.66Hz, 1H), 6.44(s, 1H), 6.95(d, *J*=8.90Hz, 2H), 7.15(d, *J*=10.10Hz, 1H), 7.99(d, *J*₁=8.89Hz, 2H). **MASS (ES+):** 693.2(M+Na)⁺ |
| IA.117 | 0.98(d, *J*=7.03Hz, 3H), 1.10(s,3H), 1.24-1.34(m, 1H), 1.53(s,3H), 1.55-1.68(m,2H), 1.73-2.04(m, 9H), 2.20-2.41(m, 4H), 2.74-2.78(m, 1H), 3.36-3.40(m, 1H), 4.27(d, *J*=16.55Hz, 1H), 4.41(d, *J*=7.75Hz, 1H), 4.49(d, *J*=16.55Hz, 1H), 5.30-5.47(s, 1H), 6.37(d,d *J*=10.15Hz,1H), 6.43(s,1H), 7.13(d, *J*=10.12Hz, 1H), 7.47(d, *J*=8.36Hz, 1H), 7.95(d,*J*=8.36Hz,2H). **MASS (ES+):** 661.2 |
| IA.118 | 0.96(d, *J*=7.08Hz, 3H), 1.24(s,3H), 1.24-1.33(m, 1H), 1.55(s, 3H), 1.56-1.92(m, 9H), 2.022.50(m, 5H), 2.73-2.81(m,1H), 3.32-3.36(m, 1H), 4.42(d, *J*=7.77Hz, 1H), 4.97(d, *J*=16.51Hz, 1H), 532-5.48(m, 1H), 5.68(d, *J*=16.56Hz, 1H), 6.38(d,d *J*₁=10.08Hz, *J*₂=1.22Hz, 1H), 6.44(s, 1H), 7.14-7.19(m, 3H), 7.93-7.96(m,2H). **MASS (ES+):** 629.3 |
| IA.119 | 0.98(d, *J*=6.93Hz, 3H), 1.10(s,3H), 1.16(t, *J*=6.88Hz, 6H), 1.31-1.35(m, 1H), 1.53(s, 3H), 1.58-2.01(m, 4H), 2.21-2.62(m, 5H), 3.36-3.38(m, 1H), 4.31(d, 1H, *J*=16.58Hz), 4.42(d, *J*=7.40Hz, 1H), 4.51(d, *J*=16.58Hz, 1H), 5.30-5.47(m, 1H), 6.38(d, *J*=10.15Hz, 1H), 6.43(s, 1H), 7.13(d, *J*=10.11Hz, 1H), 7.76(d, *J*=7.98Hz, 2H), 8.11(d, *J*=7.91Hz, 2H). **MASS(ES+):** 669.2 |
| IA.120 | 0.98(d, *J*=7.12Hz, 3H), 1.12(s,3H), 1.29-1.35(m, 1H), 1.53(s,3H), 1.56-1.66(m,5H), 1.70-2.00(m, 7H), 2.20-2.40(m, 4H), 2.73-2.77(m, 1H), 3.35-3.40(m, 1H), 4.26(d,d *J*₁=17.47Hz, *J*₂=2.37Hz, 1H), 4.42(d, *J*=5.81Hz, 1H), 4.54(d,d *J*₁=17.35Hz, *J*₂=2.61Hz, 1H), 5.31-5.47(m, 1H), 6.38(d,d *J*₁=10.14Hz, *J*₂=11.38Hz, 1H), 6.43(s, 1H), 6.89-7.01(m, 1H), 7.13(d, *J*=10.09Hz, 1H), 7.94(dd. *J*₁=15.10Hz), *J*₂=8.46Hz, 2H). **MASS (ES+):** 663.3 |
| IA.121 | 0.95(d, *J*=7.14Hz, 3H), 1.13(d, *J*=7.00Hz, 6H), 1.22(s, 3H), 1.28-1.34(m, 1H), 1.55(s,3H), 1.74-2.18(m, 4H), 2.28-2.57(m, 4H), 2.58-2.62(m, 1H), 2.30-2.38(m, 1H), 3.30-3.38(m, 1H), 4.42(d, *J*=8.92Hz, 1H), 4.97(d, *J*=16.5Hz, 1H), 5.32-5.49(m, 1H), 5.69(d, *J*=16.53Hz, 1H), 6.38(d,d *J*₁=10.13Hz, *J*₂=1.75Hz, 1H), 6.44(s, 1H), 7.14-7.20(m, 3H), 7.93-7.97(m, 2H), **MASS (FS+):** G03.3. |
| IA.122 | 0.98(d, *J*=7.14Hz, 3H), 1.11(s,3H), 1.16(d, *J*=2.98Hz, 3H), 1.17(d, *J*=3.00Hz, 3H), 1.31-1.34(m, 1H), 1.53(s,3H), 1.71-2.03(m,3H), 2.24-2.42(m, 4H), 2.57-2.61(m, 1H), 3.40-3.43(m, 1H), 3.88(s, 3H), 4.28(d, *J*=16.49Hz, 1H), 4.42(d, *J*=8.74Hz, 1H), 4.54(d, *J*=16.50Hz, 1H), 5.30-5.47(m, 1H), 6.37(d,d *J*₁=10.13Hz, *J*₂=1.77Hz, 1H), 6.43(s, 1H), 6.95(d, *J*=8.89Hz, 2H), 7.14(dd, *J*₁=10.18Hz, *J*₂=1.12Hz, 3H), 7.99(d, *J*=8.87Hz, 2H). **MASS (ES+):** 653.3 |
| IA.123 | 0.97(d, *J*=7.14Hz, 3H), 1.13(s, 3H), 1.16(d,d, *J*₁=6.99Hz, *J*₂=3.08Hz, 6H), 1.29-1.33(m, 3H), 1.53(s,3H), 1.73-2.02(m,4H), 2.23-2.47(m, 4H), 2.55-2.62(m, 1H), 3.36-3.41(m, 1H), 4.28(d,d, *J*₁=17.54Hz, *J*₂=2.63Hz, 1H), 4.42(d. 1H), 4.44(d,d, *J*₁=17.54Hz, *J*₂=2.90Hz, 1H), 5.31-5.48(m, 1H), 6.38(d,d, *J*₁=10.13Hz, *J*₂=1.79Hz, 1H), 6.44(s, 1H), 6.89-7.01(m, 2H), 7.13(d,d, *J*₁=10.10Hz, *J*₂=1.16Hz, 1H), 7.92-7.98(m, 1H). **MASS (ES+):** 637.3 |
| IA.124 | 1.07(d, *J*=7.13Hz, 3H), 1.17(s,3H), 1.33-1.41(m, 1H), 1.55(s, 3H), 1.73-2.04(m, 3H), 2.28-2.54(m, 5H), 3.43-3.48(m, 1H), 4.35(d, *J*=16.65Hz, 1H), 4.46(d, *J*=8.34Hz, 1H), 4.54(d, *J*=16.65Hz, 1H), 5.33-5.49(m, 1H), 6.41(dd, *J*₁=10.12Hz, *J*₂=1.69Hz 1H), 6.42(s, 1H), 6.51(dd, *J*₁=3.46Hz, *J*₂=1.69Hz, 1H), 7.09-7.21(m, 2H), 7.59(d, *J*=0.72Hz, 1H), 7.77(d, *J*=8.25Hz, 2H), 8.72(d, *J*=8.18Hz 2H). **MASS (ES+):** 693.3 |
| IA.125 | 0.59(s,3H), 0.93(d, *J*=6.99Hz, 3H), 1.07(t, *J*=7.54Hz, 3H), 1.21-1.88(m, 14H), 2.09.2.36(m, 6H), 3.13-3.17(m, 1H), 4.31(d, *J*=7.52Hz, 1H), 5.27-5.42(m, 1H), 6.35(dd, *J*₁=10.08Hz, *J*₂=1.16Hz 1H), 6.41(s, 1H), 7.07(d, *J*=10.11Hz, 1H), 7.36(d, *J*=8.47Hz, 2H), 7.2(d, *J*=8.49Hz, 2H). **MASS (ES+):** 649.2 |
| IA.126 | 1.07(d, *J*=7.12Hz, 3H), 1.11(s, 3H), 1.16-1.87(m,11H), 1.90-2.09(m, 2H), 2.13-2.44(m, 4H), 3.38-3.42(m, 1H), 4.06-4.19(m, 2H), 4.31(d, *J*=16.58Hz, 1H), 4.40(d, *J*=8.68,Hz, 1H), 4.55(d, *J*=16.55Hz, 1H), 5.29-5.47(m, 1H), 6.37(dd *J*₁=10.17Hz, *J*₂=1.45, 1H), 6.42(s, 1H), 6.96(d, *J*=8.81Hz, 2H), 7.13(d, *J*=10.19Hz, 1H), 8.00(d, *J*=8.83Hz, 2H). **MASS (ES+) :** 633 |
| IA.127 | 1.07(d, *J*=7.06Hz, 3H), 1.17(s,3H), 1.23-2.10(m, 8H), 2.17-2.53(m, 4H), 3.46-3.50(m, 1H), 3.88(s, 3H), 4.31(d, *J*=16.63Hz, 1H), 4.45(d, *J*=8.39Hz, 1H), 4.57(d, *J*=16.60Hz, 1H), 5.32-5.49(m, 1H), 6.40(dd, *J*₁=10.14Hz, *J*₂=1.36Hz 1H), 6.45(s, 1H), 6.50(dd, *J*₁=3.4Hz, *J*₂=1.47Hz 1H), 6.96(d, *J*=8.82Hz, 2H), 7.13-7.21(m, 2H), 7.59(s, 1H), 8.00(d, *J*=8.80Hz 2H). **MASS(ES+):** 655.2 |
| IA.128 | 0.88(t, *J*=Hz, 3H), 0.99(d, *J*=Hz, 1H), 1.11(s, 3H), 1.22-1.32(m, 4H),1.53-1.97(m, 8H), 2.20-2.46(m, 6H), 3.36-3.40(m, 1H), 4.3(d, *J*=Hz, 1H, 4.41(d, *J*=Hz, 1H), 4.49(d, *J*=Hz, 1H), 5.30-5.47(m, 1H), 6.38(dd *J*₁=Hz, *J*₂=Hz, 1H), 6.43(s. 1H), 7.13(dd *J*₁=Hz, *J*₂=Hz, 1H), 7.76(d, *J*=Hz, 2H), 8.12(d, *J*=Hz, 2H). **MASS (ES+) :** 683.2 |
| IA.129 | 0.88(t, *J*=7.32Hz, 3H), 0.99(d, *J*=7.10Hz, 3H), 1.11(s, 3H), 1.19-1.32(m, 4H), 1.48-1.97(m, 8H), 2.20-2.48(m, 6H), 3.36-3.40(m, 1H), 4.30(d, *J*=16.53Hz, 1H), 4.41(d, *J*=7.86Hz, 1H), 4.47(d, *J*=16.56Hz, 1H), 5.30-5.47(m, 1H), 6.38(dd *J*₁=10.12Hz, *J*₂=1.53Hz, 1H), 6.43(s, 1H), 7.13(d, *J*=10.02Hz, 1H), 7.47(d, *J*=8.48Hz, 2H), 7.95(d, *J*=8.48Hz, 2H). **MASS (ES+):** 649.3 |
| IA.130 | 0.88(t, *J*=7.32Hz, 3H), 0.98(d, *J*=7.11Hz, 3H), 1.13(s, 3H), 1.18-1.42(m, 4H), 1.45-1.99(m, 8H), 2.20-2.46(m, 6H), 3.36-3.39(m, 1H), 4.30(d, *J*=17.38Hz, 1H), 4.41-4.46(m, 2H), 5.30-5.47(m, 1H), 6.38(dd *J*₁=10.13Hz, *J*₂=1.55Hz, 1H), 6.43(s, 1H), 6.89-6.95(m, 1H), 6.97-7.01(m, 1H), 7.13(d, *J*=9.99Hz, 1H), 7.92-7.98(m, 1H). **MASS (ES+) :** 651.4 |
| IA.131 | 0.88(t, *J*=7.35Hz, 3H), 0.99(d, *J*=12Hz, 3H), 1.11(s, 3H), 1.21-1.38(m, 3H), 1.45-1.99(m, 9H), 2.20-2.46(m, 6H), 3.36-3-40(m, 1H), 4.31(d, *J*=16.51Hz, 1H), 4.41(d, *J*=7.65Hz, 1H), 4.49(d, *J*=16.50Hz, 1H), 5.30-5.47(m, 1H), 6.38(dd *J*₁=10.12Hz, *J*₂=1.69Hz, 1H), 6.43(s, 1H), 7.12-7.18(m, 3H), 8.03-8.06(m, 2H). **MASS (ES+) :** 633.4 |
| IA.132 | 0.98(d, *J*=7.04Hz, 3H), 1.11(s,3H), 1.16(d, *J*=5.6Hz, 6H), 1.30-1.35(m, 1H), 1.53(s,3H), 1.73-2.01(m, 4H), 2.21-2.60(m, 5H), 3.37-3.40(m, 1H), 4.28(d, *J*=16.48Hz, 1H), 4.42(d, *J*=7.47Hz, 1H), 4.48(d, *J*=16.59Hz, 1H), 5.30-5.47(m, 1H), 6.38(d, *J*=10.17Hz, 1H), 6.43(s, 1H), 7.13(d, *J*=10.11Hz, 1H), 7.473(d, *J*=8.33Hz, 2H), 7.95(d, *J*=8.39Hz, 2H). **MASS (ES+):** 635.2 |
| IA.133 | 0.96(d, *J*=7.13Hz, 3H), 1.24(s,3H), 1.27-1.33(m, 1H), 1.55(s,3H), 1.75-2.06(m,6H), 2.19-2.38(m, 8H), 3.13-3.19(m, 1H), 3.33-3.37(m, 1H), 4.41(d, *J*=9.04Hz, 1H), 5.00(d, *J*=16.54Hz, 1H), 5.31-5.48(m, 1H), 5.71(d, *J*=16.57Hz, 1H), 6.38(d,d *J*₁=10.15Hz, *J*₂=1.70Hz, 1H), 6.44(s, 1H), 7.15(d,d *J*₁=9.99Hz, *J*₂=1.10Hz, 1H), 7.18(t, *J*=8.54Hz, 2H), 7.94-7.97(m, 2H). **MASS (ES+) :** 615.1 |
| IA.134 | 0.98(d, *J*=7.16Hz, 3H), 1.10(s,3H), 1.30-1.36(m, 1H), 1.53(s, 3H), 1.53-1.98(m, 12H), 2.23-2.42(m, 4H), 2.72-2.80(m, 1H), 3.36-3.40(m, 1H), 4.33(d, *J*=16.54Hz,1H), 4.42(d, *J*=8.83Hz, 1H), 4.49(d, *J*=16.53Hz,1H), 5.30-5.47(m, 1H), 6.38(d,d *J*₁=10.14Hz, *J*₂=1.80Hz,1H), 6.43(s, 1H), 7.12(d,d, *J*₁=10.19Hz, *J*₂=1.24Hz, 1H), 7.76(d, *J*=8.27Hz, 2H), 8.11(d, *J*=8.14Hz, 2H) **MASS (ES+) :** 695.3 |
| IA.135 | 0.99(d, *J*=7.13Hz, 3H), 1.11(s,3H), 1.25-1.32(m, 1H), 1.52(s,3H), 1.72-2.04(m,6H), 2.18-2.40(m, 8H), 3.14-3.16(m, 1H), 4.31(d, *J*=16.5Hz, 1H), 4.40(d, *J*=8.48Hz, 1H), 4.48(d, *J*=16.52Hz, 1H), 6.37(d,d *J*₁=10.12Hz, *J*₂=1.72Hz, 1H), 6.43(s, 1H), 7.12(d,d *J*₁=10.12Hz, *J*₂=0.8Hz, 1H), 7.47(d, *J*=8.57Hz, 2H), 7.95(d, *J*=8.59Hz, 2H). **MASS (ES+) :** 647.2 |
| IA.136 | 0.99(d, *J*=7.12Hz, 3H), 1.10(s,3H), 1.29-1.35(m, 1H), 1.53(s, 3H), 1.76-1.99(m, 6H), 2.17-2.45m, 8H), 3.12-3.21(m, 1H). 3.35-3.41(m, 1H), 4.33(d, 1H, *J*=16.59Hz), 4.41(d, *J*=8.63Hz, 1H), 4.51(d, 1H, *J*=16.56Hz), 5.30-5.47(m, 1H), 6.37(d,d *J*₁=10.16Hz, *J*₂=1.61Hz, 1H), 6.43(s, 1H), 7.13(d,d *J*₁=10.18Hz, *J*₂=0.73Hz, 1H), 7.77(d, *J*=8.24Hz, 2H), 8.12 (d, *J*=8.14Hz, 2H). **MASS (ES+) :** 681.2 |
| IA.137 | 0.98(d, *J*=7.13Hz, 3H), 1.12(s, 3H), 1.29-1.35(m, 1H), 1.53(s, 3 H), 1.70-2.00(m, 5H), 2.16-2.45(m, 7H), 3.13-3.18(m, 1H), 3.36-3.41(m, 1H), 4.29(d,d, *J*₁=17.52Hz, *J*₂=2.62Hz, 1H), 4,41(d, *J*=9.87Hz, 1H), 4.45 (d,d, *J*₁=17.67Hz, *J*₂=2.71Hz. 1H), 5.31-5.47(m, 1H), 6.38(d,d, *J*₁=10.12Hz, *J*₂=1.71Hz, 1H), 6.43(s, 1H), 6.89-7.02(m, 2H), 7.14(d, *J*=10.13Hz, 1H), 7.92-7.98(m, 1H). **MASS (ES+) :** 649.3 |
| IA.138 | 0.96(d, *J*=7.16Hz, 3H), 1.11(s,3H), 1.29-1.34(m, 1H), 1.52(s,3H), 1.84-2.04(m,6H), 2.17-2.39(m, 8H), 3.14-3.19(m, 1H), 3.38-3.43(m, 1H), 3.88(s, 3H), 4.30(d, *J*=16.51Hz, 1H), 4.40(d, *J*=8.92Hz, 1H), 4.53(d, *J*=16.51Hz, 1H), 5.30-5.47(m, 1H), 6.37(d,d *J*₁=10.13Hz, *J*₂=1.74Hz, 1H), 6.43(s, 1H), 6.96(d, *J*=8.85Hz, 2H), 7.14(d, *J*=10.11Hz, 1H), 7.99(d, *J*=8.85Hz, 2H), **MASS (ES+) :** 643.3 |
| IA.139 | 1.06(d, *J*=7.12Hz,3H), 1.17(s,3H), 1.33-1.38(m, 1H), 1.56(s, 3H), 1.60-1.99(m, 5H), 2.29-2.65(m, 4H), 3.41-3.46(m, 1H), 4.35(d,1H, *J*=16.58Hz), 4.46(d,1H, *J*=8.88Hz), 4.53(d, 1H, *J*=16.55Hz), 6.15(s,1H), 6.37(d,d *J*₁=10.13Hz, *J*₂=1.68Hz,1H), 6.49(d,d *J*₁=3.42Hz, *J*₂=1.65Hz,1H), 7.12(d, *J*=3.47Hz,1H), 7.22(d, *J*=10.12Hz, 1H), 7.58(d, *J*=1.48Hz, 1H), 7.76(d, *J*=8.35Hz, 2H), 8.12(d, *J*=8.26Hz, 2H), **MASS (ES+) :** 675.3 |
| IA.140 | 1.05(d, *J*=7.1Hz, 3H), 1.19(s,3H), 1.32-1.37(m, 1H), 1.57(s, 3H), 1.60-2.02(m, 5H), 2.29-2.67(m, 5H), 3.42-3.47(m, 1H), 4.31(dd, *J*₁=17.52Hz, *J*₂=2.24Hz, 1H), 4.46(d, *J*=8.89Hz, 1H), 4.47(dd, 1H), 6.15(s, 1H), 6.37(d,d *J*₁=10.11Hz, *J*₂=1.09Hz, 1H), 6.48(d,d *J*₁=3.04Hz, *J*₂=1.33Hz, 1H), 6.88-7.01(m, 2H), 7.12(d, *J*=3.45Hz, 1H), 7.23(d *J*=10.12Hz, 1H), 7.58(d), 7.95(d,d *J*₁=15.1Hz, *J*₂=8.46Hz, 1H). **MASS (ES+) :** 643.2 |
| IA.141 | 1.05(d, *J*=7.09Hz, 3H), 1.17(s,3H), 1.25-1.38(m, 1H), 1.56(s, 3H), 1.60-2.00(m, 5H), 2.29-2.65(m, 4H), 3.43-3.45(m, 1H), 4.32(d, 1H, *J*=16.52Hz), 4.45(d, 1H, *J*=9.02Hz), 4.50(d, 1H, *J*=16.61Hz), 6.15(s,1H), 6.37(d,d *J*₁=10.07Hz, *J*₂=0.81Hz, 1H), 6.49(d, *J*=3.33Hz, 1H), 7.12(d, *J*=3.43Hz, 1H), 7.22(d, *J*=10.12Hz, 1H), 7.41(d, *J*=8.40Hz, 2H), 7.58(s, 1H), 7.95(d, *J*=8.45Hz, 2H). **MASS (ES+) :** 641.3 |
| IA.142 | 1.06(d, *J*=7.12Hz, 3H), 1.18(s, 3H), 1.31-1.37(m, 1H), 1.56(s, 3H), 1.60-2.04(m,4H), 2.29-2.65(m, 5H), 3.44-3.48(m, 1H), 3.88(s, 3H), 4.33(d, *J*=16.64Hz, 1H), 4.45(d, *J*=8.94Hz, 1H), 4.54(d, *J*=16.50Hz, 1H), 6.15(s, 1H), 6.36(d,d *J*₁=10.11Hz, *J*₂=1.73Hz, 1H), 6.49(d,d *J*₁=3.46Hz, *J*₂=1.70Hz,1H), 6.95(d, *J*=8.89Hz, 2H), 7.12 (d *J*=13.39Hz, 1H), 7.23(d, *J*=10.15Hz, 1H), 7.58(s, 1H), 7.99(d, *J*₁=8.66Hz, 2H), **MASS (ES+) :** 637.4 |
| IA.143 | 1.05(d, *J*=7.12Hz, 3H), 1.18(s,3H), 1.25-1.35(m, 1H), 1.57(s,3H). 1.63-1.99(m, 5H), 2.03-2.64(m, 5H), 3.45(m, 1H), 4.32(d, *J*=16.52, 1H), 4.45(d, *J*=9.01,1H), 4.53(d, *J*=16.54,1H), 6.15(s, 1H), 6.37(d,d *J*₁=10.11Hz. *J*₂=1.75Hz, 1H), 6.49(d,d *J*₁=3.47Hz, *J*₂=1.71Hz, 1H), 7.13-7.25(m, 4H), 7.58(d, *J*=0.81Hz, 1H), 8.03-8.07(m,2H). **MASS (ES+) :** 647.2 (M+Na)⁺ |
| IA.144 | 0.96(d, *J*=7.17Hz, 3H), 1.04(s,3H), 1.15(d, *J*=3.84Hz, 3H), 1.17(d, *J*=3.86Hz, 3H), 1.28-1.34(m, 1H), 1.52(s,3H), 1.76-1.89(m, 4H), 2.25(s,6H), 2.28(s, 3H), 2.22-2.41(m, 4H), 2.55-2.62(m, 1H), 3.38-3.43(m, 1H), 4.03(d, *J*=17.76Hz, 1H), 4.30(d, *J*=17.79Hz, 1H), 4.37(d, *J*=8.81Hz, 1H), 5.30-5.47(m, 1H), 6.37(dd, *J*₁=10.14Hz, *J*₂=1.86Hz, 1H), 6.43(s,1H). 6.85(s, 2H), 7.12(dd, *J*₁=10.13Hz, *J*₁=1.29Hz, 1H). **MASS (ES+) :** 643.5 |
| IA.145 | 0.95(d, *J*=7.15Hz, 3H), 1.02(s,3H), 1.20-1.42(m, 8H), 1.52(s, 3H), 1.72-1.89(m, 6H), 2.25(s, 6H), 2.28(s, 3H), 2.25-2.41(m, 5H), 3.37-3.43(m, 1H), 4.02(d, *J*=17.62Hz, 1H), 4.30(d, *J*=17.83Hz, 1H), 4.38(d, *J*=8.83Hz, 1H), 5.30-5.47(m, 1H), 6.38(dd, *J*₁=10.14Hz, *J*₂=1.86Hz, 1H), 6.44(s,1H), 6.85(s, 2H), 7.13(dd, *J*₁=10.14Hz, *J*₂=1.39Hz, 1H). **MASS (ES+) :** 705.3 (M + Na)⁻. |
| IA.146 | 0.96(d, *J*=7.16Hz, 3H), 1.03(s,3H), 1.25-1.34(m, 1H), 1.52(s, 3H), 1.54-1.69(m, 4H), 1.76-1.89(m, 7H), 2.25(s, 6H), 2.28(s, 3H), 2.27-2.80(m, 1H), 3.37-3.43(m, 1H), 4.03(d, *J*=17.77Hz, 1H), 4.30(d. *J*=17.77Hz, 1H), 4.37(d, *J*=8.7Hz, 1H), 5.30-5.47(m, 1H), 6.37(dd, *J*₁=10.14Hz, *J*₂=1.82Hz, 1H), 6.43(s,1H), 6.85(s, 2H), 7.12(dd, *J*₁=10.14Hz, *J*₂=1.12Hz, 1H). **MASS (ES+) :** 669.4 |
| IA.147 | 0.97(d, *J*=7.17Hz, 3H), 1.02(s,3H), 1.28-1.34(m, 1H), 1.52(s,3H), 1.72-1.97(m, 6H), 2.18-2.23(m, 5H), 2.26(s, 6H), 2.28(s, 3H), 2.30-2.43(m, 2H), 3.12-3.20(m, 1H), 3.38-3.43(m, 1H), 4.06(d, *J*=17.72Hz, 1H), 4.29(d, *J*=17.72Hz, 1H), 4.37(d, *J*=8.92Hz, 1H), 5.30-5.47(m, 1H), 6.38(dd, *J*₁=10.15Hz, *J*₂=1.87Hz, 1H), 6.43(s,1H), 6.85(s,2H), 7.11(dd, *J*₁=10.13Hz, *J*₂=1.27Hz, 1H). **MASS (ES+) :** 655.4 |
| IA.148 | 0.91-0.94(m, 2H), 0.98(d, *J*=7.11Hz, 3H), 1.03(s, 3H), 1.09-1.26(m, 5H), 1.52-(s,3H), 1.65-1.75(m, 8H), 1.83-1.93(m, 2H), 2.17-2.22(m, 3H), 2.25(s, 6H), 2.28(s, 3H), 2.34-2.38(m, 1H), 3.37-3.41(m, 1H), 4.06(d, *J*=17.78Hz, 1H), 4.27(d, *J*=17.79Hz, 1H), 4.27(d, *J*=9.08Hz, 1H), 5.30-5.46(m, 1H), 6.38(d,d, *J*₁=10.14Hz, *J*₂=1.85Hz, 1H), 6.43(s, 1H), 6.85(s, 2H), 7.12(d,d, *J*₁=10.12Hz, *J*₂=1.20Hz, 1H). **MASS (ES+) :** 719.4 (M+Na)⁺ |
| IA.149 | 1.07(d, *J*=7.13Hz, 3H), 1.20(s,3H), 1.35-1.40(m, 1H), 1.56(s, 3H), 1.72-1.98(m, 3H), 2.25(s, 6H), 2.28(s, 3H), 2.32-2.44(m, 4H), 3.44-3.49(m, 1H), 4.13(d, *J*=17.69Hz, 1H), 4.23(d, *J*=17.69Hz, 1H), 4.40(d, *J*=8.86Hz, 1H), 4.53-4.55(m, 1H), 5.34-5.51(m, 1H), 6.36(dd, *J*₁=10.10Hz, *J*₂=1.79Hz, 1H), 6.41(s,1H), 6.86(s, 2H), 7.10(dd, *J*₁=4.94Hz, *J*₂=3.81Hz, 1H). 7.25(dd, *J*₁=10.12Hz, *J*₂=1.05Hz, 1H), 7.58(dd, *J*₁=4.98Hz, *J*₂=1.15Hz, 1H), 7.73(dd, *J*₁=3.65Hz, *J*₂=1.16Hz, 1H). **MASS (ES+)**: 683.9 |
| IA.150 | 0.88-0.94(m, 2H), 0.99(d, *J*=7.10Hz, 3H), 1.13(s, 3H), 1.17-1.35(m, 4H), 1.53(s, 3H), 1.64-1.98(m, 10H), 2.17-2.46(m, 6H), 3.35-3.39(m, 1H), 4.30(d,d, *J*₁=15.16Hz, *J*₂=2.50Hz, 1H), 4.40(d, 1H), 4.42(d,d. *J*₁=17.58Hz, *J*₂=2.47Hz, 1H), 5.31-5.45(m, 1H), 6.38(d,d. *J*₁=10.13Hz. *J*₂=1.65Hz, 1H), 6.43(s, 1H), 6.89-7.01(m, 2H), 7.13(d,d, *J*₁=10.11Hz, *J*₂=1.11Hz, 1H), 792-7.98(m, 1H), **MASS (ES+)** : 691.5 |
| IA.141 | 0.91-0.97(m, 2H), 0.99(d, *J*=7.15Hz, 3H), 1.11(s, 3H), 1.13-1.35(m, 4H), 1.52(s, 3H), 1.61-1.95(m, 9H), 2.19-2.40(m, 6H), 3.35-3.40(m, 1H), 4.33(d, *J*=16.4Hz, 1H), 4.41(d, *J*=12.88Hz, 1H), 4.45(d, *J* =16.53Hz, 1H), 5.30-5.47 (m, 1H) 6.38(d,d *J*₁=10.12Hz, *J*=1.80Hz, 1H), 6.43(s,1H), 7.13(d,d *J*₁=10.14Hz, *J*₂=1.27Hz, 1H), 7.46(d, *J*=8.55Hz, 2H), 7.94(d, *J*=8.61Hz, 2H). **MASS (ES+)** : 711.3 (M+Na)⁺ |
| IA.152 | 0.91-0.97(m, 2H), 1.00(d, *J*=7.13Hz, 3H), 1.11(s,3H), 1.13-1.36(m, 4H), 1.53(s, 3H), 1.61-1.91(m, 10H), 2.20-2.45(m, 6H), 3.34-3.40(m, 1H), 4.35(d, *J*=16.56Hz, 1H), 4.41(d, *J*=8.94Hz, 1H), 4.48(d, *J*=16.54 Hz, 1H), 5.30-5.47(m, 1H), 6.38(d,d *J*₁=10.15Hz, *J*₂=1.87Hz. 1H). 6.43(s, 1H), 7.12(d,d, *J*₁=10.13Hz, *J*₂=1.26Hz, 1H), 7.76(d, *J*=8.28Hz, 2H), 8.11(d, *J*=8.16Hz, 2H). **MASS (ES+)** : 723.1 |
| IA.I53 | 0.91-0.97(m, 2H). 1.00(d, *J*=7.11Hz, 3H), 1.12(s, 3H), 1.09-1.36(m, 4H), 1.52(s, 3H). 1.61-1.87(m, 10H). 2.20-2.39(m, 6H), 3.37-3.42(m, 1H), 3.88(s, 3H), 4.34(d, *J*=16.49H, 1H), 4.40(d, *J*=9.19Hz, 1H), 4.48(d, *J*=16.43Hz, 1H), 5.30-5.47(m, 1H), (6.37(d,d *J*₁=10.14Hz), *J*₂=1.76Hz, 1H), 6.43(s, 1H), 6.95(d, *J*=8.87Hz, 2H), 7.13(d,d *J*₁=10.13Hz, *J*₂=1.02Hz, 1H), 7.99(d, *J*=8.89Hz, 2H), **MASS (ES+)** : 707.3 (M + Na)⁺ |
| IA.154 | 0.89(t, *J*=7.34Hz, 3H), 0.97(d, *J*=7.10Hz, 3H), 1.03(s, 3H), 1.25-1.96(m, 8H), 2.09-2.40(m, 15H), 3.38-3.42(m, 1H), 4.05(d, *J*=17,80Hz, 1H), 4.29(d, *J*=17.72Hz, 1H), 4.39(d, *J*=7.99Hz, 1H), 5.30-5.47(m, 1H), 6.39(dd *J*₁=10.10Hz, *J*₂=1.65Hz, 1H), 6.43(s, 1H), 6.85(s, 2H). 7.13(d, *J*=10.07Hz, 1H). **MASS (ES+)** : 657.3 |
| IA.155 | 1.03(s, 3H), 1.06(d, *J*=7.19Hz, 3H), 1.26(t, *J*=7.09Hz, 3H), 1.32-1.94(m, 10H), 2.22-2.44(m, 12H), 3.37-3.42(m, 1H), 4.04-4.18(m, 3H), 4.30(d, *J*=17.74Hz, 1H), 4.36-4.39(m, 1H), 5.30-5.45(m, 1H), 6.37(dd, *J*₁=10.14Hz, *J*₂=1.67Hz, 1H), 6.42(s, 1H), 6.86(s, 2H), 7.12(d, *J*=10.21Hz, 1H). **MASS (ES+)** : 645.1 |
| IA.156 | 1.06(d, *J*=7.19Hz, 3H), 1.11(s,3H), 1.25-1.97(m, 8H), 2.25(s, 6H), 2.28(s, 3H), 2.30-2.SJ(m. 3H), 3.46-3.51(m, 1H), 4.07(d,*J*=17.84Hz, 1H), 4.31(d,*J*=17.82Hz, 1H), 5.34-5.46(m, 1H),6.41(dd, *J*₁=10.14Hz, *J*₂=1.87Hz, 1H), 6.51(dd, *J*₁=3.50Hz.J₂=1.73Hz, 1H). 6.85(s. 2H), 7.12-7.16(m, 2H), 7.59(d, *J*=1.01Hz, 1H). **MASS (ES+)** : 667.3 |
| IA.157 | 1.07(d, *J*=7.10Hz, 3H), 1.17(s, 3H), 1.21-2.03(m, 8H), 2.17-2.55(m, 4H), 3.45-3.48(m, 1H), 4.32(d, *J*=16.56Hz, 1H), 4.46(d, *J*=8.46Hz, 1H), 4.51(d, *J*=15.90Hz, 1H), 5.7-5.49(m, 1H), 6.40(dd, *J*₁=10.14Hz, *J*₂=1.70Hz 1H), 6.45(s. 1H), 6.51(dd. *J*₁=3.44Hz, *J*₂=1.67Hz 1H), 7.13-7.16(m, 2H), 7.47(d, *J*=8.56Hz, 2H), 7.59(s, 1H), 7.96(d, *J*=8.59Hz 2H). **MASS (ES+)** : 659.5 |
| IA.158 | 0.97(s,3H), 1.14(t, *J*=7.54Hz, 3H), 1.19-1.25(m, 1H), 1.38(d, *J*=7.35Hz, 3H), 1.54(s, 3H), 1.92-2.14(m, 4H), 2.26-2.48(m, 5H), 3.97(d, *J*=16.90Hz, 1H), 4.45(d, *J*=8.33Hz, 1H), 4.56(d, *J*=16.89Hz, 1H), 5.31-5.48(m, 1H), 6.38(dd *J*₁=10.14Hz, *J*₂=1.85Hz 1H), 6.44(s,1H), 7.14(dd *J*₁=10.12Hz, *J*₂=1.22Hz, 1H), 7.47(d, *J*=8.60Hz, 2H), 7.93(d, *J*=8.59Hz, 2H). **MASS (ES+)**: 643.3 (M+Na)⁺ |
| IA.159 | 1.08(d, *J*=7.14Hz, 3H), 1.17(s, 3H), 1.37-1.42(m, 1H), 1.55(s,3H), 1.78-1.94(m, 4H), 2.00-2.57(m, 4H), 3.44-3.49)(m, 1H), 4.37(d, *J*=16.62Hz, 1H), 4.47(d, *J*=6.55Hz, 1H), 4.53(d, *J*=16.57Hz, 1H), 5.33-5.55(m, 1H), 6.40(d,d *J*₁=10.15Hz, *J*₂=1.80Hz, 1H), 6.46(s, 1H), 7.11)(d,d *J*₁=4.95Hz, *J*₂=3.87Hz,1H), 7.15(d, *J*=10.14Hz, 1H), 7.58(d,d *J*₁=5.02Hz, *J*₂=1.10Hz,1H), 7.74(d,d *J*₁=3.85Hz, *J*₂=1.12Hz, 1H). 7.76(d, *J*=8.39Hz, 2H), 8.10(d, *J*=8.23Hz, 2H). **MASS (ES+)** : 709.3 |
| IA.160 | 0.99(d, *J*=7.10Hz, 3H), 1.09(s,3H), 1.11(t, *J*=7.59Hz, 3H), 1.31-1.36(m, 1H), 1.53(s, 3H). 1.72-1.97(m. 4H). 2.23-2.44(m, 6H), 3.32-3.37(m, 1H), 4.38-4.48(m, 3H), 5.30-5.47(m, 1H), 6.38(d, *J*₁=10.14Hz. J₂-1.5Hz. 1H). 6.43 (s, 1H), 7.13(d. *J*=10.08Hz, 111), 7.72 (t. *J*=7.99Hz, 1H), 8.34(d, *J*=7.78Hz, 1H), 8.46(d, *J*=8.17 Hz, *J*₂=1.14Hz, 1H), 8.82( 1H). **MASS (ES+)** : 654.3 (M+Na)⁺ |
| IA.161 | 0.99(d. *J*=7.16Hz, 3H), 1.12(t, *J*=7.58Hz, 3H), 1.13(s,3H), 1.31-1.35(m, 1H), 1.52(s, 3H), 1.76-2.01(m, 3H), 2.23-2.45(m, 6H), 2.32(s, 3H). 2.33(s, 3H), 3.38-3.43(m, 1H), 4.34(d, *J*=16.65Hz, 1H), 4.39-4.41(m, 1H), 4.52(d, *J*=16.66Hz, 1H), 5.30-5.47(m, 1H), 6.37(dd, *J*₁=10.14Hz, *J*₂=1.81Hz, 1H), 6.43(s, 1H), 7.13(dd, *J*₁=10.13Hz, *J*₂=1.23 Hz, 1H), 7.24(d, *J*=7.86Hz, 1H), 774(dd, *J*₁=8.51Hz, *J*₂=1.38Hz. 1H), 7.77(s, 1H). **MASS (ES+)**: 615.4 |
| 1A.162 | 0.97(s, 3H), 1.14(t, *J*=7.51Hz, 3H), 1.21-1.27(m, 1H), 1.38(d, *J*=7.37Hz, 3H), 1.54(s, 3H), 1.73-2.12(m, 5H), 2.26-2.50(m, 6H), 4.01(d, 1H, *J*=16.98Hz), 4.46(d, *J*=8.09Hz, 1H), 4.58(d, *J*=16.98Hz, 1H), 5.31-5.48(m, 1H), 6.38(dd, *J*₁=10.11Hz, *J*₂=1.78Hz, 1H), 6.44 (s. 1H). 7.13(dd, *J*₁=10.17Hz, *J*₂=1.27Hz, 1H), 7.77(d, *J*=8.35Hz, 2H), 8.09(d, *J*=8.05Hz, 2H), **MASS (ES+)** : 655.4 |
| IA.163 | 0.93(t, *J*=7.36Hz, 3H), 1.00(d, *J*=7.20Hz, 3H), 1.11(s, 3H), 1.25-1.97(m, 10H), 2.20-2.41(m, 6H), 3.35-3.39(m, 1H), 4.33(d, *J*=16.58Hz. 1H). 4.41(d, *J*=8.84Hz, 1H), 4.50(d, *J*=16.57Hz, 1H), 5.30-5.47(m, 1H), 6.38(dd *J*₁=10.14Hz, *J*₂=1.81Hz, 1H), 6.43(s, 1H), 7.12(dd *J*₁=10.12Hz, *J*₂=1.17Hz, 1H), 7.76(d, *J*=8.29Hz, 2H), 8.12(d, *J*=8.12Hz, 2H) **MASS (ES+)** : 669.3 |
| IA.164 | 0.94(t, *J*=7.40Hz, 3H), 0.99(d, *J*=7.15Hz, 3H), 1.11(s, 3H), 1.24-1.98(m, 10H), 2.22-2.41(m, 6H), 3.36-3.40(m, 1H), 4.29(d, *J*=16.51Hz, 1H), 4.41(d, *J*=8.74Hz, 1H), 4.47(d, *J*=16.54Hz, 1H), 5.30-5.47(m, 1H),6.37(dd *J*₁=10.12Hz, *J*₂=1.75Hz, 1H), 6.43(s, 1H), 7.13(dd *J*₁=10.12Hz, *J*₂=1.14Hz, 1H), 7.46-7.48(m, 2H), 7.95(d, *J*=8.61Hz, 2H). **MASS (ES+)** : 635.3 |
| IA.165 | 0.94(t, *J*=7.39Hz, 3H), 0.99(d, *J*=7.16Hz, 3H), 1.13(s, 3H), 1.22-2.01(m, 10H), 2.20-2.45(m, 6H), 3.36-3.40(m, 1H), 4.30(d, *J*=16.52Hz, 1H), 4.28(dd *J*₁=17.53Hz, *J*₂=2.68Hz), 4.42-4.47(m, 2H), 5.30-5.47(m, 1H), 6.38(dd *J*₁=10.14Hz, *J*₂=1.76Hz, 1H), 6.43(s. 1H), 6.89-7.01 (m, 2H), 7.12-7.15(m, 1H), 7.92-7.98(m, 1H). **MASS (ES+)**: 637.3 |
| IA.166 | 0.94(t, *J*=7.36Hz, 3H), 0.99(d, *J*=7.16Hz, 3H), 1.11(s, 3H), 1.24-1.99(m, 10H), 2.20-2.41(m, 6H), 3.37-3.41(m, 1H), 4.30(d, *J*=16.52Hz, 1H). 4.41(d, *J*=8.86Hz, 1H), 4.49(d, *J*=16.51Hz, 1H), 5.30-5.47(m, 1H), 6.38(dd *J*₁=10.14Hz, *J*₂=1.74Hz, 1H), 6.43(s, 1H), 7.12-7.18(m, 3H), 8.03-8.06(m, 2H). **MASS (ES+)** : 619.4 |
| IA.167 | 0.94(t, *J*=7.40Hz, 3H), 0.99(d, *J*=7.15Hz, 3H), 1.20(s, 3H), 1.17-1.87(m, 10H), 2.20-2.45(m, 6H), 3.38-3.43(m, 1H), 3.88(s, 3H), 4.30(d, *J*=16.44Hz, 1H). 4.41(d, *J*=9.06Hz, 1H), 4.52(d, *J*=16.51Hz, 1H), 5.30-5.47(m, 1H), 6.38(dd *J*₁=10.15Hz, *J*₂=1.85Hz, 1H), 6.43(s, 1H). 6.94-6.97(m, 2H), 7.13(dd *J*₁=10.12Hz, *J*₂=1.21Hz 1H), 7.98-8.00(m, 2H). **MASS (ES+)** : 631.3 |
| IA.168 | 1.07(d, *J*=7.15Hz, 3H), 1.16(s,3H), 1.35-1.41(m, 1H), 1.55(s,3H), 1.74-2.03(m, 4H), 2.28-2.55(m, 1H), 3.43-3.48(m, 1H), 4.33(d, *J*=16.67Hz, 1H), 4.46(d, *J*=11.14Hz, 1H), 449(d, *J*=16.89Hz, 1H), 5.32-5.49(m, 1H), 6.40(dd, *J*₁=10.14Hz, *J*₂=1.69Hz 1H), 6.45(s, 1H), 6.50(dd, *J*₁=3.47Hz, *J*₂=1.69Hz, 1H), 7.13(d, *J*=2.81Hz, 1H), 7.14(dd, *J*₁=9.98Hz, *J*₂=1.20Hz 1H), 7.45(t, *J*=789Hz, 1H), 7.57(dd). *J*₁=1.86Hz, *J*₂=0.89Hz, 1H), 7.59(m, 1H), 7.88-7.91(m, 1H), 7.98(t, *J*=1.75Hz, 1H). **MASS (ES+)** : 681.3 (M+Na)⁻ |
| IA.170 | 1.07(d, *J*=7.13Hz, 3H), 1.17(s,3H), 1.35-1.41(m, 1H), 1.54(s, 3H), 1.76-2.04(m, 4H), 2.28-2.54(m, 4H), 3.45-3.50(m, 1H), 3.83(s, 3H), 4.36(d, 1H, *J*=16.61Hz), 4.45(d, *J*=8.19Hz, 1H), 4.51(d, *J*=16.68Hz, 1H), 532-5.49(m, 1H), 6.39(dd, *J*₁=10.15Hz, *J*₂=1.84Hz, 1H), 6.45(s, 1H), 6.50(dd, *J*₁=3.50Hz, *J*₂=1.70Hz, 1H). (6.68(t, *J*=2.27Hz, 1H), 7.13-7.17(m, 4H), 7.59 (m, 1H). **MASS (ES+)** : 685.2 |
| IA.171 | 1.07(d, *J*=7.12Hz, 3H), 1.17(s,3H), 1.34-1.42(m, 1H), 1.55(s, 3H), 1.73-1.99(m, 4H), 2.28-2.55(m, 4H), 3.41-3.47(m, 1H), 4.35(d, 1H. *J*=16.49Hz), 4.46(d, *J*=8.61Hz, 1H), 4.52(d, *J*=16.49Hz, 1H), 5.33-5.49(m, 1H), 6.40(dd. *J*₁=10.12Hz, *J*₂=1.73Hz, 1H), 6.45(s, 1H), 6.51(dd, *J*₁=3.47Hz, *J*₂=1.71Hz. 1H), 7.13(d, *J*=3.46Hz, 1H), 7.14(d, *J*=10.8Hz, 1H), 7.59-7.60(m, 1H), 8.17(d, *J*=8.8Hz, 2H), 8.34(d, *J*=8.72Hz, 2H). **MASS (ES+)** : 692.3 (M+Na)⁺ |
| IA.172 | 1.03(d, *J*=7.16Hz, 3H), 1.13(s,3H), 1.33-1.38(m, 1H), 1.52(s, 3H), 1.66-1.96(m, 4H), 2.18-2.38(m, 4H), 3.36-3.43(m, 1H), 3.41(s, 3H). 4.05(s, 2H), 4.35(d, 1H, *J*=16.67Hz), 4.40(d, *J*=9.01Hz, 1H), 4.49(d, *J*=16.66Hz, 1H), 5.30-5.47(m, 1H), 6.37(dd, *J*₁=10.12Hz. *J*₂=1.73Hz,1H), 6.43(s, 1H), 7.11(dd, *J*₁=10.11Hz, *J*₂=1.01Hz, 1H), 7.77(d, *J*=8.33Hz, 2H), 8.11(d, *J*=8.18Hz, 2H). **MASS (ES+)** : 693.3 (M + Na)' |
| IA.173 | 1.01(d, *J*=7.14Hz, 3H), 1.07(s,3H), 1.31-1.35(m. 1H), 1.52(s,3H), 1.75-1.95(m, 4H), 2.19-2.35(m, 4H), 2.25(s, 6H), 2.28(s, 3H), 3.41(s, 3H), 3.41-3.49(m, 1H), 4.05(s, 3H), 4.07(d, *J*=13.34Hz, 1H). 4.27(d, *J*=17.85Hz, 1H), 4.37(d, *J*=8.64Hz, 1H), 5.30-5.46(m, 1H), 6.37(dd, *J*₁=10.13Hz, *J*₂=1.65Hz, 1H), 6.43(s, 1H), 6.86(s, 2H), 7.12(d, *J*₁=10.09Hz, 1H). **MASS (ES+)** : 667.2(M+Na)⁺ |
| IA.174 | 0.99(d, *J*=7.00Hz, 3H), 1.10-1.14(m, 6H), 1.22-1.97(m, 8H), 2.20-2.45(m, 6H). 3.35-3.39(m, 1H), 4.27(d, *J*=16.36Hz, 1H), 4.40-4.45(m, 2H),5.30-5.47(m, 1H), 6.37(dd, *J*₁=10.14Hz, *J*₂=1.74Hz 1H), 6.43(s, 1H), 7.13(dd, *J*₁=10.15Hz, *J*₂=0.90Hz 1H), 7.24-7.28(m, 1H), 7.91-7.95(m, 1H), 8.08-8.10(m; 1H). **MASS (ES+)** : 639.4 |
| IA.175 | 0.91(s,3H), 1.25-1.27(m, 1H), 1.44(d, *J*=7.23Hz, 3H), 1.55(s,3H), 1.74-2.17(m, 4H), 2.23(s, 6H), 2.28(s, 3H), 2.31-2.58(m,4H), 3.69(d. *J*=17.64Hz, 1H), 4.43(d, *J*=17.63Hz, 1H), 4.46(d, *J*=11.28Hz, 1H), 5.32-5.48(m, 1H), 6.40(dd, *J*₁=10.11Hz, *J*₂=1.68Hz, 1H), 6.44(s,1H), 6.52(dd, *J*₁=3.63Hz, *J*₁=1.51Hz, 1H), 6.85(s, 2H), 7.16(dd, *J*₁=10.15Hz, *J*₂=0.99Hz, 1H), 7.19(d. *J*=3.31Hz, 1H), 7.61(d, *J*=0.79Hz, 1H). **MASS (ES+)** :667.1 |
| IA.176 | 1.01(d, *J*=7.14Hz, 3H), 1.06(s, 3H), 1.21(d, *J*=7.00Hz, 3H), 1.32-1.36(m, 1H), 1.52(s, 3H), 1.75-1.94(m, 4H). 2.25(s,6H), 2.28(s, 3H), 2.20-2.36(m, 4H), 3.35-3.45(m, 1H), 3.54(q, *J*=7.02Hz, 2H), 4.06(d, *J*=17.75Hz, 1H), 4.09(s, 2H), 4.26(d, *J*=17.82Hz, 1H), 4.36(d, *J*=8.94Hz. 1H), 5.29-5.47(m, 1H), 6.37(dd, *J*₁=10.14Hz, *J*₂=1.78Hz, 1H), 6.43(s, 1H), 6.86(s, 2H). 7.10(dd, *J*₁=10.11Hz, *J*₂=0.82Hz, 1H). **MASS (ES+):** 659.1 |
| IA.177 | 1.03(d, *J*=7.13Hz, 3H), 1.13(s, 3H), 1.21(t, *J*=7.00Hz, 3H), 1.32-1.37(m, 1H), 1.52(s, 3H), 1.68-1.95(m, 4H), 2.20-2.45(m, 4H), 3.38-3.43(m, 1H), 3.54(qd, *J*₁=6.98Hz, *J*₂=1.49Hz, 2H), 4.09(s,2H), 4.25(d, *J*=16.66Hz, 1H), 4.40(d, *J*=10.48Hz, 1H), 4.49(d, *J*=16.63Hz, 1H). 5.30-5.46(m, 1H), 6.37(dd, *J*₁=10.15Hz, *J*₂=1.72Hz, 1H), 6.43 (s, 1H), 7.11(dd, *J*₁=10.11Hz, *J*₂=0.73Hz, 1H), 7.77(d, *J*=8.28Hz, 2H), 8.11(d, *J*=8.15Hz, 2H). **MASS (ES+)** : 685.1 |
| IA.178 | 1.07(d, *J*=7.12Hz, 3H), 1.19(s,3H), 1.31-1.40(m, 1H), 1.55(s,3H), 1.77-2.06(m, 4H), 2.21-2.54(m, 4H), 2.37(s, 6H), 3.46-3.50(m, 1H), 4.35(d, *J*=16.85Hz, 1H), 4.45(d, *J*=8.6Hz, 1H), 4.56(d, *J*=16.77Hz, 1H). 5.32-5.49(m, 1H), 6.39(dd, *J*₁=10.15Hz, *J*₂=1.7Hz, 1H), 6.45(s, 1H), 6.50(dd, *J*₁=3.43Hz, *J*₂=1.68Hz, 1H), 7.13(s,1H), 7.14(s, 1H), 7.16(dd, *J*₁=11.79Hz, *J*₂=0.67Hz, 1H), 7.24(s, 1H), 7.59(s, 1H), 7.61(s, 1H). **MASS (ES+)** : 653.1 |
| IA.179 | 1.08(d, *J*=7.11Hz, 3H), 1.16(s,3H), 1.33-1.41 (s, 1H), 1.53(s,3H), 1.71-2.04(m, 4H), 2.28-2.51(m, 4H), 3.46-3.51(m, 1H), 4.44(d, *J*=8.45. 1H), 4.53(d, *J*=16.43Hz, 1H), 4.69(d, *J*=16.48Hz, 1H), 5.31-5.48(m, 1H), 6.39(dd, *J*₁=10.15Hz, *J*₂=1.84Hz 1H), 6.45(s, 1H), 6.50(dd, *J*₁=3.48Hz, *J*₂=1.69Hz, 1H), 7.13(s, 1H), 7.14(dd, *J*₁=8.47Hz, *J*₂=1.99Hz 1H), 7.56-7.65(m, 3H), 7.87-8.04(m, 4H), 8.58R(s, 1H). **MASS (ES+) :** 675.1 |
| IA.180 | 0.98(s, 3H), 1.06(d, *J*=7.15Hz, 3H), 1.33-1.39(m, 1H), 1.51(s,3H), 1.71-1.93(m, 4H), 2.35-2.49(m, 4H), 3.42-3.47(m, 1H), 4.37(d, *J*=13.12Hz, 1H). 4.39(d, *J*=4.87Hz, 1H), 4.59(d, *J*=16.48Hz, 1H), 5.30-5.47(m, 1H), 6.37(dd, *J*₁=10.12Hz, *J*₂=1.80Hz 1H), 6.44(s, 1H), 6.50(dd, *J*₁=3.14Hz, J₂=1.36Hz, 1H), 7.11(dd, *J*₁=6.33Hz, *J*₂=1.21Hz, 1H), 7.13(s, 1H), 7.52-7.61(m, 4H), 7.89(dd, *J*₁=8.54Hz, *J*₂=1.43Hz 1H), 8.02(d, *J*=8.22Hz, 1H), 8.07(dd, *J*₁=7.22Hz, *J*₂=0.94Hz, 1H), 8.49(d, *J*=8.42Hz, 1H), **MASS (ES+)** : 674.9 |
| IA.181 | 1.06(d, *J*=7.10Hz, 3H), 1.16(s, 3H), 1.34-1.42(m, 1H), 1.54(s, 3H), 1.65-2.02(m, 4H), 2.29(s, 3H), 2.24-2.61(m, 4H), 3.44-3.49(m, 1H). 4.37(d, 1H, *J*=16.53Hz), 4.44(d, *J*=8.48Hz, 1H), 4.53(d, *J*=16.5Hz, 1H), 5.31-5.48(m, 1H), 6.34(d, *J*=1.44Hz, 1H), 6.38(dd *J*₁=10.12Hz, *J*₂=1.75Hz, 1H), 6.44 (s, 1H), 7.13(dd *J*₁=10.11Hz, *J*₂=1.05Hz, 1H). 7.44 (d. *J*=1.43Hz. 1H), 7.76(d, *J*=8-26Hz. 2H), 8.12(d, *J*=8.13Hz, 2H). **MASS (ES+)** : 707.2 |
| IA.182 | 1.05(d, *J*=7.13Hz, 3H), 1.09(s,3H), 1.36-1.43(m, 1H), 1.54(s,3H), 1.75-2.00(m, 4H), 2.26(s, 6H), 2.28(s, 3H), 2.30(s, 3H), 2.41-2.60(m, 4H), 3.47-3.50(m, 1H), 4.09(d, *J*=17.81Hz, 1H). 4.32(d, *J*=17.76Hz, 1H), 4.41(d, *J*=8.28Hz, 1H). 5.32-5.48(m, 1H), 6.34(d, *J*=1.21Hz, 1H), 6.38(dd, *J*₁=10.16Hz, *J*₂=1.66Hz, 1H), 6.44(s,1H), 6.85(s, 2H), 7.14(d, *J*=10.11Hz), 7.43(d, *J*=1.18Hz, 1H). **MASS (ES+)** : 681.1 |
| IA.183 | 1.07(d, *J*=7.10Hz, 3H), 1.12(s, 3H), 1.32-1.42(m, 1H), 1.54(s, 3H), 1.73-2.00(m, 4H), 227-2.53(m, 4H). 3.40-3.44(m, 1H), 4.05(s. 3H). 4.39(s, 2H), 4.46(d, *J*=8.45Hz, 1H), 5.32-5.48(m, 1H), 6.40(dd, *J*₁=10.12Hz, *J*₂=1.68Hz, 1H), 6.45(s, 1H). 6.50(dd, *J*₁=3.44Hz. J₂=1.68Hz, 1H), 7.13(s, 1H), 7.15(dd, *J*₁=11.15Hz, *J*₂=0.94Hz, 1H), 7.19(d, *J*=8.88Hz, 1H), 7.59(d, *J*=0.69Hz, 1H), 8.22(dd, *J*₁=8.81Hz, *J*₂=2.16Hz, 1H), 8.49(d, *J*=2.15Hz, 1H). **MASS (ES+)** : 700.1 |
| IA.184 | 1.07(d, *J*=7.16Hz, 3H), 1.18(s,3H), 1.32-1.40(m, 1H), 1.55(s, 3H), 1.73-2.08(m, 4H), 2.25-2.43(m,4H), 2.32(s, 3H), 2.32 (s, 3H), 3.46-3.51(m, 1H), 4.34(d, *J*=16.73Hz, 1H), 4.45(d, *J*=8.08Hz, 1H), 4.57(d, *J*=16.76Hz, 1H), 5.32-5.49(m, 1H), 6.39(dd, *J*₁=10.14Hz, *J*₂=1.82Hz, 1H), 6.45(s, 1H), 6.50(dd, *J*₁=3.53Hz, *J*₂=1.74Hz, 1H), 7.13(dd, *J*₁=3.63Hz, *J*₂=0.62Hz, 1H), 7.16(dd. *J*₁=10.14Hz, *J*₂=1.18Hz, 1H), 7.24(d, *J*=7.87Hz, 1H), 7.59(m, 1H), 7.74(dd, *J*₁=8.69Hz, *J*₂=1.66Hz, 1H), 7.77(s, 1H). **MASS (ES+)** : 653.5,(M+H)⁺ |
| IA.185 | 1.05(d, *J*=7.17Hz. 3H), 1.16(s, 3H), 1.14-1.40(m, 1H), 1.54(s, 3H), 1.72-2.03(m, 4H), 2.29-2.48(m, 4H), 3.42-3.47(m, 1H), 4.35(d, 1H, *J*=16.60Hz), 4.46(d, *J*=8.55Hz, 1H), 4.53(d, *J*=16.65Hz, 1H), 5.32-5.49(m, 1H), 6.39(dd *J*₁=10.14Hz, *J*₂=1.84Hz, 1H), 6.45(s, 1H), 6.65(d, *J*=1.52Hz, 1H). 7.13(dd *J*₁=10.12Hz, *J*₂=1.21Hz, 1H), 7.42(t, *J*=1.70Hz, 1H), 7.76(d, *J*=8.27Hz, 2H). 7.97(d, *J*=0.75Hz, 1H), 8.12(d, *J*=8.08Hz, 2H). **MASS (ES+)** : 693.2,(M+H)⁺ |
| IA.186 | 1.04(d, *J*=7.12Hz, 3H), 1.10(s,3H), 1.33-1.38(m, 1H), 1.54(s,3H), 1.75-2.04(m, 4H), 2.25(s, 6H), 2.28(s, 3H), 2.17-2.46(m, 4H), 3.46-3.49(m, 1H), 4.09(d, *J*=17.58Hz, 1H), 4.30(d, *J*=17.79Hz, 1H), 4.42(d, *J*=7.85Hz, 1H), 5.32-5.48(m, 1H), 6.40(dd, *J*₁=10.12Hz, *J*₂=1.70Hz, 1H), 6.45(s, 1H), 6.65(d, *J*=)1.50Hz, 1H), 6.85(s, 2H), 7.13(dd, *J*₁=10.09Hz, *J*₁=0.79Hz, 1H), 7,42(t, *J*=1.40Hz, 1H). 7.97(s, 1H), **MASS (ES+)** : 667.1 (M+H)⁺ |
| IA.187 | 0.44(s,3H), 0.95(d, *J*=7.02Hz, 3H), 1.08(t, *J*=7.53Hz, 3H), 1.22-1.75(m, 15H), 2.17-2.32(m, 6H), 3.15(m, 1H), 4.29(d, *J*=6.81Hz, 1H), 5.40(m, 1H), 6.35(dd, *J*₁=10.11Hz, *J*₂=1.43Hz 1H), 6.40(s, 1H), 7.05(d, *J*=9.98Hz 1H), 7.36(t, J=7.70Hz, 2H), 7.46(t, *J*=7.20Hz, 1H), 7.95(d, *J*=7..43Hz, 2H). **MASS (ES+)** : 705.4(M+Na)⁺ |
| IA.188 | 0.60(s,3H), 0.93(d, *J*=7.04Hz, 3H), 1.06(t, *J*=7.54Hz, 3H), 1.10-1.89(m, 14H), 2.09-2.36(m, 6H), 3.14-3.17(m, 1H), 4.30(d, *J*=7.66Hz, 1H), 5.26-5.43(m, 1H), 6.34(dd, *J*₁=10.14Hz, *J*₂=1.67Hz 1H), 6.41(s, 1H), 7.06(m, 3H), 8.02-8.05(m, 2H), **MASS (ES+)**: 655.5 (M+Na)⁺. |
| IA.199 | 0.42(s,3H), 0.93(d, *J*=7.1Hz, 3H), 1.08(i *J*=7.57Hz, 3H), 1.20-1.25(m, 1H), 1.49(s,3H), 1.51-1.79(m,4H), 1.75(s, 6H), 1.80-2.37(m, 7H), 3.31(m, 1H), 4.28(d, *J*=8.59Hz, 1H), 6.10(s,1H), 6.31(d,d *J*₁=10.12Hz, *J*₂=1.80Hz, 1H), 7.13(d, *J*=10.14Hz, 1H), 7.38(t, *J*=7.53Hz, 2H). 7.45-7.47(m, 1H), 7.95-7.97(m, 1H), **MASS (ES+)**: 619.6 (M+Na)⁺. |
| IA.190 | 0.44(s, 3H), 0.82-1.15(m, 2H), 0.92(d, *J*=6.92Hz, 3H), 1.21-1.42(m, 8H), 1.48(s,3H), 1.52-1.84(m, 6H), 1.71(s,6H), 2.10-2.29(m, 5H), 3.14-3.17(m, 1H), 4.40(d, *J*=7.44Hz, 1H), 5.24-5.44(m, 1H), 6.35(d, *J*=9.7Hz, 1H). 6.41(s,1H). 7.07(d, *J*=10.1Hz, 1H), 7.37(t, *J*=7.54Hz, 2H), 7.46(t, *J*=7.28Hz, 1H), 7.95(d, *J*=7.61Hz, 2H), **MASS (ES+)**: 691.6 (M+Na)⁺. |
| IA.191 | 0.44(s,3H), 0.93(d, *J*=7.07Hz, 3H), 1.23-1.25(m, 1H), 1.47(s,3H), 1.51-1.83(m,12H), 1.74(s, 6H), 2.19-2.25(m, 4H), 2.67-2.71(m, 1H), 3.13-3.17(m, 1H), 4.30(d, *J*=7.27Hz, 1H), 5.25-5.42(m, 1H), 6.34(d,d *J*₁=10.19Hz, *J*₂=1.75Hz. 1H). 6.41(s, 1H), 7.06(d, *J*=10.22Hz, 1H), 7.37(t, *J*=7.57Hz, 2H), 7.46(t, 1H, *J*=7.34Hz*),* 7.95(d,2H, *J*=7.22Hz). **MASS (ES+)** : 655.2. |
| IA. 192 | 0.45(s, 3H), 0.93(d, *J*=7.14Hz, 3H), 1.11(d, *J*=6.96Hz, 3H), 1.21-1.26(m, 1H), 1.48(s, 3H), 1.55-1.75(m, 4H), 1.75(d, *J*=1.51Hz, 6H), 2.10-2.27(m, 4H), 2.47-2.52(m, 1H), 3.13-3.18(m, 1H), 4.30(d, *J*=7.45Hz, 1H), 5.25-5.42(m, 1H), 6.34(d,d *J*₁=10.13Hz, *J*₂=1.77Hz, 1H). 6.41(s,1H), 7.06(dd. *J*₁=10.13Hz, *J*₂=1.06Hz, 1H), 737(t, *J*=7.59Hz, 2H), 7.47(t, *J*=7.59Hz, 2H), 7.47(t, *J*=7.35Hz, 1H), 7.95(d, *J*=7.28Hz. 2H), **MASS (ES+)** : 629.2. |
| IA.193 | 0.45(s, 3H), 0.94(d, *J*=7.06Hz, 3H), 1.16-1.26(m, 2H), 1.47(s, 3H), 1.55-1.96(m, 5H), 1.7(Two s,6H). 2.09-2.29(m, 8H), 3.06-3.17(m, 2H), 4.28(d, *J*=7.88Hz, 1H), 5.25-5.43(m, 1H), 6.33(dd, *J*₁=10.13Hz, *J*₂=1.66Hz, 1H), 6.40(s,1H), 7.05(d, *J*=10.13Hz, 1H), 7.38(t, *J*=7.58Hz. 2H), 7.47(t, *J*=7.83Hz, 1H), 7.96(d, *J*=8.66Hz, 2H) **MASS (ES+)**: 641.5. |
| IA.194 | 0.5(s, 3H), 1.0(d, *J*=6.97Hz, 3H), 1.22-1.28(m, 1H), 1.51(s,3H), 1.41-1.74(m,5H), 1.77(s, 3H), 1.78(s, 3H), 215-2.60(m, 5H), 3.19-3.33(m, 1H), 4.33(d, *J*=8.07Hz, 1H), 612(s,1H), 6.35(d, *J*=10.10Hz, 1H), 6.46(d, *J*=3.25Hz, 1H), 7.04(d, *J*=3.27Hz, 1H), 7.16(d, *J*=1.10Hz. 1H), 7.38(t, *J*=7.57Hz, 2H), 7.47(t, *J*=730Hz, 1H), 7.56(s, 1H), 7.95(d, *J*=7.71Hz, 2H), **MASS (ES+)** : 635.4. |
| IA.195 | 0.44(s, 3H), 0.88-0.91(m, 2H), 0.94(d, *J*=7.11Hz, 3H), 1.09-1.36(m,5H), 1.47(s,3H), 1.52-1.73(m, 8H), 1.74(s, 6H). 2.12-2.22(m, 6H). 3.13-3.16(m, 1H), 5.29(d, *J*=8.09Hz, 1H), 5.25-5.42(m,1H), 6.35(dd, *J*₁=10.12Hz, *J*₂=1.79Hz, 1H), 6.40(s,3H), 7.06(dd, *J*₁=10.14Hz. *J*₁=1.36Hz, 1H), 7.37(t, *J*=7.54Hz, 2H), 7.44-7.47(m, 1H), 7.95(dd, *J*₁=8.31Hz, *J*₂=1.23Hz, 2H). **MASS (ES+)** : 683.4. |
| IA.196 | 0.66(s,3H), 1.02(d, *J*=7.03Hz, 3H), 1.10-1.43(m, 2H), 1.50(s, 3H), 1.54-1.82(m, 9H), 2.22-2.41(m, 4H), 3.22-3.25(m, 1H). 4.34(d, *J*=6.85Hz, 1H),5.28-5.45(m, 1H), 6.38(dd, *J*₁=10.17Hz, *J*₂=1.68Hz 1H), 6.43(s, 1H), 6.47(dd, *J*₁=3.39Hz, *J*₂=1.86Hz 1H), 2.03-7.09(m, 4H), 7.56(s, 1H), 8.01-8.01(m, 2H), **MASS (ES+)** : 671.3. |
| IA.197 | 0.58(s,3H), 1.02(d, *J*=7.09Hz, 3H), 1.08-1.37(m, 3H), 1.50(s,3H), 1.61-1.80(m, 8H), 2.22-2.38(m, 4H), 3.20-3.25(m, 1H 4.34-4.36(m, 1H), 5.28-5.48(m, 1H), 6.39(dd, *J*₁=10.12Hz, *J*₂=1.77Hz 1H), 6.43(s, 1H), 6.48(dd, *J*₁=3.47Hz, *J*₂ =1.71Hz 1H 7.05(d, *J*=3.34Hz 1H), 7.09(dd, *J*₁=10.12Hz, *J*₂=1.07Hz 1H), 7.57(t, *J*=0.85Hz, 1H), 7.65(d, *J*=8.33Hz, 2H), 8.01(d, *J*=8.21Hz, 2H), **MASS (ES+)** : 721.2 |
| IA.198 | 1.05(d, *J*=7.15Hz, 3H), 1.17(s,3H), 1.34-1.39(m, 1H), 1.55(s, 3H), 1.74-1.92(m, 4H), 2.09-2.50(m, 4H).2.32(s, 3H), 2.33(s, 3H), 3.45-3.49(m, 1H), 4.32(d, *J*=16.76Hz, 1H), 4.46(d, *J*=8.58Hz, 1H), 4.59(d, *J*=16.79Hz, 1H), 5.32-5.49(m, 1H), 6.40(dd, *J*₁=10.12Hz. *J*₂=1.65Hz, 1H), 6.46(s,1H), 6.93(d, *J*=4.02Hz, 1H), 7.17(d, *J*=10.13Hz, 1H), 7.24(d, *J*=7.93Hz, 1H), 7.53(dd, *J*=3.95Hz, 1H), 7.75(d, *J*=7.93Hz, 1H), 7.78(s, 1H). **MASS (ES+)** : 703.7 |
| IA-1.1 | 0.33(s,3H), 0.92(d, *J*=7.14Hz, 3H), 1.07(t, *J*=7.55Hz, 3H), 1.17-1.88(m, 12H), 2.08-2.30(m, 8H), 2.66-2.63(m, 2H), 3.10-3.13(m, 1H), 4.28-4.30(m, 1H), 5.25-5.41(m, 1H), 6.36(dd, *J*₁=10.13Hz, *J*₂=1.77Hz 1H), 6.40(s, 1H), 7.03-7.07(m, 3H), 7.94-7.97(m, 2H). **MASS (ES+)** : 660.5 |
| IA-1.2 | 0.15(s,3H), 0.91(d, *J*=7.10Hz, 3H), 1.08(t, *J*=7.68Hz, 3H), 1.17-1.88(m, 12H), 2.04-2.31(m, 8H), 2.63-2.67(m, 2H). 3.05-3.15(m, 1H). 4.26(d, *J*=7.89Hz, 1H), 5.23-5.39(m, 1H), 6.35(dd, *J*₁=10.11Hz, *J*₂=1.47Hz 1H), 6.39(s, 1H), 7.0.5(d. *J*=10.Hz, 2H), 7.36-7.39(m, 2H), 7.45-7.49(m, 2H), 7.90(d, *J*=7.49Hz, 2H). **MASS (ES+)** : 663.0 (M+Na)⁺. |
| IB.1 | 0.94(d, *J*=7.16Hz, 3H), 1.10(s,3H), 1.12(t, *J*=7.53Hz, 3H), 1.23-1.35(m, 1H), 1.53(s,3H), 1.69-1.85(m,3H), 2.17-2.49(m,7H), 3.29-3.36(m, 1H), 4.30-4.40(m,3H), 4.55(d, *J*=15.48, 1H), 5.04(d, *J*=15.46, 1H), 5.27-5.52(m, 1H),6.37(d,d *J*₁=10.11Hz, *J*₂=1.69Hz, 1H), 6.43(s, 1H), 7.13(d, *J*=10.10Hz, 1H), **MASS (ES+)**: 543.5 (M+Na)⁺. |
| IB.2 | 0.97(d, *J*=6.99Hz, 3H), 1.11(s,3H), 1.12(t, *J*=7.33Hz, 3H), 1.29-1.33(m, 1H), 1.53(s,3H), 1.70-1.98(m,3H), 2.17-2.54(m, 7H), 3.33-3.34(m, 1H), 3.67(d, *J*=14.69, 1H), 3.74(d, *J*=16.44, 1H), 4.19(s,2H), 4.32(d, *J*=7.28. 1H), 4.59(s, 1H), 5.31-5.48(m,1H), 6.33(d, *J*=10.16Hz. 1H), 6.37(s, 1H), 7.22(d, *J*=10.02Hz, 1H), **MASS (ES+)**: 559.5 (M+Na)⁺. |
| IB.3 | 0.99(d. *J*=7.10Hz, 3H), 1.10(s,3H), 1.12(t, *J*=7.59Hz, 3H), 1,31-1.36(m,1H), 1.52(s,3H), 1.70-1.91(m,4H), 2.19-2.45(m, 6H), 3.33-3.37(m, 1H), 3.72-3.78(m, 2H), 4.41(d, *J*=6.54,1H), 4.94(d, *J*=47.47Hz, 2H), 5.30-5.47(m,1H), 6.38(d, *J*=10.13Hz, 1H), 6.43(s, 1H), 7.12(d, *J*=10.13Hz,1H), **MASS (ES+)**: 561.2 (M+Na)⁺. |
| IB.4 | 1.02(d, *J*=7.08Hz, 3H), 1.13(s,3H), 1.32-1.37(m, 1H), 1.55(s, 3H), 1.66-2.02(m, 4H), 2.07-2.32(m,4H), 3.38-3.41(m, 1H), 3.70(d, *J*=16.32.1H), 3.78(d, *J*=16.35Hz, 1H), 1.12-4.15(m,2H), 4.33(d, *J*=6.37,1H), 4.88(t, *J*=592.1H), 5.14(m,1H), 5.36-5.53(m,1H), 6.29-6.32(m, 2H), 6.54(dd, *J*₁=3.47Hz, *J*₂=1.72Hz 1H), 7.11(d, *J*= 3.46,1H), 7.25(d, *J*=10.45Hz, 1H), 7.66(m, 1H). **MASS (ES+)**: 597.5 (M+Na)⁺. |
| IB.5 | 0.98(d, *J*=7.03Hz, 3H), 1.11(s,3H), 1.12(t, *J*=7.72Hz, 3H), 1.27-1.33(m, 1H), 1.54(s,3H), 1.78-1.88(m,5H), 2.04-2.31(m,2H), 2.33-2.63(m,6H), 3.31-3.34(m, 1H), 3.66(d, *J*=16.48, 1H), 3.74(d, *J*=16.56, 1H), 4.24(s,2H), 4.40(d, *J*=8.44, 1H), 6.13(s,1H), 6.35(d, *J*=10.06Hz, 1H), 7.20(d, *J*=10.07Hz,1H). **MASS (ES+)**: 541.6 (M+Na)⁺. |
| IB.6 | 1.07(d, *J*=7.08Hz, 3H), 1.15(s,3H), 1.32-1.41(m, 1H), 1.54(s,3H), 1.58-1.95(m, 5H), 2.29-2.53(m,3H), 3.41-3.45(m, 1H), 3.75(dd, *J*₁=15.74Hz, *J*₂=5.95Hz, 1H), 3.83(dd, *J*₁=16.61Hz, *J*₂=6.93Hz, 1H), 4.46(d, *J*=8.11Hz,1H), 4.94d, *J*=47.47Hz, 2H), 5.32-5.49(m, 1H), 6.40(dd, *J*₁=10.11Hz, *J*₂=1.43Hz, 1H), 6.45(s, 1H), 6.50(dd, *J*₁=3.14Hz, *J*₂=1.5Hz,1H), 7.12(s,1H), 7.14(d, *J*=10.36Hz,1H), 7.59(s, 1H), **MASS (ES+)**:599.5 (M+Na)⁺. |
| IB.7 | 0.95(d, *J*=7.14Hz, 3H), 1.10(s,3H), 1.14(d, *J*=4.89Hz, 3H), 1.16(d, *J*=4.92Hz, 3H), 1.23-1.33(m, 1H), 1.53(s,3H), 1.68-2.00(m,3H), 2.16-2.29(m,2H), 2.54-2.62(m, 2H), 3.32-3.36(m, 1H), 3.64-3.78(m, 2H), 4.15-4.17(m, 2H), 4.30-4.33(m, 1H), 4.72(t, *J*=5.89, 1H), 4.99(t, *J*=2.77, 1H), 5.34-5.50(m,1H), 6.31(d, *J*₁=12.18Hz, *J*₂=1.97Hz,1H), 6.32(s, 1H), 7.24(d, *J*=10.03Hz,1H), **MASS (ES+)**: 551.2 |
| IB.8 | 0.99(d, *J*=7.31Hz, 3H), 1.11(s,3H), 1.27-1.35(m, 1H), 1.52(s, 3H), 1.72-1.99(m, 7H), 2.04-2.37(m, 8H), 3.14-3.18(m, 1H), 3.33-3.36(m, 1H), 3.65-3.77(m, 2H), 4.24 (d, *J*=2.11, 2H), 4.39 (d, *J*=8.66, 1H), 5.28-5.48(m, 1H), 6.37 (dd, *J*₁=10.12Hz, *J*₂=1.60Hz, 1H), 6.43 (s, 1H), 7.12 (d, *J*=10.18Hz, 1H), **MASS (ES+)**: 564.3 |
| IB.9 | 1.02(d, *J*=7.10Hz, 3H), 1.16(s,3H), 1.13-1.36(m, 1H), 1.57(s,3H), 1.60-2.00(m,4H), 2.24-2.66(m, 6H), 3.38-3.42(m, 1H), 3.70(d, *J*=16.38, 1H), 3.78(d, *J*=16.39, 1H), 4.15-4.17(m, 2H), 4.34-4.37(m, 1H), 4.73(t, *J*=5.85, 1H), 4.78(s, 1H), 6.09(s,1H), 6.29(dd, *J*₁=10.10Hz, *J*₂=1.45Hz, 1H), 6.52(dd, *J*₁=3.33Hz, *J*₂=1.60Hz, 1H), 7.11(d, *J*=3.43Hz, 1H), 7.32(d, *J*=10.12Hz, 1H), 7.63(s, 1H), **MASS (ES+)**: 557.3 |
| IB.10 | 1.03(d, *J*=6.98Hz, 3H), 1.13(s,3H), 1.33-1.37(m, 1H), 1.55(s, 3H), 1.62-1.98(m, 3H), 2.24-2.54(m, 4H), 3.3-(m, 1H), 3.71(d, *J*=16.40,1H), 3.79(d, *J*=16.39Hz, 1H), 4.08(d, *J*=7.17Hz, 1H), 4.12(d, *J*=5.83Hz, 2H), 4.99(t, *J*=5.92,1H), 5.27(s,1H), 5.40-5.50(m,1H), 6.28(s, 1H), 6.29(d, *J*=13.05 Hz, 1H), 7.14(t, *J*=4.33Hz, 1H), 7.27(d, *J* = 10.03, 1H), 7.71(m, 2H). **MASS (ES+)**: 591.4 |
| IB.11 | 0.99(d, *J*=7.15Hz, 3H), 1.09(s,3H), 1.12(t, *J*=7.66Hz, 3H), 1.30-1.36(m, 1H), 1.52(s,3H), 1.64-1.90(m,4H), 2.18-2.44(m,7H), 3.34-3.38(m, 1H), 3.65(dd, *J*₁=16.57Hz, *J*₂=2.60Hz,1H), 3.71(dd, *J*₁=16.58Hz, *J*₂=2.58Hz, 1H), 4.41(d, *J*=7.42, 1H), 5.30-5-47(m, 1H), 6.37(dd, *J*₁=10.14 Hz, *J*₂=1.54Hz,1H), 6.43(s, 1H), 7.12(d, *J*=10.05Hz,1H), **MASS(ES+)**: 507.3 |
| IB.12 | 1.06(d, *J*=7.11Hz, 3H), 1.18(s, 3H), 1.35-1.61(m, 11H), 1.55(s, 3H), 1.80-1.97(m, 3H). 2.39-2.60(m, 5H), 3.43-3.46(m, 1H), 3.70-3.82(m, 3H), 4.06-4.33(m, 4H), 4.45(d, *J*=7.47Hz, 1H), 4,69-4.71(m, 2H), 5.32-5.49(m, 1H), 6.39(d, *J*=10.13Hz 1H), 6.45(s, 1H), 6.50(d, *J*₁=3.26Hz, *J*₂=1.48Hz, 1H), 7.13(d, *J*=3.42Hz, 1H), 7.16(d, *J*=10.32Hz, 1H), 7.59(s, 1H), **MASS (ES+)** : 773.2 |
| IB.13 | 0.70(d, *J*=7.16Hz, 3H), 1.07(s,3H), 1.11(t, *J*=7.53Hz, 3H), 1.25-1.35(m, 1H), 1.53(s,3H), 1.70-2.03(m,3H), 2.18-2.60(m, 7H), 3.34-3.37(m, 1H), 3.56-3.60(m, 2H), 4.04(d, *J*=3.79Hz, 2H), 4.28(d, *J*=9.37Hz, 1H), 4.93(s, 1H), 5.32-5.51(m,1H), 5.63-5.81(m,1H), 5.80-5.88(m,1H), 6.30(dd, *J*₁=10.12Hz, *J*₂=1.88Hz, 1H), 6.33(s, 1H), 7.24(d, *J*=9.87Hz, 1H). **MASS (ES+):** 539.2 |
| IB.14 | 1.04(d, *J*=7.10Hz, 3H), 1.12(s,3H), 1.12-1.37(m, 1H), 1.55(s, 3H), 1.70-1.95(m, 4H), 2.29-2.59(m, 4H), 3.40-3.44(m, 1H), 3.55-3.68(m, 2H), 4.02(d, *J*=4.19Hz, 1H), 4.23(br-s, 1H), 4.33(br-d, *J*=10.86Hz, 1H), 5.07-5.09(m,1H), 5.36-5.53(m, 1H), 5.65-5.71(m,1H), 5.81-5.87(m, 1H), 6.31(dd, *J*₁=10.68Hz, *J*₂=1.76Hz, 1H), 6.33(s, 1H), 6.53(dd, *J*₁=3.46Hz, *J*₂=1.75Hz, 1H), 7.10(d, *J*=3.47Hz, 1H), 7.26(d, *J*=10.00Hz, 1H), 7.64(d, *J*=1.37H, 1H). **MASS (ES+)**: 599.3(M+Na)⁺ |
| IB. 15 | 1.02(d, *J*=7.10Hz, 3H), 1.10(s,3H), 1.31-1.38(m, 1H), 1.54(s, 3H), 1.65-1.96(m, 3H), 2.26-2.53(m, 4H), 3.38-3.41(m, 1H), 3.64(d, *J*=7.8Hz, 2H), 4.16(d, *J*=5.94Hz, 2H), 4.29(b,d, 1H), 4.48(bs, 1H), 5.27(m, 1H), 5.40-5.57(m, 2H), 5.62-5.69(m, 1H), 6.26(s, 1H), 6.28(dd, *J*₁=10.12Hz, *J*₂=1.79Hz, 1H), 6.56(dd, *J*₁=3.47Hz, *J*₂=1.72Hz, 1H), 7.10(d, *J*=3.48Hz, 1H), 7.26(d, *J*=10.10Hz, 1H), 7.72(d, *J*=1.51Hz, 1H). **MASS (ES+)**: 599.3(M+Na)⁺ |
| IB.16 | 1.03(d, *J*=7.09Hz, 3H), 1.11(s, 3H), 1.31-1.37(m, 1H), 1.55(s, 3H), 1.65-2.01(m, 4H), 2.28-2.58(m, 4H). 3.36-3.41(m, 1H), 3.64(d, *J*=7.76Hz, 2H), 4.16(m, 2H), 3.31(d, *J*=6.77Hz, 1H), 4.49(t, *J*=5.37Hz, 1H), 5.28(m, 1H), 5.41-5.57(m, 2H), 5.64-5.69(m, 1H), 6.26(s, 1H), 6.29(dd, *J*₁=10.11Hz, *J*₂=1.82Hz, 1H), 7.14(t, *J*=4.36Hz, 1H), 7.27(dd, *J*₁=10.11Hz, *J*₂=0.74Hz, 1H), 7.70-7.71(m, 2H). **MASS (ES+)**: 615.0(M+Na)⁻ |
| IB.17 | 1.04(d, *J*=7.11Hz, 3H), 1.12(s,3H), 1.32-1.38(m, 1H), 1.55(s, 3H), 1.60-1.99(m, 4H), 2.29-2.59(m, 4H), 3.39-3.43(m, 1H), 3.56-3.67(m, 2H), 4.00(d, *J*=5.11Hz, 1H), 4.22(dd, *J*₁=4.85Hz, *J*₂=0.81Hz, 1H), 4.32(d, *J*=6.73Hz, 1H), 4.43(t, *J*=5.49Hz, 1H), 5.21-5.22(m, 1H), 5.39-5.56(m, 1H), 5.63-5.70(m, 1H), 5.83(d,t, *J*₁=15.17Hz, *J*₂=5.03Hz, 1H), 6.29(s, 1H), 6.30(dd, *J*₁=10.58Hz, *J*₂=1.77Hz), 1H), 7.13(dd, *J*₁=4.87Hz, *J*₂=3.87Hz, 1H), 7.27(d, *J*=9.68Hz, 1H), 7.67(dd, *J*₁=4.97Hz, *J*₂=0.99Hz, 1H), 7.70(dd, *J*₁=3.70Hz, *J*₂=1.05Hz, 1H), **MASS (ES+)** : 592.7 |
| IB.18 | 1.06(d, *J*=7.11Hz, 3H), 1.18(s, 3H), 1.35-1.41(m, 1H), 1.55(s, 3H), 1.67-1.97(m, 1H), 2.28-2.56(m, 5H), 3.44(m, 1H), 3.66-3.81(m, 3H), 3.99-4.31(m, 4H), 4.45(d, *J*=7.88Hz, 1H), 4.65-4.75(m, 2H), 5.32-5.49(m, 1H), 6.39(dd, *J*₁=10.15Hz, *J*₂=1.29Hz,1H), 6.45(s, 1H), 6.50(dd, *J*₁=3.34Hz, *J*₂=1.69Hz, 1H), 7.13(d, *J*=3.45Hz, 1H), 7.16(d, *J*=10.13Hz, 1H), 7.59(d, *J*=0.66Hz, 1H),**MASS (ES+)** : 759.1 |
| IB.19 | 1.06(d, *J*=7.05Hz, 3H), 1.18(s, 3H), 1.36-1.41(m, 1H), 1.54(s, 3H), 1.74-1.97(m, 7H), 2.29-2.50(m, 5H), 3.42-3.45(m, 1H), 3.72-3.74(m, 2H), 4.20(t, *J*=5.74Hz, 2H), 4.44-4.47(m, 1H), 4.48(t, *J*=5.90Hz, 2H), 4.65-4.74(m. , 2H), 5.33-5.50(m,1H), 6.40(dd, *J*₁=10.14Hz, *J*₂=1.52Hz, 1H), 6.45(s, 1H), 6.50(dd, *J*₁=3.22Hz, *J*₂=1.48Hz, 1H), 7.13-7.16(m, 2H), 7.59(s, 1H). **MASS (ES+)** : 736.1 |
| IB.20 | 1.03(d, *J*=7.10Hz, 3H), 1.15(s,3H), 1.33-1.39(m, 1H), 1.55(s, 3H), 1.60-200(m, 4H), 2.25-2.60(m, 4H), 3.30-342(m, 1H), 3.71(d, *J*=16.35Hz, 1H), 3.78(d,t, *J*₁=16.38Hz, *J*₂=1.86Hz, 1H), 4.15-4.17(m, 2H), 4.36(d, *J*=9.1Hz, 1H), 4.71(t, *J*=5.90, 1H), 5.03(d, *J*=2.42Hz, 1H), 5.16-5.52(m,1H), 6.34(dd *J*₁=10.97Hz, *J*₂=1,70Hz, 1H), 6.36(s, 1H), 6.97(d, *J*=13.05Hz, 1H), 7.26(d, *J*=10.17Hz, 1H) 7.52(s, 1H), **MASS (ES+)** : 647.0(M+Na)⁻ |
| IB.21 | 1.06(d, *J*=7.13Hz, 3H), 1.14(s,3H), 1.35-1.40(m, 1H), 1.54(s, 3H), 1.79(t, *J*=2.34Hz, 3H), 1.73-1.96(m, 4H), 2.18-2.54(m, 4H), 3.41-3.46(m, 1H), 3.66(dd, *J*₁=16.08Hz, *J*₂=2.46Hz, 1H), 3.73(dd, *J*₁=16.12Hz, *J*₂=2.45Hz, 1H), 4.45(d, *J*=8.43Hz, 1H), 5.32-5.48(m, 1H), 6.40(d, *J*=10.71Hz, 1H), 6.45(s, 1H), 6.50(dd, *J*₁=3.21Hz, *J*₂=1.11Hz, 1H), 7.11(d, *J*=3.39,1H), 7.13(d, *J*=9.96Hz, 1H), 7.58(m, 1H). **MASS (ES+)** : 559.5 |
| IB.22 | 1.06(d, *J*=7.10Hz, 3H), 1.17(s,3H), 1.36-1.43(m, 1H), 1.53(s, 3H), 1.56-1.96(m, 4H), 2.30(s, 3H), 2.38-2.58(m, 4H), 3.41-3.45(m, 1H), 3.70(d, *J*=16.56.1H), 3.79(d, *J*=16.56Hz, 1H), 4.22(d, *J*=14.06,1H), 4.27(d, *J*=15.99Hz, 1H), 4.42(d, *J*=8.53, 1H), 5.31-5.48(m, 1H), 6.34(d, *J*=0.96, 1H), 6.38(dd, *J*₁=10.16Hz, *J*₂=1.43Hz 1H), 6.44(s, 1H), 7.14(d, *J*= 10.2,1H), 7.44(s, 1H). **MASS (ES+)** : **MASS (ES+) :** 589.3 |
| IB.23 | 1.03(d, *J*=7.10Hz, 3H), 1.12(s,3H), 1.21(t, *J*=6.99Hz, 3H), 1.32-1.37(m, 1H), 1.52(s, 3H), 1.66-1.92(m, 4H), 2.17-2.45(m, 5H), 3.35-3.38(m, 1H), 3.54(q, *J*=6.98Hz, 2H), 3.69(d, *J=*14.78Hz, 1H), 3.73(d, *J*=14.72Hz, 1H), 4.09(s, 2H), 4.19-4.28 (m, 2H), 4.39 (d, *J*=8.39Hz, 1H), 5.30-5.46(m, 1H), 6.37(dd, *J*₁=10.13Hz, *J*₁=1.65Hz, 1H), (s, 1H), 7.13 (dd, *J*₁=10.07Hz, *J*₂=0.65Hz, 1H), **MASS (ES+)**: 567.1 |
| IB.24 | 1.03(d, *J*=7.11Hz, 3H), 1.12(s,3H), 1.32-1.38(m, 1H), 1.52(s, 3H), 1.68-1.93(m, 5H), 2.16-2.32(m, 4H), 3.35-3.39(m, 1H), 3.41(s, 3H), 3.71-3.73(m, 2H), 4.05(s, 2H), 4.23(t, *J*=2.22, 1H), 4.25(t, *J*=2.23, 1H), 4.39(d, *J*=8.92, 1H), 5.30-5.47(m, 1H), 6.37(dd, *J*₁=10.17Hz, *J*₂=1.78Hz, 1H), 6.43 (s, 1H), 7.14 (d, *J*=10.13 Hz, 1H). **MASS (ES+)** : 553.2 |
| IB.25 | 1.07(d, *J*=7.15Hz, 3H), 1.18(s,3H), 1.28-1.42(m, 1H), 1.46-1.67(m, 10H), 1.54(s,3H), 1.74-2.02(m, 9H), 2.28-2.50(m. 1H), 3.40-3.46(m, 1H), 3.70(d, *J*=16.45Hz, 1H), 3.78(d, *J*=16.43Hz, 1H), 4.44(d, *J*=8.82Hz, 1H), 5.32-5.49(m, 1H), 6.39(dd, *J*₁=10.13Hz, *J*₂=1.66Hz, 1H), 6.45(s, 1H), 6.49(dd, *J*₁=3.42Hz, *J*₂=1.66Hz,1H), 7.12(d, *J*=3.71Hz, 1H), 7.14(d, *J*=12.28Hz, 1H), 7.59(s, 1H), **MASS (ES+)** : 643.3 |
| IB.26 | 0.89.0.94(m, 2H), 1.0(d, *J*=7.14Hz, 3H), 1.12(s,3H), 1.08-1.36(m, 4H), 1.52(s,3H), 1.61-1.91(m,10H), 2.16-2.44(m,7H), 3.31-3.35(m, 1H), 3.65-3.76(m, 2H), 4.19-4.28(m, 2H), 4.40(d, *J*=8.88, 1H), 5.30-5.47(m, 1H), 6.38(d,d, *J*₁=10.12Hz, *J*₂=1.78Hz, 1H), 6.43(s, 1H), 7,14(d,d, *J*₁=10.12Hz, *J*₂=1.31Hz, 1H), **MASS (ES+)** : 605.5 |
| IB.27 | 0.89(t, *J*=7.32Hz, 3H), 0.99(d, *J*=7.11Hz, 3H), 1.11(s,3H), 1.34(q, *J*=7.53Hz, 2H), 1.52(s,3H), 1.55-1.59(m, 2H), 1.73-2.04(m,4H), 2.22-2.37(m, 7H), 3.34(m, 1H), 3.69-3.73(m, 2H), 4.24(d, *J*=1.36Hz, 2H), 4.40(d, *J*=8.57, 1H), 5.30-5.48(m, 1H), 6.38(dd, *J*₁=10.13Hz, *J*₂=1.70Hz 1H), 6.43(s, 1H), 7.13(d, *J*=10.12Hz, 1H): **MASS (ES+)**: 565.3 |
| IB.28 | 0.94(d, *J*=7.17Hz, 3H), 1.08(s,3H), 1.12(t, *J*=7.58Hz, 3H), 1.27-1.32(m, 1H), 1.53(s,3H), 1.64-1.83(m,4H), 2.22-2.48(m,7H), 3.30-3.34(m, 1H), 4.40(d, *J*=7.59, 1H), 4.60(d,d *J*₁=15.58Hz, *J*₂=1.63Hz, 1H), 4.84(d,d *J*₁=15.60Hz, *J*₂=2.18Hz, 1H), 5.30-5.47(m, 1H), 6.37(d,d *J*₁=10.17Hz, *J*₂=1.46Hz, 1H), 6.43(s, 1H), 7.12(d, *J*=10.10Hz, 1H), **MASS (ES+) :** 491.2 |
| IB.29 | 0.99(d, *J*=7.12Hz, 3H), 1.12(s,3H), 1.31-1.36(m, 1H), 1.52(s,3H), 1.54-1.70(m, 5H), 1.73-1.92(m,6H), 2.18-2.38(m, 6H), 2.72-2.80 (m, 1H), 3.32-3.37(m, 1H), 3.64-3.76(m, 2H), 4.24(s, 2H), 4.41(d, *J*=8.75, 1H), 5.31-5.47(m, 1H), 6.38(d,d, *J*₁=10.16Hz, *J*₂=164Hz, 1H), 6.44(s, 1H), 7.14(d, *J*=10.17Hz, 1H). **MASS (ES+) :** 577.2 |
| IB.30 | 0.99(d, *J*=7.13Hz, 3H), 1.06(s,3H); 1.11 (t, *J*=7.59Hz, 3H), 1.30-1.35(m, 1H), 1.52(s,3H), 1.72-2.04(m,4H), 2.17-2.43(m, 7H), 3.31-3.36(m, 1H), 3.57(dd, *J*₁=13.64Hz, *J*₂=7.49Hz, 1H), 3.73(dd, *J*₁=13.86Hz, *J*₂=8.50Hz, 1H), 4.21(dd, *J*₁=12.64Hz, *J*₂=6.89Hz, 1H), 4.33(dd, *J*₁=12.89Hz, *J*₂=8.13Hz, 1H), 4.37(d, *J*=10.81Hz, 1H), 5.30-5.46(m, 1H), 5.52-5.59(m, 1H), 5.73-5.79(m, 1H), 6.37(dd, *J*₁=10.14Hz, *J*₂=1.74Hz, 1H), 6.43(s, 1H), 7.22(dd, *J*₁=10.10Hz, *J*₂=1.03Hz, 1H), **MASS (ES+) :** 539.3 |
| IB.31 | 0.98(d, *J*=7.11Hz, 3H), 1.10-1.17(m, 6H), 1.15(s,3H), 1.30-1.36(m, 1H), 1.54(s,3H), 1.73-1.90(m,3H), 2.20-2.44(m, 8H), 3.03(d, *J*=2.56Hz, 1H), 3.34.3.38(m, 1H), 3.68(dd, 2H), 4.40(d, *J*=8.47, 1H), 4.55(d, *J*=15.4Hz, 1H), 7.72(d, *J*=15.4Hz, 1H), 5.31-5.49(m, 1H), 6.36(dd, *J*₁=10.11Hz, *J*₂=1.1.62Hz 1H), 6.43(s, 1H), 7.16(d, *J*=10.11Hz, 1H). **MASS (ES+):** 615.5 (M+Na)⁺. |
| IB.32 | 0.98(d, *J*=7.1Hz, 3H), 1.12(t, *J*=7.49Hz,3H), 1.13(s,3H), 1.20-1.36(m, 1H), 1.54(s,3H), 1.70-1.93(m,3H), 2.10(s, 3H), 2.19-2.42(m,6H), 2.88(s,1H), 3.34-3.37(m, 1H), 3.64(d, *J*=16.77, 1H), 3.72(d, *J*=16.82, 1H), 4.411.12(d, *J*=8.07Hz, 1H), 4.56(d, *J*=15.4Hz,1H), 4.691.12(d, *J*=15.4Hz, 1H), 5.30-5.47(m,1H), 6.38(dd, *J*₁=10.12Hz, *J*₂=1.50Hz, 1H), 6.43(s, 1H), 7.17(d, *J*=10.15Hz,1H), **MASS (ES+)** : 601.0 (M+Na)⁻. |
| IB.33 | 0.97(t, *J*=7.7.4Hz, 3H), 0.98(d, *J*=7.44Hz, 3H), 1.12(t, *J*=7.64Hz, 3H), 1.15(s,3H), 1.30-1.36(m, 1H), 1.54(s,3H), 1.66(q, *J*=7.42Hz, 2H), 1.70-1.94(m, 4H), 2.19-2.46(m, 8H), 3.01(d, *J*=2.63Hz,1H), 3.33-3.38(m, 1H), 3.64(d, *J*=16.88Hz, 1H), 3.72(d, *J=*16.75Hz, 1H), 4.40(d, *J*=8.68Hz, 1H), 4.53(t,d, *J*₁=15.38Hz, *J*₂=1.70Hz, 1H), 4.72(dd, *J*₁=15.39Hz, *J*₂=1.62Hz, 1H), 5.31-5.48(m, 1H), 6.37(dd, *J*₁=10.12Hz, *J*₂=1.77Hz, 1H), 6.43(s, 1H), 7.16(dd, *J*₁=10.15Hz, *J*₂=1.26Hz). **MASS (ES+)**:607.5 |
| IB.34 | 0.08(d, *J*=7.15Hz, 3H), 1.12(t, *J*=7.59Hz, 3H), 1.16(s,3H), 1.18(d, *J*=7.00Hz, 3H), 1.19(d, *J*=6.96Hz, 3H), 1.30-1.36(m, 1H), 1.54(s,3H), 1.70-1.94(m, 4H), 2.19-2.48(m, 4H), 2.36(q, *J*=7.55Hz, 2H), 2.56-2.63(m, 1H), 3.11(d, *J*=3.05Hz, 1H), 3.33-3.37(m, 1H), 3.65(d, *J*=16.93Hz, 1H), 3.71(d, *J*=17.26Hz, 1H), 4.40(d, *J*=8.83Hz, 1H), 4.53(t,d, *J*₁=15.38Hz, *J*₂=1.72Hz 1H), 4.73(dd, *J*₁=15.36Hz, *J*₂=1.61Hz, 1H), 5.31-5.48(m,1H), 6.37(dd, *J*₁=10.12Hz, *J*₂=1.79Hz, 1H), 6.43(s, 1H), 7.16(dd, *J*₁=10.12Hz, *J*₂=1.17Hz 1H). **MASS (ES+)** : 607.7 |
| IB.35 | 0.93(t, *J*=7.31Hz, 3H), 0.98(d, *J*=7.15Hz, 3H), 1.12(t, *J*=7.71Hz, 3H), 1.15(s, 3H), 1.25-1.41(m, 3H), 1.54(s, 3H), 1.55-1.94(m, 6H), 2.19-2.46(m, 8H), 3.03(d, *J*=2.91Hz, 1H), 3.33-3.38(m, 1H), 3.64(d, *J*=16.76Hz, 1H), 3.72(d, *J*=16.71Hz, 1H), 4.40(d, *J*=8.90Hz, 1H), 4.33(t,d, *J*₁=15.40Hz, *J*₂=1.75Hz, 1H), 4.71(dd, *J*₁=15.41Hz, *J*₂=1.43Hz, 1H), 5.31-5.48(m, 1H), 6.37(dd, *J*₁=10.13Hz, *J*₂=1.78Hz, 1H), 6.43(s, 1H), 7.16(dd, *J*₁=10.11Hz, *J*₂=1.19Hz 1H). **MASS (ES+)** : 621.5 |
| IB.36 | 0.98(d, *J*=7.18Hz, 3H), 1.12(t, *J*=7.59Hz, 3H), 1.15(s, 3H), 1.25-1.36(m, 1H), 1.55(s, 3H), 1.73-2.04(m, 6H), 2.20-2.47(m, 10H), 3.16-3.20(m, 2H), 3.34-3.37(m, 1H), 3.68(Two d, 2H), 4.41(d, *J=*8.64Hz, 1H), 4.54(t,d, *J*₁=15.38Hz, *J*₂=1.74Hz 1H), 4.72(t,d, *J*₁=15.37Hz, *J*₂=1.66Hz, 1H), 5.33-5.45(m,1H), 6.37(dd, *J*₁=10.16Hz, *J*₂=1.81Hz, 1H), 6.43(s, 1H), 7.16(dd, *J*₁=10.10Hz, *J*₂=1.26Hz, 1H), **MASS (ES+) :** 619.7 |
| IB.37 | 0.91(t, *J*=6.86Hz, 3H), 0.98(d, *J*=7.15Hz, 3H), 1.12(t, *J*=7.59Hz, 3H), 1.15(s, 3H), 1.25-1.36(m, 3H), 1.54(s, 3H), 1.61-1.94(m, 6H), 2.19-2.38(m, 8H), 3.05(d, *J*=3.03Hz, 1H), 3.34-3.38(m, 1H), 3.64(d, *J*=16.70Hz, 1H), 3.72(d, *J*=16.93Hz, 1H), 4.40(d, *J*=8.58Hz, 1H), 4.52(d, *J*=15.37Hz, 1H), 4.72(d, *J*=15.39Hz, 1H), 5.31-5.47(m, 1H), 6.37(dd, *J*₁=10.14Hz, *J*₂=1.75Hz, 1H), 6.43(s, 1H), 7.16(dd, *J*₁=10.63Hz. *J*₂=1.04Hz, 1H). **MASS (ES+)** : 634.8 |
| IB.38 | 1.07(s,3H), 1.14(t, *J*=7.53Hz, 3H), 1.21-1.28(m, 1H), 1.42(d, *J*=7.27Hz, 3H), 1.55(s, 3H), 1.60-1.65(m,1H), 1.90-2.00(m,5H), 2.26-2.42(m,7H), 2.61-2.71(m, 1H),3.36(d, *J*=16.65, 1H), 3.80(d, *J*=16.65, 1H), 4.26(m,2H), 4.40(d, *J*=8.38, 1H), 6.14(s,1H), 6.35 (dd, *J*₁=10.06Hz, *J*₂=1.39Hz, 1H), 7.24(d, *J*=10.23Hz,1H), **MASS (ES+)**: 541.6 (M+Na)⁺. |
| IB.39 | 1.06(s,3H), 1.45(t, *J*=7.54Hz, 3H), 1.21-1.29(m, 1H), 1.43(d, *J*=7.35Hz, 3H), 1.53(s,3H), 1.70-2.06 (m,6H), 2.27-2.41(m,6H), 3.35(d,t, *J*₁=16.67, *J*₂=2.34 1H), 3.81 (d,t, *J*₁=16,67, *J*₂=2.08, 1H), 4.25-4.28(m, 2H), 4.40(d, *J*=8.3Hz, 1H), 5.31-5.48(m,1H), 6.37(dd, *J*₁=10.12Hz, *J*₂=1.77Hz, 1H), 6.44(s, 1H), 7.16(dd, *J*₁=10.12Hz, *J*₂=1.16Hz, 1H), **MASS (ES+)** : 559.2 (M+Na)⁻ |
| IB.40 | 1.04(d, *J*=7.13Hz, 3H), 1.16(s,3H), 1.25-1.39(m, 2H), 1.43(s,3H), 1.44(s,3H), 1.55(s, 3H), 1.72-1.94(m, 3H), 2.28-2.51(m, 4H), 3.39-3.44(m, 1H), 3.67(d, *J*=16.29Hz, 1H), 3.75(d, *J*=16.24Hz, 1H), 4.60(s, 1H), 4.98-4.99(m, 1H), 5.37-5.50(m,1H), 6.32-6.35(m, 2H), 6.53(dd, *J*₁=3.41Hz, *J*₂=1.65Hz, 1H), 7.12(d, *J*=3.48Hz, 1H), 7.26(d, *J*=10.03Hz, 1H), 7.64(s, 1H), **MASS (ES+)** : 625.0(M+Na)⁺ |
| IB.41 | 096 (d, 3H, *J*=7.16Hz), 1.06 (s, 3H), 1.12 (t, 3H, *J*=7.57Hz), 1.26-1.34 (m, 1H), 1.53 (s, 3H), 1.71-1.91 (m, 3H), 2.24-2.39 (m, 6H), 3.30-3.35 (m, 1H), 3.76 (s, 3H), 4.41 (m, 1H), 4.71 (1H, ddd, *J*₁=15.93Hz, *J*₂=4.75Hz, *J*₃=1.81Hz), 4.84 (ddd, 1H, *J*₁=16.00Hz, *J*₂=4.75Hz, *J*₃=1.82Hz), 5.34 & 5.46 (m, 1H), 6.03 (d, 1H, *J*=15.77Hz), 6.37 (dd, 1H, *J*₁=10.15Hz, J₂=1.78Hz), 6.44 (s, 1H), 6.93 -6.97(m, 1H), 7.12 (dd, *J*₁=10.13Hz, *J*₂=1.18Hz), **MASS (ES+)**: 573.1(M+Na)⁺. |
| IB.42 | 1.00 (d, 3H, *J*=7.15Hz), 1.06 (s, 3H), 1.12 (t, 3H, *J*=1.56Hz), 1.30-1.35(m, 1H), 1.52(s, 3H), 1.73-1.87 (m, 4H), 222-240 (m, 6H), 3.34-3.36 (m, 1H), 3.71 (d, 2H, *J*=7.09Hz), 3.74 (s, 3H), 4.40 (d, 1H, *J*=8.85Hz), 5.32 & 5.44(m*,* 1H), 6.02 (d 1H, *J*=15.46Hz), 6.38 (dd, 1H), *J*₁=10.15Hz, *J*₂=1.79Hz), 6.43 (s, 1H), 6.85-6.89 (m, 1H), 7.12 (dd, 1H, *J*₁=10.15Hz, *J*₂=1.18Hz), **MASS (ES+)**: 589.1(M+Na)⁺. |
| IB.43 | 1.03 (s, 3H), 1.15-1.23 (m, 3H), 1.27 (t, 3H, *J*=7.12Hz), 1.45 (s, 3H), 1.47-1.55 (m, 1H), 1.63-1.70 (m, 2H), 1.80-1.87 (m, 1H), 1.93-2.00 (m, 1H). 2.09-2.16 (m, 3H). 2.32-2.36 (m, 1H), 2.53-2.61 (m, 1H), 2.91-2.98 (m, 1H), 3.76 (s, 3H), 4.13 (d of q, 2H, *J*₁=14.22Hz, *J*₂=7.10Hz, *J*₃=2.13Hz), 4.51 (s, 1H), 4.79 (d, 1H, *J*=1.62Hz), 4.80 (d, 1H, *J*=1.62Hz), 6.02 (s, 1H), 6.06 (d, 1H, *J*=15.77Hz), 6.27 (dd, 1H, *J*₁= 10.09Hz, *J*₂=1.75Hz), 6.94 (d of t, 1H, *J*₁=15.76Hz, *J*₂=4.71Hz), 7.23 (d, 1H, *J*=10.11Hz), **MASS (EI)** : 516 |
| IB.44 | 0.99(d, *J*=7.13Hz, 3H), 1.09(s,3H), 1.11(t, *J*=7.38Hz, 3H), 1.29-1.35(m, 1H), 1.39(t, *J*=7.18Hz, 3H) 1.53(s,3H), 1.72-1.89(m, 3H), 2.20-2-50(m, 6H), 3.32-3.37(m, 1H), 3.52(d, *J*=13.4Hz, 1H), 3.62(dd, *J*₁=15.97Hz, *J*₂=4.53Hz, 1H), 4.37(q, *J*=7.15Hz, 2H), 4.38-4.40(m, 1H), 4.70-4.82(m, 1H), 5.30-5.47(m,1H), 5.82-5.83(m, 2H), 6.37(dd, *J*₁=10.13Hz, *J*₂=1.77Hz, 1H), 6.43(s, 1H), 7.15(dd, *J*₁=10.12Hz, *J*₂=1.18Hz, 1H), |
| IB.45 | 1.04(d, *J*=7.06Hz, 3H), 1.16(s,3H), 1.29-1.34(m, 1H), 1.68(s, 3H), 1.71-1.97(m, 4H), 2.37-2.70(m,5H), 3.38-3.41(m, 1H), 3.70(d, *J*=16.35, 1H), 3.79(d, *J*=16.32Hz, 1H), 4.15-4.16(m, 2H), 4.53(d, *J*=2.4Hz, 1H), 4.74(t, *J*=5.94, 1H), 5.03(d, *J*=3.67Hz, 1H), 6.05(s, 1H), 6.28(dd, *J*₁=10.07Hz, *J*₂=1.27Hz, 1H), 6.53(dd, *J*₁=3.15Hz, *J*₂=1.47Hz, 1H), 7.18(d, *J*=3.35, 1H), 7.31 (d, *J*=10.1Hz, 1H), 7.63(br-s, 1H), **MASS (ES+)**: 595.4(M+Na)⁺. |
| IB.46 | 1.07(d, *J*=7.16Hz, 3H), 1.15(s,3H), 1.35-1.38(m, 1H), 1.39(t, *J*=7.14Hz, 3H), 1.56(s, 3H), 1.76-1.97(m, 3H), 2.28-2.50(m, 5H), 3.40-3.44(m, 1H), 3.55(dd, *J*₁=14.06Hz, *J*₂=2.91Hz. 1H), 3.65(dd, *J*₁=15.79Hz, *J*₂=4.86Hz, 1H), 4.36(q, *J*=7.17Hz, 2H), 4.43(d, *J*=9.21Hz, 1H), 4.70-4.83(m, 1H), 5.32-5.49(m, 1H), 5.83-5.85(m,2H), 6.39(dd, *J*₁=10.13Hz, *J*₂=1.82Hz, 1H), 6.45(s, 1H), 6.48(dd, *J*₁=3.49Hz, *J*₂=1.73Hz, 1H), 7.11(dd, *J*₁=3.49Hz, *J*₂=0.64Hz, 1H), 7.18(dd, *J*₁=10.11Hz, *J*₂=1.20Hz, 1H), 7.57(dd, *J*₁=1.6Hz, *J*₂=0.70Hz, 1H) |
| IB.47 | 1.06(d, *J*=7.13Hz, 3H), 1.12(s,3H), 134-1.38(m, 1H), 38(t, *J*=7.16Hz, 3H), 1.55(s, 3H), 1.77-1.98(m, 3H), 2.25-2.48(m, 5H), 3.42-3.45(m, 1H), 3.63(dd, *J*₁=13.76Hz, *J*₂=7.57Hz, 1H), 3.76(dd, *J*₁=13.79Hz, *J*₂=848Hz, 1H), 4.36(q, *J*=7.17Hz, 2H), 4.43(d, *J*=8.99Hz, 1H), 4.95-5.05(m, 2H), 5.31-5.50(m,1H), 5.67-5.71(m,1H), 5.77-5.81(m, 1H), 6.39(dd, *J*₁=10.14Hz, *J*₂=1.55Hz, 1H), 6.45(s, 1H), 6.49(dd, *J*₁=3.32Hz, *J*₂=1.55Hz, 1H), 7.11(dd, *J*=3.48Hz, 1H), 7.16(d, *J*=10.14Hz, 1H), 7.58(s, 1H), |
| IB.48 | 1.05(d, *J*=7.15Hz, 3H), 1.17(s,3H), 1.37-1.40(m, 1H), 1.49(d, *J*=7.07Hz, 3H), 1.55(s, 3H), 1.77-1.99(m, 4H), 2.13(s, 3H), 2.17-2.48(m,4H), 3.42-3.46(m, 1H), 3.67(d, *J*=16.90, 1H), 3.77(d, *J*=16.82Hz, 1H), 4.44(d, *J*=8.50Hz, 1H), 4.64(t,d, *J*₁=15.4Hz, *J*₂=1.87Hz, 1H), 4.78(t,d, *J*₁=15.41Hz, *J*₂=1.86Hz, 1H), 5.09(q, *J*=6.37Hz, 1H), 5.31-5.51 (m, 1H), 6.39(dd, *J*₁=10.12 Hz, *J*₂=1.78Hz, 1H), 6.45(s, 1H), 6.50(dd, *J*₁=3.49Hz, *J*₂=1.73Hz, 1H), 7.12(dd, *J*₁=3.47Hz, *J*₂=0.49Hz, 1H), 7.16(dd, *J*₁=10.14Hz, *J*₂=1.31Hz, 1H), 7.59(d, *J*=0.75Hz, 1H), **MASS (ES+)** : 689.0 |
| IB.49 | 0.97(t, *J*=7.7.47Hz, 3H), 0.98(d, *J*=7.44Hz, 3H), 1.12(t, *J*=7.64Hz, 3H), 1.15(s,3H), 1.30-1.36(m, 1H), 1.54(s,3H), 1.66(q, *J*=7.42Hz, 2H), 1.70-1.94(m, 4H), 2.19-2.46(m, 8H), 3.01(d, *J*=2.63Hz, 1H), 3.33-3.38 (m, 1H), 3.64(d, *J*=16.88Hz, 1H), 3.72(d, *J*=16.75Hz, 1H), 4.40(d, *J*=8.68Hz, 1H), 4.53(t,d, *J*₁=15.38Hz, *J*₂=1.70Hz, 1H), 4.72(dd, *J*₁=15.39Hz, *J*₂=1.62Hz, 1H), 5.31-5.48(m, 1H), 6.37(dd, *J*₁=10.12Hz, *J*₂=1.77Hz, 1H), 6.43(s, 1H), 7.16(dd, *J*₁=10.15Hz, *J*₂=1.26Hz, 1H), **MASS (ES+**) : 607.5 |
| IB.50 | 1.06(d, *J*=7.16Hz, 3H), 1.17(d, *J*=2.39Hz, 3H), 1.18(d, *J*=2.37Hz, 3H), 1.21(s, 3H), 1.35-1.41(m, 1H), 1.56(s, 3H), 1.77-2.00(m, 3H), 2.29-2.62(m, 5H), 3.21(d, *J*=2.62Hz, 1H), 3.42-3.49(m, 1H), 3.67(d, *J*=16.86Hz, 1H), 3.75(d, *J*=16.81Hz, 1H), 4.45(d, *J*=8.59Hz, 1H), 4.52(d, *J*=15.45Hz, 1H), 4.74(d, *J*=15.33Hz, 1H), 5.33-5.50(m, 1H), 6.40(dd, *J*₁=10.05Hz, *J*₂=1.65Hz, 1H), 6.45(s, 1H), 6.50(dd, *J*₁=3.50Hz, *J*₂=1.69Hz, 1H), 7.13(dd, *J*₁=3.56Hz, *J*₂=0.57Hz, 1H), 7.19(dd, *J*₁=0.75Hz, *J*₂=0.22Hz, 1H), 7.58(s, 1H). **MASS (ES+)** : 667.0(M+Na)⁺ |
| IB.51 | 1.06(d, *J*=7.16Hz,3H), 1.21(s, 9H), 1.22(s, 3H), 1.35-1.41(m, 1H), 1.56(s, 3H), 1.77-2.00(m, 1H), 2.29-2.53(m, 1H), 3.27(d, *J*=3.09Hz, 1H), 3.42-3.47(m, 1H), 3.67(d, *J*=16.88Hz, 1H), 3.74(d, *J*=16.89Hz, 1H), 4.45(d, *J*=8.36Hz, 1H), 4.50(d, *J*=15.35Hz, 1H), 4.75(d, *J*=15.40Hz, 1H), 5.33-5.40(m, 1H), 6.40(dd, *J*₁=10.19Hz, *J*₂=1.84Hz, 1H), 6.45(s, 1H), 6.50(dd, *J*₁-3.51Hz, *J*₂=1.68Hz, 1H), 7.14(d, *J*=3.43Hz, 1H), 7.17(dd, *J*₁=15.12Hz, *J*₂=0.99Hz, 1H), 7.58(d, *J*=0.98Hz, 1H). **MASS (ES+)** ; 680.9(M+Na)⁺ |
| IB.52 | 0.98(d, *J*=7.14Hz, 3H), 1.12(t, *J*=7.56Hz, 3H), 1.17(s, 3H), 1.22(s, 9H), 1.30-1.36(m, 1H), 1.54(s, 3H), 1.73-1.95(m, 3H), 2.21-2.40(m, 6H), 3.20(d, *J*=3.29Hz, 1H), 3.34-3.38(m, 1H), 3.68(br-s, 2H), 4.40 (d, *J*=8.77Hz, 1H), 4.50(d. *J*=14.59Hz, 1H), 474(d, *J*=15.33Hz. 1H), 5.30-5.50(m,1H),6.37(dd, *J*₁=10.12Hz, *J*₂=1.77Hz, 1H), 643(s,1H), 7.15(dd, *J*₁=10.09Hz, *J*₂=1.04Hz, 1H), MASS(ES+) 643.0(M+Na) |
| IB.53 | 0.94(d, *J*=7.45Hz, 3H), 1.06(d, *J*=7.18Hz, 3H), 1.12(s, 3H), 1.34-1.40(m, 1H), 1.55(s, 3H), 1.66(t, *J*=7.41Hz, 2H), 1.77-2.05(m, 3H).. 2.30(t, *J*=7.45Hz, 2H), 2.35-2.48(m, 4H), 3.43-3.47(m, 1H), 3.66(d, *J*=7.79Hz, 1H), 3.43(d, *J*=9.05Hz, 1H), 4.76-4.79(m, 2H), 5.59-5.72(m, 1H), 6.39(dd, *J*₁=10.17Hz, *J*₂=1.80Hz, 1H), 6.45(s, 1H), 6.49(dd, *J*₁=3.48Hz, *J*₂=1.81Hz), 1H), 7.12(d, *J*=3.42 Hz, 1H), 7.17(dd, *J*₁=10.08Hz, *J*=1.02Hz, 1H), 7.58(d, *J*=0.93Hz, 1H), **MASS (ES+)**: 669.0(M+Na)⁺ |
| IB.54 | 1.06(d, 3H, *J*=7.14Hz), 1.21(s, 3H), 1.38-1.41(m, 1H), 1.39(t, 3H, *J*=7.15Hz), 1.56(s, 3H), 1.77-1.96(m, 3H), 2.28-2.64(m, 5H), 3.37-3.45(m, 1H), 3.72(br-s, 2H), 4.38(q, *J*=7.12Hz, 2H), 4.46(d, *J*=8.96Hz, 1H), 4.79(d, *J*=15.27Hz, 1H), 4.89(d, *J*=15.31Hz, 1H), 5.32-5.51(m, 1H), 6.40(dd, *J*₁=10.14Hz, *J*₂=1.77Hz, 1H), 6.45(s, 1H), 6.50(dd, *J*₁=3.47Hz, *J*₂=1.69Hz, 1H), 7.12(d, *J*=3.25Hz, 1H), 7.19(dd, *J*₁=10.11Hz, *J*₂=0.85Hz, 1H), 7.58(d, *J*=0.72Hz, 1H). |
| IB.55 | 0.96(t, *J*=7.42Hz, 3H), 1.07(d, *J*=7.18Hz, 3H), 1.21(s, 3H), 1.36-1.42(m, 1H), 1.56(s, 3H), 1.65(q, *J*=7.43Hz, 2H), 1.70-2.02(m, 3H), 2.29-1.53(m, 6H), 3.09(d, *J*=2.87Hz, 1H), 3.43-3.47(m, 1H), 3.67(d, *J*=16.68Hz, 1H), 3.77(d, *J*=16.66Hz, 1H), 4.46d, *J*=8.60Hz, 1H), 4.53(d, *J*=18.41Hz, 1H), 4.73(d, *J*=15.35Hz, 1H), 5.33-5.50(m, 1H), 6.40(dd, *J*₁=10, 17Hz, *J₂=*1.81Hz, 1H), 6.46(, 1H), 7.10(dd, *J*₁=4.83Hz, *J*₂=3.94 1H), 7.19(dd, *J*₁=10.10Hz, *J*₂=1.02Hz, 1H), 7.57(dd, *J*₁=5.01Hz, *J*₂=1.05Hz, 1H), 7.75(dd, *J*₁=3.71Hz, *J₂*=1.03Hz, 1H). **MASS (ES+)** : 682.9(M+Na)⁺ |
| IB.56 | 0.96(t, *J*=7.40Hz, 3H), 1.05(d, *J*=7.16Hz.3H), 1.21(s, 3H),1.37-1.40(m, 1H), 1.56(s, 3H), 1.66(q, *J*=7.42Hz, 2H), 1.78-2.0(m, 3H), 2.28-2.41(m, 6H), 3.11(d, *J*=3.03Hz, 1H), 3.38-3.45(m, 1H), 3.71(d, *J=*11.09Hz, 1H), 4.46(d, *J*=8.70Hz, 1H), 4.52(d, *J*=15.37Hz, 1H), 4.74(d, *J*=15.34Hz, 1H), 6.42 (dd, *J*₁=10.18Hz, *J*₂=1.82Hz, 1H), 6.46(s, 1H), 6.94(d, *J*=4.09Hz, 1H), 7.19(dd, *J*₁=10.09Hz, *J*₂=1.06Hz, 1H), 7.54(d, *J*=4.07Hz, 1H), **MASS (ES+)** : 716.9(M+Na)⁺ |
| IB.57 | 0.92-1.03(m, 4H), 1.05(d, *J*=7.26Hz, 3H), 1.19(s, 3H), 1.35-1.40(m, 1H), 1.56(s,3H), 1.61-1.98(m, 4H), 2.28-2.51(m, 4H), 3.06(d, *J*=2.41Hz, 1H), 3.43-3.46(m, 1H), 3.66(d, *J*=16.84Hz, 1H), 3.77(d, *J*=16.84Hz, 1H), 4.44(d, *J*=8.44Hz, 1H), 4.55(d, *J*=16.23Hz, 1H), 4.70(d, *J*=14.68Hz, 1H), 5.33-5.50(m,1H), 6.40(dd, *J*₁=10.15Hz, *J*₂=1.79Hz, 1H), 6.45(s, 1H), 6.50(dd, *J*₁=3.47Hz, *J*₂=1.65Hz, 1H), 7.13(d, *J*=3.3Hz, 1H), 7.18(dd, *J*₁=10.10Hz, *J*₂=1.04Hz, 1H), 7.59(d, *J*=0.83Hz, 1H). **MASS (ES+)** : 693.0(M+Na)⁻ |
| IB.58 | 1.06(d, *J*=7.14Hz, 3H), 1.21(s,3H), 1.35-1.41(m, 1H), 1.57(s, 3H), 1.77-2.04(m, 5H), 2.21-2.53(m, 8H), 3.15-3.21(m, 2H), 3.42-3.47(m, 1H), 3.67(d, *J*=*16.81*Hz, 1H), 3.75(d, *J*=17.06Hz, 1H), 4.45(d, *J*=*8.61*Hz, 1H), 4.43(d, *J*=*15.33*Hz, 1H), 4.73(d, *J*=15.39Hz, 1H), 5.33-5.50(m, 1H), 6.40(dd, *J*₁=10.14Hz, *J*₂=1.82Hz, 1H), 6.45(s, 1H), 6.50(dd, *J*₁=3.47Hz, *J*₂=1.60Hz, 1H), 7.13(d, *J*=*3.26*Hz, 1H), 7.18(dd, *J*₂=10.18Hz, *J*₂=1.26Hz, 1H), 7.58(d, *J*=1.71Hz, 1H), **MASS (ES+)** : 678.9(M+Na)⁺ |
| IB.59 | 1.06(d, *J*=7.16Hz, 3H), 1.21(s,3H), 1.35-1.41(m, 1H), 1.56(s,3H), 1.62-1.9(m, 11H), 2.28-2.51(m, 4H), 2.73-2.81(m, 1H), 3.17(d, *J*=*2.84*Hz, 1H), 3.42-3.49(m, 1H), 3.66(d, *J*=16.87 Hz, 1H), 3.75(d, *J*=16.86Hz, 1H), 4.44(d, *J*=8.46Hz, 1H), 4.51(d, *J*=15.31Hz, 1H), 4.74(d, *J*=15.33Hz, 1H), 5.33-5.50(m, 1H), 6.40(dd. *J*₁=10.18Hz, *J*₂=1.82Hz, 1H), 6.45(s, 1H), 6.50(dd, *J*₁=3.48Hz, *J*₂=1.79Hz, 1H), 7.13(d, *J*=3.4Hz, 1H), 7.17(dd, *J*₁=10.08Hz, *J*₂=10.08Hz, 1H), 7.59(d. *J*=1.89Hz, 1H). **MASS (ES+)** : 693.0(M+Na)⁺ |
| IB.60 | 0.95(t. *J*=7.38Hz, 3H), 1.06(d, *J*=7.18Hz, 3H), 1.12(s, 3H), 1.34-1.40(m, 1H), 1.55(s, 3H 1.64(q, *J*=7.44Hz, 2H), 1.74-1.94(m, 3H), 2.30(t, *J*=7.46Hz, 2H), 2.36-2.50(m, 4H), 2.72(s, 1H), 3.42-3.47(m, 1H), 3.53(dd, *J*₁=14.08Hz, *J*₂=6.92Hz, 1H), 3.67(dd, *J*₁=14.0Hz. *J*₂=5.33Hz, 1H), 4.42(d, *J*=8.53Hz, 1H), 4.50(dd, *J*₁=13.23Hz, *J*₂=4.74Hz, 1H), 4.62(dd, *J*₁=13.20Hz, *J*₂=5.70Hz, 1H), 5.32-5.49(m, 1H). 5.67-5.81(m, 2H), 6.39(dd, *J*₁=10.19Hz, *J*₂=1.80Hz, 1H), 6.45(s, 1H), 6.49(dd, *J*₂=1.56Hz, *J*₂=1.56Hz, 1H), 7.12(d, *J*=3.42Hz, 1H), 7.16(dd, *J*₁=10.15Hz. *J*₂=1.02Hz, 1H), 7.58(d, *J*=0.79Hz, 1H). **MASS (ES+)** : 669.0(M+Na)⁺ |
| IB.61 | 1.03(d, *J*=7.12Hz, 3H). 1.19(s,3H), 1.25-1.42(m, 2H), 1.55(s, 3H), 1.65-1.96(m, 3H), 2.21-2.46(m, 4H), 3.45(m, 1H), 3.71(d, *J*=16.51Hz. 1H), 3.79(d, *J*=16.46Hz, 1H), 4.20-4.30(m, 2H), 4.46(d, *J*=8.42Hz, 1H), 5.30-5.50(m,1H), 6.42(dd, *J*₁=10.05Hz, *J*₂= 1.80Hz, 1H), 6.47(s, 1H), 7.15(dd, *J*₁=10.15Hz, *J*₂=1.21Hz, 1H), 7.43(d, *J*=*8.59*Hz, 2H), 7.86(d, *J*=8.55Hz, 2H). **MASS (ES+)** : 640.9(M+ Na)⁺ |
| IB.62 | 1.03(d, *J*=7.05Hz, 3H), 1.20(s,3H), 1.22-1.93(m, 8H), 2.29-2.51(m, 4H), 3.44-2.50(m, 1H), 3.71(d, *J*=16.42Hz, 1H), 3.80(d, *J*=16.52Hz, 1H), 4.26-4.32(m, 2H), 4.46(d, *J*=8.81Hz, 1H), 5.33-5.50(m,1H), 6.40-6.42(m, 1H), 6.46(s, 1H), 7.16(dd, *J*=10.15Hz, 1H), 7.39(d, *J*=7.91Hz, 1H), 7.55(d, *J*=7.56Hz, 1H)7.79(d, *J*=7.76Hz, 1H), 7.91(s, 1H). **MASS (ES+)** : 640.9(M+ Na)⁺ |
| IB.63 | 1.06(d, *J*=7.14Hz, 3H), 1.19(s.3H), 1.36-1.41(m, 1H), 1.55(s, 3H), 1.74.1.99(m, 3H), 2.17-2.51(m, 5H), 3.42.3-46(m, 1H), 3.69(d, *J*=16.73Hz 1H), 3.76(d, *J*=16.91Hz, 1H), 3.81(s, 3H), 4.45(d, *J*=8.63Hz, 1H), 4.66(td, *J*₁=1.70Hz, *J*₁=1.70Hz, 1H), 4.73(td, *J*₁=15.49Hz, *J*₂=1.71Hz, 1H), 5.33-5.49(m.1H), 6.40(dd, *J*₁=10.12Hz, *J*₂=1.72Hz, 1H),6.45(s,1H), 6.50(dd, *J*₁=3.48Hz, *J*₂=1.68Hz, 1H), 7.14(d, *J*=3.43Hz, 1H), 7.16(dd, *J*₁=10.09Hz, *J*₂=0.99Hz, 1H), 7.59(d, *J*=0.92Hz, 1H). **MASS (ES+)** : 654.9 (M + Na)⁺. |
| IB.64 | 1.06(d, *J*=7.16Hz, 3H), 1.19(s,3H), 1.33(t, *J*=7.05Hz, 3H), 1.36-1.41(m, 1H), 1.55(s, 3H), 1.74-1.99(m, 3H), 2.28-2.50(m, 4H), 2.56(d, *J*=1.85Hz, 1H), 3.42-3.47(m, 1H), 3.69(d, *J*=16.88Hz, 1H), 3.76(d, *J*=16.88Hz, 1H), 4.22(q, *J*=7.13Hz, 2H), 4.44(d, *J*=8.65Hz, 1H), 4.65(d, *J*=15.49Hz, 1H), 4.72(d, *J*=15.41Hz, 1H), 5.33-5.49(m,1H), 6.40(dd, *J*₁=10.13Hz, *J*₂=1.63Hz, 1H), 6.45(s,1H), 6.51(dd, *J*₁=3.44Hz *J*₂=1.69Hz. 1H), 7.14(d, *J*=3.50Hz, 1H), 7.16(d, *J*=10.20Hz, 1H), 7.59(d, *J*=0.87Hz, 1H). **MASS (ES+)** : 669.1(M + Na)⁺. |
| IB.65 | 1.06(d, *J*=7.19Hz, 3H), 1.20(s,3H), 1.34-1.43(m, 1H), 1.50(s, 9H), 1.55(s, 3H), 1.62-1.99(m, 3H), 2.28-2.54(m, 5H), 3.43-3.48(m, 1H), 3.68(d, *J*=16.70Hz, 1H), 3.77(d, *J*=16.65Hz, 1H), 4.44(d, *J*=8.51Hz, 1H), 4.57-4.68(m, 2H), 5.33-5.49(m, 1H), 6.40(dd. *J*₁=10.06Hz, *J*₂=1.79Hz, 1H),6.45(s,1H), 6.50(dd, *J*₁=3.48Hz, *J*₂=1.78Hz, 1H), 7.11-7.14(m, 2H), 7.59(d, *J*=1.00Hz, 1H). **MASS (ES+)** : 697.0(M + Na)⁺ |
| IB.66 | 0.96(d, *J*=6.74Hz, 6H),1.06(d, *J*=7.13Hz, 3H), 1.19(s,3H), 1.35-1.41(m, 1H), 1.55(s, 3H), 1.74-2.00(m, 4H), 2.28-2.52(m, 5H), 3.43-3.46(m, 1H), 3.69(d, *J*=16.80Hz, 1H), 3.77(d, *J*=16.79Hz, 1H), 3.93(d, *J*=6.72Hz, 2H), 4.44(d, *J*=8.62Hz, 1H), 4.66(d, *J*=15,49Hz, 1H), 4.71(d, *J*=15.48Hz, 1H), 5.33-5.49(m,1H), 6.40(dd, *J*₁=10.18Hz, *J*₂=1.48Hz, 1H), 6.45(s,1H), 6.50(dd, *J*₁=3.36Hz, *J*₂=1.59Hz, 1H), 7.13-7.16(m,2H), 7.59(s, 1H). **MASS (ES+)** : 697.0(M + Na)⁺. |
| IB.67 | 1.06(d, *J*=7.14Hz, 3H), 1.19(s, 3H), 1.35-1.41(m, 1H), 1.55(s, 3H), 1.77-2.14(m,5H), 2.28-2.72(m, 5H), 2.55(t, *J*=7.2Hz, 2H), 3.42-3.47(m, 1H), 3.59(t, *J*=6.37Hz, 2H), 3.64(d, *J*=16.76Hz, 1H), 3.76(d, *J*=16.58Hz, 1H), 4.46(d, *J*=8.65Hz, 1H), 4.58(d, *J*=15.39Hz, 1H). 4.73(d, *J*=15.40Hz, 1H), 5.33-5.49(m,1H), 6.40(dd, *J*₁=10.14Hz, *J*₂=1.64Hz, 1H), 6.45(s, 1H), 6.50(dd, *J*₁=3.4Hz, *J*₂=1.57Hz, 1H), 7.13(d, *J*=3.45Hz, 1H), 7.18(d, *J*=9.98Hz, 1H), 7.59(d, *J*=0.75Hz, 1H). **MASS (ES+)** : 679.0 |
| IB.68 | 1.05(d, *J*=7,14Hz, 3H), 1.12(s, 3H), 1.19(s, 9H), 1.34-1.40(m, 1H), 1.55(s, 3H), 1.71-2.06(m, 3H), 2.27-2.52(m, 4H), 3.42-3.47(m, 1H), 3.63(d, *J*=*11.76*Hz, 1H), 3.68(d, *J*=11.84Hz, 1H), 4.43(d, *J*=*9.20*Hz, 1H), 4.74(dd, *J*₁=13.06Hz, *J*₂=6.26Hz, 1H), 4.79(dd, *J*₁=13.58Hz, *J*₂=6.06Hz, 1H), 5.32-5.49(m, 1H), 5.55-5.74(m, 1H), 6.39(dd, *J*₁=10.19Hz, *J*₂=1.41Hz, 1H), 6.45(s, 2H), 6.49(dd, *J*₁=3.34Hz, *J*₂=1.53Hz, 1H), 7.12(d, *J*=3.45Hz, 1H), 7.15(d, *J*=10.17Hz, 1H), 7.58(s, 1H). **MASS (ES+)** : 683.0(M+Na)⁺ |
| IB.69 | 1.06(d, *J*=7.15Hz, 3H), 1.13(s, 3H), 1.20(s, 9H), 1.34-1.42(m, 1H), 1.55(s, 3H), 1.71-1.95(m, 3H), 2.17-2.53(m, 4H), 2.81(s, 1H), 3.42-3.49(m, 1H), 3.54(dd, *J*₁=14.17Hz, *J*₂=7.36Hz, 1H), 3.67(dd, *J*₁=14.01Hz, *J*₂=5.52Hz, 1H), 4.42(d, *J*=8.46Hz. 1H), 4.50(dd, *J*₁=13.61Hz, *J*₂=4.34Hz, 1H), 4.62(dd, *J*₁=13.51Hz, *J*₂=5.59Hz, 1H), 5.32-5.49(m, 1H), 5.64-5.80(m, 2H), 6.39(dd, *J*₁=10.18Hz, *J*₂=1.77Hz, 1H), 6.45(s, 1H), 6.49(dd, *J*₁=3.43Hz, *J*₂=1.80Hz, 1H), 7.12(d, *J*=3.31Hz, 1H), 7.15(dd, *J*₁=10.24Hz, *J*₂=0.78Hz, 1H), 7.58(d, *J*=0.85Hz. 1H). **MASS (ES+)** : 683.1(M+Na)⁺ |
| IB.70 | 1.06(d, 3H, *J*=7.16Hz), 1.19(s, 3H), 1.36-1.42(m, 1H), 1.56(s, 3H), 1.17-1.96(m, 3H), 2.29-2.52(m, 5H), 3.41-3.45(m, 1H), 3.70(d, 1H, *J*=16.78Hz), 3.17(d, *J*=16.93Hz, 1H), 4.46(d, *J*=8.62Hz, 1H), 4.77(d, *J*=15.30Hz, 1H), 4.89(d, *J*=15.31Hz, 1H), 5.33-5.50(m, 1H), 5.99(s, 1H), 6.40(dd, *J*₁=10.16Hz, *J*₂=1.75Hz, 1H), 6.45(s, 1H), 6.50(dd, *J*₁=3.47Hz, *J*₂=1.67Hz, 1H), 7.12(d, *J*=3.41Hz. 1H), 7.17(d, *J*=10.19Hz, 1H). 7.59(s, 1H). **MASS (ES+)** : 597.2[(M+Na)⁺ of IB.4] |
| IB.71 | 0.92(q, *J*=4.52Hz, 3H), 1.06(d, *J*=7.11Hz, 3H), 1.15(dd, 3H), 1.22(s, 3H), 1.22(s, 3H), 1.35-1.41(m, 1H), 1.56(s,3H), 1.46-2.00(m, 5H), 2.29-2.53(m, 5H), 3.22-3.26(dd, 1H), 3.40-3.50(m, 1H), 3.66(d, *J*=*16.58*Hz, 1H), 3.75(d, *J*=16.83Hz. 1H), 4.45(d, *J*=*8.39*Hz. 1H), 4.48-4.53(m, 1H),5.33-5.50(m, 1H), 6.40(dd, *J*₁=10.15(dd, *J*₂=1.71Hz, 1H), 6.45(s, 1H), 6.50(dd, *J*₁=3.39Hz, *J*₂=1.67Hz, 1H), 7.13(d, *J*=3.49Hz, 1H), 7.19(d, *J*=10.13Hz, 1H), 7.58(s, 1H). **MASS (ES+)** : 659.3 |
| IB.72 | 1.03(s, 9H), 1.05(d, *J*=7.35Hz, 3H), 1.20(s, 3H), 1.35-1.40(m, 1H), 1.56(s,3H), 1.72-2.03(m, 3H), 2.20(d, *J*=13.11Hz, 1H), 2.24(d, *J*=13.16Hz, 1H), 2.29-2.54(m, 4H), 3.07(s, 1H), 3.44-3.48(m, 1H), 3.63(d, *J*=16.81Hz, 1H), 3.78(d, *J*=16.84Hz, 1H), 4.44(d, *J*=8.47Hz, 1H), 4.50(d, *J*=15.41Hz, 1H), 4.73(d, *J*=15.35Hz, 1H), 5.33-5.50(m, 1H), 6.40(dd, *J*₁=10.17Hz, *J*₂=1.78Hz, 1H), 6.45(s, 1H). 6.50(dd. *J*₁=3.45Hz, *J*₂=1.78Hz, 1H), 7.14(d, *J*=3.37Hz, 1H), 7.18(dd, *J*₁=9.63Hz, *J*₂=1.07Hz, 1H), 7.59(s, 1H). **MASS (ES+)** : 673.3 |
| IB.73 | 1.06(d, *J*=7.17Hz, 3H), 1.15(t, *J*=7.57Hz, 3H), 1.20(s, 3H), 1.35-1.41(m, 1H), 1.56(s, 3H), 1.74-2.00(m, 3H), 2.28-2.52(m, 6H), 3.10(d, *J*=2.84Hz, 1H), 3.42-3.46(m, 1H), 3.66(d, *J*=16.80Hz, 1H), 3.75(d, *J*=16.82Hz, 1H), 4.45(d, *J*=8.54Hz, 1H), 4.54(d, *J*=15.48Hz, 1H), 4.73(d, *J*=15.38Hz, 1H), 5.33-5.40(m, 1H), 6.40(dd, *J*₁=10.11Hz, *J*₂=1.76Hz, 1H), 6.45(s, 1H), 6.50(dd, *J*₁=3.49Hz, *J*₂=1.65Hz, 1H), 7.13(d, *J*=3.35Hz, 1H), 7.18(dd, *J*₁=10.06Hz, *J*₂=1.04Hz, 1H), 7.59(d, *J*=0.80Hz, 1H). **MASS (ES+)** : 631.3 |
| IB.74 | 0.98(d, *J*=7.10Hz, 3H), 1.16(s, 3H), 1.30-1.36(m, 1H), 1.40(t, *J*=7.12Hz. 3H), 1.54(s, 3H), 1.73-1.99(m, 5H), 2.10-2.47(m, 8H), 2.61(m, 1H), 3.11-3.19(m, 1H), 3.31-3.35(m, 1H), 3.67(d, *J*=16.90Hz, 1H), 3.73(d, *J*=16.78Hz, 1H), 4.36-4.7(m, 3H), 4.79(d, *J*=15.23Hz, 1H). 4.88(d, *J*=15.28Hz, 1H), 5.31-5.48(m, 1H), 6.38(d, *J*₁=10.17Hz, 1H), 6.43(s, 1H), 7.17 (d, *J*=10.05Hz, 1H).**MASS (ES+)** : 585.22 [ (M+Na)⁺ of IB.08] |
| IB.75 | 0.98(d, *J*=7.18Hz, 3H), 1.16(s,3H), 1.18(d, *J*=3.67Hz, 3H), 1.20(d, *J*=3.71Hz 3H), 1.29-1.35(m, 1H), 1.54 (s, 3H), 1.70-1.99(m. 5H), 2.16-2.61(m, 9H), 3.12(d, *J*=3.61Hz, 1H), 3.12-3.20(m, 1H), 3.33-3.38(m, 1H), 3.65(d, *J*=16.64Hz, 1H), 3.72(d, *J*=16.26Hz, 1H), 4.40(d, *J*=*8.93*, 1H), 4.52(d, *J*= 15.32, 1H), 4.73(d, *J*=15.33Hz, 1H), 5.31-5.48(m, 1H), 6.37(dd, *J*₁=10.09Hz, *J*₂=1.77Hz, *J*₂=1.77Hz, 1H), 6.43(s, 1H), 7.16(d, *J*₁=10.10Hz, *J*₂=1.10Hz, 1H), **MASS (ES+)** : 633.25 |
| IB.76 | 1.06(d, *J*=1.17Hz, 3H), 1.22(s, 3H), 1.35-1.41(m, 1H), 1.57(s,3H), 1.67-2.03(m, 1H), 2.29-2.53(m, 4H), 3.31(d, *J*=*3.06*Hz, 1H), 3.45(m, 1H), 3.67(d, *J*=*16.78*Hz, 1H), 3.73(d, *J*=17.10Hz, 1H), 4.45(d, *J*=*7.42*Hz, 1H), 4.48(d, *J*=*15.41*Hz, 1H), 4.73(d, *J*=15.40Hz, 1H), 5.33-5.40(m, 1H), 6.40(dd, *J*₁=10.07Hz, *J*₂=1.79Hz, 1H), 6.45(s, 1H), 6.50(dd, *J*₁=3.47Hz, *J*₂=1.81Hz, 1H), 7.14(d, *J*=3.28Hz, 1H), 7.18(dd, *J*₁=10.07Hz, *J*₂=1.07Hz, 1H), 7.58(d, *J*=0.97Hz, 1H). **MASS (ES+)** : 737.4 |
| IB.77 | 1.06(d, *J*=7.12Hz, 3H), 1.19(s,3H), 1.25-1.43(m, 2H), 1.55(s, 3H), 1.74-1.97(m, 3H), 2.28-2.50(m, 4H), 3.24(q, *J*=10.07Hz, 2H),3,42-3.45(m, 1H), 3.68(d, *J*=16.80Hz, 1H), 3.76(d, *J*=16.82Hz, 1H), 4.45(d, *J*=8.56Hz, 1H), 4.65(d, *J*=15.34Hz, 1H), 4.83(d, *J*=15.37Hz, 1H), 5.33-5.49)(m,1H), 6.41(dd, *J*₁=10.14Hz, *J*₂=1.72Hz, 1H), 6.41(s,1H), 6.51(dd, *J*₁=3.42Hz, *J*₂=1.62Hz, 1H), 7.13(d, *J*=3.42Hz, 1H), 7.16(d, *J*=10.14Hz, 1H), 7.59(s, 1H). **MASS (ES+)** : 707.13(M + Na)⁺⁺ |
| IB.78 | 1.06(d, *J*=7.14Hz, 3H), 1.23(s, 3H), 1.35-1.41(m, 1H), 1.57(s,3H), 1.77-1.99(m, 3H), 2.28-2.53(m, 4H). 3.11(s, 1H), 3.39-3.45(m, 1H), 3.45(s, 3H), 3.67(d, *J*=17.19Hz, 1H), 3.73(d, *J*=16.96Hz, 1H), 4.03(d, *J*=16.48Hz, 1H), 4.10(d, *J*=16.55Hz, 1H), 4.43(d, *J*=8.74Hz, 1H), 4.58(d, *J*=15.30Hz, 1H), 4.58(d, *J*=15.33Hz, 1H), 5.33-5.50(m, 1H), 6.39(dd, *J*₁=10.14Hz, *J*₂=1.71Hz, 1H), 6.45(s. 1H), 6.50(dd, *J*₁=3.41Hz, *J*₂=1.76Hz, 1H), 7.12(d, *J*=3.55Hz 1H). 7.17(dd, *J*₁=10.19Hz *J*₂=0.74Hz, 1H), 7.58(d, *J*=0.71Hz, 1H). **MASS (ES+)**: 647.24 |
| IB.79 | 1.06(d, *J*=7.16Hz, 3H), 1.23(s, 3H), 1.27(t, *J*=7.04Hz, 3H), 1.35-1.41(m, 1H), 1.56(s,3H), 1.73-1.99(m, 3H), 2.28-2.53(m, 4H), 3.17(s, 1H), 3.39-3.42(m, 1H), 3.57(dd, *J*₁=7.03Hz, *J*₂=2.38Hz, 1H), 3.60(dd, *J*₁=4.44Hz, *J*₂=2.44Hz, 1H), 3.65(d, *J*=16.18Hz, 1H), 3.73(d, *J*=16.94Hz, 1H), 4.07(d, *J*=16.64Hz, 1H), 4.14(d, *J*=16.63Hz, 1H), 4,43(d, *J*=8.74Hz, 1H), 4.56(d, *J*=15,41 Hz, 1H), 4.85(d, *J*=15.30Hz, 1H), 5.33-5.50(m, 1H), 6.38(dd, *J*₁=10.11Hz, *J*₂=1.72Hz, 1H), 6.45(s, 1H), 6.50(dd, *J*₁=3.49Hz, *J*₂=1.65Hz, 1H), 7.12(d, *J*=3.37Hz, 1H), 7.17(d, *J*=10.17Hz, 1H), 7.58(d, *J*=1.57Hz, 1H). **MASS (ES+)** : 661.22 |
| IB.80 | 1.05(d, *J*=7.10Hz, 3H), 1.18(s,3H), 1.25-1.42(m, 2H), 1.55(s, 3H), 1.71-2.00(m, 3H), 2.17-2.51(m, 4H), 2.62-2.68(m, 4H), 3.43-3.49(m, 1H) 3.68(d, *J*=16.72Hz, 1H), 3.75(d, *J*=16.78Hz, 1H), 4.45(d, *J*=8.41Hz, 1H), 4.58(d, *J*=15.45Hz, 1H), 4.74(d, *J*=15.45Hz, 1H), 5.33-5.49(m,1H), 6.42(dd, *J*₁=10.09Hz, *J*₂=1.58Hz, 1H),6.45(s,1H), 6.51(dd, *J*₁=3.41Hz, *J*₂=1.69Hz, 1H), 7.14(d, *J*=3.41Hz, 1H), 7.22(d, *J*=10.04Hz, 1H). 7.59(s, 1H). **MASS (ES+)** : 697.16(M+Na)⁺ |
| IB.81 | 1.06(d, 3H, *J*=7.17Hz), 1.18(s, 3H), 1.35-1.41(m, 1H), 1.56(s, 3H), 1.77-1.99(m, 3H), 2.09(s, 3H), 2.28-2.51(m, 4H), 2.91(d, 1H, *J*=2.60Hz), 3.42-3.47(m, 1H), 3.66(d, 1H, *J*=16.74Hz), 3.76(d, *J*=16,78Hz, 1H), 4.46(d, *J*=8.63Hz, 1H), 4.56(d,t, *J*₁=15.46Hz, *J*₂=1.78 Hz, 1H), 4.70(d, *J*₁=15.39Hz, *J*₂=1.65Hz,1H), 5.32-5.50(m, 1H), 639(dd, *J*₁=10.07Hz, *J*₂=1.79Hz, 1H), 6.45(s, 1H), 4.50(dd, *J*₁=3.53Hz, *J*₂=1.68Hz, 1H), 7,14(d, *J*=3.37Hz, 1H), 7.20(dd, *J*₁=10.18Hz, *J*₂= 1.23Hz, 1H), 7.59(d, *J*=0.86Hz, 1H). **MASS (ES+)** : 617.1 |
| IB.82 | 1.06(d, *J*=7.18Hz, 3H), 1.13(s,3H), 1.16(d, *J*=1.26Hz, 3H), 1.18(d, *J*=1.30Hz, 3H), 1.34-1.43(m, 1H), 1.55(s, 3H), 1.77-1.95(m, 3H), 2.28-2.53(m, 4H), 253-2.80(m, 1H), 2.81(m, 1H), 3.43-3.48(m, 1H), 3.54(dd, *J*₁=14.12Hz, *J*₂=7.29Hz, 1H), 3.66(dd, *J*₁=14.07Hz, *J*₂=5.45Hz, 1H), 4.43(d, *J*=8.57Hz, 1H), 4.50(dd, *J*₁=13.32Hz, *J*₂=4.52Hz, 1H), 4.62(dd, *J*₁=13.25Hz, *J*₂=5,69Hz, 1H), 5.32-5.49(m, 1H), 5.65-5:80(m,2H), 6.39(dd, *J*₁=10.15Hz, *J*₂=1.79Hz, 1H), 6.45(s, 1H), 6.50(dd, *J*₁=3.46Hz, *J*₂=1.64Hz, 1H), 7.11(d, *J*=3.43Hz, 1H), 7.16(dd, *J*₁=10.03Hz, *J*₂=0.93Hz, 1H), 7.58(d, *J*=0.86Hz, 1H). **MASS (ES+)** : 647.1 |
| ID.83 | 1.06(d, *J*=7.17Hz, 3H), 1.12(s, 3H), 1.16(d, *J*=6.98Hz, 6H), 1.34-1.40(m, 1H), 1.55(s, 3H), 1.77-2.06(m, 3H), 2.28-2.59(m, 5H), 3.42-3.47(m, 1H), 3.66(d, *J*=7.82Hz, 2H), 4.44(d, *J*=9.08Hz, 1H), 4.75-4.79(m, 2H), 5.34-5.47(m, 1H), 5.58-5.72(m, 2H), 6.39(dd, *J*₁=10.16Hz, *J*₂=1.79Hz, 1H), 6.45(s, 1H), 6.49(dd, *J*₁=3.41Hz, *J*=1.82Hz, 1H), 7.12(d, *J*=3.19Hz, 1H), 7.16(dd, *J*₁=10.08Hz, *J*₂=1.06Hz, 1H), 7.58(br-s, 1H). **MASS (ES+)** : 647.1 |
| IB.84 | 0.98(d, *J*=7.15Hz, 3H), 1.17(s,3H), 1.22(s, 9H), 1.27-1.38(m, 1H), 1.54(s,3H), 1.70-1.99(m, 5H), 2.17-2.50(m, 8H), 3.12-3.18(m, 1H), 3.21(d, *J*=3.24Hz, 1H), 3.35-3.38(m, 1H), 3.65(d, *J*=16.86Hz, 1H), 3.65(d, *J*=16.86Hz, 1H), 3.72(d, *J*=7.02Hz. 1H), 4.39(d, *J*=8.61Hz, 1H), 4.51(d, *J*=15.31Hz, 1H), 4.73(d, *J*=15.33Hz, 1H), 5.31-5.48(m,1H), 6.37(dd, *J*₁=10.11Hz, *J*₂=1.61Hz, 1H=. 6.43(s, 1H). 7.15(d, *J*=10.16Hz, 1H). **MASS (ES+)**: 647.1 |
| IB.85 | 0.86(t, *J*=7.46Hz, 3H), 1.05(d, *J*=7.15Hz, 3H), 1.17(s, 6H), 1.23(s, 3H), 1.35-1.41(m, 1H), 1.56(s,3H), 1.73-2.01(m, 3H), 2.29-2.56(m, 4H), 3.36(br-s, 1H), 3.43-3.40(m, 1H), 4.40(d, 1H), 4.48(d, *J*=15.37Hz, 1H), 4.76(d, *J*=15.31Hz, 1H), 5.34-5.50(m, 1H), 6.40(dd, *J*₁=10.17Hz, *J*₂=1.72Hz, 1H), 6.45(s, 1H), 6.50(dd, *J*₁=3.44Hz, *J*₂=1.67Hz, 1H), 7.13(d, *J*=3.37Hz, 1H), 7.17(dd, *J*₁=10.07Hz, *J*₂=0.60Hz, 1H), 7.58(s, 1H). **MASS (ES+)** : 673.26 |
| IB.86 | 0.96(s, 3H), 1.06(d, *J*=5.33Hz, 3H), 1.07(s, 3H), 1.13(s, 3H), 1.25(s, 3H), 1.35-1.41(m, 1H), 1.56(s,3H), 1.69-2.13(m, 6H), 2.28-2.57(m, 5H), 3.10(s, 1H), 3.39-3.42(m, 1H), 3.66(d, *J*=16.62Hz, 1H), 3.73(d, *J*=17.10Hz, 1H), 4.53(d, *J*=*8.29*Hz. 1H), 4.64(d, *J*=15.20Hz, 1H), 4.92(d, *J*=*15.13*Hz, 1H), 5.33-5.50(m, 1H), 6.41(dd, *J*₁=10.07Hz, *J*₂=1.78Hz, 1H), 6.44(s, 1H), 6.49(dd, *J*₁=3.5Hz, *J*₂=1.70Hz, 1H), 7.12(d, *J*=3.41Hz, 1H), 7.29(dd, *J*₁=10.04Hz, *J*₂=0.99Hz, 1H), 7.58(d, *J*=0.95Hz, 1H). **MASS (ES+)** : 777.18(M+Na)⁺ |
| IB.87 | 1.06(d, *J*=7.15Hz, 3H), 1.23(s, 3H), 1.33(s, 9H), 1.33-1.38(m, 1H), 1.56(s, 3H), 1.77-2.08(m, 3H), 2.29-2.53(m, 4H), 3.47(m. 1H), 3.61(d, *J*=16.75Hz, 1H), 3.80(d, *J*=16.80Hz, 1H), 4.38(d, *J*=16.03Hz, 1H), 4.44(d, *J*=8.47Hz 1H), 4.72(d, *J*=15.70Hz, 1H), 4.78(s, 1H), 5.33-5.49(m, 1H), 6.39(dd, *J*₁=10.09Hz, *J*₂=1.72Hz, 1H), 6.45(s, 1H), 6.50(dd, *J*₁=3.45Hz, *J*₂=1.62Hz, 1H), 7.14(m, 2H), 7.59(d, *J*=0.74Hz, 1H), **MASS (ES+)** : 696.20(M+Na)⁺ |
| IB.88 | 1.06(d, *J*=7.17Hz, 3H), 1.18(s,3H), 1.35-1.41(m, 2H), 1.55(s, 3H), 1.77-1.96(m, 3H), 2.17-2.47(m, 4H), 3.42-3.46(m, 1H), 3.66(d, *J*=16.77Hz, 1H), 3.76(d, *J*=16.80Hz, 1H), 4.15(q, *J*=7.15Hz, 2H), 4.45(d, *J*=8.67Hz, 1H), 4.59(d, *J*=15.47Hz, 1H), 4.73(d, *J*=15.36Hz, 1H), 5.32-5.49(m,1H), 6.40(dd, *J*₁=10.09Hz, *J*₂=1.75Hz, 1H), 6.41(s,1H), 6.51(dd, *J*₁=3.46Hz, *J*₂=1.81 Hz, 1H), 7.13(d, *J*=3.3Hz. 1H). 7.20(dd, *J*₁=10.14Hz *J*₂=1.08Hz, 1H), 7.59(s, 1H). **MASS (ES+)** : 725.18(M + Na)⁺ |
| IB.89 | 0.78(d, *J*=6.98Hz, 3H), 0.88(d, J=7.03Hz, 3H), (d, *J*=6.41Hz, 3H), 1.02-1.10(m, 5H), 1.20(s,3H), 1.30-1.49(m, 3H), 1.55(s, 3H), 1.69-2.00(m, 7H), 2.28-2.51(m, 4H), 2.59(s, 1H), 3.42-3.46(m, 1H), 3.69(d, *J*=16.56Hz, 1H),3.76(d, *J*=16.91Hz, 1H), 4.42(d, *J*=8.58Hz, 1H) 4.59(td, *J*₁=10.91Hz, *J*₂=4.39Hz, 1H), 4.62(d, *J*=15.52Hz, 1H), 4.72(d, *J*=15.41Hz, 1H), 5.32-5.49(m, 1H), 6.40(dd, *J*₁=10.18Hz, *J*₂=1.81Hz 1H), 6.45(s,1H), 6.50(dd, *J*₁=3.47Hz, *J*₂=1.78Hz, 1H), 7.12-7.14(m, 2H), 7.59(s,1H). **MASS (ES+)** : 779.32(M - Na)⁺ |
| IB.90 | 1.06(d, *J*=7.14Hz, 3H) 1.17(s, 3H), 1.34-1.40(m, 1H), 1.55(s, 3H), 1.76-1.97(m, 3H), 2.06(s, 3H), 2.28-2.54(m, 4H), 3.41-0.46(m, 1H), 3.67-3.75(m, 2H), 4.00(dd, *J*₁=18.22Hz, *J*₂=5.46Hz, 1H), 4.09(dd, *J*₁=18.22Hz, *J*₂=5.84Hz, 1H), 4.41(d, *J*=5.81Hz, 1H), 4.71(s, 2H), 5,32-5.49(m, 1H), 6.11(s, 1H), 6.39(dd, *J*₁=10.16Hz, *J*₂=1.79Hz, 1H), 6.45(s, 1H), 6.50(dd, *J*₁=3.45Hz, *J*₂=1.77Hz, 1H), 7.12(d, *J*=3.51Hz, 1H), 7.19(dd, *J*₁=10.09Hz, *J*₂=0.96Hz, 1H), 7.58(d, *J*=0.90Hz, 1H). **MASS (ES+)** : 696.13(M+Na)⁺ |
| IB.91 | 0.96(d, *J*=6.72Hz, 3H), 1.05(d, *J*=7.15Hz, 3H), 1.19(s, 3H), 1.35-1.41(m, 1H), 1.55(s, 3H), 1.78-2.02(m, 4H), 2.30-2.63(m, 5H), 3.41-3.45(m, 1H), 3.70(d, *J*=16.81Hz, 1H), 3.76(d, *J*=16.76Hz, 1H), 3.93(d, *J*=*6.80*Hz, 2H), 4.44-4.46(m, 1H), 4.66(d, *J*=15.53Hz, 1H), 4.71(d, *J*=15.50Hz, 1H), 5.31-5.49(m, 1H), 6.40(dd, *J*₁=10.13Hz, *J*₂=1.67Hz, 1H), 6.46(s, 1H), 6.94(d, *J*=4.00Hz, 1H), 7.15(d, *J*=10.22Hz, 1H), 7.53(d, *J*=4.01Hz, 1H). **MASS (ES+)**: 747.0(M+Na)⁺ |
| IB.92 | 1.05(d, *J*=7.14Hz, 3H), 1.18(d, *J*=6.98Hz, 3H), 1.19(d, *J*=7.13Hz, 3H), 1.22(s, 3H), 1.35-1.41(m, 1H), 1.56(s, 3H), 1.78-2.00(m, 3H), 2.28-2.63(m, 5H), 3.23(d, *J*=3.19Hz, 1H), 3.40-3.45(m, 1H), 3.67-3.75(m, 2H), 3.45-3.47(m.1H), 3.49-3.77(m, 2H), 5.33-5.50(m, 1H), 6.41(dd, *J*₁=10.15Hz, *J*₂=1.79Hz, 1H), 6.46(s, 1H), 6.93(d, *J*=4.0Hz, 1H), 7.18(dd, *J*₁=10.14Hz, *J*₂=10.14Hz, 1H), 7.53(d, *J*=4.08Hz, 1H). **MASS (ES+)** : 717.12(M+Na)⁺ |
| IB.93 | 1.06(d, *J*=7.16Hz, 3H), 1.17(s,3H), 1.33(t, *J*=7.12Hz, 3H), 1.36-1.40(m, 1H), 1.55(s, 3H), 1.71-1.97(m, 3H), 2.28-2.48(m, 4H), 2.71(s, 1H), 3.42-3.48(m, 1H), 3.68(d, *J*=16.79Hz, 1H), 3.77(d, *J*=16.60Hz, 1H), 4.25-4.30(m, 2H), 4.44(m, 1H), 4.68(d, *J*=15.47Hz. 1H), 4.81(d, *J*=15.45Hz, 1H), 5.32-5.49(m, 1H), 6.21(d, *J*=12.01Hz, 1H), 6.32(d, *J*=11.98Hz, 1H), 6.40(dd, *J*₁=10.10Hz, *J*₂=1.75 Hz, 1H), 6.45(s, 1H), 6.5(dd, *J*₁=3.47Hz, *J*₂=1.75Hz, 1H), 7.13(d, *J*=3.42Hz, 1H), 7.19(dd, *J*₁=10.25Hz, *J*₂=0.81Hz, 1H), 7.59(d, *J*=0.88Hz, 1H). **MASS (ES+)** 723.22(M+Na)⁺ |
| IB.94 | 1.06(d, *J*=7.17Hz, 3H), 1.18 (s, 3H), 1.35-1.41(m, 1H), 1.56(s, 3H), 1.74-1.99(m, 3H), 2.28-2.47(m, 4H), 2.63-2.69(m, 4H), 2.93(s, 1H), 3.43-3.47(m, 1H), 3.66(d, *J*=16.86 Hz, 1H), 3.70(s, 3H), 3.76(d, *J*=17.21Hz, 1H), 4.45(d, *J*=8.60Hz, 1H), 4.59(d, *J*=15.38Hz, 1H), 4.68(d, *J*=15.45Hz, 1H), 5.33-5.50(m, 1H), 6.41(dd, *J*₁=10.15Hz, *J*₂=1.76Hz, 1H), 6.46(s, 1H), 6.51(dd, *J*₁=3.45Hz, *J*₂=3.45Hz, 1H), 7.13(d, *J*=3.43Hr, 1H), 7.19(d, *J*=9.97Hz, 1H), 7.59(d, *J*=0.73Hz, 1H), **MASS (ES+)** : 711.19(M+Na)⁺ |
| IB.95 | 1.06(d, *J*=7.07Hz, 3H), 1.18(s, 3H), 1.23(d, *J*=6.20Hz, 3H), 1246(d, *J*=6.25Hz, 3H), 1.35-1.41(m, 1H), 156(s, 3H), 1.74-2.00(m, 3H), 2.28-2.51(m, 4H), 2.58-2.67(m, 4H), 3.00(s, 1H), 3.43-3.47(m, 1H), 3.65(d, *J*=16.97Hz 1H), 3.76(d, *J*=17.57Hz. 1H), 4.46(d, *J*=8.05Hz, 1H), 4.59(d, *J*=15.43Hz, 1H), 4.72(d, *J*=15.40Hz, 1H), 4.96-5.06(m, 1H), 5.33-5.49(m, 1H), 6.41(dd, *J*₁=10.20Hz, *J*₂=1.24Hz, 1H), 6.46(s, 1H), 6.51 (dd, *J*₁=3.13Hz, *J*₂=1.24Hz, 1H), 7.13(d, *J*=3.33Hz, 1H), 7.19(d, *J*=10.11Hz, 1H), 7.59(s, 1H), **MASS (ES+)** : 739.31(M+Na)⁺ |
| IB.96 | 0.92(d, *J*=6.64Hz, 6H), 1.06(d, *J*=6.64Hz, 3H), 1.18(s, 3H), 1.36-1.41(m, 1H), 1.56(5. 3H), 1.74-2.06(m, 4H), 2.29-2.51(m, 4H), 2.66-2.69(m, 4H); 2.99(s, 1H), 3.43-3.45(m, 1H), 3.66(d, *J*=16.76Hz, 1H), 3.77(d, *J*=16.64Hz, 1H 3.88(d, *J*=6.57Hz, 2H), 4.46(d, J=6.66Hz. 1H), 4.59(d, *J*=15.40Hz, 1H), 4.13(d, *J*=15.33Hz, 1H), 5.33-5.49(m,1H), 6.41(d, *J*₁=10.11Hz, 1H), 6.45(s, 1H), 6.50(dd, *J*₁=2.62Hz, *J*₂=1.21Hz, 1H), 7.13(d, *J*=3.43Hz, 1H), 7.19(d, *J*=10.11Hz, 1H), 7.59(s, 1H). **MASS (ES+)** : 753.35(M+Na)⁺ |
| IB.97 | 0.92(1, *J*=7.32Hz, 3H), 1.05(d, *J*=7.15Hz, 3H), 1.18(s,3H), 1.20-1.38(m, 5H), 1.40-1.62(m,4H), 1.77-2.00(m, 3H), 2.25-2.50(m, 4H), 2.61-2.69(m, 4H), 2.97(s, 1H), 3.42-3.47(m, 1H), 3.65(d, *J*=16.71 Hz, 1H), 3.76(d, *J*=16.87Hz, 1H), 4.10(1, *J*=6.71 Hz, 2H), 4.45(d, *J*=8.59Hz. 1H), 4.59(d, *J*=15.45Hz, 1H), 4.72(d, *J*=15.43Hz, 1H), 5.32-5.49(m, 1H), 6.39(dd, *J*₁=10.16Hz, *J*₂=1.78Hz, 1H), 6.46(s,1H), 6.50(dd. *J*₁=3.74Hz, *J*₂=1.47Hz, 1H), 7.13(d, *J*=3.44Hz, 1H), 7.19(dd, *J*₁=10.10Hz, J₂=0.91Hz, 1H), 7.59(d, *J*=1.08Hz, 1H). **MASS (ES+)** : 753.18(M+Na)⁺ |
| IB.98 | 1.06(d, *J*=7.12Hz, 3H), 1.21(s, 3H), 1.31(s, 6H), 1.35-1.41(m, 1H), 1.56(s, 3H), 1.77-2.01(m, 3H), 2.29-2.53(m, 4H), 2.91(d, *J=2.26*Hz, 1H), 3.43-3.47(m, 1H), 3.58-3.64(m, 2H), 3.66(d, *J*=17.24Hz, 1H), 3.76(d, *J*=16.76Hz, 1H), 4.45-4.47(m, 1H), 4.57(d, *J=15.36*Hz, 1H). 4.78(d. *J*=15.36Hz, 1H), 5.34-5.50(m, 1H), 6.40(dd, *J*₁=10.15Hz, *J*₂=1.46Hz, 1H), 6.45(s, 1H), 6.50(dd, *J*₁=3.28Hz, *J*₂=1.48Hz, 1H), 7.14(d, *J*=3.49Hz, 1H), 7.18(dd, *J*=10.12Hz, 1H), 7.59(s, 1H). **MASS (ES+)** : 715.02(M+Na)⁻ |
| IB.99 | 0.94(d, *J*=6.70Hz, 6H), 1.06(d, *J*=7.12Hz, 3H), 1.17(s, 3H), 1.25-1.40(m, 1H), 1.55(s, 3H), 1.73-1.99(m, 4H), 2.28-2.46(m, 4H), 2.73(s, 1H) 3.43-3.47(m, 1H), 3.67(d, *J*=16.91Hz, 1H), 3.78(d, *J*=17.01Hz, 1H), 3.99(d, *J*=6.69Hz, 2H ), 4.44(d, *J*=8.42Hz, 1H), 4.67(d, *J*=15.51Hz, 1H), 4.81(d, *J*=15.46Hz, 1H), 5.32-5.49(m, 1H), 6.22(d, *J*=12.05Hz, 1H), 6.34(d, *J*=12.03Hz, 1H), 6.39(dd, *J*₁=6.39(dd, *J*₁=10.13Hz, J₂=1.65Hz, 1H), 6.45(s, 1H), 6.50(dd, *J*₁=3.57Hz, *J*₂=1.85Hz, 1H), 7.13(d, *J*=3.44Hz, 1H), 7.19(d, *J*=10.20Hz, 1H), 7.59(d, *J*=0.67Hz, 1H), **MASS (ES+)** : 751.16(M+Na)⁺ |
| IB.100 | 1.06(d, *J*=7.15Hz, 3H), 1.16(s,3H), 1.25-1.41(m, 1H), 1.56(s, 3H), 1.77-1.96(m, 3H), 1.77-1.96(m, 3H), 2.28-2.49(m, 4H), 2,71(s, 1H), 3.41-3.45(m, 1H), 3.67(d, *J*=16.72Hz, 1H), 3.77(d, *J*=16.87Hz, 1H), 3.82(s, 3H), 4.46(m, 1H), 4.74(d, *J*=15.44Hz, 1H), 4.80(d, *J*=15.44Hz, 1H), 5.32-5.49(m 1H).6.42(dd, *J*₁=10.16Hz, *J*₂=1.79Hz, 1H), 6.46(s, 1H), 6.50(dd, *J*₁=3.45Hz, *J*₁=1.76Hz. 1H), 6.86(d, *J*=15.85Hz, 1H), 6.90(d, *J*=15,82Hz. 1H), 7.13(d, *J*=3.51Hz, 1H), 7.20(dd, *J*₁=10.00Hz, *J*₂=1.00Hz, 1H), 7.59(d, *J*=0.73Hz, 1H). **MASS (ES+)** : 709.11(M+Na)⁺ |
| IB.101 | 0.96(d, *J*=6.59Hz, 6H), 1.05(d, *J*=7.115Hz, 3H), 1.20(s, 3H), 1.35-1.41(m, 1H), 1.56(s, 3H), 1.77-2.01(m, 3H), 2.052.12(m, 1H), 2.21-2.52(m, 6H), 3.07(d, *J*=2.84Hz, 1H), 3.43-3.47(m, 1H), 3.65(d, *J*=16.86Hz, 1H), 3.77(d, *J*=16.79Hz, 1H), 4.45(d, *J*=8.63Hz, 1H), 4.52(d, *J*=*15.42*Hz, 1H), 4.73(d, *J*=15.34Hz, 1H), 5.33-5.48(m, 1H), 6.40(dd, *J*₁=10.12Hz, *J*₂=1.71 Hz, 1H), 6.45(s, 1H), 6,50(dd, *J*₁=3.43Hz, *J*₂=1.61Hz, 1H), 7.13(d, *J*=3.43Hz, 1H), 7.19(dd, *J*=10.19Hz, 1H), 7.58(d, *J*=0.85Hz, 1H). **MASS (ES+)** : 681.0(M+Na)⁻ |
| IB.102 | 0.90(q, *J*=7.40Hz, 3H), 1.05(d, *J*=7.14Hz, 3H), 1.23(s, 3H), 1.35-1.41(m, 1H), 1.50-1.65(m, 4H), 1.57(s, 3H), 1.77-2.01(m, 3H), 2.21-2.55(m, 5H), 3.31(d, *J*=2.47Hz, 1H), 3.44-3.47(m, 1H), 3.64(d, *J*=16.73Hz, 1H), 3.76(d, *J*=16.70Hz, 1H), 4.43-4.46(m, 1H), 4.49(d.: *J*=*15.39*Hz, 1H), 4.78(d, *J*=15.38Hz, 1H), 5.34=5.50(m, 1H), 6.40(dd, *J*₁=10.08Hz, *J*₂=1.62Hz. 1H), 6.45(s, 1H), 6.50(dd, *J*₁=3.37Hz, *J*₂= 1.5Hz, 1H), 7.13(d, *J*=3.45Hz, 1H), 7.19(dd, *J*=10.16Hz, 1H), 7.59(s, 1H). **MASS (ES+)** : 695.0(M+Na)⁺ |
| IB.103 | 1.05(d, *J*=7.18Hz, 3H), 1.23(s, 3H), 1.34-1.40(m, 1H), 1.56(s, 3H), 1.77-2.08(m, 3H), 2.17-2.53(m, 4H), 2.92(s, 6H), 3.44-3.48(m, 1H), 3.64(d, *J*=16.62Hz, 1H), 3.76(d, *J*=16.16Hz, 1H), 4.01(d, *J*=3.45Hz, 1H), 4.41-4.43(m, 1H), 4.49(d, *J*=15.61Hz, 1H), 4.76(d, *J*=15.59Hz. 1H), 5.33-5.50(m, 1H), 6.39(dd, *J*₁=10.11Hz, *J*₂=1.75Hz, 1H), 6.45(s, 1H), 6.50(dd, *J*₁=3.47Hz, *J*₂=1.74Hz, 1H), 7.14(d, *J*=3.40Hz), 1H), 7.18(dd. *J*₁=10.16Hz, *J*₂=0.91Hz, 1H), 7.59(s, 1H). **MASS (ES+)** : 668.0(M+Na)⁺ |
| IB. 104 | 1.06(d, *J*=7.12Hz, 3H), 1.21(s, 3H), 1.35-1.41 (m, 1H), 1,56(s, 3H), 1.79(s, 3H), 1.80(s, 3H), 1.80-1.98(m, 3H), 2.28-2.52(m, 4H), 2.70(d, *J*=2.16Hz, 1H), 3.42-3.46(m, 1H), 3.67-3.77(m, 2H), 3.44-3.46(m, 1H), 4.62-4.85(m, 2H), 5.33-5.49(m, 1H), 6.39(dd, *J*₁=10.15Hz, *J*₂=1.70Hz, 1H), 6.45(s, 1H), 6.50(dd, *J*₁=3.44Hz, *J*₂=1.71Hz, 1H), 7.12(dd, *J*=3.42Hz, 1H), 7.16(dd, *J*₁=10.07Hz, *J*₂=0.93 Hz, 1H), 7.58(s, 1H). **MASS (ES+)** : 679.0 |
| IB.105 | 0.90(d, *J*=6.31Hz, 3H), 0.91(d, *J*=6.24Hz, 3H), 1.06(d, *J*=7.14Hz, 3H), 1.20(s, 3H), 1.35-1.41(m, 1H), 1.47-1.59(m, 3H), 1.56(s, 3H), 1.77-2.00(m, 3H), 2.29-2.52(m, 4H), 2.35(1. *J*=7.72Hz, 2H), 3.10(d, *J=2*.65Hz, 1H), 3.43-3.46(m, 1H), 3.65(d, *J*=*16.80*Hz, 1H), 3.77(d, *J*=16.81Hz, 1H), 4.44-4.46(m, 1H), 4.53(d, *J*=*15.38*Hz, 1H), 4.71(d, *J*=15.33Hz, 1H), 5.33-5.50(m, 1H), 6.39(dd, *J*₁=10.16Hz, J₂=1.74Hz, 1H), 6.45(s, 1H), 6.50(dd, *J*₁=3.43Hz, *J*₂=1.77Hz, 1H), 7.13(d, *J*=3.60Hz, 1H), 7.18(dd, *J*₁=0.72Hz, 1H), 7.59(s, 1H), **MASS (ES+) :** 673.0 |
| IB.106 | 1.06(d, *J*=7.18Hz, 3H), 1.17(s, 3H), 1.35-1.41(m, 1H), 1.55(s, 3H), 1.74-1.97(m, 3H), 2.28-2.48(m.4H), 2.65(s. 1H), 2.69-2.77(m, 4H), 3.43-3.47(m, 1H), 3.67(d, *J*=16.80Hz, 1H), 3.76(d, *J*=16.76Hz, 1H), 4.45(d, *J*=8.68Hz, 1H), 4.61(d, *J*=15.48Hz, 1H), 4.74(d, *J*=15.38Hz, 1H), 5.32-5.49(m, 1H), 5.71(d, *J*=50.60Hz, 2H), 6.41(dd, *J*₁=10.18Hz, *J₂*=1.81Hz, 1H), 6.45(s, 1H), 6.51(dd. *J₁*=3.49Hz,*J*=1.64Hz, 1H), 7.13(d, *J*=3.29Hz, 1H), 7.18(dd, *J*₁=10.16Hz, *J*₂=0.91Hz, 1H), 7.59(d, *J*=0.79Hz, 1H), **MASS (ES+)** : 729 :28(M+Na)⁺ |
| IB.106 | 1.06(d, *J*=7.17Hz, 3H), 1.17(s, 3H), 1.25-1.41(m, 1H), 1.54(s, 3H), 1.77-1.98(m, 3H), 2.28-2.49(m, 5H), 3.42-3.47(m, 1H), 3.69(d, *J*=16.78Hz, 1H), 3.78(d, *J*=16.84Hz, 1H), 4.44(d, *J*=8.74Hz, 1H), 4.66(d, *J*=17.09Hz, 1H), 4.84(d, *J*=17.31Hz, 1H), 5.32-5.49(m, 1H), 5.78(dd, *J*₁=50.72Hz, *J*₂=1.89Hz, 1H), 5.82(dd, *J*₁=50.31Hz, *J*₂=1.91Hz, 1H), 6.34(d, *J*=12.00Hz, 1H ), 6.38(d, *J*=12.14Hz, 1H ), 6.39(dd, *J₁*=3.46Hz, *J₂*=1.60Hz, 1H), 6.45(s, 1H), 6.51(dd, *J*₁=3.46Hz, *J*₂=1.60Hz, 1H), 7.13(d, *J*=3.42Hz, 1H ), 7.17(d, *J*=10.16Hz, 1H), 7.59(d, *J*=0.84Hz, 1H), **MASS (ES+)** : 727.26(M+Na)⁺ |
| IB.108 | 1.06(d, *J*=7.15Hz, 3H), 1.19(s, 3H), 1.25-1.41(m, 1H), 1.55(s, 3H), 1.74-1.97(m, 3H), 2.29-2.50(m, 4H), 2.81(s, 1H ), 3.43(m, 3H), 3.68(d, *J*=17.09Hz, 1H), 3.75(d, *J*=16.15Hz, 1H ), 3.77(s, 3H), 4.46(d, *J*=8.71 Hz, 1H), 4.62(d, *J*=15.45Hz, 1H), 4.80(d, *J*=15.32Hz, 1H), 5.33-5.50(m, 1H), 6.41(dd, *J*₁=10.09Hz, *J*₂-1.69Hz, 1H), 6.45(s, 1H), 6.51(dd, *J*₁=3.43Hz, *J*₂=1.69Hz, 1H), 7.13(d, *J*=3.39Hz, 1H), 7.18(d, *J*=10.15Hz, 1H), 7.59(d, *J*=0.88Hz, 1H), **MASS (ES+) :** 697.29(M+Na)⁺ |
| IB.109 | 1.06(d, *J*=7.15Hz, 3H), 1.21(s, 3H), 1.37-1.41(m, 1H), 1.44(s, 3H), 1.45(s, 3H), 1.56(s, 3H), 1.77-1.97(m, 3H), 2.29-2.52(m, 4H), 3.05(d, *J*=2.12Hz, 1H), 3.42-3.47(m, 3H). 2.71-3.75(m, 5H), 4.46(d, *J*=8.69Hz, 1H), 4.62(d, *J*=15.42Hz, 1H), 4.74(d, *J*=15.43Hz, 1H), 5.33-5.50(m, 1H), 6.41(dd, *J*₁=10.15Hz, *J*₂=1.76Hz, 1H), 6.45(s, 1H), 6.50(dd, *J*₁=3.48Hz, *J*₂=1.64Hz, 1H), 7.13(d, *J*=3.41Hz, 1H), 7.18(dd, *J*₁=10.12Hz, *J*₂=0.95Hz, 1H), 7.59(d, *J*=0.88Hz, 1H), **MASS (ES+) :** 725.30(M+Na)⁺ |
| IB.110 | 1.06(d, *J*=7.15Hz, 3H), 1.18(s, 1H), 1.35-1.42(m, 1H), 1.15(s, 3H). 1.47(s, 3H), 1.55(s, 3H), 1.59-1.98(m, 3H), 2.17-2.511(m, 4H), 3.88(m, 1H), 2.97(s, 1H), 3.39-3.45(m, 1H), 3.72(s, 2 H), 4.43-4.45(m, 1H), 4.64-4.81(m, 2H), 5.33-5.49(m, 1H), 6.39(dd, *J*₁=10.16Hz, *J*₂=1.75Hz, 1H), 6.45(s, 1H), 6.50(dd, *J*₁=3.45Hz, *J*₂=1.76Hz, 1H), 7.13(d, *J*=3.44Hz, 1H), 7.17(dd, *J*₁=10.12Hz, *J*₂=0.76Hz, 1H), 7.58(d, *J*=0.90Hz, 1H), **MASS (ES+) :** 683.10(M+Na)⁺ |
| IB.111 | 1.06(d. *J*=7.13Hz, 3H), 1.23(s, 3H), 1.36-1.41(m, 1H), 1.56(d, *J*=21.29Hz, 3H), 1.62(d, *J*=21.37Hz, 3H), 1.77-1-99(m, 3H), 2.28-2.53(m, 4H), 2.95(s, 1H), 3.39-3.43(m, 1H), 3.67(d, *J*=16.95Hz, 1H), 3.74(d, *J*=16.49Hz, 1H), 4.44(d, *J*=8.03Hz, 1H), 4.60(d, *J*=15.23Hz, 1H), 4.86(d, *J*=15.34Hz, 1H), 5.33-5.50(m, 1H), 6.39(dd, *J*₁=10.11Hz, *J*₂=1.63Hz, 1H), 6.45(s, 1H), 6.50(dd, *J*₁=3.35Hz, *J*₂=1.5Hz, 1H), 7.12(d, *J*=3.42Hz, 1H), 7.19(d, *J*=10.13Hz, 1H), 7.58(s, 1H), **MASS (ES+) :** 685.10(M+Na)⁺ |
| IB.112 | 1.06(d, *J*=7.15Hz, 3H), 1.17(s, 3H), 1.35-1.40(m, 1H), 1.55(s, 3H), 1.76-1.97(m, 3H), 2.28-2.47(m, 4H), 2.71(s, 1H), 3.42-3.47(m, 1H), 3.69(d, *J*=16.76Hz, 1H), 3.78(d, *J*=16.97Hz, 1H), 3.82(S, 3H), 4.44(d, *J*=8.22Hz, 1H), 4.69(d, *J*=15.50Hz, 1H), 4.82(d, *J*=15.54Hz, 1H), 5.30-5.60(m, 1H), 6.23(d, *J*=11.91Hz, 1H), 6.33(d, *J*=12.06Hz, 1H), 6.40(dd, *J*₁=10.14Hz, *J*₂=1.58Hz, 1H), 6.45(s.1 H), 6.50(dd, *J*₁=3.3 1Hz, *J*₂=1.51Hz, 1H), 7.13(d, *J*=3.47Hz, 1H), 7.19(d, *J*=10.14Hz, 1H), 7.59(s, 1H), **MASS (ES+) :** 687.73 |
| IB.113 | 1.06(d, *J*=7.12Hz, 3H), 1.16(s, 3H), 1.32(t, 3H), 1.35-1.42(m, 2H), 1.56(s, 3H), 1.72-1.97(m, 3H), 2.28-2.48(m, 4H), 2.86(s, 1H), 3.42-3.45(m, 1H), 3.68(d, *J*=16.77Hz, 1H), 3.77(d, *J*=16.62Hz, 1H), 4.26(q, *J*=7.07Hz, 2H), 4.46(d, *J*=8.36Hz, 1H), 4.75(d, *J*=15.39Hz, 1H), 4.80(d, *J*=15.39Hz, 1H), 5.30-5.49(m, 1H), 6.41(dd, *J*₁=10.14Hz, *J*₂=1.76Hz, 1H), 6.46(s, 1H), 6.50(dd, *J*₁=3.45Hz, *J*₂=1.76Hz, 1H), 6.85(d, *J*=15.75Hz, 1H), 6.90(d, *J*=15.90Hz, 1H), 7.13(d, *J*=3.74Hz, 1H), 7.20(dd, *J*₁=10.19Hz, *J*₂=0.84Hz, 1H), 7.59(d, *J*=087Hz, 1H), **MASS (ES+) :** 701.0 |
| IB.114 | 0.96(d, *J*=6.72Hz, 6H). 1.06(d, *J*=7.13Hz, 3H), 1.17(s, 3H), 1.25-1.41(m, 1H), 1.56(s, 3H), 1.74-2.00(m, 4H), 2.28-2.49(m, 4H), 2.86(s, 1H) 3.42-3.45(m, 1H), 3.68(d, *J*=16.76Hz, 1H), 3.78(d, *J*=16.83Hz, 1H), 4.00(d, *J*=6.70Hz, 2H), 4.46(d, *J*=8.56Hz, 1H), 4.75(d, *J*=15.47Hz, 1H), 4.80(d, *J*=15.38Hz, 1H), 5.30-5.49(m, 1H), 6.41(dd, *J*₁=10.15Hz, *J*₂=1.80Hz, 1H), 6.46(s, 1H), 6.50(dd, *J*₁=3.50Hz, *J*₂=1.73Hz, 1H), 6.85(d, *J*=15.79Hz, 1H), 6.92(d, *J*=15.93Hz, 1H), 7.13(d, *J*=3.32Hz, 1H), 7.20(dd, *J*₁=10.12Hz, *J*₂=0.96Hz, 1H), 7.59(d, *J*=0.90Hz, 1H), **MASS (ES+)** : 729.1 |
| IB.115 | 0.95(t, *J*=7.37Hz, 6H), 1.06(d, *J*=7.10Hz, 3H), 1.17(s, 3H), 1.36-1.45(m, 3H), 1.56(s, 3H), 1.67-1.97(m, 7H), 2.28-2.51(m, 4H), 2.82(s, 1H), 3.42-3.45(m, 1H), 3.72(d, *J*=16.78Hz, 1H), 3.77(d, *J*=16.74Hz, 1H), 4.22(t, *J*=6.67Hz, 2H), 4.47(d, *J*=8.32Hz, 1H), 4.75(d, *J*=15.54Hz, 1H), 4.79(d, *J*=15.52Hz, 1H), 5.33-5.49(m, 1H), 6.41(dd, *J*₁=10.13Hz, *J*₂=1.66Hz, 1H), 6.46(s, 1H), 6.50(dd, *J*₁=3.33Hz, *J*₂=1.52Hz, 1H), 6.84(d, *J*=15.81Hz, 1H), 6.90(d, *J*=15.77Hz, 1H), 7.13(d, *J*=3.40Hz, 1H), 7.20(d, *J*=10.18Hz, 1H), 7.59(s, 1H), **MASS (ES+) :** 729.1 |
| IB.116 | 1.06(d, *J*=7.16Hz, 3H), 1.18(d, *J*=7.07Hz, 3H), 1.19(d, *J*=6.98Hz, 3H), 1.23(s. 3H), 1.31-1.36(m, 1H), 1.62(s, 3H), 1.75-2.04(m, 4H), 2.17-2.82(m, 6H), 3.19(s, 1H), 3.41-3.45(m, 1H), 3.65-3.75(m, 1H), 4.53(d, *J*=15.43Hz, 1H), 4.63(s, 1H), 4.73(d, *J*=15.42Hz, 1H), 6.11(s, 1H), 6.38(dd, *J*₁=10.04Hz, *J*₂=1.72Hz, 1H), 6.49(dd, *J*₁=3.43Hz, *J*₂=1.62Hz, 1H), 7.18(d, *J*=3.35Hz, 1H), 7.24(d, *J*=10.57Hz, 1H), 7.58(d, *J*=0.75Hz, 1H), **MASS (ES+) :** 665.1(M+Na)⁺ |
| IB.117 | 0.91(t, *J*=7.48Hz, 3H), 1.05(d, *J*=7.13Hz, 3H), 1.15(d, 3H, *J*=7.02Hz), 1.22(s, 3H), 1.38-2.00(m, 6H), 1.56(s, 3H), 2.28-2.46(m, 5H), 3.19(d, 1H, *J*=3.09Hz), 3.45(m, 1H), 3.68-3.73(m, 2H), 4.44-4.46(m, 1H), 4.51(d, *J*=15.29Hz, 1H), 4.75(d, *J*=15.38Hz, 1H), 5.33-5.52(m, 1H), 6.40(dd, *J*₁=10.18Hz, *J*₂=1.78Hz, 1H), 6.45(s, 1H), 6.50(dd, *J*₁=3.46Hz, *J*₂=1.61Hz, 1H), 7.13(d, *J*=3.32Hz, 1H), 7.18(d, *J*₁=10.03 Hz, *J*₂=0.94Hz, 1H), 7.58(d, *J*=0.89Hz, 1H), **MASS (ES+) :** 659.1 |
| IB.118 | 1.05(d, *J*=7.15Hz, 3H), 1.20(s, 3H), 1.35-1.40(m, 1H), 1.59(s, 3H), 1.77-2.00(m, 3H), 2.28-2.52(m, 4H), 3.12(d, *J*=2.71Hz, 1H), 3.41-3.45(m, 1H), 3.68(d, *J*=16.77Hz, 1H), 3.75(d, *J*=16.87Hz, 1H), 4.48-4.50(m, 1H), 4.75-4.96(m, 2H), 5.33-5.50(m, 1H), 6.41(dd, *J*₁=10.08Hz, *J*₂=1.70Hz, 1H), 6.46(s, 1H), 6.49(dd, *J*₁=3.44Hz, *J*₂=1.76Hz, 1H), 6.55(dd, *J*₁=3.51Hz, *J*₂=1.65Hz, 1H), 7.11(d, *J*=3.34Hz, 1H), 7.22-7.25(m, 2H), 7.57(d, *J*=0.83Hz, 1H), 7.61(d, *J*=0.85Hz, 1H), **MASS (ES+) :** 669.1 |
| IB.119 | 1.06(d, *J*=7.12Hz, 3H), 1.17(s, 3H), 1.30(d, *J*=6.20Hz, 6H), 1.36-1.41(m, 1H), 1.56(s, 3H), 1.72-1.97(m, 3H), 2.28-2.50(m, 4H), 2.82(s, 1H), 3.42-3.45(m, 1H), 3.67(d, *J*=16.71Hz, 1H), 3.76(d, *J*=16.51Hz, 1H), 4.46(d, *J*=8.56Hz, 1H), 4.74(d, *J*=15.47Hz, 1H), 4.79(d, *J*=15.41Hz, 1H), 5.08-5,13(m, 1H), 5.33-5.49(m, 1H), 6.41(dd, *J*₁=10.16Hz, *J*₂=1.58Hz, 1H), 6.46(s, 1H), 6.50(dd, *J*₁=3.36Hz, *J*₂=1.62Hz, 1H), 6.83(d, *J*=15.87Hz, 1H), 6.88(d, *J*=15.76Hz, 1H), 7.13(d, *J*=3.39Hz, 1H), 7.20(d, *J*=10.29Hz, 1H), 7.58(s, 1H), **MASS (ES+) :** 715.1 |
| IB.120 | 1.03(d, 3H, *J*=7.05Hz), 1.15(s, 3H), 1.16(s, 6H), 1.33-1.39(m, 1H), 1.55(s, 1H), 1.67-2.00(m, 3H), 2.29-2.59(m, 4H), 3.39-3.49(m, 1H), 3.51(d, 1H, *J*=4.81Hz), 3.54(d, 1H, *J*=11.65Hz), 3.71(d, 1H, *J*=16.53Hz), 3.77(d, 1H, *J*=16.53Hz), 4.73(t, 1H, *J*=5.86Hz), 4.13-4.35(m, 1H), 4.64(d, 1H, *J*=15.64Hz), 4.68(d, 1H, *J*=15.66Hz), 5.07(s, 1H), 5.37-5.53(m, 1H), 6.31-6.33(m, 1H), 6.33(s, 1H), 6.54(dd, 1H, *J*₁=3.41Hz, *J*₂=1.58Hz), 7.12(d, 1H, *J*=3.37Hz), 7.26(d, 1H, *J*=10.30Hz), 7.66(s, 1H), **MASS (ES+) :** 697.07(M+Na)⁺ |
| IB.121 | 105(d, 3H, *J*=7.11Hz), 1.18(s, 3H), 1.35-1.40(m, 1H), 1.56(s, 1H), 1.73-2.00(m, 3H), 2.28-2.55(m, 4H), 3.01(d, 1H, *J*=2.36Hz), 3.42-3.46(m, 1H), 3.66(d, 1H, *J*=16.75Hz), 3.77(d, 1H, *J*=16.77Hz), 4.46(d, 1H, *J*=8.20Hz), 4.67(d, 1H, *J*=15.43Hz), 4.78(d, 1H, *J*=15.43Hz), 5.33-5.49(m, 1H), 5.92(d, 1H, *J*=10.35Hz), 6.12(dd, 1H, *J*₁=17.32Hz, *J*₂=10.52Hz), 6.39-6.46(m, 1H), 6.43(d, 1H, *J*=13.3Hz), 6.50(dd, 1H, *J*₁=3.27Hz, *J*₂=1.52Hz), 7.12(d, 1H, *J*=3.39Hz), 7.19(d, 1H, *J*=10.12Hz), 7.58(s, 1H), **MASS (ES+) :** 629.05 |
| IB.122 | 0.86(t, *J*=6.57Hz, 6H), 1.06(d, *J*=7.13Hz, 3H), 1.16(s, 3H), 1.27-1.41(m, 1H), 1.55(s, 3H), 1.61-1.98(m, 8H), 2.29-2.51(m, 4H), 3.38-3.42(m, 1H), 3.-64-3.79(m, 3H), 4.36(d, *J*=6.47Hz, 1H), 4.68-4.82(m, 3H), 5.35-5.51(m, 1H), 6.35(dd, *J*₁=10.15Hz, *J*₂=1.67Hz, 1H), 6.38(s, 1H), 6.52(dd, *J*₁=3.40Hz, *J*₂=1.68Hz, 1H), 7.12(d, *J*=3.46Hz, 1H), 7.27(d, *J*=9.97Hz, 1H), 7.62(s, 1H), **MASS (ES+) :** 689.1 |
| IB.123 | 0.30-0.34(m, 2H), 0.60-0.64(m, 2H), 1.06(d, *J*=7.15Hz, 3H), 1.19(s, 3H), 1.36-1.41(m, 1H), 1.55(s, 3H), 1.64-1.99(m, 4H), 2.29-2.51(m, 4H), 2.61(s, 1H), 3.42-3.47(m, 1H), 3.69(d, *J*=16.73Hz, 1H), 3.77(d, *J*=16.72Hz, 1H), 3.99(d, *J*=7.39Hz, 2H), 4.44(d, *J*=8.47Hz, 1H), 4.65-4.74(m, 2H), 5.33-5.49(m, 1H), 6.41(dd, *J*₁=10.13Hz, *J*₂=1.66Hz, 1H), 6.46(s, 1H), 6.51(dd, *J*₁=3.43Hz, *J*₂=1.68Hz, 1H), 7.13-7.17(m, 2H), 7.59(s, 1H), **MASS (ES+) :** 673.0 |
| IB.124 | 1.05(d, *J*=7.07Hz, 3H), 1.15(s, 3H), 1.29-1.41(*m*, 1H), 1.54(s, 3H), 1.72-2.00(m, 3H), 2.28-2.50(m, 4H), 2.76(s, 3H), 3.39-3.43(m, 1H), 3.73(d, *J*=16.53Hz, 1H), 3.79(d, *J*=16.54Hz, 1H), 4.16(m, 2H), 4.36(d, *J*=6.03Hz, 1H), 4.93(s, 1H), 5.35-5.51(m, 1H), 6.35(d, *J*=10.19Hz, 1H), 6.37(s, 1H), 6.52(dd, *J*₁=3.09Hz, *J*₂-1.32Hz, 1H), 7.12(d, *J*=3.40Hz, 1H), 7.27(d, *J*=10.15Hz, 1H), 7.62(s, 1H), **MASS (ES+) :** 707.1(M+Na)⁺ |
| IB.125 | 1.06(d, *J*=7.13Hz, 3H), 1.20-1.42(m, 8H), 1.55(s, 3H), 1.71-2.00(m, 7H), 2.28-2.49(m, 4H), 2.66(s, 1H), 3.43-3.47(m, 1H), 3.69(d, *J*=17.44Hz, 1H), 3.76(d, *J*=16.97Hz, 1H), 4.44(d, *J*=8.32Hz, 1H), 4.63(d, *J*=15.47Hz, 1H), 4.70(d. *J*=15.45Hz, 1H), 5.05-5.09(m, 1H), 5.33-5.49(m, 1H), 6.40(dd, *J*₁=10.14Hz, *J*₂=1.48Hz, 1H), 6.46(s, 1H), 6.51(dd, *J*₁=3.32Hz, *J*₂=1.53Hz, 1H), 7.13-7.16(m, 2H), 7.59(s, 1H) **MASS (ES+) :** 687.1 |
| IB.126 | 1.06(d, *J*=7.07Hz, 3H), 1.20-1.99(m, 15H), 2.20-2.49(m, 5H), 2.60(s, 1H), 3.44(m, 1H), 3.69(d, *J*=16.73Hz, 1H), 3.77(d, *J*=16.67Hz, 1H), 4.04(d, *J*=7.15Hz, 2H), 4.44(d, *J*=7.91Hz, 1H), 4.64-4.73(m, 2H), 5.33-5.49(m, 1H), 6.40(d, *J*=10.02Hz, 1H), 6.46(s, 1H), 6.50(m, 1H), 7.13-7.17(m, 2H), 7.59(s, 1H), **MASS (ES+) :** 701.1 |
| IB.127 | 1.06(d, *J*=7.16Hz, 3H), 1.17(d, *J*=1.39Hz, 3H), 1.18(d, *J*=2.37Hz, 3H), 1.21(s, 3H), 1.35-1.41(m, 1H), 1.56(s, 3H), 1.77-2.00(m, 3H), 2.29-2.62(m, 5H), 3.21(d, *J*=2.62Hz, 1H), 3.42-3.49(m, 1H), 3.67(d, *J*=16.86Hz, 1H), 3.75(d, *J*=16.81Hz, 1H), 4.45(d, *J*=8.59Hz, 1H), 4.52(d, *J*=15.45Hz, 1H), 4.74(d, *J*=15.33Hz, 1H), 5.33-5.50(m, 1H), 6.40(dd, *J*₁=10.05Hz, *J*₂=1.65Hz, 1H), 6.45(s, 1H), 6.50(dd, *J*₁=3.50Hz, *J*₂=1.59Hz, 1H), 7.13(dd, *J*₁=3.56Hz, *J*₂=0,57Hz, 1H), 7.19(dd, *J*₁=0.75Hz, *J*₂=0.22Hz, 1H), 7.58(s,1H), **MASS (ES+) :** 673.1 |
| IB.128 | 1.06(d, *J*=7.12Hz, 3H), 1.18(s, 3H), 1.35(s, 3H), 1.38-1.41(m, 1H), 1.43(s, 3H), 1.55(s, 3H), 1.80-1.97(m, 3H), 2.40-2.64(m, 5H), 3.39-3.50(m, 1H), 3.71-3.83(m, 3H), 4.07-4.33(m, 4H). 4.44(d, *J*=8.59Hz, 1H), 4.69-4.72(m, 2H). 5.31-5.51(m, 1H), 6.40(dd, *J*₁=10.17Hz, *J*₂=1.73Hz, 1H), 6.45(s, 1H), 6.50(dd, *J*₁=3.47Hz, *J*₂=1.64Hz, 1H), 7.13(d, *J*=3.43Hz, 1H). 7.16(d, *J*=10.04Hz, 1H), 7.39(s, 1H), **MASS (ES+) :** 733.0 |
| IB.129 | 1.05(d, *J*=7.13Hz, 3H), 1.12-1.14(m, 6H), 1.24(s, **3H),** 1.35-1.39(m, 1H), 1.56(s, 3H), 1.80-2.11(m, **3H),** 2.30-2.42(m, 4H), 3.19-3.35(m, 4H), 3.43-3.52(m, 1H), 3.60-3.82(m, 2H), 4.12(d, *J*=3.12Hz, 1H), 4.42(d, *J*=8.25Hz, 1 H), 4.48(d, *J*=15.61Hz, 1H), 4.76(d, *J*=15.96Hz, 1H), 5.32-5.52(m, 1 H), 6.39(dd, *J*₁=10.12Hz, *J*₂=1.75Hz, 1 H), 6.45(s, 1H), 6.50(dd, *J*₁=3.47Hz, *J*₂=1.67Hz, I H ), 7.14(d, *J*=3.39Hz, 1H), 7.16(dd, *J*₁=10.13Hz, *J*₂=0.97Hz, 1H), 7.58(d, *J*=0.78Hz, 1H), **MASS (ES+) :** 674.1 |
| IB.130 | 1.05(d, *J*=7.17Hz, 3H), 1.22(s, 3H), 1.34-1.40(m, 1H), 1.56(s, 3H), 1.56-1.60(m, 6H), 1.77-2.07(m, 3H), 2.28-2.42(m, 4H), 3.40-3.49(m, 5H), 3.62-3.82(m, 2H), 3.86(d, *J*=1.15Hz, 1H), 3.42(d, *J*=8.41Hz, 1H), 4.52(d, *J*=15.61Hz, 1H), 4.74(d, *J*=15.58Hz, 1H), 5.32-5.51(m, 1H), 6.39(dd, *J*₁=10.11Hz, *J*₂=1.68Hz, 1H), 6.45(s, 1H), 6.50(dd, *J*₁=3.41Hz, *J*₂=1.58Hz, 1H), 7.13(d, *J*=3.41Hz, 1H), 7.18(d, *J*=10.00Hz, 1H), 7.59(d, *J*=0.74Hz, 1H), **MASS (ES+) :** 686.1 |
| IB.131 | 1.05(d, *J*=7.1 1Hz, 3H), 1.20(s, 3H), 1.34-1.40(m, 1H), 1.55(s, 3H), 1.77-2.04(m, 3H), 2.30(s, 3H), 2.39-2.53(m, 8H), 3.48(m, 5H), 3.64(d, *J*=15.98Hz, 1H), 3.66(s, 1H), 3.77(d, *J*=17.39Hz, 1H), 4.42(d, *J*=8.06Hz, 1H), 4.57(d, *J*=15.59Hz, 1H), 4.73(d, *J*=15.61 Hz, 1H), 5.32-5.49(m, 1H), 6.39(dd, *J*₁=10.15Hz, *J*₂=1.22Hz, 1H), 6.45(s. 1H), 6.50(dd, *J*₁=3.11Hz, *J*₂=1.26Hz, 1H), 7.14(d, *J*=3.49Hz, 1H), 7.17(d, *J=*10.13Hz, 1H), 7.59(s, 1H), **MASS (ES+) :** 701.1 |
| IB.132 | 1.12(s, 3H), 1.29-1.34(m, 1H), 1.48(d, *J*=7.34Hz*,* 3H), 1.55(s, 3H), 1.74-2.16(m, 4H) 2.33-2.81(m, 4H). 3.35(t, d, *J*₁=16.65Hz, *J*₂=2.31Hz, 1H), 3.81(t, d, *J*₁=16.66Hz, *J*₂=1.98Hz, 1H), 4.26(m, 2H), 4.46(d, *J*=8.42, 1H), 5.32-5.49(m, 1H), 6.39(dd, *J*₁=10.13Hz, *J*₂=1.75Hz, 1H), 6.44(s, 1H), 6.51(dd, *J*₁=3.50Hz, *J*₂=1.65Hz, 1H), 7.17-7.19(m, 1H), 7.61(s, 1H), **MASS (ES+) :** 575.1 |
| IB.133 | 1.14(s, 3H),1.19(d, *J*=6.97Hz, 3H), 1.25-1.32(m, 1H), 1.48(d, *J*=7.33Hz, 3H), 1.58(s, 3H), 1.75-2.15(m, 4H), 2.33-2.62(m, 5H), 3.20(dd, *J*₁=17.13Hz, *J*₂=0.74Hz, 1H), 3.84-3.90(m, 2H), 4.40(m, 2H), 4.72(dd, *J*₁=15.25Hz, *J*₂=1.33Hz, 1H), 5.33-5.51(m, 1H), 6.40(dd, *J*₁=10.13Hz, *J*₂=1.69Hz, 1H), 6.44(s, 1H), 6.51(dd. *J*₁=3.43Hz, *J*₂=1.57Hz, 1H), 7.18-7.20(m, 2H), 7.61(d, *J*=0.73Hz, 1H), **MASS (ES+) :** 645.0 |
| IB.134 | 1.06(d, *J*=7.16Hz, 3H), 1.17(d, *J*=2.39Hz, 3H), 1.18(d, *J*=2.37Hz, 3H), 1.21(s, 3H), 1.35-1.41(m, 1H), 1.56(s, 3H), 1.77-2.00(m, 3H), 2.29-2.62(m, 5H), 3.21(d, *J*=2.62Hz, 1H), 3.42-3.49(m, 1H), 3.67(d, *J*=16.86Hz, 1H), 3.75(d, *J*=16.81Hz, 1H), 4.45(d, *J*=8.59Hz, 1H), 4.52(d, *J*=15.45Hz, 1H), 4.74(d, *J*=15.33Hz, 1H), 5.33-5.50(m, 1H), 6.40(dd, *J*₁=10.05Hz, *J*₂=1.65Hz, 1H), 6.45(s, 1H), 6.50(dd, *J*₁=3.50Hz, *J*₂=1.69Hz, 1H), 7.13(dd, *J*₁=3.56Hz, *J*₂=0.57Hz, 1H), 7.19(dd, *J*₁=0.75Hz, J₂=0.22Hz, 1H), 7.58(s, 1H), **MASS (ES+) :** 675.0 |
| IB.135 | 1.06(d, *J*=7.12Hz, 3H), 1.19(s, 3H), 1.36-1.41(m, 1H), 1.55(s, 3H), 1.77-1.97(m, 3H), 2.32(s, 3H), 2.29-2.49(m, 5H), 3.41-3.58(m, 1H), 3.73-3.75(m, 2H), 4.45(d, *J*=8.67Hz, 1H), 4.77(d, *J*=15.28Hz, 1H), 4.98(d, *J*=15.27Hz, 1H), 5.31-5.50(m, 1H), 6.40(dd, *J*₁=10.15Hz, *J*₂=1.65Hz, 1H), 6.45(s, 1H), 6.50(dd, *J*₁=3.39Hz, *J*₂=1.64Hz, 1H), 7.12(d, *J*=3.45Hz, 1H), 7.15(dd, *J*=9.90Hz, 1H), 7.59(s, 1H), **MASS (ES+) :** 699.0 |
| IB.136 | 1.05(t, *J*=7.09Hz, 6H), 1.05(d, *J*=6.67Hz, 3H), 1.34-1.40(m, 1H), 1.55(s, 3H). 1.76-2.02(m, 3H), 2.28-2.62(m, 4H), 2.56(q, *J*=7.22Hz, 2H), 3.26-3.39(m, 3H), 3.44-3.47(m. 1H). 3.63(d, *J*=16.46Hz, 1H), 3.85(d, *J*=16.45Hz, 1H), 4.42(d, *J*=8.63Hz, 1H), 5.30-5.81(m, 1H), 6.39(dd, *J*₁=10.l7Hz, *J*₂=1.45Hz, 1H), 6.45(s, 1H), 6.50(dd, *J*₁=3.30Hz, *J*₂=1.61Hz, 1H), 7.14(d, *J*=3.21Hz, 1H), 7.15(d, *J*=9.85Hz, 1H), 7.9(s, 1H), **MASS (ES+) :** 630.1 |
| IB.137 | 1.00(d, *J*=7.131Hz, 3H), 1.20(s, 3H), 1.32(d, *J*=6.25Hz, 3H), 1.32(d, *J*=6.13Hz, 3H), 1.35-1.39(m, 1H), 1.55(s, 3H), 1.71-1.99(m, 3H), 2.28-2.49(m, 4H), 2.53(s, 1H), 3.42-3.46(m, 1H), 3.69(d, *J*=16.80Hz, 1H), 3.76(d, *J*=16.64Hz, 1H), 4.44(d, *J*=8.28Hz, 1H), 4.64(d, *J*=15.56Hz, 1H), 4.70(d, *J*=15.45Hz, 1H), 4.82-4.90(m, 1H), 5.33-5.49(m, 1H), 6.41(dd, *J*₁=10.17Hz, *J*₂=1.38Hz, 1H), 6.46(s, 1H), 6.5.(dd, *J*₁=3.26Hz *J*₂=1.44Hz, 1H), 7.13-7.16(m, 2H), 7.59(s, 1H), **MASS (ES+)** : 661.1 |
| IB.138 | 1.06(d, *J*=7.13Hz, 3H), 1.20(s.3H), 1.35-1.41(m, 1H), 1.55(s, 3H), 1.72(s.1H), 1.76(s, 3H), 1.74-1.98(m, 3H), 2.28-2.53(m, 5H), 3.42-3.46(m, 1H), 3.69(d, *J*=16.85Hz, 1H), 3.76(d, *J*=16.94Hz, 1H), 4.43(d, *J*=8.85Hz, 1H), 4.64-4.68(m, 3H), 5.33-5.49(m, 1H), 6.39(dd *J*₁=10.11Hz, *J*₂=1.71Hz, 1H), 6.46(s,1H), 6.50(dd, *J*₁=3.45Hz, *J*₂=1.71Hz, 1H), 7.13-7.16(m, 2H), 7.59(d, *J*=0.83Hz, 1H), **MASS (ES+)** 687,1 |
| IB.139 | 0.87(t, *J*=6.15Hz, 3H), 1.06(d, *J*=7.15Hz, 3H), 1.19(s,3H), 1.28-1.40(m, 1H), 1.55(s,3H), 1.64-1.99(m, 5H), 2.28-2.59(m, 5H), 3.43-3.46(m, 1H), 3.69(d, *J*=16.84Hz, 1H), 3.76(d, *J*=16.70Hz, 1H), 4.14(t, *J*=6.75Hz, 1H), 4.44(d, *J*=8.29Hz, 1H), 4.65(d, *J*=15.47Hz, 1H), 4.65(d, *J*=15.48Hz, 1H), 5.32-5.49(m, 1H), 6.40(dd, *J*₁=10.131, *J*₂=1.66Hz 1H), 6.46(s, 1H), 6.50(dd, *J*₁=3.42Hz, *J*₂=1.70Hz, 1H), 7.13-7.16(m, 2H), 7.59(s, 1H), **MASS (ES+)** : 703.1 |
| IB.140 | 0.94-1.02(m, 2H), 1.06(d, *J*=7.14Hz, 3H), 1.19(s,3H), 1.23-1.41(m,3H), 1.55(s, 3H), 1.66-1.99(m,9H), 2.29-2.49(m, 4H), 2.58(d, *J*=2.05Hz 1H), 3.43-3.46(m, 1H), 3.68(d, *J*=16.65Hz, 1H), 3.77(d, *J*=16.93Hz, 1H), 3.97(d, *J*=6.45Hz, 2H), 4.44(d, *J*=8.23Hz, 1H), 4.65(d, *J*=15.56Hz, 1H), 4.71(d, *J*=15.48Hz, 1H), 5.33-5.49(m, 1H), 6.40(dd, *J*₁=10.16Hz, *J*₂=1.50Hz, 1H), 6.46(s, 1H), 6.51(dd, *J*₁=3.33Hz, *J*₂=1.61Hz, 1H), 7.13-7.16(m,, 2H), 7.59(s, 1H), **MASS (ES+)** : 715.2 |
| IB.141 | 1.06(d, *J*=7.13Hz, 3H), 1.20(s,3H), 1.25-1.52(m, 7H), 1.55(s, 3H), 1.73-1.99(m, 7H), 2.29-2.53(m, 4H), 2.63(s, 1H), 3.43-3.47(m, 1H) 3.68(d, *J*=16.79Hz, 1H), 3.77(d, *J*=16.71Hz, 1H), 4.44(d, *J*=8.52Hz, 1H), 4.56-4.62(m, 1H), 4.64(d, *J*=15.48Hz, 1H), 4.70(d, *J*=15.53Hz 1H), 5.33-5.50(m, 1H), 6.40(dd, *J*=10.1Hz, *J*₂=1.71Hz, 1H), 6.46(s, 1H), 6.50(dd, *J*₂=3.45Hz, *J*₂=1.58Hz, 1H), 7.13-7.16(m,. 2H), 7.59(d *J*=0.9Hz, 1H), **MASS (ES+)** : 701.1 |
| IB.142 | 0.88(m, 9H), 0.94-1.25(m, 14H), 1.38-1.41(m, 1H), 1.49-1.72(m, 6H), 1.78-1.99(m, 3H), 2.28-2.55(m, 5H), 3.43-3.46(m, 1H), 3.68(d, *J*=17.42Hz, 1H), 3.76(d, *J*=16.72Hz, 1H), 4.17(m, 2H), 4.44(d, *J*=7.54Hz, 1H), 4.64(d, *J*=15.74Hz, 1H), 4.76(d, *J*=15.62Hz, 1H), 5.33-5.49(m, 1H). 6.40(d, *J*=10.10Hz, 1H), 6.46(s, 1H), 6.50(m, 1H), 7.13-7.16(m., 2H), 7-59(m, 1H). **MASS (ES+)** : 745.1 |
| IB.143 | 0.96(s, 9H), 1.06(d, *J*=7.05Hz, 3H), 1.19(s, 3H), 1.25-1.40(m, 7H), 1.55(s, 3H), 1.74-1.99(m, 3H), 2.30-2.56(m, 5H), 3.44(m, 1H), 3.68(d, *J*=16.89z, 1H), 3.79(d, *J*=17.24Hz, 1H), 3.85(s, 2H), 4.45(d, *J*=7.73Hz, 1H), 4.67(d, *J*=15.98Hz, 1H), 4.71(d, *J*=15.98Hz, 1H), 5.33-5.49(m, 1H), 6.40(d, *J*=10.09Hz, 1H), 6.46(s, 1H), 6.50-6.51(m, 1H), 7.13-7.16(m, 2H), 7.59(s, 1H), **MASS (ES+)** : 689.1 |
| IB.144 | 1.06(d, *J*=7.16Hz, 3H). 1.19(s, 3H), 1.25-1.39(m, 1H), 1.55(s, 3H), 1.76-2.10(m, 9H), 2.28-2.67(m, 6H), 3.43-3.47(m, 1H), 33.67-3.79(m 2H), 4.12(d, *J*=8.32Hz, 1H), 4.65-4.75(m, 2H), 5.33-5.50(m, 1H), 6.40(dd, *J*₁=10.11Hz, *J*₂=1.64Hz. 1H), 6.46(s, 1H), 6.51(dd, *J*₁=3.45Hz *J*₂=1.60Hz, 1H), 7.13-7.17(m,, 2H), 7.59(m, 1H) **MASS(ES+)**: 687.1 |
| IB.145 | 1.06(d, *J*=7.07Hz, 3H). 1.19(s, 3H), 1.36-1.41(m, 1H), 1.54(s, 3H), 1.70-1.94(m, 3H), 2.17-2.49(m, 5H), 3.44(m, 1H), 3.68(d, *J*=16.70Hz 1H), 3.76(d, *J*=16.66Hz, 1H), 4.42(d, *J*=7.94Hz, 1H), 4.70(s, 2H), 5.13(s, 2H), 5.32-5.49(m, 1H), 6.38-6.50(m, 5H), 7.12-7.14(m, 2H), 7.43(s, 1H). 7.59(s, 1H). **MASS (ES+)** : 699.1 |
| IB.146 | 0.94(t, *J*=7.29Hz, 3H), 1.06(d, *J*=7.15Hz, 3H), 1.19(s, 3H), 1.25-1.43(m, 3H), 1.55(s. 3H). 1.61-1.99(m, 5H), 2.28-2.49(m, 4H), 2.59(s, 1H), 3.44-3.47(m, 1H), 3.68-3.79(m, 2H), 4.15(t, *J*=6.69Hz, 1H), 4.44(d, *J*=8.63Hz, 1H), 4.65(d, *J*=15.51Hz, 1H), 4.71(d, *J*=15.47Hz, 1H), 5.33-5.49(m, 1H), 6.40(dd, *J*₁=10.14Hz, *J*₂=1.59Hz, 1H), 6.46(s, 1H), 6.50(dd, *J*₁=3.39Hz, *J*₂=1.60Hz, 1H), 7.13-7.17(m, 2H), 7.59(s, 1H), **MASS (ES+)** : 675.1 |
| IB.147 | 0.90(t, *J*=6.86Hz, 3H), 1.06(d, *J*=7.11Hz, 3H), 1.19(s, 3H), 1.33-1.43(m, 5H), 1.55(s, 3H), 1.65-1.99(m, 5H), 2.28-2.53(m, 4H), 2.60(s, 1H) 3.42-3.47(m, 1H), 3.69(d, *J*=16.86Hz, 1H), 3.76(d, *J*=16.62Hz, 1H), 4.15(t, *J*=6.78Hz, 2H), 4.44 (d, *J*=8.38Hz, 1H), 4.66(d, *J*=15.54H, 1H), 4.71(d, *J*=15.39Hz, 1H), 5.33-5.49(m, 1H), 6.40(dd, *J*₁=10.08Hz, *J*₂=1.55Hz, 1H), 6.46(s, 1H), 6.50(dd, *J*₁=3.36Hz, *J*₂=1.74Hz, 1H), 7.13-7.17(m, 2H), 7.59(s, 1H), **MASS (ES+)** : 689.2 |
| IB.148 | 1.06(d, *J*=7.17Hz, 3H), 1.20(s, 3H), 1.24-1.25(m, 6H), 1.35-1.41(m, 1H), 1.56(s, 3H), 1.77-1.98(m, 3H), 2.28-2.52(m, 4H), 2.82(s, 1H), 3.42-3.46 (m, 1H), 3.67 (d, *J*=16.93Hz, 1H), 3.75(d, *J*=16.87Hz, 1H), 4.33(dd, *J*=13.35Hz, *J*₂=8.87Hz, 1H), 4.45(dd, *J*₁=13.22Hz, *J*₂=8.77Hz, 1H), 4.42-4.48(m, 1H), 4.58(d, *J*=15.35Hz, 1H), 4.78(d, *J*=15.36Hz, 1H), 5.31-5.49(m, 1H), 6.40(dd, *J*₁=10.12Hz, *J*₂=1.68Hz, 1H), 6.45(s, 1H), 6.50(dd, *J*₁=3.43Hz, *J*₂=1.64Hz, 1H), 7.12(d, *J*=3.44Hz, 1H), 7.17(dd, *J*=10.21Hz, 1H), 7.58(d, *J*=0.71Hz, 1H), **MASS (ES+)** : 677.1 |
| IB.149 | 1.06(d, *J*=7.10Hz, 3H), 1.18(s, 3H), 1.36(s, 3H), 1.38-1.43(m, 1H), 1.43(s, 3H), 1.55(s, 3H), 1.77-1.98(m, 3H), 2.28-2.55(m, 5H), 3.43-3.46(m, 1H), 3.66-3.83(m, 3H), 4.07-4.36(m, 4H), 4.44(d, *J*=7.86Hz, 1H), 4.67-(d, *J*=15.48Hz, 1H), 4.73(d, *J*=15.48Hz, 1H), 5.32-5.49(m, 1H), 6.39(d, *J*=10.08Hz, 1H), 6.45(s, 1H), 6.50(d, *J*=1.59Hz, 1H), 7.13(d, *J*=3.39Hz, 1H), 7.15(d, *J*=10.24Hz, 1H), 7.59(s, 1H), **MASS (ES+) :** 733.1 |
| IB.150 | 1.06(d, *J*=7.08Hz, 3H), 1.18(s, 3H), 1.35(s, 3H), 1.38-1.43(m, 1H), 1.43(s, 3H), 1.55(s. 3H), 1.77-1.97(m, 3H), 2.28-2.49(m, 5H), 3.42-3.46(m, 1H), 3.69(d, *J*=16.77Hz, 1H), 3.77(d, *J*=15.45Hz, 1H), 3.80(d, *J*=5.40Hz, 1H), 3.82(d, *J*=5.56Hz, 1H), 4.08(d, *J*=6.42Hz, 1H), 4.10(d. *J*=6.85Hz, 1H), 4.18(d, *J*=5.35Hz, 2H), 4.32(m, 1H), 4.45(d, *J*=7.55Hz, 2H). 4.70(m, 1H). 5.31-5.51(m, 1H), 6.39(d, *J*=10.13Hz, 1H), 6.45(s, 1H), 6.50(dd, *J*₁=3.47Hz, *J*₂=1.52Hz, 1H), 7.12(d, *J*=3.44Hz, 1H), 7.14(d, *J*=10.15Hz, 1H), 7.59(s, 1H), **MASS (ES+)** : 733.1 |
| IB.151 | 1.05(d, *J*=7.11Hz, 3H), 1.20(s, 3H), 1.35-1.40(m, 1H), 1.54(s, 3H). 1.77-2.03(m, 3H), 2.28-2.52(m, 4H). 3.39(d, *J*=72.79Hz, 1H), 3.39-3.46(m, 5H), 3.67-3.80(m, 6H), 4.42(d, *J*=8.10Hz, 1H), 4.59(d, *J*=15.59Hz, 1H), 4.74(d, *J*=15.59Hz, 1H), 5.32-5.49(m, 1H), 6.39(dd, *J*₁=10.16Hz, *J*₂=1.48Hz, 1H), 6.45(s, 1H), 6.50(dd, *J*₁=3.26Hz, *J*₂=1.59Hz, 1H), 7.14(d, *J*=2.57Hz, 1H), 7.16(d, *J*=10.25Hz, 1H), 7.59(s, 1H ). **MASS (ES+)** : 688.1 |
| IB.152 | 1.05(d, *J*=7.10Hz, 3H), 1.20(s, 3H), 1.34(s, 3H), 1.34-1.40(m, 1H), 1.43(s, 3H), 1.55(s, 3H), 1.77-2.03(m, 3H), 2.28-2.53(m, 4H), 3.23-3.30(m, 1H), 3.37-3.45(m, 3H), 3.63-3.79(m, 3H), 4.02-4.24(m, 2H), 4.43(d, *J*=7.99Hz, 1H), 4.53(d, *J*=15.80Hz, 1H), 4.70(d, *J*=15.6Hz, 1H), 5.15(s, 1H), 5.33-5.49(m, 1H), 6.39(dd, *J*₁=10.16Hz, *J*₂=1.42Hz, 1H), 6.45(s, 1H), 6.50(d, *J*₁=3.31Hz, *J*₂=1.54Hz, 1H), 7.14(d, *J*=3.39Hz, 1H), 7.18(d. *J*=9.90Hz, 1H), 7.59(s, 1H), **MASS (ES+)** : 732.1 |
| IB.153 | 1.06(d, *J*=7.12Hz, 3H), 1.09(m, 2H), 1.20(s, 3H), 1.36-1.41(m, 1H), 1.51-1.99(m, 15H), 2.29-2.49(m, 4H), 2.64(d, *J*=1.80Hz, 1H), 3.43-3.47(m, 1H), 3.69(d, *J*=16.81Hz, 1H), 3.76(d, J=16.88Hz, 1H), 4.17(t, *J*=6.97Hz, 2H), 4.44(d, *J*=8.65Hz, 1H), 4.65(d, *J*=15.48Hz, 1H) 4.71(d, *J*=15.44Hz, 1H), 5.33-5.49(m, 1H), 6.40(dd, *J*₁=10.13Hz, *J*₂=1.56Hz, 1H), 6.46(s, 1H), 6.51(dd, *J*₁=3.37Hz, *J*₂=1.53Hz, 1H), 7.13-7.17(m, 2H), 7.59(d, *J*=0.70Hz, 1H), **MASS (ES+)** : 715.1 |
| IB.154 | 0.93(d, *J*=6.53Hz, 6H), 1.06(d, *J*=7.11Hz, 3H), 1.20(m, 2H), 1.35-1.41(m. 1H), 1.55(s, 3H), 1.57-1.66(m, 2H), 1.66-1.99(m, 4H). 2.29-2.49(m, 4H), 2.57(s, 1H), 3.43-3.46(m, 1H), 3.69(d, *J*=16.54Hz, 1H), 3.77(d, *J*=16.68Hz, 1H), 4.19(t, *J*=6.87Hz, 2H), 4.44(d, *J*=8.08Hz 1H), 4.65(d. *J*=15.53Hz, 1H), 4.71(d, *J*=15.51Hz, 1H), 5.33-5.49(m, 1H), 6.40(d, *J*=10.11Hz, 1H), 6.46(s, 1H), 6.50(dd, *J*₁=3.10Hz *J*₂=1.63Hz, 1H), 7.13-7.17(m, 2H) 7.59(s, 1H), **MASS(ES+)** : 689.1 |
| IB.155 | 1.06(d, *J*=7.08Hz, 3H), 1.17(s, 3H), 1.36-1.40(m, 1H), 1.55(s, 3H), 1.74-2.11(m, 5H), 2.29-2.63(m, 6H), 3.44(m, 1H), 3.67(d, *J*=16.71Hz, 1H), 3.76(d, *J*=16.62Hz, 1H), 4.45(d, *J*=8.31Hz, 1H), 4.51(s, *J*=6.24Hz, 2H), 4.61(d, *J*=15.49Hz, 1H), 4.73(d, *J*=15.42Hz, 1H), 4.40(d, *J*=11.18Hz, 1H), 5.33-5.50(m, 1H), 6.40(d, *J*=11.18Hz, 1H), 6.45(s, 1H), 6.50(m, 1H), 7.13(d, *J*=3.30Hz, 1H), 7.17(d, *J*=10.24Hz, 1H). 7.59(s. 1H), **MASS (ES+)** : 706.1 |
| IB.156 | 1.05(d, *J*=7.06Hz, 3H), 1.19(s, 3H), 1.31(s, 3H), 1.13(s, 3H), 1.37-1.41(m, 1H), 1.44(s, 3H), 1.50(s, 3H), 1.55(s, 3H), 1.77-2.00(m, 3H), 2.28-2.50(m, 4H), 2.68(s, 1H), 3.45(m, 1H), 3.66(d, *J*=13.96Hz, 1H), 3.77(d, *J*=16.58Hz, 1H), 4.05(t, *J*=5.83Hz, 1H), 4.24-4.34(m, 4H), 4.46(d, *J*=7.50Hz, 1H), 4.62(dd, *J*₁=7.80Hz, *J*₂=2.15Hz, 1H), 4.69(s, 2H), 5.33-5.49(m, 1H), 5.52(d, *J*=4.93Hz, 1H), 6.38(d, *J*=10.13Hz, 1H), 6.45(s, 1H), 6.50(dd, *J*₁=3.19Hz, *J*₂=1.47Hz, 1H), 7.13(d, *J*=3.18Hz, 1H), 7.18(d, *J*=10.10Hz, 1H), 7.59(s, 1H), **MASS (ES+)** : 861.1 |
| IB.157 | 1.06(d, *J*=7.08Hz, 3H), 1.17(s, 3H), 1.36-1.40(m, 1H), 1.55(s, 3H), 1.74-2.11(m, 5H), 2.29-2.G3(m. GH), 3.44(m, 1H), 3.67(d, *J*=16.71Hz, 1H), 3.76(d, *J*=16.62Hz, 1H), 4.45(d, *J*=8.31Hz, 1H), 4.51(t, *J*=6.24Hz, 2H), 4.61(d, *J*=15.49Hz, 1H), 4.73(d, *J*=15.42Hz, 1H), 4.40(d, *J*=11.18Hz, 1H), 5.33-5.50(m, 1H), 6.40(d, *J*=11.18Hz, 1H), 6.45(s, 1H), 6.50(m, 1H), 7.13(d, *J*=330Hz, 1H), 7.17(d, *J*=10.24Hz, 1H), 7.59(s, 1H), MASS (ES+) : 720.1 |
| IB.158 | 1.06(d, *J*=7.14Hz, 3H), 1.16(s, 3H), 1.35-1.41(m, 1H), 1.55(s. 3H), 1.77-1.97(m, 3H), 2.12-2.51(m, 5H), 3.36(s. 3H), 3.43-3.47(m, 1H) 3.71(d, *J*=16.48Hz, 1H), 3.83(d, *J*=16.48Hz, 1H), 4.05(d, *J*=15.61Hz, 1H), 4.10(d, *J*=15.58Hz. 1H), 4.44(d, J=8.60Hz, 1H), 5.34-5.47(m, 1H), 6.41(dd, *J*₁=10.16Hz, *J*₂=1.69Hz, 1H), 6.46(s, 1H), 6.51(dd, *J*₁=3.44Hz, *J*₂=1.75Hz 1H), 7.13(d, *J*=3.40Hz, 1H), 7.15(d *J*=10.20Hz, 1H), 7.59(d, *J*=0.86Hz, 1H), **MASS (ES+)** : 589.1 |
| IB.159 | 1.06(d, *J*=7.16Hz, 3H), 1.16(s, 3H), 1.22(t, *J*=7.01Hz, 3H), 1.36-1.41(m, 1H), 1.54(s, 3H), 1.71-2.00(m, 3H). 2.28-2.51(m, 4H), 3.42-3.46(m, 1H), 3.46-3.59(m, 2H), 3.70(d, *J*=16.51Hz, 1H), 3.83(d, *J*=16.49Hz, 1H), 4.07-4.17(m, 2H), 4.44(d, *J*=8.80Hz, 1H), 5.32-5.49(m, 1H), 6.41(dd, *J*₁=10.12Hz, *J*₂=1.68Hz, 1H), 6.46(s, 1H), 6.51(dd, *J*₁=3.43Hz, *J*₂=1.63Hz, 1H), 7.13-7.16(m, 2H), 7.59(s, 1H), **MASS (ES+)** : 603.1 |
| IB.160 | 0.91(d, *J*=6.70Hz, 6H), 1.06(d, *J*=7.07Hz, 3H), 1.15(s, 3H), 1.25-1.41(m, 1H), 1.54(s, 3H), 1.67-1.97(m, 5H), 2.28-2.53(m, 4H), 3.22-3.28(m, 2H), 3.42-3.46(m, 1H), 3.71(d, *J*=16.42Hz, 1H), 3.84(d, *J*=16.45Hz, 1H), 4.06-4.16(m, 2H), 4.44(d, *J*=7.72Hz, 1H), 5.32-5.49(m, 1H), 6.39-6.41(m, 1H), 6.46(s, 1H), 6.51(dd, *J*₁=3.19Hz, *J*₂=1.44Hz, 1H), 7.12-7.15(m, 2H). 7.59(s, 1H). **MASS (ES+)** : 631.1 |
| IC.1 | 1.03(d, *J*=7.07Hz, 3H), 1.13(d, *J*=7.68Hz, 3H), 1.15(s, 3H), 1.20-1.89(m, 8H), 2.15-2.44(m. 6H), 2.85-2.88(m, 1H), 4.30-4.33(m, 1H), 4.82(s, 2H), 5.30-5.47(m, 1H), 6.34(dd, *J*₁=10.13Hz, *J*₂=1.17Hz, 2H), 6.38(s, 1H), 7.05(dd, *J*=8.67Hz, 2H), 7.21(d, *J*=10.17Hz, 1H), 7.43-7.47(m, 2H), **MASS (ES+)**: 615.5 (M+Na⁺). |
| IC.2 | 1.03(d, *J*=7.01Hz, 3H), 1.11-1.14(m, 6H), 1.32-1.89(m, 8H), 4.20-2.44(m, 6H), 2.80-2.84(m, 1H), 4.40(d, *J*=8.11Hz, 1H), 4.78(d, *J*=11.17Hz, 1H), 4.4.81(d, *J*=11.19Hz, 1H), 5.32-5.49(m, 1H), 6.37(d, *J*=10.14Hz, 1H), 7.10(d, *J*=10.15Hz, 1H), 7.29(d, *J*=8.19Hz, 1H), 7.44(d, *J*=8. 19Hz, 1H) 7.57(s, 1H), **MASS (ES+)**: 665.4 (M+Na)⁺. |
| IC.3 | 1.03(d, *J*=7.09Hz, 3H), 1.12(t, *J*=7.50Hz, 3H), 1.16(s, 3H), 1.16-1.95(m, 8H), 2.20-2.40(m, 6H), 2.78-2.84(m, 1H), 4.41(d, *J*=8.70Hz, 1H), 5.28-5.48(m, 1H), 5.39(d,d *J*₁=50.31 Hz, *J*₂=2.66Hz, 1H), 5.51(d,d *J*₁=49.30Hz, *J*₂=2.61Hz, 1H), 6.37(dd *J*₁=10.15Hz, *J*₂=1.74Hz, 1H). 6.43(s, 1H), 7.10(dd *J*₁=10.12Hz, *J*₂=1.11Hz, 1H), **MASS(ES+)**: 517.2 |
| IC.4 | 1.11(d, *J*=7.09Hz, 3H), 1.21(s, 3H), 1.24-1.97(m, 8H), 2.28-2.50(m, 4H), 2.86-2.90(m, 1H), 4.45-4.47(m, 1H), 5.30-5.52(m, 1H), 5.41(d.d *J*₁=53.67Hz, *J*₂=2.34Hz 1H),5.56(d,d *J*₁=52.24Hz, *J*₂=2.33Hz, 1H), 6.39(d.d *J*₁=10.13Hz, *J*₂=1.77MHz 1H),6.45(s, 1H), 6.51(d.d *J*₁=3.50Hz, *J*₂=1.72Hz 1H), 7.12-7.15(m, 2H) 7.60(d, *J*=0.88Hz, 1H). **MASS(ES+):**555.2 |
| IC.5 | 1.10(d, *J*=7.17Hz, 3H), 1.21(s, 1H), 1.25-1.38(m, 2H), 1.55(s, 1H), 1.85-1.99(m. 3H), 2.16-2.59(m, 4H), 2,88-2.93(m, 1H). 4.83(d *J*=10.67Hz, 1H), 4.83(d, *J*=10.69Hz, 1H), 5.03-5.09(m, 1H), 6.31-6.35(m, 2H), 6.54(dd, *J*₁=3.47Hz, *J*₂=1.71Hz, 1H), 7.03-7.07(m, 2H), 7.26(d *J*=10.03Hz, 1H), 7.45-7.49(m, 2H), 7.65(d *J*=0.82Hz, 1H). **MASS(ES+**) : 631.2 |
| IC.6 | 1.12(d, *J*=7.07Hz, 3H), 1.21(s, 3H), 1.26-1.48(m, 1H), 1.53(s, 3H), 1.59-1.96(m, 4H), 2.28-2.52(m, 4H). 2.82-2.86-2.89(m, 1H,. 4.44-4.46(m, 1H), 4.93(d, *J*=11.263Hz, 1H), 5.03(d, *J*=11.24Hz, 1H), 5.32-5.48(m, 1H), 6.39(dd, *J*=10.11Hz, *J*₂=1.68Hz, 1H), 6.45(s, 1H). 6.52(dd, *J*₁=3.41Hz, *J*₂=1.70Hz, 1H), 7.11(d, *J*=5.71Hz, 1H), 7.13(s, 1H), 7.58-7.64(m, 4H). **MASS(ES+)**: 681.3 |
| IC.7 | 1.09(d, *J*=6.89Hz, 3H), 1.20(s, 3H), 1.25-1.50(m, 1H), 1.55(s, 3H), 1.60-2.03(m, 4H), 2.16-2.52(m, 4H), 2.92-3.13(m, 1H), 4.32(m, 1H), 4.83(d, *J*=10.85Hz, 1H), 4.87(d, *J*=10.88Hz, 1H), 5.13-5.51(m, 1H), 6.30-6.33(m, 2H), 6.55(s, 1H), 7.13(s, 1H), 7.26(d, *J*=10.22Hz, 1H). 7.33-7.58(m, 4H), 7.58-7.77(m, 1H), **MASS(ES+)** : 647.2 |
| IC.8 | 0.99(d, *J*=7.09Hz, 3H), 1.09(t, *J*=7.52Hz, 3H), 1.12(s, 3H), 1.22-1.88(m, 8H), 2.13-2.35(m, 6H). 2.82-2.86(m, 1H), 4.25-4.27(m, 1H), 4.89(s, 2H), 4.96-4.98(m, 1H), 5.30-5.50(m, 1H), 6.26-6.29(m, 2H), 7.21(dd, *J*₁=10.03Hz, *J*₂=1.05Hz, 1H), 7.56(d, *J*=8.26Hz, 2H), 7.61(d, *J*=8.30Hz, 2H). **MASS(ES+)**: 643.0 |
| IC.9 | 1.05(d, *J*=7.09Hz, 3H), 1.18(s, 1H), 1.20-1.87(m, 8H), 2.19-2.56(m, 4H), 2.58-2.70(m, 1H), 2.86-2.90(m, 1H), 4.30-4.321(m, 1H). 4.80(d, *J*=10,70Hz, 1H), 4.85(d, *J*=10.70Hz, 1H), 4.92-4.94(m, 1H), 6.05(m, 1H), 6.25(dd, *J*₁=10.13Hz, *J*₂=1.77Hz, 1H), 6.51(dd, *J*₁=3.45Hz, *J*₂=1.70Hz, 1H), 7.00-7.02(m, 2H), 7.29(d, *J*=10.11Hz, 1H), 7.43-7.46(m, 2H). 7.63(d, *J*=0.85Hz, 1H). **MASS(ES+)** : 613.0 |
| IC.10 | 1.00(d, *J*=7.06Hz, 3H), 1.07(t. *J*=7.54 Hz, 3H), 1.15(s, 3H), 1.25-1.92(m, 8H), 2.16-2.44(m, 6H), 2.81-2.84(m, 1H), 4.41(d, *J*=8.51Hz, 1H), 5.10(d, *J*=16.55Hz, 1H), 5.21(d, *J*=16.64Hz, 1H), 5.30-5.46(m, 1H), 6.38(dd, *J*₁=10.13Hz, *J*₂=1.69Hz, 1H), 7.14(d, *J*=10.13Hz, 1H). 7.46(d, *J*=8.54Hz, 2H), 7.85(d, *J*=8.58Hz, 2H). **MASS(ES+)** :637.1 |
| IC11 | 1.10(d, *J*=7.08Hz, 3H), 1.22(s, 3H), 1.25-1.94(m, 8H), 2.27-2.65(m, 5H), 2.80-2.92(m, 1H), 4.45-4.56(m, 1H), 5.41 (dd *J*₁=52.28Hz, *J*₁=2.3Hz, 1H), 5.56(dd *J*₁=53.72Hz, *J*₂=2.34Hz, 1H), 6.15(s, 1H), 6.37(dd *J*₁=10.13Hz, *J*₂=1.82Hz, 1H), 6.50(dd *J*₁=3.48Hz, *J*₂=1.70Hz, 1H), 7.12(d, *J*=3.40Hz, 1H), 7.21(d, *J*=10.12Hz, 1H), 7.59(d, *J*=0.86Hz, 1H), **MASS(ES+) ;** 537.1 |
| IC12 | 0.89(t, *J*=7.33Hz, 3H), 1.03(d, *J*=7.07Hz, 3H), 1.16(s, 3H), 1.25-1.91(m, 12H), 2.17-2.44(m, 6H), 2.80-2.84(m, 1H), 4.1-4.33 (m, 1H). 5.30-5.58(m, 3H), 6.38(dd, *J*₁=10.13Hz, *J*₂=1.69Hz, 1H), 6.43(s, 1H), 7.11(dd, *J*=10.12Hz, J₂=0.98 1H), MASS(ES+) : 537.1 |
| IC13 | 1.02(d, *J*=7.08Hz, 3H), 1.12(t, *J*=7.70Hz, 3H), 1.15(s, 3H), 1.32-1.37(m, 1H), 1.52(s, 3H), 1.68-1.92(m, 4H), 2.22-2.39(m, 4H), 2.76-2.81(m, 1H), 3.84(s, 3H), 4.40-4.42(m, 1H), 5.29-5.47(m, 1H), 6.38(dd *J*₁=10.12Hz, *J*₂=1.79Hz, 1H), 6.43(s, 1H), 7.12(dd *J*₁=10.14Hz, *J*₂=1.32Hz, 1H), **MASS(ES+) :** 499.0 |
| IC14 | 1.08(d, *J*=7.08Hz, 3H), 1.20(s, 3H), 1.29-1.40(m, 1H), 1.55(s, 3H), 1.59-2.00(m, 4H), 2.27-2.51(m, 4H), 2.88-2.92(m, 1H), 3.85(s, 3H), 4.33-4.35(m, 1H), 4.95-4.97(m, 1H), 5.34-5.51(m, 3H), 6.33(dd *J*₁=10.10Hz, *J*₂=1.85Hz, 1H), 6.36(s, 1H), 6.52(dd *J*₁=3.49Hz, *J*₂=1.72Hz. 1H), 7.11(dd, *J*₁=3.48Hz, *J*₂=0.53Hz, 1H), 7.26(dd *J*₁=10.05Hz, *J*₂=0.96Hz, 1H), 7.62-7.63m, 1H), **MASS(ES+):** 537.0 |
| IC15 | 1.04(d, *J*=7.09Hz, 3H), 1.H-1.15(m, 6H), 1.34-1.85(m, 8H), 2.20-2.39(m, 6H), 2.80-2.90(m, 1H), 4.38(d, *J*=8.66Hz, 1H), 4.86(d, *J*=10.78Hz, 1H), 4.92(d, *J*=10.77Hz, 1H), 5.27-5.49(m, 1H), 6.36(dd, *J*₁=10.14Hz, *J*₂=1.80Hz 1H), 6.43(s, 1H), 7.09(dd, *J*₁=10.13Hz, *J*₂=1.20Hz, 1H), 7.35-7.45(m, 5H), **MASS(ES+)** :575.0 |
| IC16 | 1.08(d, *J*=7.08Hz, 3H), 1.20(s, 3H), 1.22-1.97(m, 8H), 2.26-2.54(m, 4H), 2.91-2.95(m, 1H), 4.31(d, *J*=6.59Hz, 1H), 4.85(d, *J*=10.66Hz. 1H), 4.88(d, *J*=10.66Hz, 1H), 5.24-5.56(m, 1H), 6.28-6.31(m, 2H), 6.56(dd, *J*₁=3.39Hz, *J*₂=1.64Hz 1H), 7.31(d, *J*=3.45Hz, 1H), 7.18. 7.46(m, 6H), 7.72(s, 1H), **MASS(ES+)**:613.0 |
| IC17 | 0.99(d, *J*=7.07Hz, 3H), 1.09(1, *J*=7.51Hz, 3H), 1.14(s, 3H), 1.19-1.96(m, 8H), 2.09-2.47(m, 12H), 2.77-2.81 (m, 1H), 4.40(d, *J*=6.30Hz, 1H), 4.74(d, *J*=18.25Hz, 1H), 4.82(d, *J*=18.27Hz, 1H), 5.30-5.46(m, 1H), 6.38(dd, *J*₁=10.15Hz, *J*₂=1.79Hz, 1H), 6.43(s, 1H), 6.84(s, 2H), 7.12(d, *J*=9.89Hz, 1H), **MASS(ES+)**:645.3 |
| IC18 | 1.03(d, *J*=7.09Hz, 3H), 1.14(s, 3H), 1.13(t, *J*=7.54Hz, 3H), 1.25-1.89(m, 8H), 2.17-2.40(m, 6H), 2.81-2.83(m, 1H), 4.38-4.41(m, 1H), 4.81(d, *J*=10.91Hz, 1H), 4.88(d, *J*=10.91Hz, 1H), 5.25-5.50(m, 1H), 6.35(d,d *J*₁=10.13Hz, *J*₂=1.65Hz), 6.43(s, 1H), 7.10(d,d *J*₁=10.10Hz, *J*₂=0.67Hz 1H), 7.34(d, *J*=8.40Hz, 2H), 7.40(d, *J*=8.39Hz, 2H), **MASS(ES+)** : 609.0 |
| IC19 | 1.10(d, *J*=7.04Hz, 3H), 1.21(s, 3H), 1.30-1.96(m, 8H), 2.29-2.54(m, 4H), 2.90-2.93(m, 1H), 4.33-4.36(m, 1H), 4.82(d, *J*=11.11Hz, 1H), 4.87(d, *J*=11.10Hz, 1H), 4.95-4.98(m, 1H), 5.36-5.51(m, 1H), 6.33(dd, *J*₁=10.13Hz, *J*₂=1.75Hz, 1H), 7.14(d, *J*=3.45Hz, 1H), 7.25(d, *J*=10.09Hz, 1H), 7.32(dd, *J*₁=8.22Hz, *J*₂=1.89Hz 1H), 7.44-7.46(m, 1H), 7.64(s, 1H). **MASS(ES+):**680.9 |
| IC20 | 1.09(d, *J*=7.00Hz, 3H), 1.20(s, 3H), 1.30-2.02(m, 8H), 2.26-2.53(m, 4H), 2.91-2.95(m, 1H), 4.32(d, *J*=6.99Hz, 1H), 4.97(d, *J*=11.64Hz. 1H), 5.03(d, *J*=11.59Hz, 1H), 5.37-5.54(m, 1H), 6.31-6.32(m, 2H), 6.56(dd, *J*₁=3.28Hz, *J*₂=1.56Hz, 1H), 7.13(d, *J*=3.41Hz, 1H), 7.18, 7.31(m, 2H), 7.42(d, *J*=1.43Hz, 1H), 7.58(d, *J*=8.25Hz, 1H), 7.70(s, 1H), **MASS(ES+):** 681.5 |
| IC21 | 1.H(d, *J*=6.97Hz, 3H), 1.22(s, 3H), 1.25-1.96(m, 8H), 2.28-2.58(m, 4H), 2.90-3.95(m, 1H), 4.36(m, 1H), 4.83-4.88(m, 3H), 5:30-5.55(m, 1H), 6.34(d,d *J*₁=10.10Hz, *J*₂=1.68Hz, 1H), 6.38(m, 1H), 6.53(d,d *J*₁=3.39Hz, *J*₂=1.65Hz, 1H), 7.13(d *J*=3.43Hz, 1H), 7.124-7.42(m, 4H), 7.50(s, 1H), 7.63(s, 1H), **MASS(ES+) :** 647.7 |
| IC22 | 1.09(d, *J*=7.04Hz, 3H), 1.20(s,3H), 1.25-1.97(m, 8H), 2.03-2.57(m, 4H), 2.89-2.93(m, 1H), 4.33(d, *J*=6.78Hz, 1H), 4.95(d, *J*=10.92Hz, 1H), 5.05(d, *J*=10.89Hz, 1H), 5.12(m, 1H), 5.30.5.30(m, 1H), 6.30-6.33(m, 2H), 6.54(dd, *J*₁=3.39Hz, *J*₂=1.63Hz 1H), 6.74-6.78(m, 2H), 7.H(d, *J*=3.36Hz, 1H), 7.26(d, *J*=10.07Hz, 1H), 7.66(s, 1H), MASS(ES+) : 667.0 |
| IC23 | 1.10(d, *J*=7.05Hz, 3H). 1.22(s, 3H), 1.36-1.42(m, 1H), 1.55(s, 3H), 1.72-2.00(m, 4H), 2.29-2.52(m, -4H), 2.87-2.92(m, 1H), 4.32-4.35(m, 1H), 4.93(d, *J*=11.04Hz, 1H), 4.97(d, *J*=11.07Hz, 1H), 5.05-5.06(m, 1H), 5.30-5.52(m, 1H), 6.33(dd, *J*₁=10.09Hz, *J*₂=1.88Hz, 1H), 6.35(s, 1H), 6.54(dd, *J*₁=3.49Hz, *J*₂=1.72Hz 1H), 7.13(d, *J*=3.39Hz 1H), 7.26(dd, *J*₁=10.07Hz, *J*₂=1.10Hz, 1H), 7.51-7.69(m, 4H), 7.74(s, 1H), MASS(ES+) : 681.5 |

### Example 30 : Pharmacological Activity:

### (a) Steroid Receptor Binding Assays:

The steroid receptor binding assay were performed by MDS Pharma Services at their Taipei (Taiwan) pharmacology laboratory.

### (i) Glucocorticoid Receptor Binding Assay

The activity of test compounds on glucocorticoid receptor was assessed using radioligand binding assays. The Human HeLa cells transfected with glucocorticoid receptors were incubated with [³H]dexamethasone at 25°C for 2hrs in RPMI-1640, 10nM HEPES, pH 7.2 incubation medium, in the absence or presence of test compounds at 3nM and/or 10nM concentration. Free [³H]dexamethasone was removed from the medium by centrifugation and concentration of receptor bound ligand was determined in the supernatant by liquid scintillation counting.

Dexamethasone as reference compound was tested concurrently as an integral part of each assay to ensure the validity of the results obtained. The % inhibition was evaluated using a single concentration of test compound and the corresponding radiolabeled ligand. Results are presented as the percent inhibition of specific binding. The values are average of duplicate determinations for each concentration.

**Table 7: Glucocoticoid Receptor binding (in vitro) screen**

| **Compound No.** | **Glucocorticoid receptor binding % inhibition** |
|---|---|
| IA.143 | 58^{b} |
| IA.161 | 55^{b} |
| IA.157 | 56^{b} |
| IA.159 | 36^{b} |
| IA.168 | 40^{a} |
| IB.2 | 55^{a} |
| IB.4 | 88^{b} |
| IB.6 | 92^{b} |
| IB.7 | 82^{b} |
| IB.8 | 93^{b} |
| IB.10 | 82^{b} |
| IB.13 | 79^{b} |
| IB.14 | 85^{b} |
| IB.15 | 83^{b} |
| IB.50 | 74^{b} |
| IB.51 | 67^{b} |
| IB.54 | 82^{b} |
| IB.66 | 72^{b} |
| IB.67 | 77^{b} |
| IB.70 | 43^{a} |
| IB.73 | 45^{a} |
| IB.88 | 46³ |
| IB.100 | 45^{a} |
| IB.101 | 45^{a} |
| IB.103 | 78^{b} |
| IB.104 | 72^{b} |
| IB.105 | 42^{a} |
| IC.3 | 72^{c} |
| IC.4 | 73^{b} |
| IC.5 | 75^{c} |
| IC.7 | 41^{b} |
| Fluticasone propionate | 88^{b} |
| Budesonide | 85^{b} |

| | |
|---|---|
| *^{a} %Inhibition at 3nM:^{b}* % *Inhibition at 10nM; ^{c} % Inhibition at 100nM*. | |

### (b) Croton oil ear edema

Mice (male, CD-1) were made into different groups. Test compounds were dissolved in pyridine-water-acetone (4:2:14) solution 10µl of compound or vehicle solution was applied to the left ear of the mice. The right ear of each mouse was applied simultaneously with 10µl of pyridine: water: acetone (4:2:14) solution. After 2hrs of compound or vehicle application, 10µl of croton oil solution was applied to the left ear of all animals. After 6hr of croton oil treatment the animals were sacrificed; ears were excised and weighed separately.

**Table 8: Croton oil Ear Edema Screen**

| **Compound No.** | **Mean % Inhibition** |
|---|---|
| IA.3 | 45.31^{a} |
| IA.56 | 34.59^{a} |
| IA.184 | 49.06^{a} |
| IA.185 | 49.06^{a} |
| IB.20 | 61.49^{a} |
| IB.33 | 76.16^{a} |
| IB.34 | 64.39^{a} |
| IB.35 | 60.18^{a} |
| IB.37 | 57.12^{a} |
| IB.44 | 75.19^{a} |
| IB.46 | 75.97^{a} |
| IB.49 | 71.82^{a} |
| IB.52 | 78.12^{a} |
| IB.53 | 71.19^{a} |
| IB.55 | 74.78^{a} |
| IB.57 | 74.43^{a} |
| IB.64 | 72.75^{a} |
| IB 73 | 76.24^{a} |
| IB.77 | 71.71^{a} |
| IB.81 | 77.75^{a} |
| IB.98 | 73.71^{b} |
| IB.102 | 73.63^{b} |
| IB.112 | 66.94^{b} |
| IB.138 | 72.48^{b} |
| IB.150 | 77.76^{b} |
| IB.159 | 66.02^{b} |
| IC.13 | 41.79^{a} |
| IC.14 | 61.46^{a} |
| Fluticasone propionate | 81.09^{a} |
| Dexamethasone | 50.05^{a} |

| | |
|---|---|
| *" Inhibition at 1.6µg: ^{b} Inhibition at 0.5µg* | |

### (c) Cotton pellet granuloma

SD rats were used for assessing anti-inflammatory activity of test compound using cotton pellet granuloma method. Sterilized cotton pellets weighing 20mg were prepared. Test compounds were dissolved in acetone so as to get the required quantity for each cotton pellet in 500µl. Different pellets were impregnated with 500µl of acetone to contain required quantity of test compounds and allowed to dry. Vehicle control pellets were impregnated with 500µl acetone. Rats were made into different groups. Two cotton pellets were surgically implanted into the scapular region of each rat. On sixth day of such implantation, pellets along with granuloma were taken out. Thymus gland was also separated from each animal and weighed. The pellets with granuloma were dried for 20 hrs at 60°C and weighed. Mean weights of dry granuloma and thymus, body weight gain/100g body weights were calculated.

**Table 9: Cotton pellet granuloma test**

| **Compound No.** | **Dose (mcg)** | **Mean % Inhibition of Granuloma** | **Thymus wt.* (% Decrease)** |
|---|---|---|---|
| IA.3 | 1000 | 74.76 | -11.72 |
| IA.6 | 1000 | 61.25 | -6.95 |
| IA.11 | 1000 | 61.36 | -4.72 |
| IA.13 | 1000 | 62.52 | 6.46 |
| IA.14 | 1000 | 70.74 | -13.26 |
| IA-20 | 1000 | 67.57 | 0.73 |
| IA.27 | 10 | 31.28 | 8.71 |
| | 1000 | 69.84 | 11.74 |
| IA.28 | 1000 | 73.73 | 13.5 |
| IA.35 | 1000 | 67.16 | -11.43 |
| IA.36 | 1000 | 70.74 | -13.26 |
| IA.38 | 10 | 67.17 | -1.28 |
| IA.42 | 1000 | 65.63 | -1.33 |
| IA.43 | 10 | 27.72 | -0.55 |
| | 1000 | 72.03 | 1.47 |
| IA.44 | 1000 | 71.79 | 4.58 |
| IA.45 | 1000 | 80.06 | -4.46 |
| IA.58 | 10 | 59.00 | 6.00 |
| IA.59 | 1000 | 76.86 | -2.51 |
| IA.122 | 3 | 37.29 | 2.25 |
| IA.136 | 10 | 37.74 | 4.44 |
| | 1000 | 67.79 | 2.14 |
| IA.137 | 1000 | 65.58 | 1.59 |
| IA.138 | 1000 | 66.16 | 15.45 |
| IA.140 | 1000 | 62.54 | -36.16 |
| IA.141 | 1000 | 63.1 | -18.26 |
| IA.142 | 10 | 45.36 | -8.43 |
| IA.197 | 1000 | 79.04 | -8.89 |
| IB.5 | 10 | 39.79 | -0.39 |
| IB.7 | 1000 | 78.82 | 14.89 |
| IB.16 | 3 | 68.92 | -11.06 |
| | 1000 | 77.36 | -11.65 |
| IB.163 | 3 | 45.8 | -5.44 |
| | 10 | 76.13 | 13.77 |
| IC.6 | 1000 | 84.96 | 10.96 |
| IC.8 | 3 | 45.15 | 3.38 |
| IC.9 | 3 | 34.3 | 2.51 |
| Fluticasone propionate | 1000 | 75.03 | 82.36 |
| Dexamethasone | 1000 | 44.73 | 68.66 |

| | | | |
|---|---|---|---|
| ** In all the compounds (other than the reference compounds) the thymus weight change and the body weight gain were statistically insignificant when compared with vehicle control* | | | |

### (d) Sephadex-Induced Lung Edema in the SD Rat

Sephadex G-200 was prepared in sterile saline (10mg/ml) and allowed to swell for at least 3 days at room temperature. Test compounds were prepared as suspensions by grinding and sonicating the solid in chilled saline. All the compounds were administered intratracheally at 24hrs and 2hrs prior to intratracheal administration of Sephadex (5 mg/kg) under light ether anesthesia. Vehicle control. animals were administered vehicle instead of Sepahdex. At 24hr after Sephadex instillation, lung and thymus were excised from individual animals and weighed. Wet weight of lung and thymus was corrected for 100g of initial body weight. The percentage increase in lung weight caused by Sephadex and its inhibitions by the compounds, were calculated. The percentage inhibitions of thymus compared to vehicle control group were also calculated.

**Table 10: Sephadex-Induced Lung Edema in the SD Rat**

| **Compound No.** | **Dose (mcg/kg)** | **Mean % inhibition*** | |
|---|---|---|---|
| | | **Lung edema** | **Thymus** |
| IA.168 | 3000 | 95.3 | 9.2 |
| IB.15 | 1000 | 70.3 | 4.5 |
| IB.15 | 3000 | 71.1 | 6.4 |
| IB.51 | 3000 | 120.5 | 29.8 |
| IB.54 | 3000 | 110.4 | 29.7 |
| IB.64 | 1000 | 86.4 | 2.0 |
| IB.64 | 3000 | 104.6 | 20.0 |
| IB.67 | 3000 | 121.8 | 33.4 |
| IB.79 | 3000 | 99.3 | 15.2 |
| IB.94 | 3000 | 99.7 | 8.1 |
| IB.100 | 3000 | 120.8 | 36.0 |
| IB.101 | 3000 | 114.8 | 8.9 |
| IB.150 | 3000 | 106.2 | 18.0 |
| Budesonide | 3000 | 119.38 | 67.08 |

| | | | |
|---|---|---|---|
| ** In all the compounds (other than the reference compounds) the body weight gain was statistically insignificant when compared with vehicle control* | | | |

**Table 10a: Therapeutic Index for Compounds in Sephadex Lung Edema model in SD Rat**

| **Comp No.** | **ED50 Lung edema (mg/kg, i.t.)** | **ED₅₀ Thymus (mg/kg, i.t.)** | **Therapeutic index ED₅₀ Thymus involution/ ED₅₀ Lung edema** |
|---|---|---|---|
| IB.51 | 0.641 | 13.31 | 20.76 |
| IB.67 | 0.232 | 8.84 | 38.10 |
| IB.70 | 0.323 | 7.67 | 23.75 |
| IB.88 | 0.300 | 4.09 | 13.63 |
| Budesonide | 0.101 | 0.68 | 6.73 |
| Fluticasone propionate | 0.086 | 0.36 | 4.18 |

### (e) Liver Glycogen Deposition in Rats

**Male** Sprague Dawley rats, weighing 190-220g were bilaterally adrenalectomized and maintained on 0.9% saline solution throughout the experiment. On day 5th and 6th of adrenalectomy, animals were made into different groups, test compounds prepared in cold saline (sonicated for 30min) were instilled intratracheally 2 doses @ 3mg/kg, 24hrs apart. At 15hrs after the last treatment, animals were sacrificed and livers were excised and weighed. The glycogen content of the livers was determined by anthrone method. Briefly, a weighed quantity of liver tissue was homogenized with 5% trichloroacetic acid, supernatant mixed with ethanol and kept overnight for precipitation. The glycogen thus extracted was estimated as glucose generated after acid hydrolysis by addition of anthrone reagent at 620nm. Glycogen content was expressed as mg/100g liver.

**Table 11: Liver Glycogen Deposition in Rats**

| **Comp No.** | **Dose (mg/kg)** | **Glycogen content (mg/100g liver)** | **% Inhibition of thymus** |
|---|---|---|---|
| IA.3 | 3 | -13.6 | -2.42 |
| IB.4 | 3 | -5.77 | -9.68 |
| IB.50 | 3 | -6.67 | 5.48 |
| IC.3 | 3 | -11.34 | -2.43 |
| Fluticasone propionate | 3 | 1514.65 | 70.17 |
| Mometasone furoate | 3 | 1117.32 | 43.51 |
| Budesonide | 3 | 552.77 | 65.02 |

### (f) Assessment of Systemic Activity by i.v. Route of Administration

For assessing systemic side effect on i.v. administration, cotton pellet granuloma assay (blank cotton pellet) in male SD rats was undertaken. Sterilized cotton pellets weighing 20mg were surgically implanted into the scapular region on both sides. Starting from next day of implantation, compounds dissolved in Pharmasove (Pharmasolve 66.67% in saline) or vehicle were administered intravenously at 1.6mg/kg dose for 3 successive days. On fifth day of implantation, pellets along with granuloma were taken out. The thymus gland was also separated from each animal and weighed. The pellets with granuloma were dried for 20hrs at 60°C and weighed. Mean weights of dry granuloma and thymus and body weight gain/100g body weights were compared with vehicle control groups.

The compounds of formula I of present invention did not induce any significant thymus and body weight gain suppression by i.v. administration. Further, there was no significant cotton pellet granuloma inhibition by i.v. route of administration, thus clearly indicating lack of systemic activity and hence excellent systemic safety profile for the compounds of formula I.

**Table 12: Activity by i.v. Routes of Administration**

| **Comp No.** | **Dose mcg (for 3 days), i.v.** | **Mean Inhibition of Granuloma (%)** | **Mean Thymus wt. Decrease (%)*** |
|---|---|---|---|
| IA.3 | 1600 | 3.98 | 6.37 |
| IA.11 | 1600 | 16.65 | -17.88 |
| IA.89 | 1600 | 15.90 | 6.54 |
| IC.3 | 1600 | 12.09 | -10.38 |
| IC.5 | 1600 | -22.20 | 1.67 |
| IA.143 | 1600 | -3.01 | 5.51 |
| Fluticasone propionate | 1600 | 55.62 | 75.71 |

| | | | |
|---|---|---|---|
| **The body weight gain in all the compounds was statistically not significant when compared with vehicle control* | | | |

### Conclusion

The compounds of formula I of the present invention have potent binding to the glucocorticoid receptor *in vitro*. In the *in vivo* models, which include croton oil induced ear edema, cotton pellet induced granuloma, and Sephadex induced lung edema screens, the compounds of the present invention exhibit significant anti-inflammatory activity. In the cotton pellet granuloma, the thymus involution and the body weight gain due to the compounds was either absent or insignificant even at dose levels as high as 100-1000mcg per pellet. Similar behaviour was once again observed in the case Sephadex induced lung edema models even when the compounds were administered at very high doses, clearly demonstrating that these compounds do not cause any adverse systemic effects following local administration. The absence or non-significance of the systemic side effects was further confirmed by measurement of hepatic glycogen deposition in rats after intratracheal administration, and by measurement of thymus and body weight gain suppression following i.v. administration.

Thus the compounds of formula I described in the present invention are potent anti-inflammatory glucocorticoids having insignificant or no noteworthy systemic activity even at doses significantly higher than therapeutic doses. Due to non-significant systemic effects, the compounds of the present invention have high therapeutic index for anti-inflammatory activity *vs* systemic side effects, a property which is highly desirable for therapies using glucocorticoids.

## Claims

1. A compound of formula 1, and physiologically acceptable salt thereof: 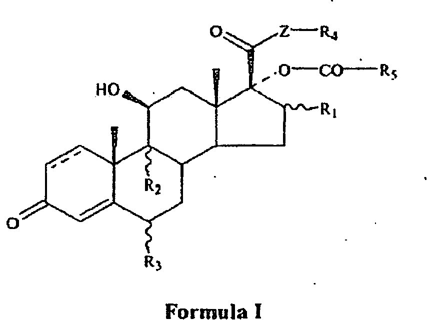 -̅-̅-̅-̅-̅-̅ represents single or double bond and 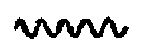 represents α or β-configuration.
wherein
Z represents O or S;
R₁ represents hydrogen or methyl which may be either in α or β-configuration, or methylene;
R₂ and R₃ are the same or different and each independently represents hydrogen, halogen or a methyl group;
R₅ represents a group selected from (C₁-C₁₀)-alkyl, (C₃-C₁₃)-cycloalkyl,
-O-(C₁-C₁₀)-alkyl, aryl or heterocyclic ring wherein the ring or ring system is unsubstituted or substituted by one or more halogen, -OH, (C₁-C₃)-alkyl, -O-(C₁-C₃)-alkyl, (C₃-C₁₃)-cycloalkyl; wherein the alkyl or cycloalkyl groups can optionally contain one or more unsaturations and/or can have one or more hetero atom incorporated therein and optionally in each case have one or more hydrogen atoms replaced by halogen, -OH, (C₁-C₃)-alkyl, -O-(C₁-C₁₃)-alkyl, (C₃-C₁₃)-cycloalkyl; and
R₄ represents moiety selected from a group consisting of A, B and C, with a proviso that when R₄ represents moiety (C), Z is S: 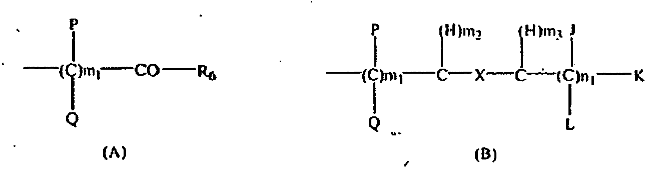 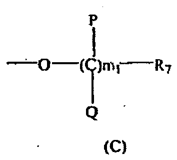 wherein
m₁ is 1, 2 or 3;
m₂ is 0 or 1;
m₃ is 0 or 1;
n₁ is 0, 1 or 2;
R₆ represents a group selected from (C₁-C₈)-alkyl, (C₃-C₁₃)-cycloalkyl, aryl and a heterocyclic radical, where the ring or ring system is unsubstituted or substituted by one or more substituents selected from (C₁-C₈)-alkyl, (C₃-C₁₃)-cycloalkyl, halogen, O-(C₁-C₈)-alkyl, O-(C₃-C₁₃)-cycloalkyl; OCO-(C₁-C₃)-alkyl, S(O)₀₋₂(C₁-C₈)-alkyl, COO-(C₁-C₈)-alkyl, -OCO-O-(C₁-C₃)-alkyl, -OCO-CO-O-(C₁-C₃)-alkyl, CONH₂, CONH-(C₁-C₈)-alkyl, CON-[(C₁-C₈)-alkyl]₂, -NHCO-(C₁-C₈)-alkyl, N-(C₁-C₈)-alkyl-CO-(C₁-C₈)-alkyl, -NHCO-O-(C₁-C₈)-alkyl, - N-(C₁-C₈)-alkyl-CO-O-(C₁-C₈)-alkyl, -NHCONH-(C₁-C₈)-alkyl, -N-(C₁-C₈)-alkyl-CONH-(C₁-C₈)-alkyl, -NHCONH-SO₂-(C₁-C₈)-alkyl; -N-(C₁-C₈)-alkyl-CONH-SO₂-(C₁-C₈)-alkyl, NO₂, -CN; wherein the alkyl or cycloalkyl groups can optionally contain one or more unsaturations and/or can have one or more hetero atom incorporated therein and optionally, in each case have one or more hydrogen atoms replaced by halogen, -OH, (C₁-C₃)-alkyl, - OCO-(C₁-C₃)-alkyl or (C₃-C₁₃)-cycloalkyl;
with a proviso that when R₄ represents moiety A wherein R₀ represents (C₁-C₁₃)-cycloalkyl optionally containing one or more hetero atom incorporated therein, the hetero atom is not nitrogen;
P and Q are independently selected from hydrogen and C₁ to C₃ alkyl;
or P and Q can be joined together with the carbon atom to which they are attached to form (C₃-C₈)-cycloalkyl as represented by moiety (A-1): 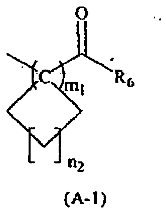 wherein m₁ is 1 and n₂ is 0,1, 2, 3, 4 or 5 and R₆ is an aryl as defined above;
or P and R₆ can be joined together to form a cyclic system as represented by moiety (A-2): 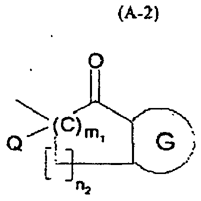 wherein m₁ is 1 and n₂ is 0, 1, 2, 3 or 4 and ring G is an aryl as defined above;
X represents either a double bond or a triple bond; and
J, K and L are independently selected from a group consisting of hydrogen, halogen, (C₁-C₁₀)-alkyl, (C₃-C₁₃)-cycloalkyl, -OH, -O-(C₁-C₁₀)-alkyl, -O-(C₃-C₁₃)-cycloalkyl, -OCO-(C₁-C₁₀)-alkyl, -OCO-(C₃-C₁₃)-cycloalkyl, -OCO-CO-O-(C₁-C₁₀)-alkyl, -OCO-CO-O-(C₃-C₁₃)-cycloalkyl, -OCO-O-(C₁-C₁₀)-alkyl, -OCO-O-(C₃-C₁₃)-cycloalkyl, -OCO-NH-(C₁-C₁₀)-alkyl, -OCO-NH-(C₃-C₁₃)-cycloalkyl, -OCO-N-[(C₁-C₁₀)-alkyl]₂, -OCO-N-[(C₃-C₁₃)-cycloalkyl]₂, -OCO-NHSO₂-(C₁-C₁₀)-alkyl, -OCO-NHSO₂-(C₃-C₁₃)-cycloalkyl, -NH₂, -NH-(C₁-C₈)-alkyl, -N-[(C₁-C₈)-alkyl]₂, -NO₂ and -CN;
wherein the alkyl or cycloalkyl groups can optionally contain, one or more unsaturations and/or can have one or more hetero atom incorporated therein and optionally, in each case have one or more hydrogen atoms replaced by halogen, -OH, (C₁-C₃)-alkyl, -O-(C₁-C₃)-alkyl, -OCO-(C₁-C₃)-alkyl, -COOH, -COO-(C₁-C₅)-alkyl, -COO-(C₁-C₅)-haloalkyl, -NHCO-(C₁-C₈)-alkyl, -ONO₂, -NH-(C₁-C₈)-alkyl, -N-[(C₁-C₈)-alkyl]₂, -NO₂, -CN, (C₃-C₁₃)-cycloalkyl, aryl or heterocyclic radical;
or J and K can be joined together with the carbon atom to which they are attached to represent a (C₃-C₁₃)-cycloalkyl or -CO- group and L is as defined above;
or J, K and L are absent when n₁ is 0;
R₇ represents a group selected from hydrogen, halogen, aryl and CO-aryl, wherein the ring or ring system is unsubstituted or substituted as defined above;

2. The compound of formula 1, and physiologically acceptable salt thereof, as claimed in claim 1, wherein R₄ represents moiety (A). 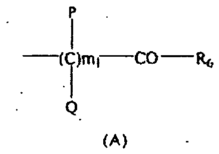 wherein m₁, P, Q and R₆ are as defined in claim 1.

3. The compound of formula I, and physiologically acceptable salt thereof, as claimed in claim 1, wherein R₄ represents moiety (B). 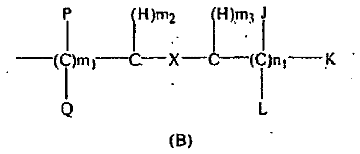 wherein m₁, m₂, m₃, n₁, P, Q, J, K, L and X are as defined in claim 1.

4. The compound of formula 1, and physiologically acceptable salt thereof, as claimed in claim 1, wherein R₄ represents moiety (C). 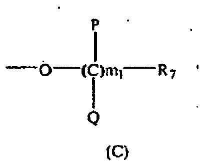 wherein m₁, P, Q and R₇ are as defined in claim 1.

5. A compound of formula I-B, and physiologically acceptable salt thereof: 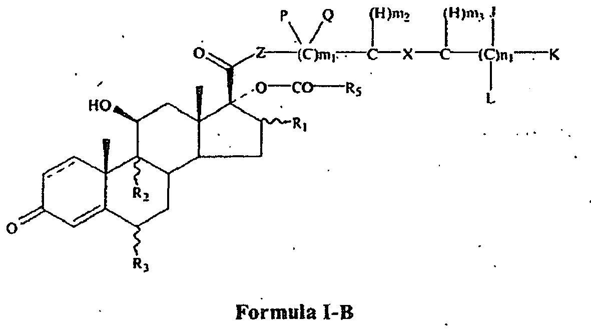 wherein
Z represents O or S;
R₁ represents hydrogen or methyl which may be either in α or β-configuration, or methylene; R₂ and R₃ are the same or different and each independently represents hydrogen, halogen or a methyl group;
R₅ represents a group selected from (C₁-C₁₀)-alkyl, (C₃-C₁₃)-cycloalkyl,
-O-(C₁-C₁₀)-alkyl, aryl or heterocyclic ring wherein the ring or ring system is unsubstituted or substituted by one or more halogen, -OH, (C₁-C₃)-alkyl, -O-(C₁-C₃)-alkyl, (C₃-C₁₃)-cycloalkyl; wherein the alkyl or cycloalkyl groups can optionally contain one or more unsaturations and/or can have one or more hetero atom incorporated therein and optionally in each case have one or more hydrogen atoms replaced by halogen, -OH, (C₁-C₃)-atky). -O-(C₁-C₃)-alkyl, (C₁-C₁₃)-cycloalkyl and
m₁ is 1;
m₂ is 0 or 1;
m₃ is 0 or 1;
n₁ is 0, 1 or 2;
P and Q are hydrogen;
X represents either a double bond or a triple bond; and
J, K and L are independently selected from a group consisting of hydrogen, halogen, (C₁-C₁₀)-alkyl, (C₃-C₁₃)-cycloalkyl, -OH, -O-(C₁-C₁₀)-alkyl, -O-(C₃-C₁₃)-cycloalkyl, -OCO-(C₁-C₁₀)-alkyl, -OCO-(C₃-C₁₃)-cycloalkyl, -OCO-CO-O-(C₁-C₁₀)-alkyl, -OCO-CO-O-(C₃-C₁₃)-cycloalkyl, -OCO-O-(C₁-C₁₀)-alkyl, -OCO-O-(C₃-C₁₃)-cycloalkyl, -OCO-NH-(C₁-C₁₀)-alkyl, -OCO-NH-(C₃-C₁₃)-cycloalkyl, -OCO-N-[(C₁-C₁₀)-alkyl]₂, -OCO-N-[(C₃-C₁₃)-cycloalkyl]₂, - OCO-NHSO₂-(C₁-C₁₀)-alkyl, -OCO-NHSO₂-(C₃-C₁₃)-cycloalkyl, -NH₂, -NH-(C₁-C₈)-alkyl,-N-[(C₁-C₈)-alkyl]₂, -NO₂ and -CN.

6. A compound of formula I-B, and physiologically acceptable salt thereof: 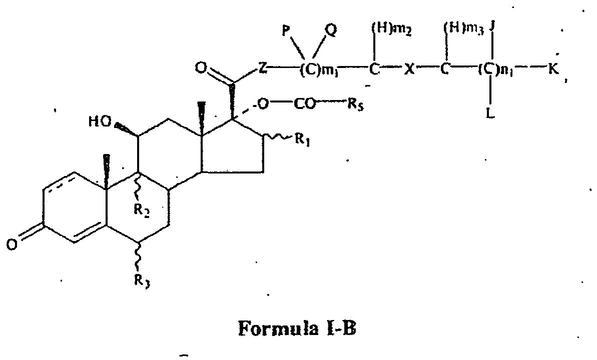 wherein
Z represents S;
R₁ represents hydrogen or methyl which may be either in α or β-configuration, or ethylene;
R₂ and R₃ are the same or different and each independently represents hydrogen, halogen or a methyl group;
R₅ represents a group selected from (C₁-C₁₀)-alkyl, (C₃-C₁₃)-cycloalkyl,
-O-(C₁-C₁₀)-alkyl, aryl or heterocyclic ring wherein the ring or ring system is unsubstituted or substituted by one or more halogen, -OH, (C₁-C₃)-alkyl, -O-(C₁-C₃)-alkyl, (C₃-C₁₃)-cycloalkyl; wherein the alkyl or cycloalkyl groups can optionally contain one or more unsaturations and/or can have one or more hetero atom incorporated therein and optionally in each case have one or more hydrogen atoms replaced by halogen, -OH, (C₁-C₃)-alkyl, -O-(C₁-C₃)-alkyl, (C₃-C₁₃)-cycloalkyl and
m₁ is 1;
m₂ is 0;
m₃ is 0;
n₁ is 0, 1 or 2;
P and Q are hydrogen;
X represents a triple bond, and
J, K and L are independently selected from a group consisting of hydrogen, halogen, (C₁-C₁₀)-alkyl, (C₃-C₁₃)-cycloalkyl, -OH, -O-(C₁-C₁₀)-alkyl, -O-(C₃-C₁₃)-cycloalkyl, -OCO-(C₁-C₁₀)-alkyl, -OCO-(C₃-C₁₃)-cycloalkyl, -OCO-CO-O-(C₁-C₁₀)-alkyl, -OCO-CO-O-(C₃-C₁₃)-cycloalkyl, -OCO-O-(C₁-C₁₀)-alkyl, -OCO-O-(C₃-C₁₃)-cycloalkyl, -OCO-NH-(C₁-C₁₀)-alkyl, -OCO-NH-(C₃-C₁₃)-cycloalkyl, -OCO-N-[(C₁-C₁₀)-alkyl]₂, -OCO-N-[(C₃-C₁₃)-cycloalkyl]₂,-OCO-NHSO₂-(C₁-C₁₀)-alkyl, -OCO-NHSO₂-(C₃-C₁₃)-cycloalkyl, -NH₂, -NH-(C₁-C₈)-alkyl, - N-[(C₁-C₈)-alkyl]₂, -NO₂ and -CN.

7. The compound as claimed in claim 1 selected from the group consisting of
*S*-[2-(4-Chlorophenyl)-2-oxoethyl] 6α,9α-difluoro-11β-hydroxy-16α-methyl-3-oxo-17α-propionyloxyandrosta-1,4-diene-17β-carbothioate,
2-(4-Fluorophenyl)-2-oxoethyl 6α,9α-difluoro-11β-hydroxy-16α-methyl-3-oxo-17α-propionyloxyandrosta-1,4-diene-17β-carboxylate,
*S*-[2-(4-Fluorophenyl)-2-oxoethyl] 6α,9α-difluoro-11β-hydroxy-16α-methyl-3-oxo-17α-propionyloxyandrosta-1,4-diene-17β-carbothioate,
*S*-[2-(2,4-Difluorophenyl)-2-oxoethyl] 6α,9α-difluoro-11β-hydroxy-16α-methyl-3-oxo-17α-propionyloxyandrosta-1,4-diene-17β-carbothioate,
*S*-[2-(2,4-Dichlorophenyl)-2-oxoethyl] 6α,9α-difluoro-11β-hydroxy-16α-methyl-3-oxo-17α-propionyloxyandrosta-1,4-diene-17β-carbothioate,
*S*-[2-(3,4-Dichlorophenyl)-2-oxoethyl] 6α,9α-difluoro-11β-hydroxy-16α-methyl-3-oxo-17α-propionyloxyandrosta-1,4-diene-17β-carbothioate,
*S*-[2-(5-Chlorothiophen-2-yl)-2-oxoethyl] 6α,9α-difluoro-11β-hydroxy-16α-methyl-3-oxo-17α-propionyloxyandrosta-,1,4-diene-17β-carboxylate,
*S*-[2-Oxopropyl]6α,9α-difluoro-11β-hydroxy-16α-methyl-3-oxo-17α-propionyloxyandrosta-1,4-diene-17β-carbothioate,
2-Oxopropyl 6α,9α-difluoro-11β-hydroxy-16α-methyl-3-oxo-17α-propionyloxyandrosta-1,4-dien-17β-carboxylate,
*S*-[2-(4-Methoxyphenyl)-2-oxoethyl] 6α,9α-difluoro-11β-hydroxy-16α-methyl-3-oxo-17α-propionyloxyandrosta-1,4-diene-17β-carbothioate,
*S*-[2-oxo-2-(4-trifluoromethyloxyphenyl)ethyl] 6α,9α-difluoro-11β-hydroxy-16α-methyl-3-oxo-17α-propionyloxyandrosta-1,4-diene-17β-carbothioate,
*S*-[2-Oxo-2-(4-trifluoromeihylphenyl)ethyl] 6α,9α-difluoro-11β-hydroxy-16α-methyl-3-oxo-17α-propionyloxyandrosta-1,4-diene-17β-carbothioate,
2-(4-Methanesulphonylphenyl)-2-oxoethyl 6α,9α-difluoro-11β-hydroxy-16α-methyl-3-oxo-17α-propionyloxyandrosta-1,4-diene-17β-carboxylate,
*S*-[2-(4-Methanesulphonyiphenyl)-2-oxoethyl] 6α,9α-difluoro-11β-hydroxy-16α-methyl-3-oxo-17α-propionyloxyandrosta-1,4-diene-17β-carbothioate,
2-Oxo-2-phenylethyl 6α,9α-difluoro-11β-hydroxy-16α-methyl-3-oxo-17α-propionyloxyandrosta-1,4-diene-17β-carboxylate,
*S-*[2-Oxo-2-phenylethyl] 6α,9α-difluoro-11β-hydroxy-16α-methyl-3-oxo-17α-propionyloxyandrosta-1,4-diene-17β-carbothioate,
*S*-[2-Oxo-2-(2,4,6-trimethylphenyl)ethyl] 6α,9α-difluoro-11β-hydroxy-16α-methyl-3-oxo-17α-propionyloxyandrosta-1,4-diene-17β-carbothioate,
*S*-[1,1-Dimethyl-2-oxo-2-phenylethyl] 6α,9α-difluoro-11β-hydroxy-16α-methyl-3-oxo-17α-propionyloxyandrosta-1,4-diene-17β-carbothioate,
*S*-[2-(4-Methoxyphenyl)-2-oxoethyl] 6α,9α-difluoro-11β-hydroxy-16α-methyl-17α-(2-methylpropionyloxy)-3-oxoandrosta-1,4-diene-17β-carbothioate,
*S*-[2-Oxo-2-(4-trifluoromethylphenyl)ethyl] 17α-cyclobutylcarbonyloxy-6α,9α-difluoro-11β-hydroxy-16α-methyl-3-oxoandrosta-1,4-diene-17β-carbothioate,
*S*-[2-(2,4-Difluorophenyl)-2-oxoethyl] 17α-cyclobutylcarbonyloxy-6α,9α-difluoro-11β-hydroxy-16α-methyl-3-oxoandrosta-1,4-diene-17β-carbothioate,
*S*-[2-(4-Methoxyphenyl)-2-oxoethyl] 17α-cyclobutylcarbonyloxy-6α,9α-difluoro-11β-hydroxy-16α-methyl-3-oxoandrosta-1,4-diene-17β-carbothioate,
*S*-[2-(2,4-Difluorophenyl)-2-oxoethyl] 9α-fluoro-17α-[(2-furanylcarbonyl)oxy]-11β-hydroxy-16α-methyl-3-oxoandrosta-1,4-diene-17β-carbothioate.
*S*-[2-(4-Chlorophenyl)-2-oxoethyl] 9α-fluoro-17α-[(2-furanylcarbonyl)oxy]-11β-hydroxy-16α-methyl-3-oxoandrosta-1,4-diene-17β-carbothioate,
*S*-[2-(4-Methoxyphenyl)-2-oxoethyl] 9α-fluoro-17α-[(2-furanylcarboxyl)oxy]-11β-hydroxy-16α-methyl-3-oxoandrosta-1,4-diene-17β-carbothioate,
*S*-[2-(4-Fluorophenyl)-2-oxoethyl]9α-fluoro-17α-[(2-furanylcarbonyl)oxy]-11β-hydroxy-16α-methyl-3-oxoandrosta-1,4-diene-17β-carbothioate,
*S*-[2-(4-Chlorophenyl)-2-oxoethyl] 6α,9α-difluoro-17α-[(2-furanylcarbonyl)oxy]-11β-hydroxy-16α-methyl-3-oxoandrosta-1,4-diene-17β-carbothioate,
*S*-[2-Oxo-2-(4-trifluoromethylphenyl)ethyl] 6α,9α-difluoro-11β-hydroxy-16α-methyl-3-oxo-17α-[(2-thienylcarbonyl)oxy]androsta-1,4-diene-17β-carbothioate,
*S*-[2-(3,4-Dimethylphenyl)-2-oxoethyl] 6α,9α-difluoro-11β-hydroxy-16α-methyl-3-oxo-17α-propionyloxyandrosta-1,4-diene-17β-carbothioate,
*S*-[2-(3-Chlorophenyl)-2-oxoethyl] 6α,9α-difluoro-17α-[(2-furanylcarbonyloxy]-11β-hydroxy-16α-methyl-3-oxoandrosta-1,4-diene-17β-carbothioate,
*S*-[2-Oxo-2-(4-trifluoromethylphenyl)ethyl] 6α,9α-difluoro-17α-[(2-furanylcarbonyl)oxy]-11β-hydroxy-16β-methyl-3-oxoandrosta-1,4-diene-17β-carbothioate.
*S*-(4-Hydroxy-but-2-ynyl) 6α,9α-difluoro-17α-[(2-furanylcarbonyl)oxy]-11β-hydroxy-16α-methyl-3-oxoandrosta-1,4-diene-17β-carbothioate,
*S*-(4-*n*-Propylcarbonyloxy-but-2-ynyl) 6α,9α-difluoro-11β-hydroxy-16α-methyl-3-oxo-17α-propionyloxyandrosta-1,4-diene-17β-carbothioate,
*S*-[4-(2-Ethoxy-1,2-dioxoethyly-(*E*)-but-2-enyl] 6α,9α-difluoro-11β-hydroxy-16α-methyl-3-oxo-17α-propionyloxyandrosta-1,4-diene-17β-carbothioate,
*S*-[4-(2-Ethoxy-1,2-dioxoethyl)oxy-(*E*)-but-2-enyl] 6α,9α-difluoro-17α-[(2-furanyl-carbonyl)oxy]-11β-hydroxy-16α-methyl-3-oxoandrosta-1,4-diene-17β-carbothioate,
*S*-[4-*n*-Propylcarbonyloxy-but-2-ynyl] 6α,9α-difluoro-17α-[(2-furanylcarbonyl)oxy]-11β-hydroxy-16α-methyl-3-oxoandrosta-1,4-diene-17β-carbothioate,
*S*-[4-(2-Methylpropionyloxy)-but-2-ynyl] 6α-9α-difluoro-17α-[(2-furanylcarbonyl)oxy]-11β-hydroxy-16α-methyl-3-oxoandrosta-1,4-diene-17β-carbothioate,
*S*-[4-(2,2-Dimethylpropionyloxy)-but-2-ynyl]6α,9α-difluoro-17α-[(2-furanyl-carbonyl)oxy]-11β-hydroxy-16α-methyl-3-oxoandrosta-1,4-diene-17β-carbothioate,
*S*-[4-(2,2-Dimethylpropionyloxy)-but-2-ynyl] 6α,9α-difluoro-11β-hydroxy-16α-methyl-3-oxo-17α-propionyloxyandrosta-1,4-diene-17β-carbothioate,
*S*-[4-*n*-Propylcarbonyloxy-(Z)-but-2-enyl] 6α,9α-difluoro-17α-[(2-furanylcarbonyl)oxy]-11β-hydroxy-16α-methyl-3-oxoandrosta-1,4-diene-17β-carbothioate,
*S*-[4-(2-Ethoxy-1,2-dioxoethyl)oxy-but-2-ynyl] 6α,9α-difluoro-17α-[(2-furanyl-carbonyl)oxy]-11β-hydroxy-16α-methyl-3-oxoandrosta-1,4-diene-17β-carbothioate.
*S*-[4-n-Propylcarbonyloxy-but-2-ynyl] 6α,9α-difluoro-11β-hydroxy-16α-methyl-3-oxo-17α-[(2-thienylcarbony)oxy]androsta-1,4-diene-17β-carbothioate,
*S*-[(4-Cyclopropylcarbonyloxy)-but-2-ynyl] 6α,9α-difluoro-17α-[(2-furanylcarbonyl)oxy]-11β-hydroxy-16α-methyl-3-oxoandrosta-1,4-diene-17β-carbothioate,
*S*-[(4-Cyclobutylcarbonyloxy)-but-2-ynyl] 6α,9α-difluoro-17α-[(2-furanylcarbonyl)oxy]-11β-hydroxy-16α-methyl-3-oxoandrosta-1,4-diene-17β-carbothioate,
*S*-[(4-Cyclopentylcarbonyloxy)-but-2-ynyl] 6α,9α-difluoro-17α-[(2-furanylcarbonyl)oxy]-11β-hydroxy-16α-methyl-3-oxoandrosta-1,4-diene-17β-carbothioate,
*S*-[4-n-Propylcarbonyloxy-(*E*)-but-2-enyl]-6α,9α-difluoro-17α-[(2-furanylcarbonyl)oxy]-11β-hydroxy-16α-methyl-3-oxoandrosta-1,4-diene-17β-carbothioate,
*S*-[(4-Methoxycarbonyloxy)-but-2-ynyl] 6α,9α-difluoro-17α-[(2-furanylcarbonyl)oxy]-11β-hydroxy-16α-methyl-3-oxoandrosta-1,4-diene-17β-carbothioate,
*S*-[(4-Ethoxycarbonyloxy)-but-2-ynyl] 6α,9α-difluoro-17α-[(2-furanylcarbonyl)oxy]-11β-hydroxy-16α-methyl-3-oxoandrosta-1,4-diene-17β-carbothioate,
*S*-[4-(2,2-Dimethylethyloxycarbonyloxy)-but-2-ynyl] 6α,9α-difluoro-17α-[(2-furanyl-carbonyl)oxy]-11β-hydroxy-16α-methyl-3-oxoandrosta-1,4-diene-17β-carbothiaate,
*S*-[4-(2-Methylpropyloxycarbony(oxy)-but-2-ynyl] 6α,9α-difluoro-17α-[(2-furanyl-carbonyl)oxy]-11β-hydroxy-16α-methyl-3-oxoandrosta-1,4-diene-17β-carbothioate,
*S*-[4-(3-Chloropropylcarbonyloxy)-but-2-ynyl] 6α,9α-difluoro-17α-[(2-furanylcarbonyl)-oxy]-11β-hydroxy-16α-methyl-3-oxoandrosta-1,4-diene-17β-carbothioate,
*S*-[4-Dichloroacetoxy-but-2-ynyl] 6α,9α-difluoro-17α-[(2-furanylcarbonyl)oxy]-11β-hydroxy-16α-methyl-3-oxoandrosta-1,4-diene-17β-carbothioate,
*S*-[4-(2-Methylpropylcarbonyloxy)-but-2-ynyl] 6α,9α-difluoro-17α-[(2-furanylcarbonyl)-oxy]-11β-hydroxy-16α-methyl-3-oxoandrosta-1,4-diene-17β-carbothioate,
*S*-[4-(3,3-Dimethylpropylcarbonyloxy)-but-2-ynyl] 6α,9α-difluoro-17α-[(2-furanyl-carbonyl)oxy]-11β-hydroxy-16α-methyl-3-oxoandrosta-1,4-diene-17β-carbothioate,
*S*-(4-Propionyloxy-but-2-ynyl) 6α,9α-difluoro-17α-[(2-furanylcarbonyl)oxy]-11β-hydroxy-16α-methyl-3-oxoandrosta-1,4-diene-17β-carbothioate,
*S*-[4-(3,3,3-Trifluoropropionyloxy)-but-2-ynyl] 6α,9α-difluoro-17α-[(2-furanylcarbonyl)-oxy]-11β-hydroxy-16α-methyl-3-oxoandrosta-1,4-diene-17β-carbothioate,
*S*-[4-(Methoxyacetyloxy)-but-2-ynyl] 6α,9α-difluoro-17α-[(2-furanylcarbonyl)oxy]-11β-hydroxy-16α-methyl-3-oxoandrosta-1,4-diene-17β-carbothioate,
*S*-[4-(Ethoxyacethoxy)-but-2-ynyl] 6α,9α-difluoro-17α-[(2-furanylcarbonyl)oxy]-11β-hydroxy-16α-methyl-3-oxoandrosta-1,4-diene-17β-carbothioale,
*S*-(4-Acetoxy-but-2-ynyl) 6α,9α-difluoro-17α-[(2-furanylcarbonyl)oxy]-11β-hydroxy-16α-methyl-3-oxoandrosta-1,4-diene-17β-carbothioate,
*S*-[4-(2,2-Dimethylbutylcarbonyloxy)-but-2-ynyl] 6α,9α-difluoro-17α-[(2-furanylcarbonyl)-oxy]-11β-hydroxy-16α-methyl-3-oxoandrosta-1,4-diene-17β-carbothioate,
*S*-[4-((1*S*)-Camphanylcarbonyloxy)-but-2-ynyl] 6α,9α-difluoro-17α-[(2-furanylcarbonyl)-oxy]-11β-hydroxy-16α-methyl-3-oxoandrosta-1,4-diene-17β-carbothioate,
*S*-[4-(4-Ethoxybutyl-1,4-dione)oxy-but-2-ynyl] 6α,9α-difluoro-17α-[(2-furanylcarbonyl)-oxy]-11β-hydroxy-16α-methyl-3-oxoandrosta-1,4-diene-17β-carbothioate.
*S*-[4-(4-Ethyloxy-(*Z*)-but-2-en-1,4-dione)oxy-but-2-ynyl] 6α,9α-difluoro-17α-[(2-furanyl-carbonyl)oxy]-11β-hydroxy-16α-methyl-3-oxoandrosta-1,4-diene-17β-carbothioate,
*S*-[4-(4-Methoxybutyl-1,4-dione)oxy-but-2-ynyl] 6α,9α-difluoro-17α-[(2-furanylcarbonyl)-oxy]-11β-hydroxy-16α-methyl-3-oxoandrosta-1,4-diene-17β-carbothioate,
*S*-[4-(4-Isopropyloxybutyl-1,4-dione)oxy-but-2-ynyl] 6α,9α-difluoro-11β-hydroxy-16α-methyl-17α-[(2-furanylcarbonyl)oxy]-3-oxoandrosta-1,4-diene-17β-carbothioate,
*S*-[4-(4-Isobutyloxybutyl-1,4-dione)oxy-but-2-ynyl] 6α,9α-difluoro-17α-[(2-furanyl-carbonyl)oxy]-11β-hydroxy-16α-methyl-3-oxoandrosta-1,4-diene-17β-carbothioate,
*S*-[4-(4-n-Butyloxybutyl-1,4-dione)oxy-but-2-ynyl] 6α,9α-difluoro-17α-[(2-furanyl-carbonyl)oxy]-11β-hydroxy-16α-methyl-3-oxoandrosta-1,4-diene-17β-carbothioate,
*S*-[4-(3-Chloro-2,2-dimethylpropionyloxy)-but-2-ynyl] 6α,9α-difluoro-17α-[(2-furanyl-carbonyl)oxy]-11β-hydroxy-16α-methyl-3-oxoandrosta-1,4-diene-17β-carbothioate,
*S*-[4-(4-Methoxy-(*E*)-but-2-en-1,4-dione)oxy-but-2-ynyl] 6α,9α-difluoro-17α-[(2-furanyl-carbonyl)oxy]-11β-hydroxy-16α-methyl-3-oxoandrosta-1,4-diene-17β-carbothioate;
*S*-[4-(3-Methyl-1-oxobutyloxy)-but-2-ynyl] 6α,9αa-difluoro-17α-[(2-furanylcarbonyl)oxy]-11β-hydroxy-16α-methyl-3-oxoandrosta-1,4-diene-17β-carbothioate,
*S*-[4-(2-Ethyl-1-oxobutyloxy)-but-2-ynyl] 6α,9α-difluoro-17α-[(2-furanylcarbonyl)oxy]-11β-hydroxy-16α-methyl-3-oxoandrosta-1,4-diene-17β-carbothioate,
*S*-[4-(*N*,*N*-Dimethylaminocarbonyloxy)-but-2-ynyl] 6α,9α-difluoro-17α-[(2-furanyl-carbonyl)oxy]-11β-hydroxy-16α-methyl-3-oxoandrosta-1,4-diene-17β-carbothioate,
*S*-[4-(2-Chloro-2-methylpropionyloxy)-but-2-ynyl] 6α,9α-difluoro-17α-[(2-furanyl-carbonyl)oxy]-11β-hydroxy-16α-methyl-3-oxoandrosta-1,4-diene-17β-carbothioate,
*S*-[4-(4-Methyl-1-oxopentyloxy)-but-2-ynyl] 6α,9α-difluoro-17α-[(2-furanylcarbonyl)oxy]-11β-hydroxy-16α-methyl-3-oxoandrosta-1,4-diene-17β-carbothioate,
*S*-[4-(2-Hydroxy-2-methylpropionyloxy)-but-2-ynyl] 6α,9α-difluoro-17α-[(2-furanyl-carbonyl)oxy]-11β-hydroxy-16α-methyl-3-oxoandrosta-1,4-diene-17β-carbothioate,
*S*-[4-(2-Fluoro-2-methylpropionyloxy)-but-2-ynyl] 6α,9α-difluoro-17α-[(2-furanyl-carbonyl)oxy]-11β-hydroxy-16α-methyl-3-oxoandrosta-1,4-diene-17β-carbothioate,
*S*-[4-(4-Methoxy-(*Z*)-but-2-en-1,4-dione)oxy-but-2-ynyl] 6α,9α-difluoro-17α-[(2-furanyl-carbonyl)oxy]-11β-hydroxy-16α-methyl-3-oxoandrosta-1,4-diene-17β-carbothioate,
*S*-[4-(4-Ethoxy-(*E*)-but-2-en-1,4-dione)oxy-but-2-ynyl] 6α,9α-difluoro-17α-[(2-furanyl-carbonyl)oxy]-11β-hydroxy-16α-methyl-3-oxoandrosta-1,4-diene-17β-carbothioate,
*S*-[4-(4-Isobutyloxy-(*E*)-but-2-en-1,4-dione)oxy-but-2-ynyl] 6α,9α-difluoro-17α-[(2-furanylcarbonyl)oxy]-11β-hydroxy-16α-methyl-3-oxoandrosta-1,4-diene-17β-carbothioate,
*S*-(4-Acryloyloxy-but-2-ynyl) 6α,9α-difluoro-17α-[(2-furanylcarbonyl)oxy]-11β-hydroxy-16α-methyl-3-oxoandrosta-1,4-diene-17β-carbothioate,
*S*-[4-(Cyclopentylmethyloxycarbonyloxy)-but-2-ynyl] 6α,9α-difluoro17α-[(2-furanyl-carbonyl)oxy]-11β-hydroxy-16α-methyl-3-oxoandrosta-1,4-diene-17β-carbothioate,
*S*-[4-(3-Methylbut-2-enoxycarbonyloxy)-but-2-ynyl] 6α,9α-difluoro-17α-[(2-furanyl-carbonyl)oxy]-11β-hydroxy-16α-methyl-3-oxoandrosta-1,4-diene-17β-carbothioate,
*S*-[4-(*n*-Pentyloxycarbonyloxy)-but-2-ynyl] 6α,9α-difluoro-17α-[(2-furanylcarbonyl)oxy]-11β-hydroxy-16α-methyl-3-oxoandrosta-1,4-diene-17β-carbothioate,
*S*-[4-(2,2-Dimethyl-3-fluoropropionyloxy)-but-2-ynyl] 6α,9α-difluoro-17α-[(2-furanyl-carbonyloxy]-11β-hydroxy-16α-methyl-3-oxoandrosta-1,4-diene-17β-carbothioate,
*S*-[4-((*S*)-2,2-Dimethyl-1,3-dioxolan-4-yl-methoxycarbonyloxy)-but-2-ynyl] 6α,9α-difluoro-17α-[(2-furanylcarbonyl)oxy]-11β-hydroxy-16α-methyl-3-oxoandrosta-1,4-diene-17β-carbothioate,
*S*-[4-(*R*)-2,2-Dimethyl-1,3-dioxolan-4-yl-methoxycarbonyloxy)-but-2-ynyl] 6α,9α-difluoro-17α-[(2-furanylcarbonyl)oxy]-11β-hydroxy-16α-methyl-3-oxoandrosta-1,4-diene-17β-carbothioate,
*S*-[4-(Isoamyloxycarbonyloxy)-but-2-ynyl] 6α,9α-difluoro-17α-[(2-furanylcarbonyl)oxy]-11β-hydroxy-16α-methyl-3-oxoandrosta-1,4-diene-17β-carbothioate,
*S*-(4-Ethoxy-but-2-ynyl) 6α,9α-difluoro-17α-[(2-furanylcarbonyl)oxy]-11β-hydroxy-16α-methyl-3-oxoandrosta-1,4-diene-17β-carbothioate,
6α,9α-Difluoro-11β-hydroxy-16α-methyl-3-oxo-17α-propionyloxyandrosta-1,4-diene-17β-carbonylsulfenic acid fluoromethyl ester,
6α,9α-Difluoro-17α-[(2-furanylcarbonyl)oxy]-11β-hydroxy-16α-methyl-3-oxoandrosta-1,4-diene-17β-carbonylsulfenic acid fluoromethyl ester,
6α,9α-Difluoro-17α-[(2-furanylcarbonyl)oxy]-11β-hydroxy-16α-methyl-3-oxoandrosta-1,4-diene-17β-carbonylsulfenic acid (4-fluorophenyl)methyl ester,
6α,9α-Difluoro-17α-[(2-furanylcarbonyl)oxy]-11β-hydroxy-16α-methyl-3-oxoandrosta-1,4-diene-17β-carbonylsulfenic acid (4-trifluoromethylphenyl)methyl ester,
6α,9α-Difluoro-17α-[(2-furanylcarbonyl)oxy]-11β-hydroxy-16α-methyl-3-oxoandrosta-1,4-diene-17β-carbonylsulfenic acid (4-chlorophenyl)methyl ester,
6α,9α-Difluoro-11β-hydroxy-16α-methyl-3-oxo-17α-propionyloxyandrosta-1,4-diene-17β-carbonylsulfenic acid (4-trifluoromethylphenyl)methyl ester,
9α-Fluoro-17α-[(2-furanylcarbonyl)oxy]-11β-hydroxy-16α-methyl-3-oxoandrosta-1,4-diene-17β-carbonylsulfenic acid (4-fluorophenyl)methyl ester,
6α,9α-Difluoro-11β-hydroxy-16α-methyl-3-oxo-17α-propionyloxyandrosta-1,4-diene-17β-carbonylsulfenic acid methyl ester,
6α,9α-Difluoro-17α-[(2-furanylcarbonyl)oxy]-11β-hydroxy-16α-methyl-3-oxoandrosta-1,4-diene-17β-carbonylsulfenic acid methyl ester,
*S*-[4-(4-Methyl-1-oxopentyloxy)-but-2-ynyl] 6α,9α-difluoro-17α-[(2-furanylcarbonyl)-oxy]-11β-hydroxy-16α-methyl-3-oxoandrosta-1,4-diene-17β-carbothioate.

## Patentansprüche

1. Verbindung der Formel I und physiologisch unbedenkliches Salz davon: 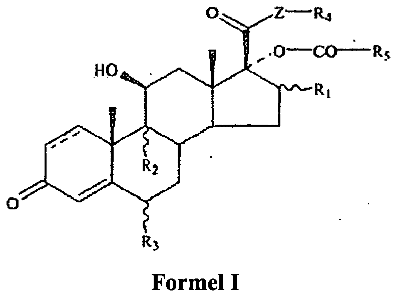 -̅-̅-̅-̅-̅-̅ steht für eine Einfach- oder Doppelbindung und 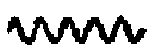 steht für eine α- oder β-Konfiguration,
wobei
Z für O oder S steht;
R₁ für Wasserstoff oder Methyl, das entweder in einer α- oder β-Konfiguration sein kann, oder Methylen steht;
R₂ und R₃ gleich oder verschieden sind und jeweils unabhängig für Wasserstoff, Halogen oder eine Methylgruppe stehen;
R₅ für eine Gruppe steht, die aus (C₁-C₁₀)-Alkyl, (C₃-C₁₃)-Cycloalkyl, -O-(C₁-C₁₀)-Alkyl, Aryl oder einem heterocyclischen Ring ausgewählt ist, wobei der Ring oder das Ringsystem unsubstituiert oder durch Halogen, -OH, (C₁-C₃)-Alkyl, -O-(C₁-C₃)-Alkyl und/oder (C₃-C₁₃)-Cycloalkyl substituiert ist; wobei die Alkyl- oder Cycloalkylgruppen gegebenenfalls eine oder mehrere Ungesättigtheiten enthalten können und/oder ein oder mehrere darin eingebundene Heteroatome aufweisen können und gegebenenfalls jeweils ein oder mehrere Wasserstoffatome aufweisen, die durch Halogen, -OH, (C₁-C₃)-Alkyl, -O-(C₁-C₃)-Alkyl, (C₃-C₁₃)-Cycloalkyl ersetzt sind; und
R₄ für eine Einheit steht, die aus einer Gruppe bestehend aus A, B und C ausgewählt ist, mit der Maßgabe, dass, wenn R₄ für eine Einheit (C) steht, Z S ist: 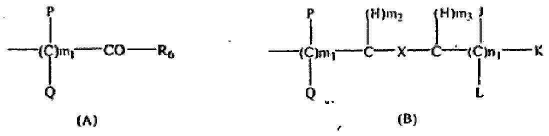 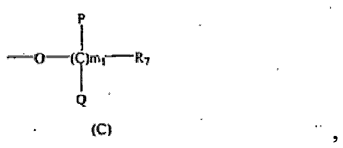 wobei
m₁ 1, 2 oder 3 ist;
m₂ 0 oder 1 ist;
m₃ 0 oder 1 ist;
n₁ 0, 1 oder 2 ist;
R₆ für eine Gruppe steht, die aus (C₁-C₈)-Alkyl, (C₃-C₁₃)-Cycloalkyl, Aryl und einem heterocyclischen Rest ausgewählt ist, wobei der Ring oder das Ringsystem unsubstituiert oder durch einen oder mehrere Substituenten substituiert ist, die aus (C₁-C₈)-Alkyl, (C₃-C₁₃)-Cycloalkyl, Halogen, O-(C₁-C₈)-Alkyl, O-(C₃-C₁₃)-Cycloalkyl, OCO-(C₁-C₃)-Alkyl, S(O)₀₋₂-(C₁-C₈)-Alkyl, COO-(C₁-C₈)-Alkyl, -OCO-O-(C₁-C₃)-Alkyl, -OCO-CO-O-(C₁-C₃)-Alkyl, CONH₂, CONH-(C₁-C₈)-Alkyl, CON-[(C₁-C₈)-Akyl]₂, -NHCO-(C₁-C₈)-Alkyl, N-(C₁-C₈)-Alkyl-CO-(C₁-C₈)-alkyl,-NHCO-O-(C₁-C₈)-Alkyl, N-(C₁-C₈)-Alkyl-CO-O-(C₁-C₈)-alkyl, -NHCONH-(C₁-C₈)-Alkyl, N-(C₁-C₈)-Akyl-CONH-(C₁-C₈)-alkyl, -NHCONH-SO₂-(C₁-C₈)-Alkyl, N-(C₁-C₈)-Alkyl-CONH-SO₂-(C₁-C₈)-alkyl, -NO₂, -CN ausgewählt sind; wobei die Alkyl-oder Cycloalkylgruppen gegebenenfalls eine oder mehrere Ungesättigtheiten enthalten können und/oder ein oder mehrere darin eingebundene Heteroatome aufweisen können und gegebenenfalls jeweils ein oder mehrere Wasserstoffatome aufweisen, die durch Halogen, -OH, (C₁-C₃)-Alkyl, -OCO-(C₁-C₃)-Alkyl oder (C₃-C₁₃)-Cycloalkyl ersetzt sind;
mit der Maßgabe, dass, wenn R₄ für eine Einheit A steht, wobei R₆ für (C₃-C₁₃)-Cycloalkyl steht, das gegebenenfalls ein oder mehrere darin eingebundene Heteroatome enthält, wobei das Heteroatom nicht Stickstoff ist;
P und Q unabhängig aus Wasserstoff und C₁- bis C₃-Alkyl ausgewählt sind
oder P und Q zusammen mit dem Kohlenstoffatom, an das sie angelagert sind, verbunden sein können, um (C₃-C₈)-Cycloalkyl zu bilden, wie durch die Einheit (A-1) dargestellt: 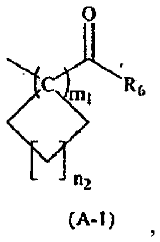 wobei m₁ 1 ist und n₂ 0, 1, 2, 3, 4 oder 5 ist und R₆ ein Aryl ist, wie oben definiert;
oder P und R₆ zusammen verbunden werden können, um ein cyclisches System zu bilden, wie durch die Einheit (A-2) dargestellt: 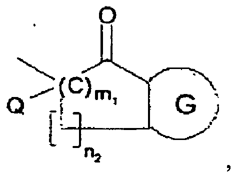 wobei m₁ 1 ist und n₂ 0, 1, 2, 3 oder 4 ist und der Ring G ein Aryl ist, wie oben definiert;
X entweder für eine Doppelbindung oder eine Dreifachbindung steht und
J, K und L unabhängig aus einer Gruppe bestehend aus Wasserstoff, Halogen, (C₁-C₁₀)-Alkyl, (C₃-C₁₃)-Cycloalkyl, -OH, -O-(C₁-C₁₀)-Alkyl, -O-(C₃-C₁₃)-Cycloalkyl,-OCO-(C₁-C₁₀)-Alkyl, -OCO-(C₃-C₁₃)-Cycloalkyl, -OCO-CO-O-(C₁-C₁₀)-Alkyl,-OCO-CO-O-(C₃-C₁₃)-Cycloalkyl, -OCO-O-(C₁-C₁₀)-Alkyl, -OCO-O-(C₃-C₁₃)-Cycloalkyl, -OCO-NH-(C₁-C₁₀)-Alkyl, -OCO-NH-(C₃-C₁₃)-Cycloalkyl, -OCO-N-[(C₁-C₁₀)-Alkyl]₂, -OCO-N-[(C₃-C₁₃)-Cycloalkyl]₂, -OCO-NHSO₂-(C₁-C₁₀)-Alkyl, - OCO-NHSO₂-(C₃-C₁₃)-Cycloalkyl, -NH₂, -NH-(C₁-C₈)-Alkyl, -N-[(C₁-C₈)-Akyl]₂, - NO₂ und -CN ausgewählt sind;
wobei die Alkyl- oder Cycloalkylgruppen gegebenenfalls eine oder mehrere Ungesättigtheiten enthalten können und/oder ein oder mehrere darin eingebundene Heteroatome aufweisen können und gegebenenfalls jeweils ein oder mehrere Wasserstoffatome aufweisen, die durch Halogen, -OH, (C₁-C₃)-Alkyl, -O-(C₁-C₃)-Alkyl, -OCO-(C₁-C₃)-Alkyl, -COOH, -COO-(C₁-C₅)-Alkyl, -COO-(C₁-C₅)-Halogenalkyl, -NHCO-(C₁-C₈)-Alkyl, -ONO₂, -NH-(C₁-C₈)-Alkyl, -N-[(C₁-C₈)-Alkyl]₂, -NO₂, -CN, (C₃-C₁₃)-Cycloalkyl, Aryl oder einen heterocyclischen Rest ersetzt sind;
oder J und K zusammen mit dem Kohlenstoffatom, an das sie angelagert sind, verbunden sein können, um eine (C₃-C₁₃)-Cycloalkyl- oder -CO-Gruppe darzustellen, und L wie oben definiert ist;
oder J, K und L fehlen, wenn n₁ 0 ist;
R₇ für eine Gruppe steht, die aus Wasserstoff, Halogen, Aryl und CO-Aryl ausgewählt ist, wobei der Ring oder das Ringsystem unsubstituiert oder wie oben definiert substituiert ist.

2. Verbindung der Formel I und physiologisch unbedenkliches Salz davon nach Anspruch 1, wobei R₄ für die Einheit (A) steht: 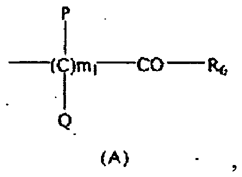 wobei m₁, P, Q und R₆ wie in Anspruch 1 definiert sind.

3. Verbindung der Formel I und physiologisch unbedenkliches Salz davon nach Anspruch 1, wobei R₄ für die Einheit (B) steht: 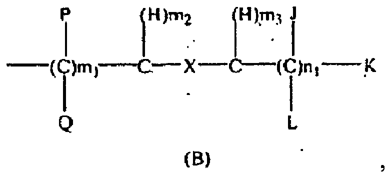 wobei m₁, m₂, m₃, n₁, P, Q, J, K, L und X wie in Anspruch 1 definiert sind.

4. Verbindung der Formel I und physiologisch unbedenkliches Salz davon nach Anspruch 1, wobei R₄ für die Einheit (C) steht: 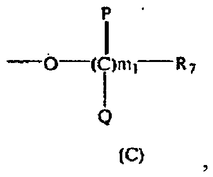 wobei m₁, P, Q und R₇ wie in Anspruch 1 definiert sind.

5. Verbindung der Formel I-B und physiologisch unbedenkliches Salz davon: 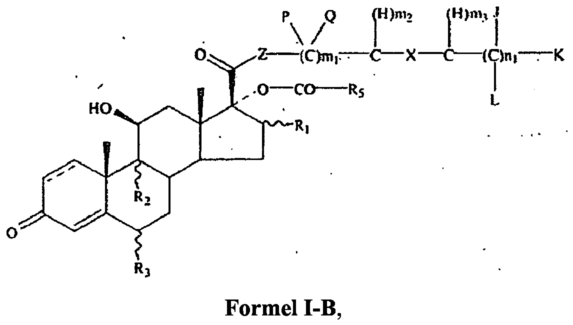 wobei
Z für O oder S steht;
R₁ für Wasserstoff oder Methyl, das entweder in einer α- oder β-Konfiguration sein kann, oder Methylen steht;
R₂ und R₃ gleich oder verschieden sind und jeweils unabhängig für Wasserstoff, Halogen oder eine Methylgruppe stehen;
R₅ für eine Gruppe steht, die aus (C₁-C₁₀)-Alkyl, (C₃-C₁₃)-Cycloalkyl, -O-(C₁-C₁₀)-Alkyl, Aryl oder einem heterocyclischen Ring ausgewählt ist, wobei der Ring oder das Ringsystem unsubstituiert oder durch Halogen, -OH, (C₁-C₃)-Alkyl, -O-(C₁-C₃)-Alkyl und/oder (C₃-C₁₃)-Cycloalkyl substituiert ist; wobei die Alkyl- oder Cycloalkylgruppen gegebenenfalls eine oder mehrere Ungesättigtheiten enthalten können und/oder ein oder mehrere darin eingebundene Heteroatome aufweisen können und gegebenenfalls jeweils ein oder mehrere Wasserstoffatome aufweisen, die durch Halogen, -OH, (C₁-C₃)-Alkyl, -O-(C₁-C₃)-Alkyl, (C₃-C₁₃)-Cycloalkyl ersetzt sind; und
m₁ 1 ist;
m₂ 0 oder 1 ist;
m₃ 0 oder 1 ist;
n₁ 0, 1 oder 2 ist;
P und Q Wasserstoff sind;
X entweder für eine Doppelbindung oder eine Dreifachbindung steht und
J, K und L unabhängig aus einer Gruppe bestehend aus Wasserstoff, Halogen, (C₁-C₁₀)-Alkyl, (C₃-C₁₃)-Cycloalkyl, -OH, -O-(C₁-C₁₀)-Alkyl, -O-(C₃-C₁₃)-Cycloalkyl,-OCO-(C₁-C₁₀)-Alkyl, -OCO-(C₃-C₁₃)-Cycloalkyl, -OCO-CO-O-(C₁-C₁₀)-Alkyl, - OCO-CO-O-(C₃-C₁₃₎-Cycloalkyl, -OCO-O-(C₁-C₁₀)-Alkyl, -OCO-O-(C₃-C₁₃)-Cycloalkyl, -OCO-NH-(C₁-C₁₀)-Alkyl, -OCO-NH-(C₃-C₁₃)-Cycloalkyl, -OCO-N-[(C₁-C₁₀)-Alkyl]₂, -OCO-N-[(C₃-C₁₃)-Cycloalkyl]₂, -OCO-NHSO₂-(C₁-C₁₀)-Akyl, - OCO-NHSO₂-(C₃-C₁₃)-Cycloalkyl, -NH₂, -NH-(C₁-C₈)-Alkyl, -N-[(C₁-C₈)-Alkyl]₂,-NO₂ und -CN ausgewählt sind.

6. Verbindung der Formel I-B und physiologisch unbedenkliches Salz davon: 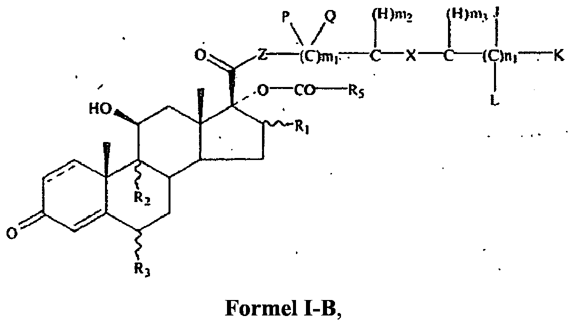 wobei
Z für S steht;
R₁ für Wasserstoff oder Methyl, das entweder in einer α- oder β-Konfiguration sein kann, oder Methylen steht;
R₂ und R₃ gleich oder verschieden sind und jeweils unabhängig für Wasserstoff, Halogen oder eine Methylgruppe stehen;
R₅ für eine Gruppe steht, die aus (C₁-C₁₀)-Alkyl, (C₃-C₁₃)-Cycloalkyl, -O-(C₁-C₁₀)-Alkyl, Aryl oder einem heterocyclischen Ring ausgewählt ist, wobei der Ring oder das Ringsystem unsubstituiert oder durch Halogen, -OH, (C₁-C₃)-Alkyl, -O-(C₁-C₃)-Alkyl und/oder (C₃-C₁₃)-Cycloalkyl substituiert ist; wobei die Alkyl- oder Cycloalkylgruppen gegebenenfalls eine oder mehrere Ungesättigtheiten enthalten können und/oder ein oder mehrere darin eingebundene Heteroatome aufweisen können und gegebenenfalls jeweils ein oder mehrere Wasserstoffatome aufweisen, die durch Halogen, -OH, (C₁-C₃)-Alkyl, -O-(C₁-C₃)-Alkyl, (C₃-C₁₃)-Cycloalkyl ersetzt sind; und
m₁ 1 ist;
m₂ 0 ist;
m₃ 0 ist;
n₁ 0, 1 oder 2 ist;
P und Q Wasserstoff sind;
X für eine Dreifachbindung steht und
J, K und L unabhängig aus einer Gruppe bestehend aus Wasserstoff, Halogen, (C₁-C₁₀)-Alkyl, (C₃-C₁₃)-Cycloalkyl, -OH, -O-(C₁-C₁₀)-Alkyl, -O-(C₃-C₁₃)-Cycloalkyl,-OCO-(C₁-C₁₀)-Alkyl, -OCO-(C₃-C₁₃)-Cycloalkyl, -OCO-CO-O-(C₁-C₁₀)-Alkyl,-OCO-CO-O-(C₃-C₁₃)-Cycloalkyl, -OCO-O-(C₁-C₁₀)-Alkyl, -OCO-O-(C₃-C₁₃)-Cycloalkyl, -OCO-NH-(C₁-C₁₀)-Alkyl, -OCO-NH-(C₃-C₁₃)-Cycloalkyl, -OCO-N-[(C₁-C₁₀)-Alkyl]₂, -OCO-N-[(C₃-C₁₃)-Cycloalkyl]₂, -OCO-NHSO₂-(C₁-C₁₀)-Alkyl, - OCO-NHSO₂-(C₃-C₁₃)-Cycloalkyl, -NH₂, -NH-(C₁-C₈)-Alkyl, -N-[(C₁-C₈)-Alkyl]₂, - NO₂ und -CN ausgewählt sind.

7. Verbindung nach Anspruch 1, die aus der Gruppe bestehend aus folgenden Verbindungen ausgewählt ist:
6α,9α-Difluor-11β-hydroxy-16α-methyl-3 -oxo-17α-propionyloxyandrosta-1,4-dien-17β-carbothiosäure-*S*-[2-(4-chlorphenyl)-2-oxoethyl]ester,
6α,9α-Difluor-11β-hydroxy-16α-methyl-3-oxo-17α-propionyloxyandrosta-1,4-dien-17β-carbonsäure-*S*-[2-(4-fluorphenyl)-2-oxoethyl]ester,
6α,9α-Difluor-11β-hydroxy-16α-methyl-3-oxo-17α-propionyloxyandrosta-1,4-dien-17β-carbothiosäure-*S*-[2-(4-fluorphenyl)-2-oxoethyl]ester,
6α,9α-Difluor-11β-hydroxy-16α-methyl-3-oxo-17α-propionyloxyandrosta-1,4-dien-17β-carbothiosäure-*S*-[2-(2,4-difluorphenyl)-2-oxoethyl]ester,
6α,9α-Difluor-11β-hydroxy-16α-methyl-3-oxo-17α-propionyloxyandrosta-1,4-dien-17β-carbothiosäure-*S*-[2-(2,4-dichlorphenyl)-2-oxoethyl]ester,
6α,9α-Difluor-11β-hydroxy-16α-methyl-3-oxo-17α-propionyloxyandrosta-1,4-dien-17β-carbothiosäure-*S*-[2-(3,4-dichlorphenyl)-2-oxoethyl]ester,
6α,9α-Difluor-11β-hydroxy-16α-methyl-3-oxo-17α-propionyloxyandrosta-1,4-dien-17β-carbonsäure-*S*-[2-(5-chlorthiophen-2-yl)-2-oxoethyl]ester,
6α,9α-Difluor-11β-hydroxy-16α-methyl-3-oxo-17α-propionyloxyandrosta-1,4-dien-17β-carbothiosäure-*S*-[2-oxopropyl]ester,
6α,9α-Difluor-11β-hydroxy-16α-methyl-3-oxo-17α-propionyloxyandrosta-1,4-dien-17β-carbonsäure-2-oxopropylester,
6α,9α-Difluor-11β-hydroxy-16α-methyl-3-oxo-17α-propionyloxyandrosta-1,4-dien-17β-carbothiosäure-*S*-[2-(4-methoxyphenyl)-2-oxoethyl]ester,
6α,9α-Difluor-11β-hydroxy-16α-methyl-3-oxo-17α-propionyloxyandrosta-1,4-dien-17β-carbothiosäure-*S*-[2-oxo-2-(4-trifluormethyloxyphenyl)ethyl]ester,
6α,9α-Difluor-11β-hydroxy-16α-methyl-3-oxo-17α-propionyloxyandrosta-1,4-dien-17β-carbothiosäure-*S*-[2-oxo-2-(4-trifluormethylphenyl)ethyl]ester,
6α,9α-Difluor-11β-hydroxy-16α-methyl-3-oxo-17α-propionyloxyandrosta-1,4-dien-17β-carbonsäure-(2-4-methansulfonylphenyl)-2-oxoethylester,
6α,9α-Difluor-11β-hydroxy-16α-methyl-3-oxo-17α-propionyloxyandrosta-1,4-dien-17β-carbothiosäure-*S*-[2-4-methansulfonylphenyl)-2-oxoethyl]ester,
6α,9α-Difluor-11β-hydroxy-16α-methyl-3-oxo-17α-propionyloxyandrosta-1,4-dien-17β-carbonsäure-2-oxo-2-phenylethylester,
6α,9α-Difluor-11β-hydroxy-16α-methyl-3-oxo-17α-propionyloxyandrosta-1,4-dien-17β-carbothiosäure-*S*-[2-oxo-2-phenylethyl]ester,
6α,9α-Difluor-11β-hydroxy-16α-methyl-3-oxo-17α-propionyloxyandrosta-1,4-dien-17β-carbothiosäure-*S*-[2-oxo-2-(2,4,6-trimethylphenyl)ethyl]ester,
6α,9α-Difluor-11β-hydroxy-16α-methyl-3-oxo-17α-propionyloxyandrosta-1,4-dien-17β-carbothio säure-*S*-[1,1-dimethyl-2-oxo-2-phenylethyl]ester,
6α,9α-Difluor-11β-hydroxy-16α-methyl-3-oxo-17α-propionyloxyandrosta-1,4-dien-oxoandrosta-1,4-dien-17β-carbothiosäure-*S*-[1-(4-methoxyphenyl)-2-oxoethyl]ester,
17α-Cyclobutylcarbonyloxy-6α,9α-difluor-11β-hydroxy-16α-methyl-3-oxoandrosta-1,4-dien-17β-carbothiosäure-*S*-[2-oxo-2-(4-trifluormethylphenyl)ethyl]ester,
17α-Cyclobutylcarbonyloxy-6α,9α-difluor-11β-hydroxy-16α-methyl-3-oxoandrosta-1,4-dien-17β-carbothiosäure-*S*-[2-(2,4-difluorphenyl)-2-oxoethyl]ester,
17α-Cyclobutylcarbonyloxy-6α,9α-difluor-11β-hydroxy-16α-methyl-3-oxoandrosta-1,4-dien-17β-carbothiosäure-*S*-[2-(4-methoxyphenyl)-2-oxoethyl]ester,
9α-Fluor-17α-[(2-furanylcarbonyl)oxy]-11β-hydroxy-16α-methyl-3-oxoandrosta-1,4-dien-17β-carbothiosäure-*S*-[2-(2,4-difluorphenyl)-2-oxoethyl]ester,
9α-Fluor-17α-[(2-furanylcarbonyl)oxy]-11β-hydroxy-16α-methyl-3-oxoandrosta-1,4-dien-17β-carbothiosäure-*S*-[2-(4-chlorphenyl)-2-oxoethyl]ester,
9α-Fluor-17α-[(2-furanylcarbonyl)oxy]-11β-hydroxy-16α-methyl-3-oxoandrosta-1,4-dien-17β-carbothiosäure-*S*-[2-(4-methoxyphenyl)-2-oxoethyl]ester,
9α-Fluor-17α-[(2-furanylcarbonyl)oxy]-11β-hydroxy-16α-methyl-3-oxoandrosta-1,4-dien-17β-carbothiosäure-*S*-[2-(4-fluorphenyl)-2-oxoethyl]ester,
6α,9α-Difluor-17α-[(2-furanylcarbonyl)oxy]-11β-hydroxy-16α-methyl-3-oxoandrosta-1,4-dien-17β-carbothiosäure-*S*-[2-(4-chlorphenyl)-2-oxoethyl]ester,
6α,9α-Difluor-11β-hydroxy-16α-methyl-3-oxo-17α-[(2-thienylcarbonyl)oxy]androsta-1,4-dien-17β-carbothiosäure-*S*-[2-oxo-2-(4-trifluormethylphenyl)ethyl]ester,
6α,9α-Difluor-11β-hydroxy-16α-methyl-3-oxo-17α-propionyloxyandrosta-1,4-dien-17β-carbothiosäure-*S*-[2-(3,4-difluormethylphenyl)-2-oxoethyl]ester,
6α,9α-Difluor-17α-[(2-furanylcarbonyl)oxy]-11β-hydroxy-16α-methyl-3-oxoandrosta-1,4-dien-17β-carbothiosäure-*S*-[2-(3-chlorphenyl)-2-oxoethyl]ester,
6α,9α-Difluor-17α-[(2-furanylcarbonyl)oxy]-11β-hydroxy-16α-methyl-3-oxoandrosta-1,4-dien-17β-carbothiosäure-*S*-[2-oxo-2-(4-trifluormethylphenyl)ethyl] ester,
6α,9α-Difluor-17α-[(2-furanylcarbonyl)oxy]-11β-hydroxy-16α-methyl-3-oxoandrosta-1,4-dien-17β-carbothiosäure-*S*-(4-hydroxybut-2-inyl)ester,
6α,9α-Difluor-11β-hydroxy-16α-methyl-3-oxo-17α-propionyloxyandrosta-1,4-dien-17β-carbothiosäure-*S*-(4-n-propylcabonyloxybut-2-inyl)]ester,
6α,9α-Difluor-11β-hydroxy-16α-methyl-3-oxo-17α-propionyloxyandrosta-1,4-dien-17β-carbothiosäure-*S*-[4-(2-ethoxy-1,2-dioxoethyl)-(E)-but-2-enyl]ester,
6α,9α-Difluor-17α-[(2-furanylcarbonyl)oxy]-11β-hydroxy-16α-methyl-3-oxoandrosta-1,4-dien-17β-carbothiosäure-*S*-[4-(2-ethoxy-1,2-dioxoethyl)oxy-(E)-but-2-enyl] ester,
6α,9α-Difluor-17α-[(2-furanylcarbonyl)oxy]-11β-hydroxy-16α-methyl-3-oxoandrosta-1,4-dien-17β-carbothiosäure-*S*-[4-*n*-propylcarbonyloxybut-2-inyl]ester,
6α,9α-Difluor-17α-[(2-furanylcarbonyl)oxy]-11β-hydroxy-16α-methyl-3-oxoandrosta-1,4-dien-17β-carbothiosäure-*S*-[4-(2-methylpropionyloxy)but-2-inyl] ester,
6α,9α-Difluor-17α-[(2-furanylcarbonyl)oxy]-11β-hydroxy-16α-methyl-3-oxoandrosta-1,4-dien-17β-carbothiosäure-*S*-[4-(2,2-dimethylpropionyloxy)but-2-inyl] ester,
6α,9α-Difluor-11β-hydroxy-16α-methyl-3-oxo-17α-propionyloxyandrosta-1,4-dien-17β-carbothiosäure-*S*-[4-(2,2-dimethylpropionyloxy)but-2-inyl]ester,
6α,9α-Difluor-17α-[(2-furanylcarbonyl)oxy]-11β-hydroxy-16α-methyl-3-oxoandrosta-1,4-dien-17β-carbothiosäure-*S*-[4-*n*-propylcarbonyloxy-(*Z*)-but-2-enyl]ester,
6α,9α-Difluor-17α-[(2-furanylcarbonyl)oxy]-11β-hydroxy-16α-methyl-3-oxoandrosta-1,4-dien-17β-carbothiosäure-*S*-[4-(2-ethoxy-1,2-dioxoethyl)oxybut-2-inyl] ester,
6α,9α-Difluor-11β-hydroxy-16α-methyl-3-oxo-17α-[(2-thienylcarbonyl)oxy]androsta-1,4-dien-17β-carbothiosäure-*S*-[4-*n*-propylcarbonyloxybut-2-inyl]ester,
6α,9α-Difluor-17α-[(2-furanylcarbonyl)oxy]-11β-hydroxy-16α-methyl-3-oxoandrosta-1,4-dien-17β-carbothiosäure-*S*-[(4-cyclopropylcarbonyloxy)but-2-inyl]ester,
6α,9α-Difluor-17α-[(2-furanylcarbonyl)oxy]-11β-hydroxy-16α-methyl-3-oxoandrosta-1,4-dien-17β-carbothiosäure-*S*-[(4-cyclobutylcarbonyloxy)but-2-inyl]ester,
6α,9α-Difluor-17α-[(2-furanylcarbonyl)oxy]-11β-hydroxy-16α-methyl-3-oxoandrosta-1,4-dien-17β-carbothiosäure-*S*-[(4-cyclopentylcarbonyloxy)but-2-inyl]ester,
6α,9α-Difluor-17α-[(2-furanylcarbonyl)oxy]-11β-hydroxy-16α-methyl-3-oxoandrosta-1,4-dien-17β-carbothiosäure-*S*-[4-*n*-propylcarbonyloxy-(*E*)-but-2-enyl]ester,
6α,9α-Difluor-17α-[(2-furanylcarbonyl)oxy]-11β-hydroxy-16α-methyl-3-oxoandrosta-1,4-dien-17β-carbothiosäure-*S*-[(4-methoxycarbonyloxy)-but-2-inyl] ester,
6α,9α-Difluor-17α-[(2-furanylcarbonyl)oxy]-11β-hydroxy-16α-methyl-3-oxoandrosta-1,4-dien-17β-carbothiosäure-*S*-[(4-ethoxycarbonyloxy)-but-2-inyl]ester,
6α,9α-Difluor-17α-[(2-furanylcarbonyl)oxy]-11β-hydroxy-16α-methyl-3-oxoandrosta-1,4-dien-17β-carbothiosäure-*S*-[4-(2,2-dimethylethyloxycarbonyloxy)-but-2-inyl]ester,
6α,9α-Difluor-17α-[(2-furanylcarbonyl)oxy]-11β-hydroxy-16α-methyl-3-oxoandrosta-1,4-dien-17β-carbothiosäure-*S*-[4-(2-methylpropyloxycarbonyloxy)-but-2-inyl]ester,
6α,9α-Difluor-17α-[(2-furanylcarbonyl)oxy]-11β-hydroxy-16α-methyl-3-oxoandrosta-1,4-dien-17β-carbothiosäure-*S*-[4-(3-chlorpropylcarbonyloxy)-but-2-inyl]ester,
6α,9α-Difluor-17α-[(2-furanylcarbonyl)oxy]-11β-hydroxy-16α-methyl-3-oxoandrosta-1,4-dien-17β-carbothiosäure-*S*-[4-dichloracetoxybut-2-inyl]ester,
6α,9α-Difluor-17α-[(2-furanylcarbonyl)oxy]-11β-hydroxy-16α-methyl-3-oxoandrosta-1,4-dien-17β-carbothiosäure-*S*-[4-(2-methylpropylcarbonyloxy)-but-2-inyl]ester,
6α,9α-Difluor-17α-[(2-furanylcarbonyl)oxy]-11β-hydroxy-16α-methyl-3-oxoandrosta-1,4-dien-17β-carbothiosäure-*S*-[4-(3,3-dimethylpropylcarbonyloxy)-but-2-inyl]ester,
6a,9α-Difluor-17α-[(2-furanylcarbonyl)oxy]-11β-hydroxy-16α-methyl-3-oxoandrosta-1,4-dien-17β-carbothiosäure-*S*-(4-propionyloxybut-2-inyl)ester,
6a,9α-Difluor-17α-[(2-furanylcarbonyl)oxy]-11β-hydroxy-16α-methyl-3-oxoandrosta-1,4-dien-17β-carbothiosäure-*S*-[4-(3,3,3-trifluorpropionyloxy)but-2-inyl] ester,
6a,9α-Difluor-17α-[(2-furanylcarbonyl)oxy]-11β-hydroxy-16α-methyl-3-oxoandrosta-1,4-dien-17β-carbothiosäure-*S*-[4-(methoxyacetyloxy)but-2-inyl]ester,
6a,9α-Difluor-17α-[(2-furanylcarbonyl)oxy]-11β-hydroxy-16α-methyl-3-oxoandrosta-1,4-dien-17β-carbothiosäure-*S*-[4-(ethoxyacetyloxy)but-2-inyl]ester,
6α,9α-Difluor-17α-[(2-furanylcarbonyl)oxy]-11β-hydroxy-16α-methyl-3-oxoandrosta-1,4-dien-17β-carbothiosäure-*S*-(4-acetoxybut-2-inyl)ester,
6α,9α-Difluor-17α-[(2-furanylcarbonyl)oxy]-11β-hydroxy-16α-methyl-3-oxoandrosta-1,4-dien-17β-carbothiosäure-*S*-[4-(2,2-dimethylbutylcarbonyloxy)but-2-inyl]ester,
6α,9α-Difluor-17α-[(2-furanylcarbonyl)oxy]-11β-hydroxy-16α-methyl-3-oxoandrosta-1,4-dien-17β-carbothiosäure-*S*-[4-((1*S*)-camphanylcarbonyloxy)but-2-inyl]ester,
6α,9α-Difluor-17α-[(2-furanylcarbonyl)oxy]-11β-hydroxy-16α-methyl-3-oxoandrosta-1,4-dien-17β-carbothiosäure-*S*-[4-(4-ethoxybutyl-1,4-dion)oxybut-2-inyl]ester,
6α,9α-Difluor-17α-[(2-furanylcarbonyl)oxy]-11β-hydroxy-16α-methyl-3-oxoandrosta-1,4-dien-17β-carbothiosäure-*S*-[4-(4-ethyloxy-(*Z*)-but-2-en-1,4-dion)oxybut-2-inyl]ester,
6α,9α-Difluor-17α-[(2-furanylcarbonyl)oxy]-11β-hydroxy-16α-methyl-3-oxoandrosta-1,4-dien-17β-carbothiosäure-*S*-[4-(4-methoxybutyl-1,4-dion)oxybut-2-inyl]ester,
6α,9α-Difluor-11β-hydroxy-16α-methyl-17α-[(furanylcarbonyl)oxy]-3-oxoandrosta-1,4-dien-17β-carbothiosäure-*S*-[4-(4-isopropyloxybutyl-1,4-dion)oxybut-2-inyl] ester,
6α,9α-Difluor-17α-[(2-furanylcarbonyl)oxy]-11β-hydroxy-16α-methyl-3-oxoandrosta-1,4-dien-17β-carbothiosäure-*S*-[4-(4-isobutyloxybutyl-1,4-dion)oxybut-2-inyl] ester,
6α,9α-Difluor-17α-[(2-furanylcarbonyl)oxy]-11β-hydroxy-16α-methyl-3-oxoandrosta-1,4-dien-17β-carbothiosäure-*S*-[4-(4-*n*-butyloxybutyl-1,4-dion)oxybut-2-inyl] ester,
6α,9α-Difluor-17α-[(2-furanylcarbonyl)oxy]-11β-hydroxy-16α-methyl-3-oxoandrosta-1,4-dien-17β-carbothiosäure-*S*-[4-(3-chlor-2,2-dimethylpropionyloxy)but-2-inyl]ester,
6α,9α-Difluor-17α-[(2-furanylcarbonyl)oxy]-11β-hydroxy-16α-methyl-3-oxoandrosta-1,4-dien-17β-carbothiosäure-*S*-[4-(4-methoxy-(*E*)-but-2-en-1,4-dion)oxybut-2-inyl] ester,
6α,9α-Difluor-17α-[(2-furanylcarbonyl)oxy]-11β-hydroxy-16α-methyl-3-oxoandrosta-1,4-dien-17β-carbothiosäure-*S*-[4-(3-methyl-1-oxobutyloxy)but-2-inyl]ester,
6α,9α-Difluor-17α-[(2-furanylcarbonyl)oxy]-11β-hydroxy-16α-methyl-3-oxoandrosta-1,4-dien-17β-carbothiosäure-*S*-[4-(2-ethyl-1-oxobutyloxy)but-2-inyl]ester,
6α,9α-Difluor-17α-[(2-furanylcarbonyl)oxy]-11β-hydroxy-16α-methyl-3-oxoandrosta-1,4-dien-17β-carbothiosäure-S-[4-(*N*,*N*-dimethylaminocarbonyloxy)but-2-inyl]ester,
6α,9α-Difluor-17α-[(2-furanylcarbonyl)oxy]-11β-hydroxy-16α-methyl-3-oxoandrosta-1,4-dien-17β-carbothiosäure-*S*-[4-(2-chlor-2-methylpropionyloxy)but-2-inyl] ester,
6α,9α-Difluor-17α-[(2-furanylcarbonyl)oxy]-11β-hydroxy-16α-methyl-3-oxoandrosta-1,4-dien-17β-carbothiosäure-*S*-[4-(4-methyl-1-oxopentyloxy)but-2-inyl]ester,
6α,9α-Difluor-17α-[(2-furanylcarbonyl)oxy]-11β-hydroxy-16α-methyl-3-oxoandrosta-1,4-dien-17β-carbothiosäure-*S*-[4-(2-hydroxy-2-methylpropionyloxy)but-2-inyl]ester,
6α,9α-Difluor-17α-[(2-furanylcarbonyl)oxy]-11β-hydroxy-16α-methyl-3-oxoandrosta-1,4-dien-17β-carbothiosäure-*S*-[4-(2-fluor-2-methylpropionyloxy)but-2-inyl]ester,
6α,9α-Difluor-17α-[(2-furanylcarbonyl)oxy]-11β-hydroxy-16α-methyl-3-oxoandrosta-1,4-dien-17β-carbothiosäure-*S*-[4-(4-methoxy-(*Z*)-but-2-en-1,4-dion)oxybut-2-inyl]ester,
6α,9α-Difluor-17α-[(2-furanylcarbonyl)oxy]-11β-hydroxy-16α-methyl-3-oxoandrosta-1,4-dien-17β-carbothiosäure-*S*-[4-(4-ethoxy-(*E*)-but-2-en-1,4-dion)oxybut-2-inyl] ester,
6α,9α-Difluor-17α-[(2-furanylcarbonyl)oxy]-11β-hydroxy-16α-methyl-3-oxoandrosta-1,4-dien-17β-carbothiosäure-*S*-[4-(4-isobutyloxy-(*E*)-but-2-en-1,4-dion)oxybut-2-inyl]ester,
6α,9α-Difluor-17α-[(2-furanylcarbonyl)oxy]-11β-hydroxy-16α-methyl-3-oxoandrosta-1,4-dien-17β-carbothiosäure-*S*-(4-acryloyloxybut-2-inyl]ester,
6α,9α-Difluor-17α-[(2-furanylcarbonyl)oxy]-11β-hydroxy-16α-methyl-3-oxoandrosta-1,4-dien-17β-carbothiosäure-S-[4-(4-cyclopentylmethyloxycarbonyloxy)but-2-inyl]ester,
6α,9α-Difluor-17α-[(2-furanylcarbonyl)oxy]-11β-hydroxy-16α-methyl-3-oxoandrosta-1,4-dien-17β-carbothiosäure-*S*-[4-(3-methylbut-2-enoxycarbonyloxy)but-2-inyl]ester,
6α,9α-Difluor-17α-[(2-furanylcarbonyl)oxy]-11β-hydroxy-16α-methyl-3-oxoandrosta-1,4-dien-17β-carbothiosäure-*S*-[4-(*n*-pentyloxycarbonyloxy)but-2-inyl]ester,
6α,9α-Difluor-17α-[(2-furanylcarbonyl)oxy]-11β-hydroxy-16α-methyl-3-oxoandrosta-1,4-dien-17β-carbothiosäure-*S*-[4-(2,2-dimethyl-3-fluorpropionyloxy)but-2-inyl]ester,
6α,9α-Difluor-17α-[(2-furanylcarbonyl)oxy]-11β-hydroxy-16α-methyl-3-oxoandrosta-1,4-dien-17β-carbothiosäure-S-[4-((*S*)-2,2-dimethyl-1,3-dioxolan-4-ylmethoxycarbonyloxy)but-2-inyl]ester,
6α,9α-Difluor-17α-[(2-furanylcarbonyl)oxy]-11β-hydroxy-16α-methyl-3-oxoandrosta-1,4-dien-17β-carbothiosäure-S-[4-((*R*)-2,2-dimethyl-1,3-dioxolan-4-ylmethoxycarbonyloxy)but-2-inyl] ester,
6α,9α-Difluor-17α-[(2-furanylcarbonyl)oxy]-11β-hydroxy-16α-methyl-3-oxoandrosta-1,4-dien-17β-carbothiosäure-*S*-[4-(isoamylcarbonyloxy)but-2-inyl]ester,
6α,9α-Difluor-17α-[(2-furanylcarbonyl)oxy]-11β-hydroxy-16α-methyl-3-oxoandrosta-1,4-dien-17β-carbothiosäure-*S*-(4-ethoxybut-2-inyl)ester,
6α,9α-Difluor-17α-[(2-furanylcarbonyl)oxy]-11β-hydroxy-16α-methyl-3-17β-carbonylsulfensäurefluormethylester,
6α,9α-Difluor-17α-[(2-furanylcarbonyl)oxy]-11β-hydroxy-16α-methyl-3-oxoandrosta-1,4-dien-17β-carbonylsulfensäurefluormethylester,
6α,9α-Difluor-17α-[(2-furanylcarbonyl)oxy]-11β-hydroxy-16α-methyl-3-oxoandrosta-1,4-dien-17β-carbonylsulfensäure-(4-fluorphenyl)methylester,
6α,9α-Difluor-17α-[(2-furanylcarbonyl)oxy]-11β-hydroxy-16α-methyl-3-oxoandrosta-1,4-dien-17β-carbonylsulfensäure-(4-trifluormethylphenyl)methylester,
6α,9α-Difluor-17α-[(2-furanylcarbonyl)oxy]-11β-hydroxy-16α-methyl-3-oxoandrosta-1,4-dien-17β-carbonylsulfensäure-(4-chlorphenyl)methylester,
6α,9α-Difluor-17α-[(2-furanylcarbonyl)oxy]-11β-hydroxy-16α-methyl-3-17β-carbonylsulfensäure(4-trifluormethylphenyl)methylester,
9α-Fluor-17α-[(2-furanylcarbonyl)oxy]-11β-hydroxy-16α-methyl-3-oxoandrosta-1,4-dien-17β-carbonylsulfensäure-(4-fluorphenyl)methylester,
6α,9α-Difluor-11β-hydroxy-16α-methyl-3-oxo-17α-propionyloxyandrosta-1,4-dien-17β-carbonylsulfensäuremethylester,
6α,9α-Difluor-17α-[(2-furanylcarbonyl)oxy]-11β-hydroxy-16α-methyl-3-oxoandrosta-1,4-dien-17β-carbonylsulfensäuremethylester,
6α,9α-Difluor-17α-[(2-furanylcarbonyl)oxy]-11β-hydroxy-16α-methyl-3-oxoandrosta-1,4-dien-17β-carbothiosäure-S-[4-(4-methyl-1-oxopentyloxy)but-2-inyl]ester.

## Revendications

1. Composé de formule I, et sel physiologiquement acceptable de celui-ci : 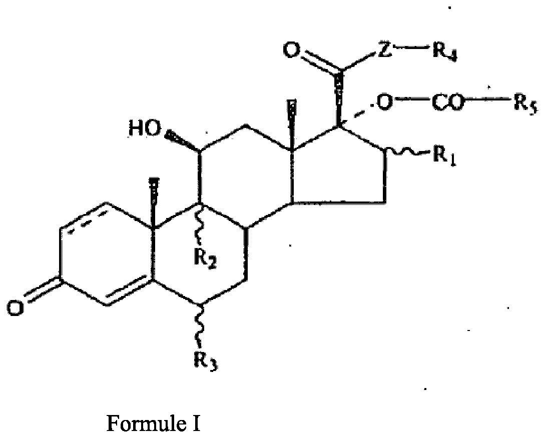
- -̅-̅-̅-̅-̅ représente une liaison simple ou double et 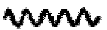 représente une configuration α ou β,
dans laquelle
Z représente O ou S ;
R₁ représente hydrogène ou méthyle qui peut être dans la configuration α ou β, ou méthylène ;
R₂ et R₃ sont identiques ou différents et représentent chacun indépendamment hydrogène, halogène ou un groupe méthyle ;
R₅ représente un groupe sélectionné parmi (C₁-C₁₀)-alkyle, (C₃-C₁₃)-cycloalkyle, -O-(C₁-C₁₀)-alkyle, aryle ou un anneau hétérocyclique, dans laquelle l'anneau ou le système d'anneau est non substitué ou substitué par un ou plusieurs halogènes, -OH, (C₁-C₃)-alkyle,-O-(C₁-C₃)-alkyle, (C₃-C₁₃)-cycloalkyle ; dans laquelle les groupes alkyle ou cycloalkyle peuvent contenir en option une ou plusieurs insaturations et/ou peuvent avoir un ou plusieurs hétéroatomes incorporés en leur sein, et ont en option dans chaque cas un ou plusieurs atomes d'hydrogène remplacés par halogène, -OH, (C₁-C₃)-alkyle, -O-(C₁-C₃)-alkyle, (C₃-C₁₃)-cycloalkyle ; et
R₄ représente un fragment sélectionné parmi un groupe constitué de A, B et C à condition que, lorsque R₄ représente le fragment (C), Z soit S : 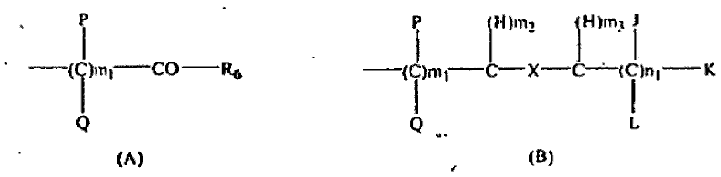 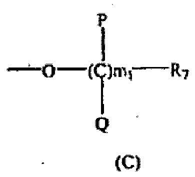 dans lesquels
m₁ est 1, 2 ou 3;
m₂ est 0 ou 1 ;
m₃ est 0 ou 1 ;
n₁ est 0, 1 ou 2 ;
R₆ représente un groupe sélectionné parmi (C₁-C₈)-alkyle, (C₃-C₁₃)-cycloalkyle, aryle et un radical hétérocyclique, où l'anneau ou le système d'anneau est non substitué ou substitué par un ou plusieurs substituants sélectionnés parmi (C₁-C₈)-alkyle, (C₃-C₁₃)-cycloalkyle, halogène, O-(C₁-C₈)-alkyle, O-(C₃-C₁₃)-cycloalkyle, OCO-(C₁-C₃)-alkyle, S(O)₀₋₂(C₁-C₈)-alkyle, COO-(C₁-C₈)-alkyle, -OCO-O-(C₁-C₃)-alkyle, -OCO-CO-O-(C₁-C₃)-alkyle, CONH₂, CONH-(C₁-C₈)-alkyle, CON-[(C₁-C₈)-alkyle]₂, -NHCO-(C₁-C₈)-alkyle, N-(C₁-C₈)-alkyl-CO-(C₁-C₈)-alkyle, -NHCO-O-(C₁-C₈)-alkyle, -N-(C₁-C₈)-alkyl-CO-O-(C₁-C₈)-alkyle, - NHCONH-(C₁-C₈)-alkyle, -N-(C₁-C₈)-alkyl-CONH-(C₁-C₈)-alkyle, NHCONH-SO₂-(C₁-C₈)-alkyle, -N-(C₁-C₈)-alkyl-CONH-SO₂-(C₁-C₈)-alkyle, -NO₂, -CN ; dans lesquels les groupes alkyle ou cycloalkyle peuvent contenir en option une ou plusieurs insaturations et/ou peuvent avoir un ou plusieurs hétéroatomes incorporés en leur sein, et ont en option dans chaque cas un ou plusieurs atomes d'hydrogène remplacés par halogène, -OH, (C₁-C₃)-alkyle, -OCO-(C₁-C₃)-alkyle ou (C₃-C₁₃)-cycloalkyle ;
à condition que, lorsque R₄ représente le fragment A dans lequel R₆ représente (C₃-C₁₃₎-cycloalkyle contenant en option un ou plusieurs hétéroatomes incorporés en son sein, l'hétéroatome n'est pas l'azote ;
P et Q sont indépendamment sélectionnés parmi hydrogène et alkyle en C₁ à C₃ ;
ou P et Q peuvent être reliés ensemble à l'atome de carbone auquel ils sont reliés pour former (C₃-C₈)-cycloalkyle tel que représenté par le fragment (A-1) : 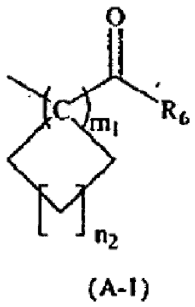 dans lequel m₁ est 1 et n₂ est 0, 1, 2, 3, 4 ou 5 et R₆ est un aryle tel que défini ci-dessus ;
ou P et R₆ peuvent être reliés ensemble pour former un système cyclique tel que représenté par le fragment (A-2) : 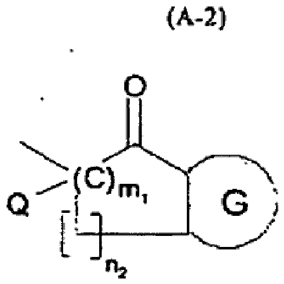 dans lequel m₁ est 1 et n₂ est 0, 1, 2, 3 ou 4 et l'anneau G est un aryle tel que défini ci-dessus ;
X représente une double liaison ou une triple liaison ; et
J, K et L sont indépendamment sélectionnés parmi un groupe constitué d'hydrogène, halogène, (C₁-C₁₀)-alkyle, (C₃-C₁₃)-cycloalkyle, -OH, O-(C₁-C₁₀)-alkyle, O-(C₃-C₁₃)-cycloalkyle, -OCO-(C₁-C₁₀)-alkyle, -OCO-(C₃-C₁₃)-cycloalkyle, -OCO-CO-O-(C₁-C₁₀)-alkyle, -OCO-CO-O-(C₃-C₁₃)-cycloalkyle, -OCO-O-(C₁-C₁₀)-alkyle, -OCO-O-(C₃-C₁₃)-cycloalkyle, -OCO-NH-(C₁-C₁₀)-alkyle, -OCO-NH-(C₃-C₁₃)-cycloalkyle, -OCO-N-[(C₁-C₁₀)-alkyle]₂, -OCO-N-[(C₃-C₁₃)-cycloalkyle]₂, -OCO-NHSO₂-(C₁-C₁₀)-alkyle, -OCO-NHSO₂-(C₃-C₁₃)-cycloalkyle, -NH₂, -NH-(C₁-C₈)-alkyle, -N-[(C₁-C₈)-alkyle]₂,-NO₂ et -CN ; dans lequel les groupes alkyle ou cycloalkyle peuvent contenir en option une ou plusieurs insaturations et/ou peuvent avoir un ou plusieurs hétéroatomes incorporés en leur sein, et ont en option dans chaque cas un ou plusieurs atomes d'hydrogène remplacés par halogène, - OH, (C₁-C₃)-alkyle, -O-(C₁-C₃)-alkyle,-OCO-(C₁-C₃)-alkyle, -COOH, -COO-(C₁-C₅)-alkyle, -COO-(C₁-C₅)-halogénoalkyle, -NHCO-(C₁-C₈)-alkyle, -ONO₂, -NH-(C₁-C₈)-alkyle, -N-[(C₁-C₈)-alkyle]₂, -NO₂, -CN, (C₃-C₁₃)-cycloalkyle, aryle ou un radical hétérocyclique ;
ou J et K peuvent être reliés ensemble à l'atome de carbone auquel ils sont reliés pour représenter un groupe (C₃-C₁₃)-cycloalkyle ou -CO- et L est tel que défini ci-dessus ;
ou J, K et L sont absents lorsque n₁ est 0 ;
R₇ représente un groupe sélectionné parmi hydrogène, halogène, aryle et CO-aryle, dans lequel l'anneau ou le système d'anneau est non substitué ou substitué tel que défini ci-dessus.

2. Composé de formule I, et sel physiologiquement acceptable de celui-ci, selon la revendication 1, dans lequel R₄ représente le fragment (A) 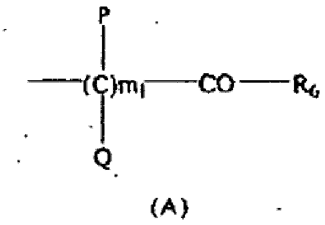 dans lequel m₁, P, Q et R₆ sont tels que définis à la revendication 1.

3. Composé de formule I, et sel physiologiquement acceptable de celui-ci, selon la revendication 1, dans lequel R₄ représente le fragment (B) 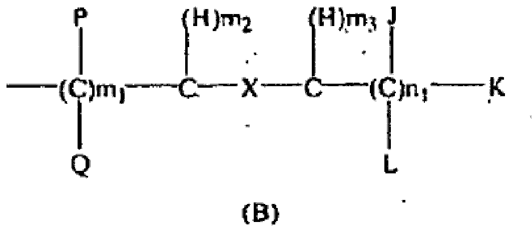 dans lequel m₁, m₂, m₃, n₁, P, Q, J, K, L et X sont tels que définis à la revendication 1.

4. Composé de formule I, et sel physiologiquement acceptable de celui-ci, selon la revendication 1, dans lequel R₄ représente le fragment (C) 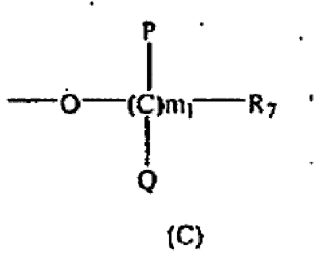 dans lequel m₁, P, Q et R₇ sont tels que définis à la revendication 1.

5. Composé de formule I-B, et sel physiologiquement acceptable de celui-ci : 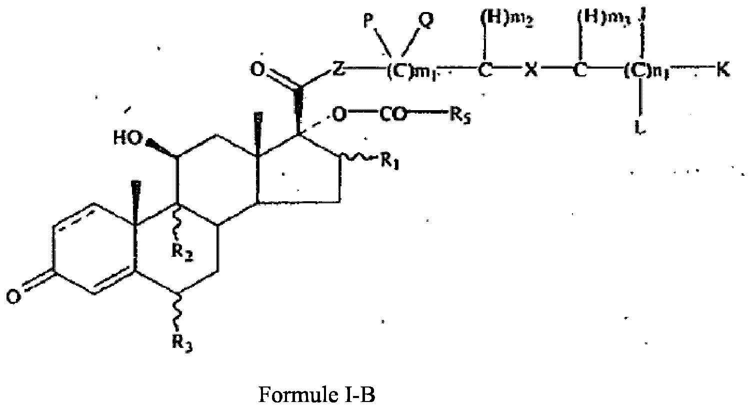 dans laquelle
Z représente O ou S ;
R₁ représente hydrogène ou méthyle qui peut être dans la configuration α ou β, ou méthylène ;
R₂ et R₃ sont identiques ou différents et représentent chacun indépendamment hydrogène, halogène ou un groupe méthyle ;
R₅ représente un groupe sélectionné parmi (C₁-C₁₀)-alkyle, (C₃-C₁₃)-cycloalkyle, -O-(C₁-C₁₀)-alkyle, aryle ou un anneau hétérocyclique, dans laquelle l'anneau ou le système d'anneau est non substitué ou substitué par un ou plusieurs halogènes, -OH, (C₁-C₃)-alkyle,-O-(C₁-C₃)-alkyle, (C₃-C₁₃)-cycloalkyle ; dans laquelle les groupes alkyle ou cycloalkyle peuvent contenir en option une ou plusieurs insaturations et/ou peuvent avoir un ou plusieurs hétéroatomes incorporés en leur sein, et ont en option dans chaque cas un ou plusieurs atomes d'hydrogène remplacés par halogène, -OH, (C₁-C₃)-alkyle, -O-(C₁-C₃)-alkyle, (C₃-C₁₃)-cycloalkyle ; et
m₁ est 1 ;
m₂ est 0 ou 1 ;
m₃ est 0 ou 1 ;
n₁ est 0, 1 ou 2 ;
P et Q sont hydrogène ;
X représente une double liaison ou une triple liaison ; et
J, K et L sont indépendamment sélectionnés parmi un groupe constitué d'hydrogène, halogène, (C₁-C₁₀)-alkyle, (C₃-C₁₃)-cycloalkyle, -OH, O-(C₁-C₁₀)-alkyle, O-(C₃-C₁₃)-cycloalkyle, -OCO-(C₁-C₁₀)-alkyle, -OCO-(C₃-C₁₃)-cycloalkyle, -OCO-CO-O-(C₁-C₁₀)-alkyle, -OCO-CO-O-(C₃-C₁₃)-cycloalkyle, -OCO-O-(C₁-C₁₀)-alkyle, -OCO-O-(C₃-C₁₃)-cycloalkyle, -OCO-NH-(C₁-C₁₀)-alkyle, -OCO-NH-(C₃-C₁₃)-cycloalkyle, -OCO-N-[(C₁-C₁₀)-alkyle]₂, -OCO-N-[(C₃-C₁₃)-cycloalkyle]₂, -OCO-NHSO₂-(C₁-C₁₀)-alkyle, -OCO-NHSO₂-(C₃-C₁₃)-cycloalkyle, -NH₂, -NH-(C₁-C₈)-alkyle, -N-[(C₁-C₈)-alkyle]₂,-NO₂ et -CN.

6. Composé de formule I-B, et sel physiologiquement acceptable de celui-ci : 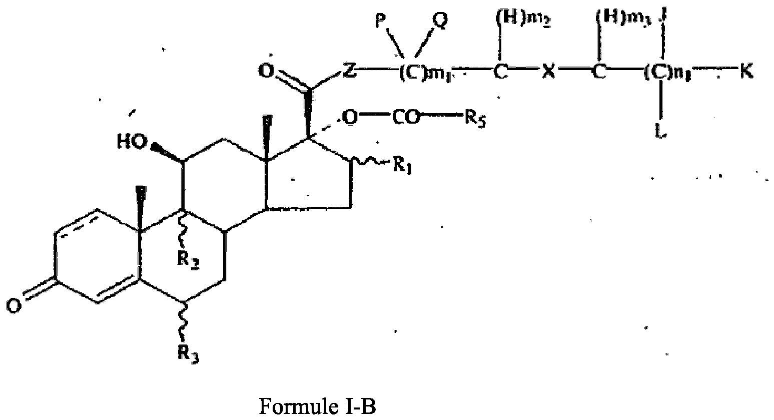 dans laquelle
Z représente S ;
R₁ représente hydrogène ou méthyle qui peut être dans la configuration α ou β, ou méthylène ;
R₂ et R₃ sont identiques ou différents et représentent chacun indépendamment hydrogène, halogène ou un groupe méthyle ;
R₅ représente un groupe sélectionné parmi (C₁-C₁₀)-alkyle, (C₃-C₁₃)-cycloalkyle, -O-(C₁-C₁₀)-alkyle, aryle ou un anneau hétérocyclique, dans laquelle l'anneau ou le système d'anneau est non substitué ou substitué par un ou plusieurs halogènes, -OH, (C₁-C₃)-alkyle,-O-(C₁-C₃)-alkyle, (C₃-C₁₃)-cycloalkyle ; dans laquelle les groupes alkyle ou cycloalkyle peuvent contenir en option une ou plusieurs insaturations et/ou peuvent avoir un ou plusieurs hétéroatomes incorporés en leur sein, et ont en option dans chaque cas un ou plusieurs atomes d'hydrogène remplacés par halogène, -OH, (C₁-C₃)-alkyle, -O-(C₁-C₃)-allcyle, (C₃-C₁₃)-cycloalkyle ; et
m₁ est 1 ;
m₂ est 0 ;
m₃ est 0 ;
n₁ est 0, 1 ou 2 ;
P et Q sont hydrogène ;
X représente une triple liaison, et
J, K et L sont indépendamment sélectionnés parmi un groupe constitué d'hydrogène, halogène, (C₁-C₁₀)-alkyle, (C₃-C₁₃)-cycloalkyle, -OH, O-(C₁-C₁₀)-alkyle, O-(C₃-C₁₃)-cycloalkyle, -OCO-(C₁-C₁₀)-alkyle, -OCO-(C₃-C₁₃)-cycloalkyle, -OCO-CO-O-(C₁-C₁₀)-alkyle, -OCO-CO-O-(C₃-C₁₃)-cycloalkyle, -OCO-O-(C₁-C₁₀)-alkyle, -OCO-O-(C₃-C₁₃)-cycloalkyle, -OCO-NH-(C₁-C₁₀)-alkyle, -OCO-NH-(C₃-C₁₃)-cycloalkyle, -OCO-N-[(C₁-C₁₀)-alkyle]₂, -OCO-N-[(C₃-C₁₃)-cycloalkyle]₂, -OCO-NHSO₂-(C₁-C₁₀)-alkyle, -OCO-NHSO₂-(C₃-C₁₃)-cycloalkyle, -NH₂, -NH-(C₁-C₈)-alkyle, -N-[(C₁-C₈)-alkyle]₂,-NO₂ et -CN.

7. Composé selon la revendication 1 sélectionné parmi le groupe constitué de
6α,9α-difluoro-11β-hydroxy-16α-méthyl-3-oxo-17α-propionyloxyandrosta-1,4-diène-17β-carbothioate S-[2-(4-chlorophényl)-2-oxoéthylique],
6α,9α-difluoro-11β-hydroxy-16α-méthyl-3-oxo-17α-propionyloxyandrosta-1,4-diène-17β-carboxylate 2-(4-fluorophényl)-2-oxoéthylique,
6α,9α-difluoro-11β-hydroxy-16α-méthyl-3-oxo-17α-propionyloxyandrosta-1,4-diène-17β-carbothioate S-[2-(4-fluorophényl)-2-oxoéthylique],
6α,9α-difluoro-11β-hydroxy-16α-méthyl-3-oxo-17α-propionyloxyandrosta-1,4-diène-17β-carbothioate S-[2-(2,4-difluorophényl)-2-oxoéthylique],
6α,9α-difluoro-11β-hydroxy-16α-méthyl-3-oxo-17α-propionyloxyandrosta-1,4-diène-17β-carbothioate S-[2-(2,4-dichlorophényl)-2-oxoéthylique],
6α,9α-difluoro-11β-hydroxy-16α-méthyl-3-oxo-17α-propionyloxyandrosta-1,4-diène-17β-carbothioate S-[2-(3,4-dichlorophényl)-2-oxoéthylique],
6α,9α-difluoro-11β-hydroxy-16α-méthyl-3-oxo-17α-propionyloxyandrosta-1,4-diène-17β-carbothioate S-[2-(5-chlorophén-2-yl)-2-oxoéthylique],
6α,9α-difluoro-11β-hydroxy-16α-méthyl-3-oxo-17α-propionyloxyandrosta-1,4-diène-17β-carbothioate S-[2-oxopropylique],
6α,9α-difluoro-11β-hydroxy-16α-méthyl-3-oxo-17α-propionyloxyandrosta-1,4-diène-17β-carboxylate 2-oxopropylique,
6α,9α-difluoro-11β-hydroxy-16α-méthyl-3-oxo-17α-propionyloxyandrosta-1,4-diène-17β-carbothioate S-[2-(4-méthoxyphényl)-2-oxoéthylique],
6α,9α-difluoro-11β-hydroxy-16α-méthyl-3-oxo-17α-propionyloxyandrosta-1,4-diène-17β-carbothioate S-[2-oxo-2-(4-trifluorométhyloxyphényl)éthylique],
6α,9α-difluoro-11β-hydroxy-16α-méthyl-3-oxo-17α-propionyloxyandrosta-1,4-diène-17β-carbothioate S-[2-oxo-2-(4-trifluorométhylphényl)éthylique],
6α,9α-difluoro-11β-hydroxy-16α-méthyl-3-oxo-17α-propionyloxyandrosta-1,4-diène-17β-carboxylate 2-(4-méthanesulfonylphényl)-2-oxoéthylique,
6α,9α-difluoro-11β-hydroxy-16α-méthyl-3-oxo-17α-propionyloxyandrosta-1,4-diène-17β-carbothioate S-[2-(4-méthanesulfonylphényl)-2-oxoéthylique],
6α,9α-difluoro-11β-hydroxy-16α-méthyl-3-oxo-17α-propionyloxyandrosta-1,4-diène-17β-carboxylate 2-oxo-2-phényléthylique,
6α,9α-difluoro-11β-hydroxy-16α-méthyl-3-oxo-17α-propionyloxyandrosta-1,4-diène-17β-carbothioate S-[2-oxo-2-phényléthylique],
6α,9α-difluoro-11β-hydroxy-16α-méthyl-3-oxo-17α-propionyloxyandrosta-1,4-diène-17β-carbothioate S-[2-oxo-2-(2,4,6-triméthylphényl)éthylique],
6α,9α-difluoro-11β-hydroxy-16α-méthyl-3-oxo-17α-propionyloxyandrosta-1,4-diène-17β-carbothioate S-[1,1-diméthyl-2-oxo-2-phényléthylique],
6α,9α-difluoro-11β-hydroxy-16α-méthyl-17α-(2-méthylpropionyloxy)-3-oxoandrosta-1,4-diène-17β-carbothioate S-[2-(4-méthoxyphényl)-2-oxoéthylique],
17α-cyclobutylcarbonyloxy-6α,9α-difluoro-11β-hydroxy-16α-méthyl-3-oxoandrosta-1,4-diène-17β-carbothioate S-[2-oxo-2-(4-trifluorométhylphényl)éthylique],
17α-cyclobutylcarbonyloxy-6α,9α-difluoro-11β-hydroxy-16α-méthyl-3-oxoandrosta-1,4-diène-17β-carbothioate S-[2-(2,4-difluorophényl)-2-oxoéthylique],
17α-cyclobutylcarbonyloxy-6α,9α-difluoro-11β-hydroxy-16α-méthyl-3-oxoandrosta-1,4-diène-17β-carbothioate S-[2-(4-méthoxyphényl)-2-oxoéthylique],
9α-fluoro-17α-[(2-furanylcarbonyl)oxy]-11β-hydroxy-16α-méthyl-3-oxoandrosta-1,4-diène-17β-carbothioate S-[2-(2,4-difluorophényl)-2-oxoéthylique],
9α-fluoro-17α-[(2-furanylcarbonyl)oxy]-11β-hydroxy-16α-méthyl-3-oxoandrosta-1,4-diène-17β-carbothioate S-[2-(4-chlorophényl)-2-oxoéthylique],
9α-fluoro-17α-[(2-furanylcarbonyl)oxy]-11β-hydroxy-16α-méthyl-3-oxoandrosta-1,4-diène-17β-carbothioate S-[2-(4-méthoxyphényl)-2-oxoéthylique],
9α-fluoro-17α-[(2-furanylcarbonyl)oxy]-11β-hydroxy-16α-méthyl-3-oxoandrosta-1,4-diène-17β-carbothioate S-[2-(4-fluorophényl)-2-oxoéthylique],
6α,9α-difluoro-17α-[(2-furanylcarbonyl)oxy]-11β-hydroxy-16α-méthyl-3-oxoandrosta-1,4-diène-17β-carbothioate S-[2-(4-chlorophényl)-2-oxoéthylique],
6α,9α-difluoro-11β-hydroxy-16α-méthyl-3-oxo-17α-[(2-thiénylcarbonyl)oxy]androsta-1,4-diène-17β-carbothioate S-[2-oxo-2-(4-trifluorométhylphényl)éthylique],
6α,9α-difluoro-11β-hydroxy-16α-méthyl-3-oxo-17α-propionyloxyandrosta-1,4-diène-17β-carbothioate S-[2-(3,4-diméthylphényl)-2-oxoéthylique],
6α,9α-difluoro-17α-[(2-furanylcarbonyl)oxy]-11β-hydroxy-16α-méthyl-3-oxoandrosta-1,4-diène-17β-carbothioate S-[2-(3-chlorophényl)-2-oxoéthylique],
6α,9α-difluoro-17α-[(2-furanylcarbonyl)oxy]-11β-hydroxy-16α-méthyl-3-oxoandrosta-1,4-diène-17β-carbothioate S-[2-oxo-2-(4-trifluorométhylphényl)éthylique],
6α,9α-difluoro-17α-[(2-furanylcarbonyl)oxy]-11β-hydroxy-16α-méthyl-3-oxoandrosta-1,4-diène-17β-carbothioate S-(4-hydroxy-but-2-ynylique),
6α,9α-difluoro-11β-hydroxy-16α-méthyl-3-oxo-17α-propionyloxyandrosta-1,4-diène-17β-carbothioate S-(4-n-propylcarbonyloxy-but-2-ynylique),
6α,9α-difluoro-11β-hydroxy-16α-méthyl-3-oxo-17α-propionyloxyandrosta-1,4-diène-17β-carbothioate S-[4-(2-éthoxy-1,2-dioxoéthyly-(E)-but-2-énylique],
6α,9α-difluoro-17α-[(2-furanylcarbonyl)oxy]-11β-hydroxy-16α-méthyl-3-oxoandrosta-1,4-diène-17β-carbothioate S-[4-(2-éthoxy-1,2-dioxoéthyl)oxy-(E)-but-2-énylique],
6α,9α-difluoro-17α-[(2-furanylcarbonyl)oxy]-11β-hydroxy-16α-méthyl-3-oxoandrosta-1,4-diène-17β-carbothioate S-[4-n-propylcarbonyloxy-but-2-ynylique],
6α,9α-difluoro-17α-[(2-furanylcarbonyl)oxy]-11β-hydroxy-16α-methyl-3-oxoandrosta-1,4-diène-17β-carbothioate S-[4-(2-méthylpropionyloxy)-but-2-ynylique],
6α,9α-difluoro-17α-[(2-furanylcarbonyl)oxy]-11β-hydroxy-16α-méthyl-3-oxoandrosta-1,4-diène-17β-carbothioate S-[4-(2,2-diméthylpropionyloxy)-but-2-ynylique],
6α,9α-difluoro-11β-hydroxy-16α-méthyl-3-oxo-17α-propionyloxyandrosta-1,4-diène-17β-carbothioate S-[4-(2,2-diméthylpropionyloxy)-but-2-ynylique],
6α,9α-difluoro-17α-[(2-furanylcarbonyl)oxy] -11β-hydroxy-16α-méthyl-3-oxoandrosta-1,4-diène-17β-carbothioate S-[4-n-propylcarbonyloxy-(Z)-but-2-énylique],
6α,9α-difluoro-17α-[(2-furanylcarbonyl)oxy]-11β-hydroxy-16α-méthyl-3-oxoandrosta-1,4-diène-17β-carbothioate S-[4-(2-éthoxy-1,2-dioxoéthyl)oxy-but-2-ynylique],
6α,9α-difluoro-11β-hydroxy-16α-méthyl-3-oxo-17α-[(2-thiénylcarbonyl)oxy]androsta-1,4-diène-17β-carbothioate S-[4-n-propylcarbonyloxy-but-2-ynylique],
6α,9α-difluoro-17α-[(2-furanylcarbonyl)oxy]-11β-hydroxy-16α-méthyl-3-oxoandrosta-1,4-diène-17β-carbothioate S-[(4-cyclopropylcarbonyloxy)-but-2-ynylique],
6α,9α-difluoro-17α-[(2-furanylcarbonyl)oxy]-11β-hydroxy-16α-méthyl-3-oxoandrosta-1,4-diène-17β-carbothioate S-[(4-cyclobutylcarbonyloxy)-but-2-ynylique],
6α,9α-difluoro-17α-[(2-furanylcarbonyl)oxy]-11β-hydroxy-16α-méthyl-3-oxoandrosta-1,4-diène-17β-carbothioate S-[(4-cyclopentylcarbonyloxy)-but-2-ynylique],
6α,9α-difluoro-17α-[(2-furanylcarbonyl)oxy]-11β-hydroxy-16α-méthyl-3-oxoandrosta-1,4-diène-17β-carbothioate S-[4-n-propylcarbonyloxy-(E)-but-2-énylique],
6α,9α-difluoro-17α-[(2-furanylcarbonyl)oxy]-11β-hydroxy-16α-méthyl-3-oxoandrosta-1,4-diène-17β-carbothioate S-[(4-méthoxycarbonyloxy)-but-2-ynylique],
6α,9α-difluoro-17α-[(2-furanylcarbonyl)oxy]-11β-hydroxy-16α-méthyl-3-oxoandrosta-1,4-diène-17β-carbothioate S-[(4-éthoxycarbonyloxy)-but-2-ynylique],
6α,9α-difluoro-17α-[(2-furanylcarbonyl)oxy]-11β-hydroxy-16α-méthyl-3-oxoandrosta-1,4-diène-17β-carbothioate S-[4-(2,2-diméthyléthyloxycarbonyloxy)-but-2-ynylique],
6α,9α-difluoro-17α-[(2-furanylcarbonyl)oxy]-11β-hydroxy-16α-méthyl-3-oxoandrosta-1,4-diène-17β-carbothioate S-[4-(2-méthylpropyloxycarbonyloxy)-but-2-ynylique],
6α,9α-difluoro-17α-[(2-furanylcarbonyl)oxy]-11β-hydroxy-16α-méthyl-3-oxoandrosta-1,4-diène-17β-carbothioate S-[4-(3-chloropropylcarbonyloxy)-but-2-ynylique],
6α,9α-difluoro-17α-[(2-furanylcarbonyl)oxy]-11β-hydroxy-16α-méthyl-3-oxoandrosta-1,4-diène-17β-carbothioate S-[4-dichloroacétoxy-but-2-ynylique],
6α,9α-difluoro-17α-[(2-furanylcarbonyl)oxy]-11β-hydroxy-16α-methyl-3-oxoandrosta-1,4-diène-17β-carbothioate S-[4-(2-méthylpropylcarbonyloxy)-but-2-ynylique],
6α,9α-difluoro-17α-[(2-furanylcarbonyl)oxy]-11β-hydroxy-16α-méthyl-3-oxoandrosta-1,4-diène-17β-carbothioate S-[4-(3,3-diméthylpropylcarbonyloxy)-but-2-ynylique],
6α,9α-difluoro-17α-[(2-furanylcarbonyl)oxy]-11β-hydroxy-16α-méthyl-3-oxoandrosta-1,4-diène-17β-carbothioate S-(4-propionyloxy-but-2-ynylique),
6α,9α-difluoro-17α-[(2-furanylcarbonyl)oxy]-11β-hydroxy-16α-méthyl-3-oxoandrosta-1,4-diène-17β-carbothioate S-[4-(3,3,3-trifluoropropionyloxy)-but-2-ynylique],
6α,9α-difluoro-17α-[(2-furanylcarbonyl)oxy]-11β-hydroxy-16α-méthyl-3-oxoandrosta-1,4-diène-17β-carbothioate S-[4-(méthoxyacétyloxy)-but-2-ynylique],
6α,9α-difluoro-17α-[(2-furanylcarbonyl)oxy]-11β-hydroxy-16α-méthyl-3-oxoandrosta-1,4-diène-17β-carbothioate S-[4-(éthoxyacétyloxy)-but-2-ynylique],
6α,9α-difluoro-17α-[(2-furanylcarbonyl)oxy]-11β-hydroxy-16α-méthyl-3-oxoandrosta-1,4-diène-17β-carbothioate S-(4-acétoxy-but-2-ynylique),
6α,9α-difluoro-17α-[(2-furanylcarbonyl)oxy]-11β-hydroxy-16α-méthyl-3-oxoandrosta-1,4-diène-17β-carbothioate S-[4-(2,2-diméthylbutylcarbonyloxy)-but-2-ynylique],
6α,9α-difluoro-17α-[(2-furanylcarbonyl)oxy]-11β-hydroxy-16α-méthyl-3-oxoandrosta-1,4-diène-17β-carbothioate S-[4-((1S)-camphanylcarbonyloxy)-but-2-ynylique],
6α,9α-difluoro-17α-[(2-furanylcarbonyl)oxy]-11β-hydroxy-16α-méthyl-3-oxoandrosta-1,4-diène-17β-carbothioate S-[4-(4-éthoxybutyl-1,4-dione)oxy-but-2-ynylique],
6α,9α-difluoro-17α-[(2-furanylcarbonyl)oxy]-11β-hydroxy-16α-méthyl-3-oxoandrosta-1,4-diène-17β-carbothioate S-[4-(4-éthyloxy-(Z)-but-2-èn-1,4-dione)oxy-but-2-ynylique],
6α,9α-difluoro-17α-[(2-furanylcarbonyl)oxy]-11β-hydroxy-16α-méthyl-3-oxoandrosta-1,4-diène-17β-carbothioate S-[4-(4-méthoxybutyl-1,4-dione)oxy-but-2-ynylique],
6α,9α-difluoro-11β-hydroxy-16α-méthyl-17α-[(2-furanylcarbonyl)oxy]-3-oxoandrosta-1,4-diène-17β-carbothioate S-[4-(4-isopropyloxybutyl-1,4-dione)oxy-but-2-ynylique],
6α,9α-difluoro-17α-[(2-furanylcarbonyl)oxy]-11β-hydroxy-16α-méthyl-3-oxoandrosta-1,4-diène-17β-carbothioate S-[4-(4-isobutyloxybutyl-1,4-dione)oxy-but-2-ynylique],
6α,9α-difluoro-17α-[(2-furanylcarbonyl)oxy]-11β-hydroxy-16α-méthyl-3-oxoandrosta-1,4-diène-17β-carbothioate S-[4-(4-n-butyloxybutyl-1,4-dione)oxy-but-2-ynylique],
6α,9α-difluoro-17α-[(2-furanylcarbonyl)oxy]-11β-hydroxy-16α-méthyl-3-oxoandrosta-1,4-diène-17β-carbothioate S-[4-(3-chloro-2,2-diméthylpropionyloxy)-but-2-ynylique],
6α,9α-difluoro-17α-[(2-furanylcarbonyl)oxy]-11β-hydroxy-16α-methyl-3-oxoandrosta-1,4-diène-17β-carbothioate S-[4-(4-méthoxy-(E)-but-2-èn-1,4-dione)oxy-but-2-ynylique],
6α,9α-difluoro-17α-[(2-furanylcarbonyl)oxy]-11β-hydroxy-16α-méthyl-3-oxoandrosta-1,4-diène-17β-carbothioate S-[4-(3-méthyl-1-oxobutyloxy)-but-2-ynylique],
6α,9α-difluoro-17α-[(2-furanylcarbonyl)oxy]-11β-hydroxy-16α-méthyl-3-oxoandrosta-1,4-diène-17α-carbothioate S-[4-(2-éthyl-1-oxobutyloxy)-but-2-ynylique],
6α,9α-difluoro-17α-[(2-furanylcarbonyl)oxy]-11β-hydroxy-16α-méthyl-3-oxoandrosta-1,4-diène-17β-carbothioate S-[4-(N,N-diméthylaminocarbonyloxy)-but-2-ynylique],
6α,9α-difluoro-17α-[(2-furanylcarbonyl)oxy]-11β-hydroxy-16α-méthyl-3-oxoandrosta-1,4-diène-17β-carbothioate S-[4-(2-chloro-2-méthylpropionyloxy)-but-2-ynylique],
6α,9α-difluoro-17α-[(2-furanylcarbonyl)oxy]-11β-hydroxy-16α-méthyl-3-oxoandrosta-1,4-diène-17β-carbothioate S-[4-(4-méthyl-1-oxopentyloxy)-but-2-ynylique],
6α,9α-difluoro-17α-[(2-furanylcarbonyl)oxy]-11β-hydroxy-16α-méthyl-3-oxoandrosta-1,4-diène-17β-carbothioate S-[4-(2-hydroxy-2-méthylpropionyloxy)-but-2-ynylique],
6α,9α-difluoro-17α-[(2-furanylcarbonyl)oxy]-11β-hydroxy-16α-méthyl-3-oxoandrosta-1,4-diène-17β-carbothioate S-[4-(2-fluoro-2-méthylpropionyloxy)-but-2-ynylique],
6α,9α-difluoro-17α-[(2-furanylcarbonyl)oxy]-11β-hydroxy-16α-méthyl-3-oxoandrosta-1,4-diène-17β-carbothioate S-[4-(4-méthoxy-(Z)-but-2-èn-1,4-dione)oxy-but-2-ynylique],
6α, 9α-difluoro-17α-[(2-furanylcarbonyl)oxy]-11β-hydroxy-16α-méthyl-3-oxoandrosta-1,4-diène-17β-carbothioate S-[4-(4-éthoxy-(E)-but-2-èn-1,4-dione)oxy-but-2-ynylique],
6α,9α-difluoro-17α-[(2-furanylcarbonyl)oxy]-11β-hydroxy-16α-méthyl-3-oxoandrosta-1,4-diène-17β-carbothioate S-[4-(4-isobutyloxy-(E)-but-2-èn-1,4-dione)oxy-but-2-ynylique],
6α,9α-difluoro-17α-[(2-furanylcarbonyl)oxy]-11β-hydroxy-16α-méthyl-3-oxoandrosta-1,4-diène-17β-carbothioate S-(4-acryloxy-but-2-ynylique),
6α,9α-difluoro-17α-[(2-furanylcarbonyl)oxy]-11β-hydroxy-16α-méthyl-3-oxoandrosta-1,4-diène-17β-carbothioate S-[4-(cyclopentylméthyloxycarbonyloxy)-but-2-ynylique],
6α,9α-difluoro-17α-[(2-furanylcarbonyl)oxy]-11β-hydroxy-16α-méthyl-3-oxoandrosta-1,4-diène-17β-carbothioate S-[4-(3-méthylbut-2-énoxycarbonyloxy)-but-2-ynylique],
6α,9α-difluoro-17α-[(2-furanylcarbonyl)oxy]-11β-hydroxy-16α-méthyl-3-oxoandrosta-1,4-diène-17β-carbothioate S-[4-(n-pentyloxycarbonyloxy)-but-2-ynylique],
6α,9α-difluoro-17α-[(2-furanylcarbonyl)oxy]-11β-hydroxy-16α-méthyl-3-oxoandrosta-1,4-diène-17β-carbothioate S-[4-(2,2-diméthyl-3-fluoropropionyloxy)-but-2-ynylique],
6α,9α-difluoro-17α-[(2-furanylcarbonyl)oxy]-11β-hydroxy-16α-méthyl-3-oxoandrosta-1,4-diène-17β-carbothioate S-[4-((S)-2,2-diméthyl-1,3-dioxolan-4-yl-méthoxycarbonyloxy)-but-2-ynylique],
6α,9α-difluoro-17α-[(2-furanylcarbonyl)oxy]-11β-hydroxy-16α-méthyl-3-oxoandrosta-1,4-diène-17β-carbothioate S-[4-((R)-2,2-diméthyl-1,3-dioxolan-4-yl-méthoxycarbonyloxy)-but-2-ynylique],
6α,9α-difluoro-17α-[(2-furanylcarbonyl)oxy]-11β-hydroxy-16α-méthyl-3-oxoandrosta-1,4-diène-17β-carbothioate S-[4-(isoamyloxycarbonyloxy)-but-2-ynylique],
6α,9α-difluoro-17α-[(2-furanylcarbonyl)oxy]-11β-hydroxy-16α-méthyl-3-oxoandrosta-1,4-diène-17β-carbothioate S-(4-éthoxy-but-2-ynylique),
ester fluorométhylique d'acide 6α,9α-difluoro-11β-hydroxy-16α-méthyl-3-oxo-17α-propionyloxyandrosta-1,4-diène-17β-carbonylsulfénique,
ester fluorométhylique d'acide 6α,9α-difluoro-17α-[(2-furanylcarbonyl)oxy]-11β-hydroxy-16α-méthyl-3-oxoandrosta-1,4-diène-17β-carbonylsulfénique,
ester (4-fluorophényl)méthylique d'acide 6α,9α-difluoro-17α-[(2-furanylcarbonyl)oxy]-11β-hydroxy-16a-méthyl-3-oxoandrosta-1,4-diène-17β-carbonylsulfénique,
ester (4-trifluorométhylphényl)méthylique d'acide 6α,9α-difluoro-17α-[(2-furanylcarbonyl)oxy]-11β-hydroxy-16α-méthyl-3-oxoandrosta-1,4-diène-17β-carbonylsulfénique,
ester (4-chlorophényl)méthylique d'acide 6α,9α-difluoro-17α-[(2-furanylcarbonyl)oxy]-11β-hydroxy-16α-méthyl-3-oxoandrosta-1,4-diène-17β-carbonylsulfénique,
ester (4-trifluorométhylphényl)méthylique d'acide 6α,9α-difluoro-11β-hydroxy-16α-méthyl-3-oxo-17α-propionyloxyandrosta-1,4-diène-17β-carbonylsulfénique,
ester (4-fluorophényl)méthylique d'acide 9α-fluoro-17α-[(2-furanylcarbonyl)oxy]-11β-hydroxy-16α-méthyl-3-oxoandrosta-1,4-diène-17β-carbonylsulfénique,
ester méthylique d'acide 6α,9α-difluoro-11β-hydroxy-16α-méthyl-3-oxo-17α-propionyloxyandrosta-1,4-diène-17β-carbonylsulfénique,
ester méthylique d'acide 6α,9α-difluoro-17α-[(2-furanylcarbonyl)oxy]-11β-hydroxy-16α-méthyl-3-oxoandrosta-1,4-diène-17β-carbonylsulfénique,
6α,9α-difluoro-17α-[(2-furanylcarbonyl)oxy]-11β-hydroxy-16α-méthyl-3-oxoandrosta-1,4-diène-17β-carbothioate S-[4-(4-méthyl-1-oxopentyloxy)-but-2-ynylique].
